# EUROPÄISCHE PATENTSCHRIFT

(11) **EP 2 563 786 B1**
(45) Veröffentlichungstag und Bekanntmachung des Hinweises auf die Patenterteilung: **02.11.2016**
(21) Anmeldenummer: 11716265.1
(22) Anmeldetag: 26.04.2011
(51) Int. Cl.: C07D 417/12, A01N 43/78, C07D 417/14

(54) **Ketoheteroarylpiperidin und -piperazin Derivate als Fungizide**
Ketoheteroarylpiperidine and piperazine derivatives as fungicides
Dérivés de kétohétéroarylpipéridine et pipérazine comme fongicides

(30) Priorität: 28.04.2010 US 328806 P; 28.04.2010 EP 10161264
(43) Veröffentlichungstag der Anmeldung: 06.03.2013
(73) Patentinhaber: Bayer Intellectual Property GmbH, 40789 Monheim am Rhein (DE)
(72) Erfinder: CRISTAU, Pierre, 69009 Lyon (FR); HOFFMANN, Sebastian, 41470 Neuss (DE); KLUTH, Joachim, 40764 Langenfeld (DE); SEITZ, Thomas, 40764 Langenfeld (DE); TSUCHIYA, Tomoki, 69009 Lyon (FR); WASNAIRE, Pierre, 40225 Düsseldorf (DE); BENTING, Jürgen, 42799 Leichlingen (DE); WACHENDORFF-NEUMANN, Ulrike, 56566 Neuwied (DE)
(74) Vertreter: BIP Patents
(86) Internationale Anmeldenummer: PCT/EP2011/056594
(87) Internationale Veröffentlichungsnummer: WO 2011/134969

(56) Entgegenhaltungen:
- WO-A1-2009/132785
- WO-A2-2007/014290
- WO-A2-2008/091594

## Beschreibung

Die vorliegende Erfindung betrifft neue Ketoheteroarylpiperidin und -piperazin Derivate, Verfahren zu deren Herstellung, deren Verwendung zum Bekämpfen von unerwünschten Mikroorganismen, insbesondere phytopathogener Pilze, im Pflanzenschutz, im Bereich Haushalt und Hygiene und im Materialschutz, sowie Pflanzenschutzmittel enthaltend diese Ketoheteroarylpiperidin und -piperazin Derivate.

Es ist bereits bekannt, dass bestimmte Heteroarylpiperidin und -piperazin Derivate als fungizide Pflanzenschutzmittel benutzt werden können (siehe WO 2007/014290, WO 2008/013925, WO 2008/013622, WO 2008/091594, WO 2008/091580, WO 2009/055514, WO 2009/094407, WO 2009/094445, WO 2009/132785, WO 2010/037479, WO 2010/065579, WO 2010/149275, WO 2010/066353, WO 2011/018401, WO 2011/018415). Die fungizide Wirksamkeit dieser Verbindungen ist jedoch gerade bei niedrigeren Aufwandmengen nicht immer ausreichend.

Da sich die ökologischen und ökonomischen Anforderungen an moderne Pflanzenschutzmittel laufend erhöhen, beispielsweise was Wirkspektrum, Toxizität, Selektivität, Aufwandmenge, Rückstandsbildung und günstige Herstellbarkeit angeht, und außerdem z.B. Probleme mit Resistenzen auftreten können, besteht die ständige Aufgabe, neue Pflanzenschutzmittel, insbesondere Fungizide zu entwickeln, die zumindest in Teilbereichen Vorteile gegenüber den bekannten aufweisen.

Überraschenderweise wurde nun gefunden, dass die vorliegenden Ketoheteroarylpiperidin und -piperazin Derivate die genannten Aufgaben zumindest in Teilaspekten lösen und sich als Pflanzenschutzmittel, insbesondere als Fungizide eignen.

Gegenstand der Erfindung sind Verbindungen der Formel (I), 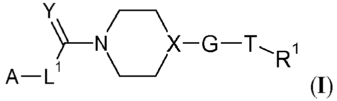 in welcher die Symbole folgende Bedeutungen haben:
- A: steht für Phenyl, das bis zu drei Substituenten enthalten kann,
wobei die Substituenten unabhängig voneinander aus R² ausgewählt sind,
oder
- A': steht für ein gegebenenfalls benzokondensiertes, unsubstituiertes oder substituiertes 5- oder 6-gliedriges Heteroaryl, wobei die Substituenten am Kohlenstoff unabhängig voneinander aus R³ und die Substituenten am Stickstoff unabhängig voneinander aus R⁴ ausgewählt sind,
- L¹: steht für NR⁵ oder C(R⁶)₂,
- X: steht für CH oder Stickstoff,
- Y: steht für Schwefel oder Sauerstoff,
G steht für: 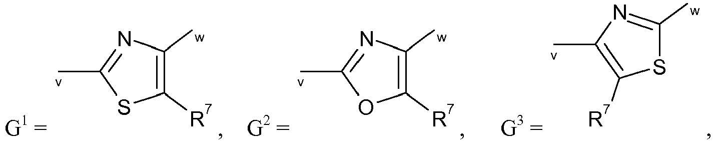 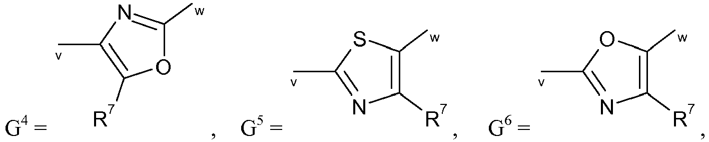 wobei die Bindung, die mit "v" identifiziert ist, direkt an X gebunden ist, und wobei die Bindung, die mit "w" identifiziert ist, direkt an T gebunden ist,
- T: steht für *-C(=O)CH₂-#, *-C(=O)CH₂C(R⁸)₂-#, *-C(=O)CH=CH-#, *-C(=O)C≡C-#, *-C(=O)CH₂C(=O)-#, *-C≡CC(=O)-#, *-CH=CHC(=O)-#, *-CH₂CH₂C(=O)-#, *-C(=S)CH₂-#, *-C(=S)CH₂C(R⁸)₂-#, *-C(=S)CH=CH-#, *-C(=S)C≡C-#, *-C≡CC(=S)-#, *-CH=CHC(=S)-#, *-CH₂CH₂C(=S)-#, *-C(=NR⁹)CH₂-# *-C(=NR⁹)CH₂C(R⁸)₂-#, *-CH₂CH₂C(=NR⁹)- #, *-C(=NR⁹)CH=CH- #, *-CH=CHC(=NR⁹)- #, *-CH=CHC(NOH)-# oder *-CH=CHC(NO-C₁-C₄-Alkyl)CH₂-#,
wobei die Bindung, die mit * identifiziert ist, direkt an G gebunden ist, und wobei die Bindung, die mit # identifiziert ist, direkt an R¹ gebunden ist,
- R¹: ausgewählt ist aus der Gruppe enthaltend R¹ₐ, R¹_{b}, R¹_{c}, R¹_{d}, R¹ₑ, R¹_{f}, R¹_{g} oder R¹ₕ, wobei
- R¹ₐ: steht für C₁-C₆-Alkyl, C₁-C₆-Halogenalkyl, C₂-C₆-Alkenyl, C₂-C₆-Alkinyl, C₂-C₈-Alkoxyalkyl oder C₅-C₉-Cycloalkoxyalkyl oder
- R¹_{b}: steht für unsubstituiertes oder substituiertes C₃-C₁₀-Cycloalkyl,
wobei die Substituenten unabhängig voneinander aus -Q oder R¹⁰ ausgewählt sind oder
- R¹_{c}: steht für unsubstituiertes oder substituiertes C₅-C₁₀-Cycloalkenyl, wobei die Substituenten unabhängig voneinander aus R¹¹ ausgewählt sind oder
- R¹_{d}: steht für unsubstituiertes oder substituiertes Phenyl,
wobei die Substituenten unabhängig voneinander aus -L²Q oder R¹² ausgewählt sind oder
- R¹ₑ: steht für unsubstituiertes oder substituiertes, Naphthalen-1-yl, Naphthalen-2-yl, 1,2,3,4-Tetrahydronaphthalen-1-y 1, 1 , 2 , 3 , 4-Tetrahydronaphthalen-2-y 1, 5, 6, 7,8-Tetrahydronaphthalen-1-yl, 5,6,7,8-Tetrahydronaphthalen-2-yl, Decalin-1-yl, Decalin-2-yl, 1H-Inden-1-yl, 1H-Inden-2-yl, 1H-Inden-3-yl, 1H-Inden-4-yl, 1H-Inden-5-yl, 1H-Inden-6-yl, 1H-Inden-7-yl, Indan-1-yl, Indan-2-yl, Indan-3-yl, Indan-4-yl oder Indan-5-yl,
wobei die Substituenten unabhängig voneinander aus R¹³ ausgewählt sind oder
- R¹_{f}: steht für einen unsubstituierten oder substituierten 5- oder 6-gliedrigen Heteroarylrest, wobei die Substituenten am Kohlenstoff unabhängig voneinander aus -L²Q oder R¹⁴ ausgewählt sind, und die Substituenten am Stickstoff unabhängig voneinander aus -L³Q oder R¹⁵ ausgewählt sind oder
- R¹_{g}: steht für benzokondensiertes unsubstituiertes oder substituiertes 5- oder 6- gliedriges Heteroaryl, wobei die Substituenten am Kohlenstoff unabhängig voneinander aus R¹⁶ ausgewählt sind, und die Substituenten am Stickstoff unabhängig voneinander aus R¹⁷ ausgewählt sind oder
- R¹ₕ: steht für unsubstituiertes oder substituiertes C₅-C₁₅-Heterocyclyl, wobei die Substituenten am Kohlenstoff unabhängig voneinander ausgewählt aus R¹⁸ sind, und die Substituenten am Stickstoff unabhängig voneinander ausgewählt aus R¹⁹ sind,
- L²: steht für eine direkte Bindung, -O-, -C(R²⁰)₂- oder -NR²¹-,
- L³: steht für eine direkte Bindung oder -C(R²⁰)₂-,
- Q: ist ausgewählt aus der Gruppe enthaltend Q', Q" und Q"', wobei
Q' steht für ein Phenyl, das bis zu zwei Substituenten enthalten kann, wobei die Substituenten unabhängig voneinander aus R²² ausgewählt sind oder
Q" steht für einen 5- oder 6-gliedrigen Heteroarylrest, der bis zu zwei Substituenten enthalten kann, wobei die Substituenten am Kohlenstoff unabhängig voneinander aus R²³ ausgewählt sind und die Substituenten am Stickstoff unabhängig voneinander aus R²⁴ ausgewählt sind oder
Q'" steht für gesättigtes oder teilweise ungesättigtes C₃-C₁₀-Cycloalkyl,
- R², R¹² und R²²: stehen unabhängig voneinander für Halogen, Cyano, Hydroxy, SH, Amino, Nitro, Phenyl, C(=O)H, C(=O)OH, CONR²⁵R²⁶, NR²⁵R²⁶, C₁-C₆-Alkyl, C₂-C₆-Alkenyl, C₂-C₆-Alkinyl, C₁-C₆-Halogenalkyl, C₂-C₆-Halogenalkenyl, C₂-C₆-Halogenalkinyl, C₃-C₈-Cycloalkyl, C₃-C₈-Halogencycloalkyl, C₄-C₁₀-Alkylcycloalkyl, C₄-C₁₀-Cycloalkylalkyl, C₆-C₁₄-Cycloalkylcycloalkyl, C₄-C₁₀-Halogencycloalkylalkyl, C₅-C₁₀-Alkylcycloalkylalkyl, C₃-C₈-Cycloalkenyl, C₃-C₈-Halogencycloalkenyl, C₂-C₆-Alkoxyalkyl, C₄-C₁₀-Cycloalkoxyalkyl, C₃-C₈-Alkoxyalkoxyalkyl, C₂-C₆-Alkylthioalkyl, C₂-C₆-Alkylsulfinylalkyl, C₂-C₆-Alkylsulfonylalkyl, C₂-C₆-Alkylaminoalkyl, C₃-C₈-Dialkylaminoalkyl, C₂-C₆-Halogenalkylaminoalkyl, C₄-C₁₀-Cycloalkylaminoalkyl, C₂-C₆-Alkylcarbonyl, C₂-C₆-Halogenalkylcarbonyl, C₄-C₈-Cycloalkylcarbonyl, C₂-C₆-Alkoxycarbonyl, C₄-C₈-Cycloalkoxycarbonyl, C₅-C₁₀-Cycloalkylalkoxycarbonyl, C₂-C₆-Alkylaminocarbonyl, C₃-C₈-Dialkylaminocarbonyl, C₄-C₈-Cycloalkylaminocarbonyl, C₂-C₆-Halogenalkoxyalkyl, C₁-C₆-Hydroxyalkyl, C₁-C₆-Alkoxy, C₁-C₆-Halogenalkoxy, C₃-C₈-Cycloalkoxy, C₃-C₈-Halogencycloalkoxy, C₄-C₁₀-Cycloalkylalkoxy, C₃-C₆-Alkenyloxy, C₃-C₆-Halogenalkenyloxy, C₃-C₆-Alkinyloxy, C₃-C₆-Halogenalkinyloxy, C₂-C₆-Alkoxyalkoxy, C₂-C₆-Alkylcarbonyloxy, C₂-C₆-Halogenalkylcarbonyloxy, C₄-C₈-Cycloalkylcarbonyloxy, C₃-C₆-Alkylcarbonylalkoxy, C₁-C₆-Alkylthio, C₁-C₆-Halogenalkylthio, C₃-C₆-Cycloalkylthio, C₁-C₆-Alkylsulfinyl, C₁-C₆-Halogenalkylsulfinyl, C₁-C₆-Alkylsulfonyl, C₁-C₆-Halogenalkylsulfonyl, C₃-C₈-Cycloalkylsulfonyl, Tri(C₁-C₄-alkyl)silyl, C₁-C₆-Alkylsulfonylamino oder C₁-C₆-Halogenalkylsulfonylamino,
- R³, R¹⁴, R¹⁶, R¹⁸ und R²³: stehen unabhängig voneinander für Halogen, Cyano, Hydroxy, SH, Amino, Nitro, NR²⁵R²⁶, C₁-C₆-Alkyl, C₂-C₆-Alkenyl, C₂-C₆-Alkinyl, C₁-C₆-Halogenalkyl, C₂-C₆-Halogenalkenyl, C₂-C₆-Halogenalkinyl, C₃-C₆-Cycloalkyl, C₃-C₆-Halogencycloalkyl, C₄-C₁₀-Alkylcycloalkyl, C₄-C₁₀-Cycloalkylalkyl, C₆-C₁₄-Cycloalkylcycloalkyl, C₅-C₁₀-Alkylcycloalkylalkyl, C₂-C₄-Alkoxyalkyl, C₂-C₄-Alkylcarbonyl, C₂-C₆-Alkoxycarbonyl, C₂-C₆-Alkylaminocarbonyl, C₃-C₈-Dialkylaminocarbonyl, C₁-C₄-Hydroxyalkyl, C₁-C₄-Alkoxy, C₃-C₆-Alkenyloxy, C₃-C₆-Alkinyloxy, C₁-C₄-Halogenalkoxy, C₂-C₆-Alkylcarbonyloxy, C₂-C₆-Alkylcarbonylthio, C₁-C₄-Alkylthio, C₁-C₄-Halogenalkylthio, C₁-C₄-Alkylsulfinyl, C₁-C₄-Halogenalkylsulfinyl, C₁-C₄-Alkylsulfonyl, C₁-C₄-Halogenalkylsulfonyl, Tri(C₁-C₄-alkyl)silyl oder Phenyl,
- R⁴, R¹⁵, R¹⁷, R¹⁹ und R²⁴: stehen unabhängig voneinander für C₁-C₆-Alkyl, C₂-C₆-Alkenyl, C₂-C₆-Alkinyl, C₁-C₆-Halogenalkyl, C₃-C₆-Halogenalkenyl, C₃-C₆-Halogenalkinyl, C₃-C₆-Cycloalkyl, C₃-C₆-Halogencycloalkyl, C₄-C₁₀-Alkylcycloalkyl, C₄-C₁₀-Cycloalkylalkyl, Phenyl, Benzyl, C₁-C₄-Alkylsulfonyl, C(=O)H, C₂-C₄-Alkylcarbonyloxy, C₂-C₅-Alkoxycarbony, C₂-C₅-oder Alkylcarbonyl,
- R⁵: steht für Wasserstoff oder C₁-C₄-Alkyl,
- R⁶: steht unabhängig voneinander für Wasserstoff, C₁-C₄-Alkyl, C₁-C₄-Halogenalkyl, Cyclopropyl, Halogen,
oder die beiden Reste R⁶ bilden zusammen mit dem Kohlenstoffatom, an das sie gebunden sind, einen Cyclopropylring,
- R⁷: steht für Wasserstoff, C₁-C₃-Alkyl, C₁-C₃-Halogenalkyl, C₁-C₄-Alkoxy, C₂-C₅-Alkoxycarbonyl oder Halogen,
- R⁸: steht unabhängig voneinander für Wasserstoff oder C₁-C₄-Alkyl,
- R⁹: steht für OH, Nitro, Cyano, C₁-C₄-Alkyl, C₁-C₄-Alkylamino, C₂-C₄-Dialkylamino, C₁-C₄-Alkoxy, C₁-C₄-Halogenalkoxy oder Cyclopropyloxy
- R¹⁰ und R¹¹: stehen unabhängig voneinander für Cyano, Halogen, C₁-C₆-Alkyl, C₁-C₆-Halogenalkyl, C₃-C₆-Cycloalkyl, C₂-C₆-Alkenyl, C₂-C₆-Halogenalkenyl, C₂-C₆-Alkinyl, C₂-C₆-Halogenalkinyl, Tri(C₁-C₄-alkyl)silyl, Phenyl, Hydroxy, Oxo, C₁-C₆-Alkoxy, C₁-C₆-Halogenalkoxy, C₃-C₆-Alkenyloxy, C₃-C₆-Alkinyloxy, C₁-C₆-Alkylthio oder C₁-C₆-Halogenalkylthio,
- R¹³: steht unabhängig voneinander für Cyano, Nitro, Halogen, C₁-C₆-Alkyl, C₁-C₄-Halogenalkyl, C₃-C₆-Cycloalkyl, C₂-C₆-Alkenyl, C₂-C₆-Halogenalkenyl, C₂-C₆-Alkinyl, C₂-C₆-Halogenalkinyl, Tri(C₁-C₄-alkyl)silyl, Benzyl, Phenyl, Hydroxy, SH, C₁-C₆-Alkoxy, C₁-C₆-Halogenalkoxy, C₃-C₆-Alkenyloxy, C₃-C₆-Alkinyloxy, C₁-C₆-Alkylthio oder C₁-C₆-Halogenalkylthio,
- R²⁰: steht unabhängig voneinander für Wasserstoff, Halogen, C₁-C₄-Alkyl oder C₁-C₄-Halogenalkyl,
- R²¹: steht für C₁-C₆-Alkyl, C₁-C₆-Halogenalkyl, C₃-C₈-Cycloalkyl, C₂-C₆-Alkylcarbonyl, C₂-C₆-Halogenalkylcarbonyl, C₂-C₆-Alkoxycarbonyl oder C₂-C₆-Halogenalkoxycarbonyl,
- R²⁵ und R²⁶: stehen unabhängig voneinander für Wasserstoff, C₁-C₄-Alkyl, C₂-C₆-Alkenyl, C₂-C₆-Alkinyl, C₁-C₃-Halogenalkyl, C₃-C₆-Cycloalkyl, Benzyl oder Phenyl,
sowie agrochemisch wirksame Salze davon.

Ein weiterer Gegenstand der Erfindung ist die Verwendung der Verbindungen der Formel (I) als Fungizid.

Erfindungsgemäße Ketoheteroarylpiperidin und -piperazin Derivate der Formel (I) sowie deren agrochemisch wirksame Salze eignen sich sehr gut zur Bekämpfung pflanzenpathogener Schadpilze. Die vorgenannten erfindungsgemäßen Verbindungen zeigen vor allem eine starke fungizide Wirksamkeit und lassen sich sowohl im Pflanzenschutz, im Bereich Haushalt und Hygiene als auch im Materialschutz verwenden.

Die Verbindungen der Formel (I) können sowohl in reiner Form als auch als Mischungen verschiedener möglicher isomerer Formen, insbesondere von Stereoisomeren, wie E- und Z-, threo- und erythro-, sowie optischen Isomeren, wie R- und S-Isomeren oder Atropisomeren, gegebenenfalls aber auch von Tautomeren vorliegen. Es werden sowohl die E- als auch die Z-Isomeren, wie auch die threo- und erythro-, sowie die optischen Isomeren, beliebige Mischungen dieser Isomeren, sowie die möglichen tautomeren Formen beansprucht.
- A: steht bevorzugt für Phenyl, das bis zu zwei Substituenten enthalten kann, wobei die Substituenten unabhängig voneinander aus R² Liste ausgewählt sind und
R² bevorzugt steht für:
Halogen, Cyano, Hydroxy, Amino, Nitro, C₁-C₆-Alkyl, C₂-C₆-Alkenyl, C₂-C₆-Alkinyl, C₃-C₈-Cycloalkyl, C₁-C₆-Halogenalkyl, C₂-C₆-Halogenalkenyl, C₂-C₆-Halogenalkinyl, C₃-C₆-Halogencycloalkyl, C₁-C₄-Alkoxy, C₁-C₄-Halogenalkoxy, C₁-C₄-Alkylthio, C₁-C₄-Alkylsulfonyl, C₁-C₄-Halogenalkylthio, C₁-C₄-Halogenalkylsulfonyl, C₂-C₄-Alkoxyalkyl, C₂-C₅-Alkylcarbonyl, C₂-C₆-Alkoxycarbonyl, C₂-C₆-Alkylcarbonyloxy oder C(=O)H,
- A': steht außerdem bevorzugt für einen heteroaromatischen Rest ausgewählt aus folgender Gruppe: Furan-2-yl, Furan-3-yl, Thiophen-2-yl, Thiophen-3-yl, Isoxazol-3-yl, Isoxazol-4-yl, Isoxazol-5-yl, Pyrrol-1-yl, Pyrrol-2-yl, Pyrrol-3-yl, Oxazol-2-yl, Oxazol-4-yl, Oxazol-5-yl, Thiazol-2-yl, Thiazol-4-yl, Thiazol-5-yl, Isothiazol-3-yl, Isothiazol-4-yl, Isothiazol-5-yl, Pyrazol-1-yl, Pyrazol-3-yl, Pyrazol-4-yl, Imidazol-1-yl, Imidazol-2-yl, Imidazol-4-yl, 1,2,3-Triazol-1-yl, 1,2,4-Triazol-1-yl, Pyridin-2-yl, Pyridin-3-yl, Pyridin-4-yl, Pyridazin-3-yl, Pyridazin-4-yl, Pyrazin-2-yl, Pyrazin-3-yl, Pyrimidin-2-yl, Pyrimidin-4-yl oder Pyrimidin-5-yl, der bis zu zwei Substituenten enthalten kann, wobei die Substituenten unabhängig voneinander aus R³ und R⁴ ausgewählt sind und
R³ bevorzugt steht für einen Substituenten am Kohlenstoff:
Halogen, Cyano, Hydroxy, Amino, Nitro, C₁-C₆-Alkyl, C₂-C₆-Alkenyl, C₂-C₆-Alkinyl, C₃-C₈-Cycloalkyl, C₁-C₆-Halogenalkyl, C₂-C₆-Halogenalkenyl, C₂-C₆-Halogenalkinyl, C₃-C₆-Halogencycloalkyl, C₁-C₄-Alkoxy, C₁-C₄-Halogenalkoxy, C₁-C₄-Alkylthio, C₁-C₄-Alkylsulfonyl, C₁-C₄-Halogenalkylthio, C₁-C₄-Halogenalkylsulfonyl, C₂-C₄-Alkoxyalkyl, C₂-C₆-Alkoxycarbonyl, C₂-C₆-Alkylcarbonyloxy, C(=O)H' oder Phenyl
R⁴ bevorzugt steht für einen Substituenten am Stickstoff:
C₁-C₆-Alkyl, C₁-C₄-Halogenalkyl, C₃-C₆-Alkenyl, C₃-C₆-Halogenalkenyl, C₃-C₆-Alkinyl oder C₃-C₆-Halogenalkinyl.
- A: steht besonders bevorzugt für Phenyl, das bis zu zwei Substituenten enthalten kann, wobei die Substituenten unabhängig voneinander ausgewählt sind aus R² und
R² steht besonders bevorzugt für:
Fluor, Brom, Iod, Chlor, Cyano, Nitro, Methyl, Ethyl, Propyl, 1-Methylethyl, 1,1-Dimethylethyl, Chlorfluormethyl, Dichlormethyl, Dichlorfluormethyl, Difluormethyl, Trichlormethyl, Trifluormethyl, Cyclopropyl, Ethoxy, 1-Methylethoxy, Propoxy, Methoxy, Trifluormethoxy, Difluormethoxy, 1-Methylethylthio, Methylthio, Ethylthio, Propylthio, Difluormethylthio oder Trifluormethylthio,
- A': steht besonders bevorzugt für einen heteroaromatischen Rest ausgewählt aus folgender Gruppe: Furan-2-yl, Furan-3-yl, Thiophen-2-yl, Thiophen-3-yl, Isoxazol-3-yl, Isoxazol-4-yl, Isoxazol-5-yl, Pyrrol-1-yl, Pyrrol-2-yl, Pyrrol-3-yl, Oxazol-2-yl, Oxazol-4-yl, Oxazol-5-yl, Thiazol-2-yl, Thiazol-4-yl, Thiazol-5-yl, Isothiazol-3-yl, Isothiazol-4-yl, Isothiazol-5-yl, Pyrazol-1-yl, Pyrazol-3-yl, Pyrazol-4-yl, Imidazol-1-yl, Imidazol-2-yl, Imidazol-4-yl, 1,2,3-Triazol-1-yl, 1,2,4-Triazol-1-yl, Pyridin-2-yl, Pyridin-3-yl, Pyridin-4-yl, Pyridazin-3-yl, Pyridazin-4-yl, Pyrimidin-2-yl, Pyrimidin-4-yl oder Pyrimidin-5-yl, der bis zu zwei Substituenten enthalten kann, wobei die Substituenten unabhängig voneinander ausgewählt sind aus aus R³ und R⁴ und
R³ besonders bevorzugt steht für einen Substituenten am Kohlenstoff:
Fluor, Chlor, Brom, Iod, Cyano, Nitro, Methyl, Ethyl, Propyl, 1-Methylethyl, 1,1-Dimethylethyl, Chlorfluormethyl, Dichlormethyl, Dichlorfluormethyl, Difluormethyl, Trichlormethyl, Trifluormethyl, Cyclopropyl, Ethoxy, 1-Methylethoxy, Propoxy, Methoxy, Trifluormethoxy, Difluormethoxy, 1-Methylethylthio, Methylthio, Ethylthio, Propylthio, Difluormethylthio Trifluormethylthio oder Phenyl,
R⁴ besonders bevorzugt steht für einen Substituenten am Stickstoff:
Methyl, Ethyl, Propyl, 1-Methylethyl, 2,2-Trifluorethyl, 2,2-Difluorethyl, 2,2-Dichlor-2-fluorethyl, 2-Chlor-2-difluorethyl oder 2-Chlor-2-fluorethyl,
- A': steht ganz besonders bevorzugt für Pyrazol-1-yl, das bis zu zwei Substituenten enthalten kann, wobei die Substituenten unabhängig voneinander ausgewählt sind aus R³ und
R³ ganz besonders bevorzugt steht für
Methyl, Difluormethyl oder Trifluormethyl,
- A: steht außerdem ganz besonders bevorzugt für Phenyl, das bis zu zwei Substituenten enthalten kann, wobei die Substituenten unabhängig voneinander aus R² ausgewählt sind und
R² ganz besonders bevorzugt steht für:
Methyl, Ethyl, Iod, Chlor, Brom, Fluor, Methoxy, Ethoxy, Difluormethyl oder Trifluormethyl,
- G: steht bevorzugt für G¹ oder G²,
- G: steht besonders bevorzugt für G¹,
- T: steht bevorzugt für *-C(=O)CH₂-# *-C(=O)CH₂C(R⁸)₂-#, *-C(=O)CH=CH-#, *-C(=O)C≡C-#, *-C≡CC(=O)-#, *-CH=CHC(=O)-#, *-CH₂CH₂C(=O)-#, *-C(=S)CH₂-#, *-C(=S)CH₂C(R⁸)₂-#, *-CH₂CH₂C(=S)- #, *-C(=NR⁹)CH₂-# *-C(=NR⁹)CH=CH- #, *-CH=CHC(=NR⁹)-#, *-CH=CHC(NOH)-# oder *-CH=CHC(NO-C₁-C₄-Alkyl)CH₂-#,
- T: steht besonders bevorzugt für *-C(=O)CH₂-# *-C(=O)CH₂C(R⁸)₂-#, *-C(=O)CH=CH-#, *-C≡CC(=O)-#, *-CH=CHC(=O)-#, *-CH₂CH₂C(=O)-#, *-C(=S)CH₂-#, *-C(=S)CH₂C(R⁸)₂-#, *-CH₂CH₂C(=S)- #, *-C(=NR⁹)CH₂-# *-C(=NR⁹)CH=CH-#, *-CH=CHC(-NOH)-# oder *-CH=CHC(N-O-C₁-C₄-Alkyl)CH₂-#,
- T: steht ganz besonders bevorzugt für *-C(=O)CH₂-#, *-C(=O)CH₂C(CH₃)₂-#, *-C(=O)CH₂CH₂-#, *-C(=O)CH=CH-#, *-C≡CC(=O)-#, *-CH=CHC(=O)-#, *-CH₂CH₂C(=O)-#, *-C(=NOCH₃)CH₂-#, *-C(=N-OH)CH=CH-#, *-C(=N-OCH₃)CH=CH-#, oder *-CH=CHC(NOH)-#,
- L¹: steht bevorzugt für CH₂ oder NR⁵, besonders bevorzugt für CH₂,
- X: steht bevorzugt für CH,
- Y: steht bevorzugt für Sauerstoff,
- R¹ₐ: steht bevorzugt für C₁-C₆-Alkyl, C₁-C₆-Halogenalkyl, C₂-C₆-Alkenyl oder C₂-C₆-Alkinyl, C₂-C₆-Alkoxyalkyl,
- R¹_{b}: steht bevorzugt für unsubstituiertes oder substituiertes C₃-C₁₀-Cycloalkyl, wobei die Substituenten unabhängig voneinander aus -Q oder R¹⁰ ausgewählt sind und
R¹⁰ bevorzugt steht für:
Cyano, Halogen, C₁-C₆-Alkyl, C₁-C₆-Halogenalkyl, C₃-C₆-Cycloalkyl, C₂-C₆-Alkenyl, C₂-C₆-Halogenalkenyl, C₂-C₆-Alkinyl, C₂-C₆-Halogenalkinyl, Tri(C₁-C₄-alkyl)silyl, Hydroxy, Oxo, C₁-C₆-Alkoxy, C₁-C₆-Halogenalkoxy, C₃-C₆-Alkenyloxy, C₃-C₆-Alkinyloxy, C₁-C₆-Alkylthio, C₁-C₆-Halogenalkylthio,
- R¹_{c}: steht bevorzugt für unsubstituiertes oder substituiertes C₅-C₁₀-Cycloalkenyl, wobei die Substituenten unabhängig voneinander aus R¹¹ ausgewählt sind und
R¹¹ bevorzugt steht für:
Cyano, Chlor, Fluor, C₁-C₆-Alkyl, C₁-C₆-Halogenalkyl, C₃-C₆-Cycloalkyl, C₂-C₆-Alkenyl, C₂-C₆-Halogenalkenyl, C₂-C₆-Alkinyl, C₂-C₆-Halogenalkinyl, Phenyl, Oxo, C₁-C₆-Alkoxy, C₁-C₆-Halogenalkoxy, C₃-C₆-Alkenyloxy, C₃-C₆-Alkinyloxy, C₁-C₆-Alkylthio oder C₁-C₆-Halogenalkylthio,
- R¹_{d}: steht bevorzugt für unsubstituiertes oder substituiertes Phenyl, wobei die Substituenten unabhängig voneinander aus -L²Q oder R¹² ausgewählt sind und
R¹² bevorzugt steht für:
Halogen, Cyano, Hydroxy, SH, Amino, Nitro, C(=O)H, C(=O)OH, CONR²⁵R²⁶, NR²⁵R²⁶, C₁-C₆-Alkyl, C₂-C₆-Alkenyl, C₂-C₆-Alkinyl, C₁-C₆-Halogenalkyl, C₂-C₆-Halogenalkenyl, C₂-C₆-Halogenalkinyl, C₃-C₈-Cycloalkyl, C₃-C₈-Halogencycloalkyl, C₄-C₁₀-Alkyl-cycloalkyl, C₄-C₁₀-Cycloalkylalkyl, C₆-C₁₄-Cycloalkylcycloalkyl, C₄-C₁₀-Halogencycloalkylalkyl, C₅-C₁₀-Alkylcycloalkylalkyl, C₃-C₈-Cycloalkenyl, C₃-C₈-Halogencycloalkenyl, C₂-C₆-Alkoxyalkyl, C₄-C₁₀-Cycloalkoxyalkyl, C₃-C₈-Alkoxyalkoxyalkyl, C₂-C₆-Alkylthioalkyl, C₂-C₆-Alkylsulfinylalkyl, C₂-C₆-Alkylsulfonylalkyl, C₂-C₆-Alkylaminoalkyl, C₃-C₈-Dialkylaminoalkyl, C₂-C₆-Halogenalkylaminoalkyl, C₄-C₁₀-Cycloalkylaminoalkyl, C₂-C₆-Alkylcarbonyl, C₂-C₆-Halogenalkylcarbonyl, C₄-C₈-Cycloalkylcarbonyl, C₂-C₆-Alkoxycarbonyl, C₄-C₈-Cycloalkoxycarbonyl, C₅-C₁₀-Cycloalkylalkoxycarbonyl, C₂-C₆-Alkylammocarbonyl, C₃-C₈-Dialkylaminocarbonyl, C₄-C₈-Cycloalkylaminocarbonyl, C₂-C₆-Halogenalkoxyalkyl, C₁-C₆-Hydroxyalkyl, C₁-C₆-Alkoxy, C₁-C₆-Halogenalkoxy, C₃-C₈-Cycloalkoxy, C₃-C₈-Halogencycloalkoxy, C₄-C₁₀-Cycloalkylalkoxy, C₃-C₆-Alkenyloxy, C₃-C₆-Halogenalkenyloxy, C₃-C₆-Alkinyloxy, C₃-C₆-Halogenalkinyloxy, C₂-C₆-Alkoxyalkoxy, C₂-C₆-Alkylcarbonyloxy, C₂-C₆-Halogenalkylcarbonyloxy, C₄-C₈-Cycloalkylcarbonyloxy, C₃-C₆-Alkylcarbonylalkoxy, C₁-C₆-Alkylthio, C₁-C₆-Halogenalkylthio, C₃-C₆-Cycloalkylthio, C₁-C₆-Alkylsulfinyl, C₁-C₆-Halogenalkylsulfinyl, C₁-C₆-Alkylsulfonyl, C₁-C₆-Halogenalkylsulfonyl, C₃-C₈-Cycloalkylsulfonyl, Tri(C₁-C₄-alkyl)silyl, C₁-C₆-Alkylsulfonylamino, C₁-C₆-Halogenalkylsulfonylamino,
- R¹ₑ: steht bevorzugt für unsubstituiertes oder substituietes, Naphthalen-1-yl, Naphthalen-2-yl, 1,2,3,4-Tetrahydronaphthalen-1-yl, 1,2,3, 4-Tetrahydronaphthalen-2-yl, 5,6,7,8-Tetrahydronaphthalen-1-yl, 5,6,7,8-Tetrahydronaphthalen-2-yl, Decalin-1-yl, Decalin-2-yl, 1H-Inden-1-yl, 1H-Inden-2-yl, 1H-Inden-3-yl, 1H-Inden-4-yl, 1H-Inden-5-yl, 1H-Inden-6-yl, 1H-Inden-7-yl, Indan-1-yl, Indan-2-yl, Indan-3-yl, Indan-4-yl oder Indan-5-yl, wobei die Substituenten unabhängig voneinander aus R¹³ ausgewählt sind und
R¹³ bevorzugt steht für:
Cyano, Halogen, C₁-C₆-Alkyl, C₁-C₄-Halogenalkyl, C₂-C₆-Alkenyl, C₂-C₆-Alkinyl, Benzyl, Phenyl, C₁-C₆-Alkoxy, C₁-C₆-Halogenalkoxy, C₃-C₆-Alkenyloxy, C₃-C₆-Alkinyloxy oder C₁-C₆-Alkylthio,
- R¹_{f}: steht bevorzugt für einen unsubstituierten oder substituierten 5- oder 6-gliedrigen Heteroarylrest, wobei die Substituenten unabhängig voneinander aus -L²Q oder R¹⁴ und -L³Q oder R¹⁵ ausgewählt sind und
R¹⁴ bevorzugt steht für einen Substituenten am Kohlenstoff:
Halogen, Cyano, Hydroxy, SH, Amino, Nitro, NR²⁵R²⁶, C₁-C₆-Alkyl, C₂-C₆-Alkenyl, C₂-C₆-Alkinyl, C₁-C₆-Halogenalkyl, C₂-C₆-Halogenalkenyl, C₂-C₆-Halogenalkinyl, C₃-C₆-Cycloalkyl, C₃-C₆-Halogencycloalkyl, C₄-C₁₀-Alkylcycloalkyl, C₄-C₁₀-Cycloalkylalkyl, C₆-C₁₄-Cycloalkylcycloalkyl, C₅-C₁₀-Alkylcycloalkylalkyl, C₂-C₄-Alkoxyalkyl, C₃-C₆-Alkenyloxy, C₃-C₆-Alkinyloxy, C₂-C₄-Alkylcarbonyl, C₂-C₆-Alkoxycarbonyl, C₂-C₆-Alkylaminocarbonyl, C₃-C₈-Dialkylaminocarbonyl, C₁-C₄-Hydroxyalkyl, C₁-C₄-Alkoxy, C₁-C₄-Halogenalkoxy, C₂-C₆-Alkylcarbonyloxy, C₂-C₆-Alkylcarbonylthio, C₁-C₄-Alkylthio, C₁-C₄-Halogenalkylthio, C₁-C₄-Alkylsulfinyl, C₁-C₄-Halogenalkylsulfinyl, C₁-C₄-Alkylsulfonyl, C₁-C₄-Halogenalkylsulfonyl, Tri(C₁-C₄-alkyl)silyl,
R¹⁵ bevorzugt steht für einen Substituenten am Stickstoff:
C₁-C₆-Alkyl, C₃-C₆-Alkenyl, C₃-C₆-Alkinyl, C₁-C₆-Halogenalkyl, C₃-C₆-Halogenalkenyl, C₃-C₆-Halogenalkinyl, C₃-C₆-Cycloalkyl, C₃-C₆-Halogencycloalkyl, C₄-C₁₀-Alkyl-cycloalkyl, C₄-C₁₀-Cycloalkylalkyl, Phenyl,
- R¹_{g}: steht bevorzugt für benzokondensiertes unsubstituiertes oder substituiertes 5- oder 6-gliedriges Heteroaryl, wobei die Substituenten unabhängig voneinander ausgewählt sind aus R¹⁶ und R¹⁷ und
R¹⁶ bevorzugt steht für einen Substituenten am Kohlenstoff:
Halogen, Cyano, Hydroxy, SH, Amino, Nitro, NR²⁵R²⁶, C₁-C₆-Alkyl, C₂-C₆-Alkenyl, C₂-C₆-Alkinyl, C₁-C₆-Halogenalkyl, C₂-C₆-Halogenalkenyl, C₂-C₆-Halogenalkinyl, C₃-C₆-Cycloalkyl, C₃-C₆-Halogencycloalkyl, C₄-C₁₀-Alkylcycloalkyl, C₄-C₁₀-Cycloalkylalkyl, C₆-C₁₄-Cycloalkylcycloalkyl, C₅-C₁₀-Alkylcycloalkylalkyl, C₂-C₄-Alkoxyalkyl, C₂-C₄-Alkylcarbonyl, C₂-C₆-Alkoxycarbonyl, C₂-C₆-Alkylaminocarbonyl, C₃-C₈-Dialkylaminocarbonyl, C₁-C₄-Hydroxyalkyl, C₁-C₄-Alkoxy, C₁-C₄-Halogenalkoxy, C₂-C₆-Alkylcarbonyloxy, C₂-C₆-Alkylcarbonylthio, C₁-C₄-Alkylthio, C₁-C₄-Halogenalkylthio, C₁-C₄-Alkylsulfinyl, C₁-C₄-Halogenalkylsulfinyl, C₁-C₄-Alkylsulfonyl, C₁-C₄-Halogenalkylsulfonyl, Tri(C₁-C₄-alkyl)silyl oder Phenyl,
R¹⁷ bevorzugt steht für einen Substituenten am Stickstoff: C₁-C₆-Alkyl, C₃-C₆-Alkenyl, C₃-C₆-Alkinyl, C₁-C₆-Halogenalkyl, C₃-C₆-Halogenalkenyl, C₃-C₆-Halogenalkinyl, C₃-C₆-Cycloalkyl, C₃-C₆-Halogencycloalkyl, C₄-C₁₀-Alkyl-cycloalkyl, C₄-C₁₀-Cycloalkylalkyl oder Phenyl,
- R¹ₕ: steht bevorzugt für unsubstituiertes oder substituiertes C₅-C₁₅-Heterocyclyl, wobei mögliche Substituenten unabhängig voneinander ausgewählt sind aus R¹⁸ und R¹⁹ und
R¹⁸ bevorzugt steht für einen Substituenten am Kohlenstoff:
Halogen, Cyano, Hydroxy, SH, Amino, Nitro, NR²⁵R²⁶, C₁-C₆-Alkyl, C₂-C₆-Alkenyl, C₂-C₆-Alkinyl, C₁-C₆-Halogenalkyl, C₂-C₆-Halogenalkenyl, C₂-C₆-Halogenalkinyl, C₃-C₆-Cycloalkyl, C₃-C₆-Halogencycloalkyl, C₄-C₁₀-Alkylcycloalkyl, C₄-C₁₀-Cycloalkylalkyl, C₆-C₁₄-Cycloalkylcycloalkyl, C₅-C₁₀-Alkylcycloalkylalkyl, C₂-C₄-Alkoxyalkyl, C₂-C₄-Alkylcarbonyl, C₂-C₆-Alkoxycarbonyl, C₂-C₆-Alkylaminocarbonyl, C₃-C₈-Dialkylaminocarbonyl, C₁-C₄-Hydroxyalkyl, C₁-C₄-Alkoxy, C₁-C₄-Halogenalkoxy, C₂-C₆-Alkylcarbonyloxy, C₂-C₆-Alkylcarbonylthio, C₁-C₄-Alkylthio, C₁-C₄-Halogenalkylthio, C₁-C₄-Alkylsulfinyl, C₁-C₄-Halogenalkylsulfinyl, C₁-C₄-Alkylsulfonyl, C₁-C₄-Halogenalkylsulfonyl, Tri(C₁-C₄-alkyl)silyl oder Phenyl,
R¹⁹ bevorzugt steht für einen Substituenten am Stickstoff:
C₁-C₆-Alkyl, C₃-C₆-Alkenyl, C₃-C₆-Alkinyl, C₁-C₆-Halogenalkyl, C₃-C₆-Halogenalkenyl, C₃-C₆-Halogenalkinyl, C₃-C₆-Cycloalkyl, C₃-C₆-Halogencycloalkyl, C₄-C₁₀-Alkylcycloalkyl, C₄-C₁₀-Cycloalkylalkyl oder Phenyl,
- R¹ₐ: steht besonders bevorzugt für 1,1-Dimethylethyl, 3,3-Dimethylbutyl, 2,2-Dimethylpropyl, 1,2,2-Trimethylpropyl, Pentyl, 1-Ethylpropyl, Butyl, 2-Methylpropyl, 1-Methylethyl, Ethyl, Propyl, 4-Methylpentyl oder Hexyl,
- R¹_{b}: steht besonders bevorzugt für Cyclopentyl, Cyclohexyl oder Cycloheptyl, welche jeweils bis zu zwei Substituenten enthalten können, wobei die Substituenten unabhängig voneinander aus R¹⁰ ausgewählt sind und
R¹⁰ besonders bevorzugt steht für:
Cyano, Chlor, Fluor, Brom, Iod, Methyl, Ethyl, Propyl, 1-Methylethyl, 1,1-Dimethylethyl, Ethenyl, Phenyl, Methoxy, Ethoxy, Propyloxy, Trifluormethoxy, Ethinyl, 2-Propinyloxy, Methylthio, Ethylthio oder Trifluomethylthio,
- R¹_{c}: steht besonders bevorzugt für Cyclopentenyl, Cyclohexenyl oder Cycloheptenyl, welche jeweils bis zu zwei Substituenten enthalten können, wobei die Substituenten unabhängig voneinander aus R¹¹ ausgewählt sind und
R¹¹ besonders bevorzugt steht für:
Methyl, Ethyl, Methoxy, Ethoxy, Trifluormethoxy, Ethinyl, 2-Propinyloxy, Methylthio, Ethylthio oder Trifluormethylthio,
- R¹_{d}: steht außerdem besonders bevorzugt für Phenyl, das bis zu drei Substituenten enthalten kann, wobei die Substituenten unabhängig voneinander aus -L²Q' oder R¹² ausgewählt sind und
R¹² besonders bevorzugt steht für:
Fluor, Chlor, Brom, Iod, Cyano, Nitro, Methyl, Ethyl, Propyl, 1-Methylethyl, Butyl, 1,1-Dimethylethyl, 1,2-Dimethylethyl, Ethenyl, Ethinyl, Trifluoromethyl, Difluormethyl, Trichlormethyl, Dichlormethyl, Cyclopropyl, Methoxy, Ethoxy, Propoxy, 1-Methylethoxyl, 1,1-Dimethylethoxyl, Methylcarbonyl, Ethylcarbonyl, Trifluormethylcar-bonyl, Methoxycarbonyl, Ethoxycarbonyl, Propoxycarbonyl, 1-Methylethoxycarbonyl, 1,1-Dimethylethoxycarbonyl, 2-Propenyloxy, 2-Propinyloxy, Methylthio, Ethylthio, Methylsulfinyl oder Methylsulfonyl,
- R¹ₑ: steht besonders bevorzugt für Naphthalen-1-yl, Naphthalen-2-yl, 1,2,3,4-Tetrahydronaphthalen-1-yl, 1,2,3,4-Tetrahydronaphthalen-2-yl, 5,6,7,8-Tetrahydronaphthalen-1-yl, 5,6,7,8-Tetrahydronaphthalen-2-yl, Decalin-1-yl, Decalin-2-yl, 1H-Inden-1-yl, 1H-Inden-2-yl, 1H-Inden-3-yl, 1H-Inden-4-yl, 1H-Inden-5-yl, 1H-Inden-6-yl, 1H-Inden-7-yl, Indan-1-yl, Indan-2-yl, Indan-3-yl, Indan-4-yl oder Indan-5-yl, das bis zu drei Substituenten enthalten kann, wobei die Substituenten unabhängig voneinander aus R¹³ ausgewählt sind und
R¹³ besonders bevorzugt steht für:
Methyl, Methoxy, Cyano, Fluor, Chlor, Brom, Iod,
- R¹_{f}: steht besonders bevorzugt für Furan-2-yl, Furan-3-yl, Thiophen-2-yl, Thiophen-3-yl, Isoxazol-3-yl, Isoxazol-4-yl, Isoxazol-5-yl, Pyrrol-1-yl, Pyrrol-2-yl, Pyrrol-3-yl, Oxazol-2-yl, Oxazol-4-yl, Oxazol-5-yl, Thiazol-2-yl, Thiazol-4-yl, Thiazol-5-yl, Isothiazol-3-yl, Isothiazol-4-yl, Isothiazol-5-yl, Pyrazol-1-yl, Pyrazol-3-yl, Pyrazol-4-yl, Imidazol-1-yl, Imidazol-2-yl, Imidazol-4-yl, 1,2,4-Oxadiazol-3-yl, 1,2,4-Oxadiazol-5-yl, 1,3,4-Oxadiazol-2-yl, 1,2,4-Thiadiazol-3-yl, 1,2,4-Thiadiazol-5-yl, 1,3,4-Thiadiazol-2-yl, 1,2,3-Triazol-1-yl, 1,2,3-Triazol-2-yl, 1,2,3-Triazol-4-yl, 1,2,4-Triazol-1-yl, 1,2,4-Triazol-3-yl, 1,2,4-Triazol-4-yl, Pyridin-2-yl, Pyridin-3-yl, Pyridin-4-yl, Pyridazin-3-yl, Pyridazin-4-yl, Pyrimidin-2-yl, Pyrimidin-4-yl, Pyrimidin-5-yl oder Pyrazin-2-yl, welche jeweils bis zu zwei Substituenten enthalten können, wobei die Substituenten unabhängig voneinander aus R¹⁴ und R¹⁵ ausgewählt sind und
R¹⁴ besonders bevorzugt steht für einen Substituenten am Kohlenstoff:
Chlor, Fluor, Brom, Iod, Cyano, Nitro, Methyl, Ethyl, Propyl, 1-Methylethyl, Butyl, 1,1-Dimethylethyl, Ethenyl, Ethinyl, Trifluormethyl, Difluormethyl, Cyclopropyl, Cyclopentyl, Cyclohexyl, Methylcarbonyl, Ethylcarbonyl, Methoxylcarbonyl, Ethoxycarbonyl, Methoxy, Ethoxy, Propoxy, 1-Methylethoxy, 2-Propinyoxy, Trifluormethoxyl, Methylcabonyloxy, Methylcarbonylthio, Methylthio, Ethylthio, Trifluomethylthio, Methylsulfinyl, Ethylsulfinyl, Trifluoromethylsulfinyl, Methylsulfonyl, Ethylsulfonyl, oder Trifluormethylsulfonyl,
R¹⁵ besonders bevorzugt steht für einen Substituenten am Stickstoff:
Methyl, Ethyl, Propyl, Cyclopropyl, Cyclohexyl, Phenyl oder 2-Propinyl,
- R¹_{g}: steht besonders bevorzugt für Indol-1-yl, Indol-2-yl, Indol-3-yl, Indol-4-yl, Indol-5-yl, Indol-6-yl, Indol-7-yl, Benzimidazol-1-yl, Benzimidazol-2-yl, Benzimidazol-4-yl, Benzimidazol-5-yl, Indazol-1-yl, Indazol-3-yl, Indazol-4-yl, Indazol-5-yl, Indazol-6-yl, Indazol-7-yl, Indazol-2-yl, 1-Benzofuran-2-yl, 1-Benzofuran-3-yl, 1-Benzofuran-4-yl, 1-Benzofuran-5-yl, 1-Benzofuran-6-yl, 1-Benzofuran-7-yl, 1-Benzothiophen-2-yl, 1-Benzothiophen-3-yl, 1-Benzothiophen-4-yl, 1-Benzothiophen-5-yl, 1-Benzothiophen-6-yl, 1-Benzothiophen-7-yl, 1,3-Benzothiazol-2-yl, 1,3-Benzothiazol-4-yl, 1,3-Benzothiazol-5-yl, 1,3-Benzothiazol-6-yl, 1,3-Benzothiazol-7-yl, 1,3-Benzoxazol-2-yl, 1,3-Benzoxazol-4-yl, 1,3-Benzoxazol-5-yl, 1,3-Benzoxazol-6-yl, 1,3-Benzoxazol-7-yl, Chinolin-2-yl, Chinolin-3-yl, Chinolin-4-yl, Chinolin-5-yl, Chinolin-6-yl, Chinolin-7-yl, Chinolin-8-yl, Isochinolin-1-yl, Isochinolin-3-yl, Isochinolin-4-yl, Isochinolin-5-yl, Isochinolin-6-yl, Isochinolin-7-yl oder Isochinolin-8-yl, welche jeweils bis zu zwei Substituenten enthalten können, wobei die Substituenten unabhängig voneinander aus R¹⁶ und R¹⁷ ausgewählt sind und
R¹⁶ besonders bevorzugt steht für einen Substituenten am Kohlenstoff:
Fluor, Chlor, Brom, Iod, Methyl, Methoxy, 2-Propinyloxy, 2-Propenyloxy,
R¹⁷ besonders bevorzugt steht für einen Substituenten am Stickstoff:
Methyl, Ethyl, Propyl, Cyclopropyl, Cyclohexyl, Phenyl oder 2-Propinyl,
- R¹ₐ: steht ganz besonders bevorzugt für 1,1-Dimethylethyl,
- R¹_{b}: steht ganz besonders bevorzugt für Cyclohexyl, Cyclopentyl, 2-Methylcyclohexyl, 3-Methylcyclohexyl oder 4-Methylcyclohexyl,
- R¹_{c}: steht ganz besonders bevorzugt für Cyclohex-3-en-1-yl oder Cyclohex-2-en-1-yl,
- R¹_{d}: steht ganz besonders bevorzugt für Phenyl, das bis zu zwei Substituenten enthalten kann, wobei die Substituenten unabhängig voneinander aus R¹² ausgewählt sind und
R¹² ganz besonders bevorzugt steht für:
Chlor, Fluor, Brom, Iod, Methyl, Methoxy, Ethoxy, Trifluormethyl, Difluormethyl, 2-Propenyloxy, 2-Propinyloxy oder Phenyl,
- R¹ₑ: steht ganz besonders bevorzugt für Naphthalen-1-yl, Naphthalen-2-yl, 1,2,3,4-Tetrahydronaphthalen-1-y l , 1 , 2,3,4-Tetrahydronaphthalen-2-yl, 5,6,7,8-Tetrahydronaphthalen-1-yl, 5,6,7,8-Tetrahydronaphthalen-2-yl, Decalin-1-yl, Decalin-2-yl, 1H-Inden-1-yl, 1H-Inden-2-yl, 1H-Inden-3-yl, 1H-Inden-4-yl, 1H-Inden-5-yl, 1H-Inden-6-yl, 1H-Inden-7-yl, Indan-1-yl, Indan-2-yl, Indan-3-yl, Indan-4-yl oder Indan-5-yl,
- R¹_{f}: steht ganz besonders bevorzugt für für Furan-2-yl, Furan-3-yl, Thiophen-2-yl, Thiophen-3-yl, Isoxazol-3-yl, Isoxazol-4-yl, Isoxazol-5-yl, Pyrrol-1-yl, Pyrrol-2-yl, Pyrrol-3-yl, Oxa-zol-2-yl, Oxazol-4-yl, Oxazol-5-yl, Thiazol-2-yl, Thiazol-4-yl, Thiazol-5-yl, Isothiazol-3-yl, Isothiazol-4-yl, Isothiazol-5-yl, Pyrazol-1-yl, Pyrazol-3-yl, Pyrazol-4-yl, Imidazol-1-yl, Imidazol-2-yl, Imidazol-4-yl, 1,2,3-Triazol-1-yl, 1,2,3-Triazol-2-yl, 1,2,3-Triazol-4-yl, 1,2,4-Triazol-1-yl, 1,2,4-Triazol-3-yl, 1,2,4-Triazol-4-yl, Pyridin-2-yl, Pyridin-3-yl, Pyridin-4-yl, Pyridazin-3-yl, Pyridazin-4-yl, Pyrimidin-2-yl, Pyrimidin-4-yl oder Pyrimidin-5-yl,
- L²: steht bevorzugt für eine direkt Bindung oder - O -,
- L²: steht besonders bevorzugt für eine direkt Bindung,
- L³: steht bevorzugt für eine direkt Bindung,
- Q': steht bevorzugt für Phenyl, das bis zu zwei Substituenten enthalten kann, wobei die Substituenten unabhängig voneinander aus R²² ausgewählt sind und
R²² bevorzugt steht für:
Fluor, Chlor, Brom, Iod, Cyano, Hydroxy, SH, Nitro, C₁-C₆-Alkyl, C₁-C₆-Halogenalkyl, C₃-C₆-Cycloalkyl, C₁-C₄-Alkoxy, C₁-C₄-Halogenalkoxy, C₁-C₄-Alkylthio oder C₁-C₄-Halogenalkylthio,
- Q": steht bevorzugt für Furan-2-yl, Furan-3-yl, Thiophen-2-yl, Thiophen-3-yl, Isoxazol-3-yl, Isoxazol-4-yl, Isoxazol-5-yl, Pyrrol-1-yl, Pyrrol-2-yl, Pyrrol-3-yl, Oxazol-2-yl, Oxazol-4-yl, Oxazol-5-yl, Thiazol-2-yl, Thiazol-4-yl, Thiazol-5-yl, Isothiazol-3-yl, Isothiazol-4-yl, Isothiazol-5-yl, Pyrazol-1-yl, Pyrazol-3-yl, Pyrazol-4-yl, Imidazol-1-yl, Imidazol-2-yl, Imidazol-4-yl, 1,2,4-Oxadiazol-3-yl, 1,2,4-Oxadiazol-5-yl, 1,3,4-Oxadiazol-2-yl, Tetrazol-5-yl, 1,2,4-Thiadiazol-3-yl, 1,2,4-Thiadiazol-5-yl, 1,3,4-Thiadiazol-2-yl, 1,2,3-Triazol-1-yl, 1,2,3-Triazol-2-yl, 1,2,3-Triazol-4-yl, 1,2,4-Triazol-1-yl, 1,2,4-Triazol-3-yl, 1,2,4-Tri-azol-4-yl, Tetrazol-5-yl, Pyridin-2-yl, Pyridin-3-yl, Pyridin-4-yl, Pyridazin-3-yl, Pyridazin-4-yl, Pyrimidin-2-yl, Pyrimidin-4-yl, Pyrimidin-5-yl, Pyrazin-2-yl, 1,3,5-Triazin-2-yl oder 1,2,4-Triazin-3-yl, welche jeweils bis zu zwei Substituenten enthalten können, wobei die Substituenten unabhängig voneinander aus R²³ und R²⁴ ausgewählt sind und
R²³ bevorzugt steht für einen Substituenten am Kohlenstoff:
Fluor, Chlor, Brom, Iod, Cyano, Hydroxy, SH, Nitro, C₁-C₆-Alkyl, C₁-C₆-Halogenalkyl, C₃-C₆-Cycloalkyl, C₁-C₄-Alkoxy, C₁-C₄-Halogenalkoxy, C₁-C₄-Alkylthio oder C₁-C₄-Halogenalkylthio,
R²⁴ bevorzugt steht für einen Substituenten am Stickstoff:
C₁-C₆-Alkyl, C₃-C₆-Alkenyl, C₃-C₆-Alkinyl, C₃-C₆-Cycloalkyl oder Phenyl,
- Q': steht besonders bevorzugt für Phenyl,
- R⁵: steht bevorzugt für Wasserstoff oder Methyl, Ethyl, Propyl, *iso*-Propyl, Butyl, *iso*-Butyl oder *tert*-Butyl,
- R⁵: steht besonders bevorzugt für Wasserstoff,
- R⁷: steht bevorzugt für Wasserstoff,
- R⁸: steht unabhängig voneinander bevorzugt für Wasserstoff, Methyl oder Ethyl,
- R⁸: steht besonders bevorzugt für Wasserstoff, Methyl,
- R²⁵ und R²⁶: stehen unabhängig voneinander bevorzugt für Wasserstoff, Methyl, Ethyl, Propyl, *iso*-Propyl, Butyl, *iso*-Butyl oder *tert*-Butyl,

Die erfindungsgemäß verwendbaren Heteroarylcarbonsaüre Derivate sind durch die Formel (I) allgemein definiert. Bevorzugte Restedefinitionen der vorstehenden und nachfolgend genannten Formeln sind im Folgenden angegeben. Diese Definitionen gelten für die Endprodukte der Formel (I) wie für alle Zwischenprodukte (siehe auch unten unter "Erläuterungen der Verfahren und Zwischenprodukte") gleichermaßen.

Die oben aufgeführten allgemeinen oder in Vorzugsbereichen aufgeführten Restedefinitionen bzw. Erläuterungen können jedoch auch untereinander, also zwischen den jeweiligen Bereichen und Vorzugsbereichen beliebig kombiniert werden. Sie gelten für die Endprodukte sowie für die Vor- und Zwischenprodukte entsprechend. Außerdem können einzelne Definitionen entfallen.

Bevorzugt sind solche Verbindungen der Formel (I), in welcher alle Reste jeweils die oben genannten bevorzugten Bedeutungen haben.

Besonders bevorzugt sind solche Verbindungen der Formel (I), in welcher alle Reste jeweils die oben genannten besonders bevorzugten Bedeutungen haben.

Ganz besonders bevorzugt sind solche Verbindungen der Formel (I), in welcher alle Reste jeweils die oben genannten ganz besonders bevorzugten Bedeutungen haben.

Weiterhin bevorzugt sind Verbindungen der Formel (I), in welcher
- A: für 3,5-Bis(difluormethyl)-1H-pyrazol-1-yl steht.

Weiterhin bevorzugt sind Verbindungen der Formel (I), in welcher
- A: für 5-Methyl-3-(trifluormethyl)-1H-pyrazol-1-yl steht.

Weiterhin bevorzugt sind Verbindungen der Formel (I), in welcher
- A: für 2,5-Dimethylphenyl steht.

Weiterhin bevorzugt sind Verbindungen der Formel (I), in welcher
- A: für 2-Methyl-5-chlorphenyl steht.

Weiterhin bevorzugt sind Verbindungen der Formel (I), in welcher
- G: für G¹ steht.

Weiterhin bevorzugt sind Verbindungen der Formel (I), in welcher
- T: für *-C(=O)CH₂-# steht.

Weiterhin bevorzugt sind Verbindungen der Formel (I), in welcher
- T: für *-C(=O)CH₂C(CH₃)₂-# steht.

Weiterhin bevorzugt sind Verbindungen der Formel (I), in welcher
- T: für *-C(=O)CH₂CH-# steht.

Weiterhin bevorzugt sind Verbindungen der Formel (I), in welcher
- T: für *-C(=O)CH=CH-# steht.

Weiterhin bevorzugt sind Verbindungen der Formel (I), in welcher
- T: für *-C≡CC(=O)-# steht.

Weiterhin bevorzugt sind Verbindungen der Formel (I), in welcher
- T: für *-CH=CHC(=O)-# steht.

Weiterhin bevorzugt sind Verbindungen der Formel (I), in welcher
- T: für *-CH₂CH₂C(=O)-# steht.

Weiterhin bevorzugt sind Verbindungen der Formel (I), in welcher
- T: für *-C(=NOCH3)CH₂-# steht.

Weiterhin bevorzugt sind Verbindungen der Formel (I), in welcher
- T: für *-CH=CHC(=NOH)-# steht.

Weiterhin bevorzugt sind Verbindungen der Formel (I), in welcher
- X: für CH steht.

Weiterhin bevorzugt sind Verbindungen der Formel (I), in welcher
- Y: für Sauerstoff steht.

Weiterhin bevorzugt sind Verbindungen der Formel (I), in welcher
- L¹: für NH steht.

Weiterhin bevorzugt sind Verbindungen der Formel (I), in welcher
- L¹: für -CH₂- steht.

Weiterhin bevorzugt sind Verbindungen der Formel (I), in welcher
- R⁸: für Wasserstoff steht.

Weiterhin bevorzugt sind Verbindungen der Formel (I), in welcher
- R⁸: für Methyl steht.

Weiterhin bevorzugt sind Verbindungen der Formel (I), in welcher
- R⁵: für Wasserstoff steht.

Weiterhin bevorzugt sind Verbindungen der Formel (I), in welcher
- R¹: für Cyclohexyl (R¹_{b}) steht.

Weiterhin bevorzugt sind Verbindungen der Formel (I), in welcher
- R¹: für Phenyl (R¹_{d}) steht.

Weiterhin bevorzugt sind Verbindungen der Formel (I), in welcher
- R¹: für 2-Methoxyphenyl (R¹_{d}) steht.

Weiterhin bevorzugt sind Verbindungen der Formel (I), in welcher
- R¹: für 4-Methoxyphenyl (R¹_{d}) steht.

Weiterhin bevorzugt sind Verbindungen der Formel (I), in welcher
- R¹: für 2-Ethoxyphenyl (R¹_{d}) steht.

Weiterhin bevorzugt sind Verbindungen der Formel (I), in welcher
- R¹: für Naphthalen-1-yl (R¹ₑ )steht.

Weiterhin bevorzugt sind Verbindungen der Formel (I), in welcher
- R¹: für *tert*-Butyl (R¹ₐ) steht.

Weiterhin bevorzugt sind Verbindungen der Formel (I), in welcher
- R¹: für Thiophen-2-yl (R¹_{f}) steht.

Weiterhin bevorzugt sind Verbindungen der Formel (I), in welcher
- R¹: für Furan-2-yl (R¹_{f}) steht.

Weiterhin bevorzugt sind Verbindungen der Formel (I), in welcher
- R¹: für 2-Chlorphenyl (R¹_{d}) steht.

Weiterhin bevorzugt sind Verbindungen der Formel (I), in welcher
- R¹: für 2,4-Dichlorphenyl (R¹_{d}) steht.

Weiterhin bevorzugt sind Verbindungen der Formel (I), in welcher
- R¹: für 2-Bromphenyl (R¹_{d}) steht.

Weiterhin bevorzugt sind Verbindungen der Formel (I), in welcher
- R¹: für 2,6-Difluorphenyl (R¹_{d}) steht.

Weiterhin bevorzugt sind Verbindungen der Formel (I), in welcher
- R¹: für 2-Iodphenyl (R¹_{d}) steht.

Weiterhin bevorzugt sind Verbindungen der Formel (I), in welcher
- R¹: für 2-Methylphenyl (R¹_{d}) steht.

Weiterhin bevorzugt sind Verbindungen der Formel (I), in welcher
- R¹: für 3-Methylphenyl (R¹_{d}) steht.

Weiterhin bevorzugt sind Verbindungen der Formel (I), in welcher
- R¹: für Cyclopentyl (R¹_{b}) steht.

Weiterhin bevorzugt sind Verbindungen der Formel (I), in welcher
- R¹: für 2-Fluor-4-methoxyphenyl (R¹_{d}) steht.

Weiterhin bevorzugt sind Verbindungen der Formel (I), in welcher
- R¹: für 2-Brom-4-fluorphenyl (R¹_{d}) steht.

Weiterhin bevorzugt sind Verbindungen der Formel (I), in welcher
- R¹: für 2,6-Dimethoxyphenyl (R¹_{d}) steht.

Weiterhin bevorzugt sind Verbindungen der Formel (I), in welcher
- R¹: für 2-Methylcyclohexyl (R¹_{b}) steht.

Weiterhin bevorzugt sind Verbindungen der Formel (I), in welcher
- R¹: für 2-(Prop-2-yn-1-yloxy)phenyl (R¹_{d}) steht.

Weiterhin bevorzugt sind Verbindungen der Formel (I), in welcher
- R¹: für 2,6-Dichlorphenyl (R¹_{d}) steht.

Weiterhin bevorzugt sind Verbindungen der Formel (I), in welcher
- R¹: für 2,6-Dimethylphenyl (R¹_{d}) steht.

Weiterhin bevorzugt sind Verbindungen der Formel (I), in welcher
- R¹: für 2,4,6-Trifluorphenyl (R¹_{d}) steht.

Weiterhin bevorzugt sind Verbindungen der Formel (I), in welcher
- R¹: für 2-Chlor-5-fluorphenyl (R¹_{d}) steht.

Weiterhin bevorzugt sind Verbindungen der Formel (I), in welcher
- R¹: für 2-Chlor-6-fluorphenyl (R¹_{d}) steht.

Weiterhin bevorzugt sind Verbindungen der Formel (I), in welcher
- R¹: für 4-Fluorphenyl (R¹_{d}) steht.

Weiterhin bevorzugt sind Verbindungen der Formel (I), in welcher
- R¹: für 3-Fluorphenyl (R¹_{d}) steht.

Weiterhin bevorzugt sind Verbindungen der Formel (I), in welcher
- R¹: für 2-Fluorphenyl (R¹_{d}) steht.

Die zuvor genannten Reste-Definitionen können untereinander in beliebiger Weise kombiniert werden. Außerdem können einzelne Definitionen entfallen.

Je nach Art der oben definierten Substituenten weisen die Verbindungen der Formel (I) saure oder basische Eigenschaften auf und können mit anorganischen oder organischen Säuren oder mit Basen oder mit Metallionen Salze, gegebenenfalls auch innere Salze oder Addukte bilden. Tragen die Verbindungen der Formel **(I)** Amino, Alkylamino oder andere, basische Eigenschaften induzierende Gruppen, so können diese Verbindungen mit Säuren zu Salzen umgesetzt werden oder fallen durch die Synthese direkt als Salze an. Tragen die Verbindungen der Formel **(I)** Hydroxy, Carboxy oder andere, saure Eigenschaften induzierende Gruppen, so können diese Verbindungen mit Basen zu Salzen umgesetzt werden. Geeignete Basen sind beispielsweise Hydroxide, Carbonate, Hydrogencarbonate der Alkali- und Erdalkalimetalle, insbesondere die von Natrium, Kalium, Magnesium und Calcium, weiterhin Ammoniak, primäre, sekundäre und tertiäre Amine mit C₁-C₄-Alkyl-Gruppen, Mono-, Di- und Trialkanolamine von C₁-C₄-Alkanolen, Cholin sowie Chlorcholin.

Die so erhältlichen Salze weisen ebenfalls fungizide, herbizide und insektizide Eigenschaften auf.

Beispiele für anorganische Säuren sind Halogenwasserstoffsäuren wie Fluorwasserstoff, Chlorwasserstoff, Bromwasserstoff und Iodwasserstoff, Schwefelsäure, Phosphorsäure und Salpetersäure und saure Salze wie NaHSO₄ und KHSO₄. Als organische Säuren kommen beispielsweise Ameisensäure, Kohlensäure und Alkansäuren wie Essigsäure, Trifluoressigsäure, Trichloressigsäure und Propionsäure sowie Glycolsäure, Thiocyansäure, Milchsäure, Bernsteinsäure, Zitronensäure, Benzoesäure, Zimtsäure, Oxal-säure, gesättigte oder einfach oder doppelt ungesättigte C₆-C₂₀-Fettsäuren, Alkylschwefelsäuremonoester, Alkylsulfonsäuren (Sulfonsäuren mit geradkettigen oder verzweigten Alkylresten mit 1 bis 20 Kohlenstoffatomen), Arylsulfonsäuren oder Aryldisulfonsäuren (aromatische Reste wie Phenyl und Naphthyl welche ein oder zwei Sulfonsäuregruppen tragen), Alkylphosphon-säuren (Phosphonsäuren mit geradkettigen oder verzweigten Alkylresten mit 1 bis 20 Kohlenstoffatomen), Arylphosphonsäuren oder Aryldiphosphonsäuren (aromatische Reste wie Phenyl und Naphthyl welche ein oder zwei Phosphonsäurereste tragen), wobei die Alkyl- bzw. Arylreste weitere Substituenten tragen können, z.B. p-Toluolsulfonsäure, Salicylsäure, p-Aminosalicylsäure, 2-Phenoxybenzoesäure, 2-Acetoxybenzoesäure etc..

Als Metallionen kommen insbesondere die Ionen der Elemente der zweiten Hauptgruppe, insbesondere Calzium und Magnesium, der dritten und vierten Hauptgruppe, insbesondere Aluminium, Zinn und Blei, sowie der ersten bis achten Nebengruppe, insbesondere Chrom, Mangan, Eisen, Kobalt, Nickel, Kupfer, Zink und andere in Betracht. Besonders bevorzugt sind die Metallionen der Elemente der vierten Periode. Die Metalle können dabei in den verschiedenen ihnen zukommenden Wertigkeiten vorliegen.

Gegebenenfalls substituierte Gruppen können einfach oder mehrfach substituiert sein, wobei bei Mehrfachsubstitutionen die Substituenten gleich oder verschieden sein können.

Bei den in den vorstehenden Formeln angegebenen Definitionen der Symbole wurden Sammelbegriffe verwendet, die allgemein repräsentativ für die folgenden Substituenten stehen:
**Halogen:** Fluor, Chlor, Brom und Iod;
**Alkyl:** gesättigte, geradkettige oder verzweigte Kohlenwasserstoffreste mit 1 bis 8 Kohlenstoffatomen, z.B. (aber nicht beschränkt auf) C₁-C₆-Alkyl wie Methyl, Ethyl, Propyl, 1-Methylethyl, Butyl, 1-Methyl-propyl, 2-Methylpropyl, 1,1-Dimethylethyl, Pentyl, 1-Methylbutyl, 2-Methylbutyl, 3-Methylbutyl, 2,2-Di-methylpropyl, 1-Ethylpropyl, Hexyl, 1,1-Dimethylpropyl, 1,2-Dimethylpropyl,1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1-Ethyl-2-methylpropyl;
**Alkenyl:** ungesättigte, geradkettige oder verzweigte Kohlenwasserstoffreste mit 2 bis 8 Kohlenstoff atomen und einer Doppelbindung in einer beliebigen Position, z.B. (aber nicht beschränkt auf) C₂-C₆-Alkenyl wie Ethenyl, 1-Propenyl, 2-Propenyl, 1-Methylethenyl, 1-Butenyl, 2-Butenyl, 3-Butenyl, 1-Methyl-1-propenyl, 2-Methyl-1-propenyl, 1-Methyl-2-propenyl, 2-Methyl-2-propenyl, 1-Pentenyl, 2-Pentenyl, 3-Pentenyl, 4-Pentenyl, 1-Methyl-1-butenyl, 2-Methyl-1-butenyl, 3-Methyl-1-butenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-1-propenyl, 1,2-Dimethyl-2-propenyl, 1-Ethyl-1-propenyl, 1-Ethyl-2-propenyl, 1-Hexenyl, 2-Hexenyl, 3-Hexenyl, 4-Hexenyl, 5-Hexenyl, 1-Methyl-1-pentenyl, 2-Methyl-1-pentenyl, 3-Methyl-1-pentenyl, 4-Methyl-1-pentenyl, 1-Methyl-2-pentenyl, 2-Methyl-2-pentenyl, 3-Methyl-2-pentenyl, 4-Methyl-2-pentenyl, 1-Methyl-3-pentenyl, 2-Methyl-3-pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1,-Dimethyl-3-butenyl, 1,2-Dimethyl-1-butenyl, 1,2-Dimethyl-2-butenyl, 1,2-Dimethyl-3-butenyl, 1,3-Dimethyl-1-butenyl, 1,3-Dimethyl-2-butenyl, 1,3-Dimethyl-3-butenyl, 2,2-Dimethyl-3-butenyl, 2,3-Dimethyl-1-butenyl, 2,3-Dimethyl-2-butenyl, 2,3-Dimethyl-3-butenyl, 3,3-Dimethyl-1-butenyl, 3,3-Dimethyl-2-butenyl, 1-Ethyl-1-butenyl, 1-Ethyl-2-butenyl, 1-Ethyl-3-butenyl, 2-Ethyl-1-butenyl, 2-Ethyl-2-butenyl, 2-Ethyl-3-butenyl, 1,1,2-Trimethyl-2-propenyl, 1-Ethyl-1-methyl-2-propenyl, 1-Ethyl-2-methyl-1-propenyl und 1-Ethyl-2-methyl-2-propenyl;
**Alkinyl:** geradkettige oder verzweigte Kohlenwasserstoffgruppen mit 2 bis 8 Kohlenstoffatomen und einer Dreifachbindung in einer beliebigen Position, z.B. (aber nicht beschränkt auf) C₂-C₆-Alkinyl wie Ethinyl, 1-Propinyl, 2-Propinyl, 1-Butinyl, 2-Butinyl, 3-Butinyl, 1-Methyl-2-propinyl, 1-Pentinyl, 2-Pentinyl, 3-Pentinyl, 4-Pentinyl, 1-Methyl-2-butinyl, 1-Methyl-3-butinyl, 2-Methyl-3-butinyl, 3-Methyl-1-butinyl, 1,1-Dimethyl-2-propinyl, 1-Ethyl-2-propinyl, 1-Hexinyl, 2-Hexinyl, 3-Hexinyl, 4-Hexinyl, 5-Hexinyl, 1-Methyl-2-pentinyl, 1-Methyl-3-pentinyl, 1-Methyl-4-pentinyl, 2-Methyl-3-pentinyl, 2-Methyl-4-pentinyl, 3-Methyl-1-pentinyl, 3-Methyl-4-pentinyl, 4-Methyl-1-pentinyl, 4-Methyl-2-pentinyl, 1,1-Dimethyl-2-butinyl, 1,1-Dimethyl-3-butinyl, 1,2-Dimethyl-3-butinyl, 2,2-Dimethyl-3-butinyl, 3,3-Dimethyl-1-butinyl, 1-Ethyl-2-butinyl, 1-Ethyl-3-butinyl, 2-Ethyl-3-butinyl und 1-Ethyl-1-methyl-2-propinyl;
**Alkoxy:** gesättigte, geradkettige oder verzweigte Alkoxyreste mit 1 bis 8 Kohlenstoffatomen, z.B. (aber nicht beschränkt auf) C₁-C₆-Alkoxy wie Methoxy, Ethoxy, Propoxy, 1-Methylethoxy, Butoxy, 1-Methyl-propoxy, 2-Methylpropoxy, 1,1-Dimethylethoxy, Pentoxy, 1-Methylbutoxy, 2-Methylbutoxy, 3-Methylbutoxy, 2,2-Di-methylpropoxy, 1-Ethylpropoxy, Hexoxy, 1,1-Dimethylpropoxy, 1,2-Dimethylpropoxy,1-Methylpentoxy, 2-Methylpentoxy, 3-Methylpentoxy, 4-Methylpentoxy, 1,1-Dimethylbutoxy, 1,2-Dimethylbutoxy, 1,3-Dimethylbutoxy, 2,2-Dimethylbutoxy, 2,3-Dimethylbutoxy, 3,3-Dimethylbutoxy, 1-Ethylbutoxy, 2-Ethylbutoxy, 1,1,2-Trimethylpropoxy, 1,2,2-Trimethylpropoxy, 1-Ethyl-1-methylpropoxy und 1-Ethyl-2-methylpropoxy;
**Alkylthio:** gesättigte, geradkettige oder verzweigte Alkylthioreste mit 1 bis 8 Kohlenstoffatomen, z.B. (aber nicht beschränkt auf) C₁-C₆-Alkylthio wie Methylthio, Ethylthio, Propylthio, 1-Methylethylthio, Butylthio, 1-Methyl-propylthio, 2-Methylpropylthio, 1,1-Dimethylethylthio, Pentylthio, 1-Methylbutylthio, 2-Methylbutylthio, 3-Methylbutylthio, 2,2-Di-methylpropylthio, 1-Ethylpropylthio, Hexylthio, 1,1-Dimethylpropylthio, 1,2-Dimethylpropylthio,1-Methylpentylthio, 2-Methylpentylthio, 3-Methyl-pentylthio, 4-Methylpentylthio, 1,1-Dimethylbutylthio, 1,2-Dimethylbutylthio, 1,3-Dimethyl-butylthio, 2,2-Dimethylbutylthio, 2,3-Dimethylbutylthio, 3,3-Dimethylbutylthio, 1-Ethylbutylthio, 2-Ethylbutylthio, 1,1,2-Trimethylpropylthio, 1,2,2-Trimethylpropylthio, 1-Ethyl-1-methylpropyl-thio und 1-Ethyl-2-methylpropylthio;
**Alkoxycarbonyl:** eine Alkoxygruppe mit 1 bis 6 Kohlenstoffatomen (wie vorstehend genannt), welche über eine Carbonylgruppe (-CO-) an das Gerüst gebunden ist;
**Alkylsulfinyl:** gesättigte, geradkettige oder verzweigte Alkylsulfinylreste mit 1 bis 8 Kohlenstoffatomen, z.B. (aber nicht beschränkt auf) C₁-C₆-Alkylsulfinyl wie Methylsulfinyl, Ethylsulfinyl, Propylsulfinyl, 1-Methylethylsulfinyl, Butylsulfinyl, 1-Methyl-propylsulfinyl, 2-Methylpropylsulfinyl, 1,1-Dimethylethylsulfinyl, Pentylsulfinyl, 1-Methylbutylsulfinyl, 2-Methylbutylsulfinyl, 3-Methylbutylsulfinyl, 2,2-Di-methylpropylsulfinyl, 1-Ethylpropylsulfinyl, Hexylsulfinyl, 1,1-Dimethylpropylsulfinyl, 1,2-Dimethylpropylsulfinyl,1-Methylpentylsulfinyl, 2-Methylpentyl-sulfinyl, 3-Methylpentylsulfinyl, 4-Methylpentylsulfinyl, 1,1-Dimethylbutylsulfinyl, 1,2-Dimethylbutylsulfinyl, 1,3-Dimethylbutylsulfinyl, 2,2-Dimethylbutylsulfinyl, 2,3-Dimethylbutylsulfinyl, 3,3-Dimethylbutylsulfinyl, 1-Ethylbutylsulfinyl, 2-Ethylbutylsulfinyl, 1,1,2-Trimethylpropylsulfinyl, 1,2,2-Trimethylpropylsulfinyl, 1-Ethyl-1-methylpropylsulfinyl und 1-Ethyl-2-methylpropylsulfinyl;
**Alkylsulfonyl:** gesättigte, geradkettige oder verzweigte Alkylsulfonylreste mit 1 bis 8 Kohlenstoffatomen, z.B. (aber nicht beschränkt auf) C₁-C₆-Alkylsulfonyl wie Methylsulfonyl, Ethylsulfonyl, Propylsulfonyl, 1-Methylethylsulfonyl, Butylsulfonyl, 1-Methyl-propylsulfonyl, 2-Methylpropylsulfonyl, 1,1-Dimethylethylsulfonyl, Pentylsulfonyl, 1-Methylbutylsulfonyl, 2-Methylbutylsulfonyl, 3-Methylbutylsulfonyl, 2,2-Di-methylpropylsulfonyl, 1-Ethylpropylsulfonyl, Hexylsulfonyl, 1,1-Dimethylpropylsulfonyl, 1,2-Dimethylpropylsulfonyl,1-Methylpentylsulfonyl, 2-Methylpentyl-sulfonyl, 3-Methylpentylsulfonyl, 4-Methylpentylsulfonyl, 1,1-Dimethylbutylsulfonyl, 1,2-Dimethylbutylsulfonyl, 1,3-Dimethylbutylsulfonyl, 2,2-Dimethylbutylsulfonyl, 2,3-Dimethylbutylsulfonyl, 3,3-Dimethylbutylsulfonyl, 1-Ethylbutylsulfonyl, 2-Ethylbutylsulfonyl, 1,1,2-Trimethylpropylsulfonyl, 1,2,2-Trimethylpropylsulfonyl, 1-Ethyl-1-methylpropylsulfonyl und 1-Ethyl-2-methylpropylsulfonyl;
**Cycloalkyl:** monocyclische, gesättigte Kohlenwasserstoffgruppen mit 3 bis 10 Kohlenstoffringgliedern, z.B. (aber nicht beschränkt auf) Cyclopropyl, Cyclopentyl und Cyclohexyl;
**Halogenalkyl:** geradkettige oder verzweigte Alkylgruppen mit 1 bis 8 Kohlenstoffatomen (wie vorstehend genannt), wobei in diesen Gruppen teilweise oder vollständig die Wasserstoffatome durch Halogenatome wie vorstehend genannt ersetzt sein können, z.B. (aber nicht beschränkt auf) C₁-C₃-Halogenalkyl wie Chlormethyl, Brommethyl, Dichlormethyl, Trichlormethyl, Fluormethyl, Difluormethyl, Trifluormethyl, Chlorfluormethyl, Dichlorfluormethyl, Chlordifluormethyl, 1-Chlorethyl, 1-Bromethyl, 1-Fluorethyl, 2-Fluorethyl, 2,2-Difluorethyl, 2,2,2-Trifluorethyl, 2-Chlor-2-fluorethyl, 2-Chlor,2-difluorethyl, 2,2-Dichlor-2-fluorethyl, 2,2,2-Trichlorethyl, Pentafluorethyl und 1,1,1-Trifluorprop-2-yl;
**Halogenalkoxy:** geradkettige oder verzweigte Alkoxygruppen mit 1 bis 8 Kohlenstoffatomen (wie vorstehend genannt), wobei in diesen Gruppen teilweise oder vollständig die Wasserstoffatome durch Halogenatome wie vorstehend genannt ersetzt sein können, z.B. (aber nicht beschränkt auf) C₁-C₃-Halogenalkoxy wie Chlormethoxy, Brommethoxy, Dichlormethoxy, Trichlormethoxy, Fluormethoxy, Difluormethoxy, Trifluormethoxy, Chlorfluormethoxy, Dichlorfluormethoxy, Chlordifluormethoxy, 1-Chlorethoxy, 1-Bromethoxy, 1-Fluorethoxy, 2-Fluorethoxy, 2,2-Difluorethoxy, 2,2,2-Trifluorethoxy, 2-Chlor-2-fluorethoxy, 2-Chlor,2-difluorethoxy, 2,2-Dichlor-2-fluorethoxy, 2,2,2-Trichlorethoxy, Pentafluor-ethoxy und 1,1,1-Trifluorprop-2-oxy;
**Halogenalkylthio:** geradkettige oder verzweigte Alkylthiogruppen mit 1 bis 8 Kohlenstoffatomen (wie vorstehend genannt), wobei in diesen Gruppen teilweise oder vollständig die Wasserstoffatome durch Halogenatome wie vorstehend genannt ersetzt sein können, z.B. (aber nicht beschränkt auf) C₁-C₃-Halogenalkylthio wie Chlormethylthio, Brommethylthio, Dichlormethylthio, Trichlormethylthio, Fluormethylthio, Difluormethylthio, Trifluormethylthio, Chlorfluormethylthio, Dichlorfluor-methylthio, Chlordifluormethylthio, 1-Chlorethylthio, 1-Bromethylthio, 1-Fluorethylthio, 2-Fluorethylthio, 2,2-Difluorethylthio, 2,2,2-Trifluorethylthio, 2-Chlor-2-fluorethylthio, 2-Chlor,2-difluorethylthio, 2,2-Dichlor-2-fluorethylthio, 2,2,2-Trichlorethylthio, Pentafluorethylthio und 1,1,1-Trifluorprop-2-ylthio;
**Heteroaryl:** 5 oder 6-gliedriges, vollständig ungesättigtes monocyclisches Ringsystem, enthaltend ein bis vier Heteroatome aus der Gruppe Sauerstoff, Stickstoff oder Schwefel, enthält der Ring mehrere Sauerstoffatome, so stehen diese nicht direkt benachbart;
**5-gliedriges Heteroaryl: enthaltend ein bis vier Stickstoffatome oder ein bis drei Stickstoff atome und ein Schwefel- oder Sauerstoffatom:** 5-Ring Heteroarylgruppen, welche neben Kohlenstoffatomen ein bis vier Stickstoffatome oder ein bis drei Stickstoffatome und ein Schwefel- oder Sauerstoffatom als Ringglieder enthalten können, z.B. (aber nicht beschränkt auf) 2-Furyl, 3-Furyl, 2-Thienyl, 3-Thienyl, 2-Pyrrolyl, 3-Pyrrolyl, 3-Isoxazolyl, 4-Isoxazolyl, 5-Isoxazolyl, 3-Isothiazolyl, 4-Isothiazolyl, 5-Isothiazolyl, 3-Pyrazolyl, 4-Pyrazolyl, 5-Pyrazolyl, 2-Oxazolyl, 4-Oxazolyl, 5- Oxazolyl, 2-Thiazolyl, 4-Thiazolyl, 5-Thiazolyl, 2-Imidazolyl, 4-Imidazolyl, 1,2,4-Oxadiazol-3-yl, 1,2,4-Oxadiazol-5-yl, 1,2,4-Thiadiazol-3-yl, 1,2,4-Thiadiazol-5-yl, 1,2,4-Triazol-3-yl, 1,3,4-Oxadiazol-2-yl, 1,3,4-Thiadiazol-2-yl und 1,3,4-Triazol-2-yl;
**über Stickstoff gebundenes 5-gliedriges Heteroaryl, enthaltend ein bis vier Stickstoffatome, oder über Stickstoff gebundenes benzokondensiertes 5-gliederiges Heteroaryl, enthaltend ein bis drei Stickstoffatome:** 5-Ring Heteroarylgruppen, welche neben Kohlenstoffatomen ein bis vier Stickstoffatome bzw. ein bis drei Stickstoffatome als Ringglieder enthalten können, und in welchen zwei benachbarte Kohlenstoffringglieder oder ein Stickstoff- und ein benachbartes Kohlenstoffringglied durch eine Buta-1,3-dien-1,4-diylgruppe verbrückt sein können, in der ein oder zwei C-Atome durch N-Atome ersetzt sein können, in der ein oder zwei C-Atome durch N-Atome ersetzt sein können, wobei diese Ringe über eines der Stickstoffringglieder an das Gerüst gebunden sind, z.B. (aber nicht beschränkt auf) 1-Pyrrolyl, 1-Pyrazolyl, 1,2,4-Triazol-1-yl, 1-Imidazolyl, 1,2,3-Triazol-1-yl und 1,3,4-Triazol-1-yl;
**6-gliedriges Heteroaryl, enthaltend ein bis vier Stickstoffatome:** 6-Ring Heteroarylgruppen, welche neben Kohlenstoffatomen ein bis drei bzw. ein bis vier Stickstoffatome als Ringglieder enthalten können, beispielsweise (aber nicht beschränkt auf) 2-Pyridinyl, 3-Pyridinyl, 4-Pyridinyl, 3-Pyridazinyl, 4-Pyridazinyl, 2-Pyrimidinyl, 4-Pyrimidinyl, 5-Pyrimidinyl, 2-Pyrazinyl, 1,3,5-Triazin-2-yl, 1,2,4-Triazin-3-yl und 1,2,4,5-Tetrazin-3-yl;
**Benzokondensiertes 5-gliedriges Heteroaryl, enthaltend ein bis drei Stickstoffatome oder ein Stickstoffatom und ein Sauerstoff- oder Schwefelatom:** z.B. (aber nicht beschränkt auf) Indol-1-yl, Indol-2-yl, Indol-3-yl, Indol-4-yl, Indol-5-yl, Indol-6-yl, Indol-7-yl, Benzimidazol-1-yl, Benzimidazol-2-yl, Benzimidazol-4-yl, Benzimidazol-5-yl, Indazol-1-yl, Indazol-3-yl, Indazol-4-yl, Indazol-5-yl, Indazol-6-yl, Indazol-7-yl, Indazol-2-yl, 1-Benzofuran-2-yl, 1-Benzofuran-3-yl, 1-Benzofuran-4-yl, 1-Benzofuran-5-yl, 1-Benzofuran-6-yl, 1-Benzofuran-7-yl, 1-Benzothiophen-2-yl, 1-Benzothio-phen-3-yl, 1-Benzothiophen-4-yl, 1-Benzothiophen-5-yl, 1-Benzothiophen-6-yl, 1-Benzothiophen-7-yl, 1,3-Benzothiazol-2-yl, 1,3-Benzothiazol-4-yl, 1,3-Benzothiazol-5-yl, 1,3-Benzothiazol-6-yl, 1,3-Benzothiazol-7-yl, 1,3-Benzoxazol-2-yl, 1,3-Benzoxazol-4-yl, 1,3-Benzoxazol-5-yl, 1,3-Benzoxazol-6-yl und 1,3-Benzoxazol-7-yl,
**Benzokondensiertes 6-gliedriges Heteroaryl, enthaltend ein bis drei Stickstoffatome: :** z.B. (aber nicht beschränkt auf) Chinolin-2-yl, Chinolin-3-yl, Chinolin-4-yl, Chinolin-5-yl, Chinolin-6-yl, Chinolin-7-yl, Chinolin-8-yl, Isochinolin-1-yl, Isochinolin-3-yl, Isochinolin-4-yl, Isochinolin-5-yl, Isochinolin-6-yl, Isochinolin-7-yl, und Isochinolin-8-yl;
**Heterocyclyl:** drei- bis fünfzehngliedriger gesättigter oder partiell ungesättigter Heterocyclus, enthaltend ein bis vier Heteroatome aus der Gruppe Sauerstoff, Stickstoff oder Schwefel: mono-, bi- oder tricyclische Heterocyclen enthaltend neben Kohlenstoffringgliedern ein bis drei Stickstoffatome und/oder ein Sauerstoff- oder Schwefelatom oder ein oder zwei Sauerstoff- und/oder Schwefelatome; enthält der Ring mehrere Sauerstoffatome, so stehen diese nicht direkt benachbart; wie z.B. (aber nicht beschränkt auf) Oxiranyl, Aziridinyl, 2-Tetrahydrofuranyl, 3-Tetrahydrofuranyl, 2-Tetrahydrothienyl, 3-Tetrahydrothienyl, 2-Pyrrolidinyl, 3-Pyrrolidinyl, 3-Isoxazolidinyl, 4-Isoxazolidinyl, 5-Isoxazolidinyl, 3-Isothiazolidinyl, 4-Isothiazolidinyl, 5-Isothiazolidinyl, 3-Pyrazolidinyl, 4-Pyrazolidinyl, 5-Pyrazolidinyl, 2-Oxazolidinyl, 4-Oxazolidinyl, 5-Oxazolidinyl, 2-Thiazolidinyl, 4-thiazolidinyl, 5-Thiazolidinyl, 2-Imidazolidinyl, 4-Imidazolidinyl, 1,2,4-Oxadiazolidin-3-yl, 1,2,4-Oxadiazolidin-5-yl, 1,2,4-Thiadiazolidin-3-yl, 1,2,4-Thiadiazolidin-5-yl, 1,2,4-Triazolidin-3-yl, 1,3,4-Oxadiazolidin-2-yl, 1,3,4-Thiadiazolidin-2-yl, 1,3,4-Triazolidin-2-yl, 2,3-Dihydrofur-2-yl, 2,3-Dihydrofur-3-yl, 2,4-Dihydrofur-2-yl, 2,4-Dihydrofur-3-yl, 2,3-Dihydrothien-2-yl, 2,3-Dihydrothien-3-yl, 2,4-Dihydrothien-2-yl, 2,4-Dihydrothien-3-yl, 2-Pyrrolin-2-yl, 2-Pyrrolin-3-yl, 3-Pyrrolin-2-yl, 3-Pyrrolin-3-yl, 2-Isoxazolin-3-yl, 3-Isoxazolin-3-yl, 4-Isoxazolin-3-yl, 2-Isoxazolin-4-yl, 3-Isoxazolin-4-yl, 4-Isoxazolin-4-yl, 2-Isoxazolin-5-yl, 3-Isoxazolin-5-yl, 4-Isoxazolin-5-yl, 2-Isothiazolin-3-yl, 3-Isothiazolin-3-yl, 4-Isothiazolin-3-yl, 2-Isothiazolin-4-yl, 3-Isothiazolin-4-yl, 4-Isothiazolin-4-yl, 2-Isothiazolin-5-yl, 3-Isothiazolin-5-yl, 4-Isothiazolin-5-yl, 2,3-Dihydropyrazol-1-yl, 2,3-Dihydropyrazol-2-yl, 2,3-Dihydropyrazol-3-yl, 2,3-Dihydropyrazol-4-yl, 2,3-Dihydropyrazol-5-yl, 3,4-Dihydropyrazol-1-yl, 3,4-Dihydropyrazol-3-yl, 3,4-Dihydropyrazol-4-yl, 3,4-Dihydropyrazol-5-yl, 4,5-Dihydroopyrazol-1-yl, 4,5-Dihydropyrazol-3-yl, 4,5-Dihydropyrazol-4-yl, 4,5-Dihydropyrazol-5-yl, 2,3-Dihydrooxazol-2-yl, 2,3-Dihydrooxazol-3-yl, 2,3-Dihydrooxazol-4-yl, 2,3-Dihydrooxazol-5-yl, 3,4-Dihydrooxazol-2-yl, 3,4-Dihydrooxazol-3-yl, 3,4-Dihydrooxazol-4-yl, 3,4-Dihydrooxazol-5-yl, 3,4-Dihydrooxazol-2-yl, 3,4-Dihydrooxazol-3-yl, 3,4-Dihydrooxazol-4-yl, 2-Piperidinyl, 3-Piperidinyl, 4-Piperidinyl, 1,3-Dioxan-5-yl, 2-Tetrahydropyranyl, 4-Tetrahydropyranyl, 2-Tetrahydrothienyl, 3-Hexahydro-pyridazinyl, 4-Hexahydropyridazinyl, 2-Hexahydropyrimidinyl, 4-Hexahydropyrimidinyl, 5-Hexahydropyrimidinyl, 2-Piperazinyl, 1,3,5-Hexahydro-triazin-2-yl und 1,2,4-Hexahydrotriazin-3-yl;
Bei der Nennung von Kombinationen von mehreren Resten, wie zum Beispiel Cx-Cy-Alkylcarbonyl oder Cx-Cy-Alkoxyalkyl, bezeichnet der Ausdruck Cx-Cy jeweils die Summe aller Kohlenstoffatome, die jeweils in dem gesamten Fragment vorhanden sind. X und Y stehen dabei jeweils für eine ganze Zahl, wobei die Zahl Y größer ist als diejenige von X.
Nicht umfasst sind solche Kombinationen, die den Naturgesetzen widersprechen und die der Fachmann daher aufgrund seines Fachwissens ausgeschlossen hätte. Beispielsweise sind Ringstrukturen mit drei oder mehreren benachbarten O-Atomen ausgeschlossen.

### Erläuterung der Verfahren und Zwischenprodukte

Die Ketoheteroarylpiperidin und -piperazin Derivate der Formel (**I**) lassen sich auf unterschiedliche Weise herstellen. Im Folgenden sind die möglichen Verfahren schematisch dargestellt. Wenn nicht anders angegeben haben die angegebenen Reste die oben angegebenen Bedeutungen.

Die erfindungsgemäßen Verfahren zur Herstellung von Verbindungen der Formel (I) werden gegebenenfalls unter Verwendung eines oder mehrerer Reaktionshilfsmittel durchgeführt.

Als Reaktionshilfsmittel kommen gegebenenfalls anorganische oder organische Basen oder Säureakzeptoren in Betracht. Hierzu gehören vorzugsweise Alkalimetall- oder Erdalkalimetall- -acetate, -amide, -carbonate, -hydrogencarbonate, -hydride, -hydroxide oder -alkanolate, wie beispielsweise Natrium-, Kalium- oder Calcium-acetat, Lithium-, Natrium-, Kalium- oder Calcium-amid, Natrium-, Kalium- oder Calcium-carbonat, Natrium-, Kalium- oder Calcium-hydrogencarbonat, Lithium-, Natrium-, Kalium- oder Calcium-hydrid, Lithium-, Natrium-- Kalium- oder Calciumhydroxid, Natrium- oder Kalium- -methanolat, -ethanolat, -n- oder -i-propanolat, -n-, -i-, -s- oder - tButanolat; weiterhin auch basische organische Stickstoffverbindungen, wie beispielsweise Trimethylamin, Triethylamin, Tripropylamin, Tributylamin, Ethyl-diisopropylamin, N,N-Dimethylcyclohexylamin, Dicyclohexylamin, Ethyl-dicyclohexylamin, N,N-Dimethyl-anilin, N,N-Dimethyl-benzylamin, Pyridin, 2-Methyl-, 3-Methyl-, 4-Methyl-, 2,4-Dimethyl-, 2,6-Dimethyl-, 3,4-Dimethyl-und 3,5-Dimethyl-pyridin, 5-Ethyl-2-methyl-pyridin, 4-Dimethylamino-pyridin, N-Methyl-piperidin, 1,4-Diazabicyclo[2,2,2]-octan (DABCO), 1,5-Diazabicyclo[4,3,0]-non-5-en (DBN), oder 1,8 Diazabicyclo[5,4,0]-undec-7-en (DBU).

Die erfindungsgemäßen Verfahren werden gegebenenfalls unter Verwendung eines oder mehrerer Verdünnungsmittel durchgeführt. Als Verdünnungsmittel kommen praktisch alle inerten organischen Lösungsmittel in Frage. Hierzu gehören vorzugsweise aliphatische und aromatische, gegebenenfalls halogenierte Kohlenwasserstoffe wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether wie Diethyl-und Dibutylether, Glykoldimethylether und Diglykoldimethylether, Tetrahydrofuran und Dioxan, Ketone wie Aceton, Methylethyl-, Methyl-isopropyl-oder Methyl-isobutyl-keton, Ester wie Essigsäuremethylester oder - ethylester, Nitrile wie z.B. Acetonitril oder Propionitril, Amide wie z.B. Dimethylformamid, Dimethylacetamid und N-Methyl-pyrrolidon, sowie Dimethylsulfoxid, Tetramethylensulfon und Hexamethylphosphorsäuretriamid und DMPU.

Die Reaktionstemperaturen können bei den erfindungsgemäßen Verfahren in einem größeren Bereich variiert werden. Im Allgemeinen arbeitet man bei Temperaturen zwischen 0 °C und 250 °C, vorzugsweise bei Temperaturen zwischen 10 °C und 185 °C.

Die erfindungsgemäßen Verfahren werden im Allgemeinen unter Normaldruck durchgeführt. Es ist jedoch auch möglich, unter erhöhtem oder vermindertem Druck zu arbeiten.

Zur Durchführung der erfindungsgemäßen Verfahren werden die jeweils benötigten Ausgangsstoffe im Allgemeinen in angenähert äquimolaren Mengen eingesetzt. Es ist jedoch auch möglich, eine der jeweils eingesetzten Komponenten in einem größeren Überschuss zu verwenden.

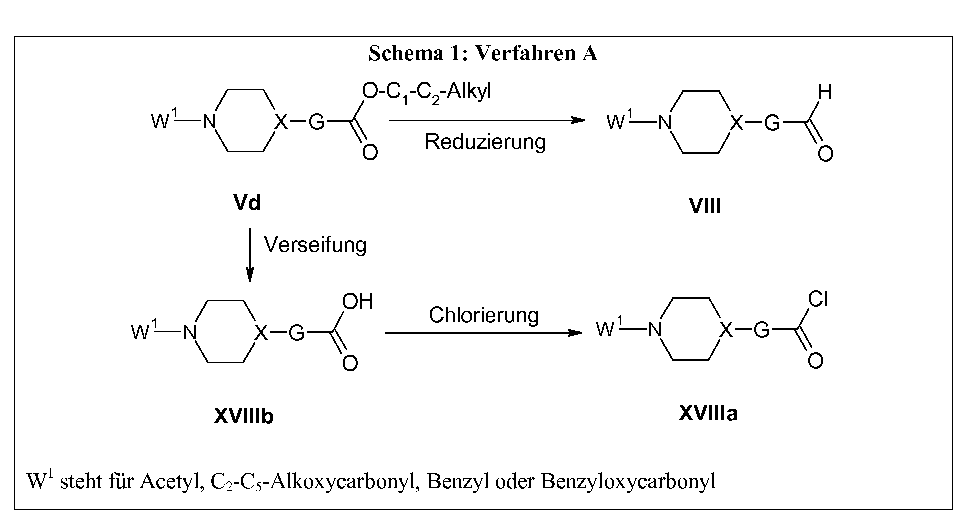

Eine Möglichkeit, Verbindungen den Formeln **(VIII), (XVIIIb)** und **(XVIIIa)** aus entsprechenden Verbindungen **(Vd)** herzustellen, ist in Schema 1 gezeigt.

Die Verbindungen der Fomeln **Vd** mit können aus kommerziell erhältlichen Vorstufen (s. z.B. **Abbildung 1)** nach literaturbeschriebenen Vorschriften hergestellt werden (s. z.B. Comprehensive Heterocyclic Chemistry, Vol. 4-6, A. R. Katritzky and C. W. Rees editors, Pergamon Press, New York, 1984; Comprehensive Heterocyclic Chemistry II, Vol. 2-4, A. R. Katritzky, C. R. Rees, and E. F. Scruveb editors, Pergamon Press, New York, 1996; und die Serie, The Chemistry of Heterocyclic Compounds, E. C. Taylor, editor, Wiley, New York; WO 2008/064474; WO 2008/006794; WO 2006/133216; US 5,234,033 A; WO 2007/039177; WO 2007/014290; Org. Biomol. Chem., 2008, 1904.)

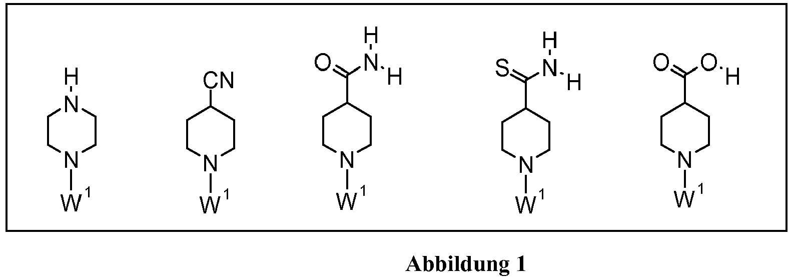

Der Aldehyd der Formel (**VIII**) kann aus C₁-C₂-Alkylestern der Formel (**Vd**) durch Reduziergung mit Reduktionsmittel (z.B. Diisobutylaluminiumhydrid) hergestellt werden. Bevorzugt wird die Reaktion in Tetrahydrofuran bei -78 °C, und zwar unter Inertatmosphäre (s. z.B. J. Med. Chem., 2001, 2319) durchgeführt.

Die Carbonsäure der Formel (**XVIIIb**) kann durch Verseifung des entsprechenden C₁-C₂-Alkylesters der Formel (**Vd**) dargestellt werden (vgl. z.B. WO 2007/014290).

Die Verbindungen der allgemeinen Formel (**XVIIIa**) werden aus den entsprechenden Säuren der Formel (**XVIIIb**) durch Chlorierung unter Verwendung literaturbekannter Prozesse dargestellt. (z.B. Tetrahedron 2005, 61, 10827-10852, und darin zitierte Literatur).

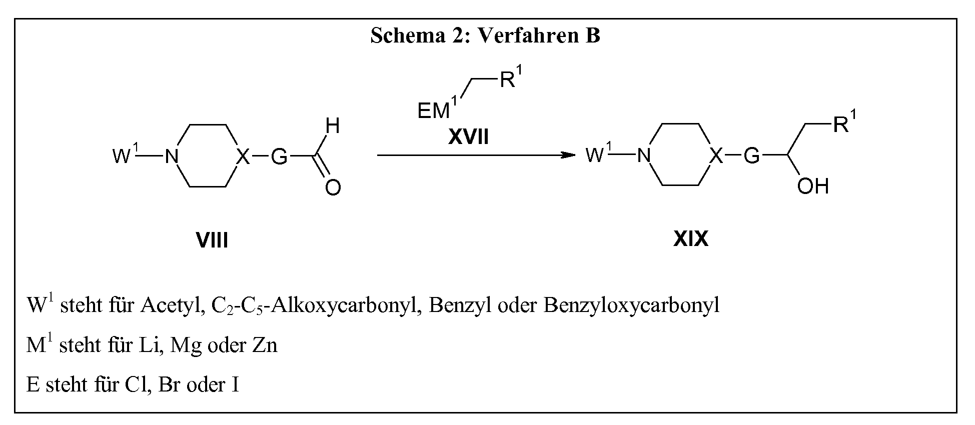

Eine Möglichkeit, Verbindungen der Formel (**XIX**) aus entsprechenden Verbindungen **(VIII)** mit einem metallorganischen Reagenz **(XVII)** herzustellen, ist in Schema 2 gezeigt.

Die Verbindungen der Formel (**XIX**) werden aus dem Aldehyd der Formel **(VIII)** durch Addition eines metallorganischen Reagenzes R¹-CH₂M¹E **(XVII,** M¹ = Mg, Li oder Zn, E = Cl, Br, oder I) hergestellt. Bevorzugt wird die Reaktion in Tetrahydrofuran oder Diethylether bei -78 °C - 35 °C durchgeführt. Besonders bevorzugt wird die Reaktion in Tetrahydrofuran bei -78 °C, und zwar unter Inertatmosphäre (s. z. B. WO 2007/039177, WO 2006/066109) durchgeführt. Bei Organozink Verbindungen werdern bevorzugt Lewis-Säuren verwendet (z.B. BF₃-Et₂O).

Das metallorganische Reagenz **(XVII)** ist kommerziell verfügbar oder kann aus kommerziell erhältlichen Vorstufen nach literaturbeschriebenen Vorschriften hergestellt werden (siehe beispielsweise *"*Handbook of Functionalized Organometallics Vol. 1 and 2", Ed. P. Knochel, Weinheim, Wiley-VCH, 2005, und darin zitierte Referenzen).

Die Reaktionszeit variiert in Abhängigkeit vom Maßstab der Reaktion und der Reaktionstemperatur, aber liegt im Allgemeinen zwischen einigen Minuten und 48 Stunden.Nach Beendigung der Reaktion, wird Verbindung (**XIX**) von der Reaktionsmischung durch eine der üblichen Trenntechniken getrennt. Falls notwendig werden die Verbindungen durch Umkristallisation, Destillation oder Chromatographie gereinigt oder können gegebenenfalls auch im nächsten Schritt ohne vorhergehende Reinigung eingesetzt werden.

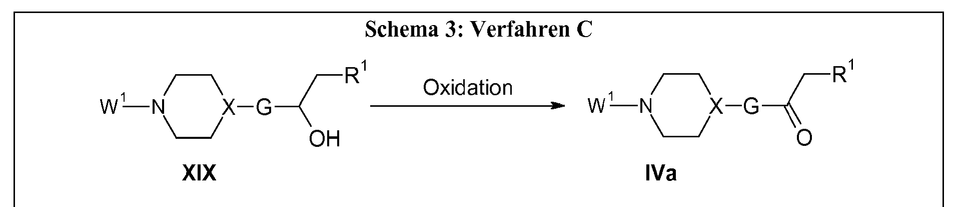

Eine Möglichkeit, Verbindungen der Formel (**IVa**) aus Verbindungen (**XIX**) herzustellen, ist in Schema 3 gezeigt.

Verbindungen der Formel (**IVa**) werden durch Oxidation des Alkohols (**XIX**) hergestellt. In der Literatur finden sich zahlreiche Methoden für die Darstellung von Ketonen aus sekundären Alkoholen (s. z.B. "Oxidation of Alcohols to Aldehydes and Ketones", Gabriel Tojo, Marcos Fernández, Springer Berlin, 2006, Seite 1-97, und darin zitierte Literatur). Bevorzugt wird die Oxidation unter Swern Bedingungen oder mit Dess-Martin Periodinan durchgeführt (siehe z.B. J. Am. Chem. Soc. 1991, 113, 7277).

Es kommen alle Lösungsmittel infrage, die selbst nicht vom Oxidationsmittel oxidiert werden, wie beispielsweise Dichlormethan, Chloroform oder Acetonitril, gegebenenfalls in Gegenwart eines Reaktionshilfsmittels, beispielsweise einer Säure (z.B. Schwefelsaüre oder Salzsäure) oder auch Base (z.B Triethylamin oder Pyridin).

Die Reaktion wird vorzugsweise bei Raumtemperatur durchgeführt. Die Reaktionszeit variiert in Abhängigkeit vom Maßstab der Reaktion und der Reaktionstemperatur, liegt aber im Allgemeinen zwischen einigen Minuten und 48 Stunden.

Nach Beendigung der Reaktion, wird Verbindung (**IVa**) von der Reaktionsmischung durch eine der üblichen Trenntechniken getrennt. Falls notwendig werden die Verbindungen durch Umkristallisation, Destillation oder Chromatographie gereinigt oder können gegebenenfalls auch im nächsten Schritt ohne vorhergehende Reinigung eingesetzt werden.

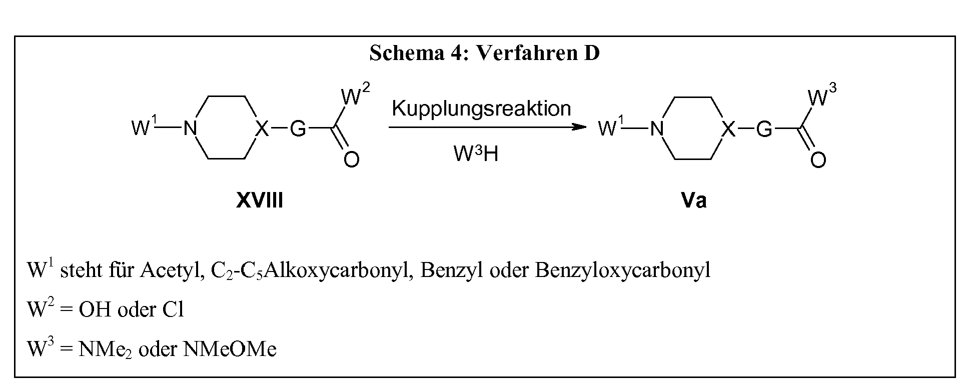

Eine Möglichkeit, Verbindungen der Formel **(Va)** aus entsprechenden Verbindungen **(XVIII)** erzustellen, ist in Schema 4 gezeigt.

Eine Verbindung mit der allgemeinen Formel **(Va)** wird durch eine Kupplungsreaktion einer Verbindung mit der allgemeinen Formel (**XVIIIa**) (wobei W² für Chlor steht) und mit einer der folgenden Verbindungen dargestellt: N,O-Dimethylhydroxamin, N,O-Dimethylhydroxamin-HCl Salz, Dialkylamin oder Dialkylamin-HCl Salz gegebenenfalls in der Gegenwart eines Säurefängers / Base (s. z.B. WO 2008/013622, WO 2008/013925 und WO 2006/018188).

Wenigstens ein Äquivalent eines Säurefängers / einer Base (z.B. Hünig Base, Triethylamin oder kommerziell erhältliche polymere Säurefänger) wird im Verhältnis zum Startmaterial der allgemeinen Formel (**XVIIIa**) verwendet. Ist das Startmaterial ein Salz, werden wenigstens zwei Äquivalente des Säurefängers benötigt.

Die Reaktion wird normalerweise bei Temperaturen von 0 °C bis 100 °C durchgeführt und vorzugsweise bei 20 °C bis 30 °C, aber sie kann auch bei Rückflusstemperatur der Reaktionsmischung durchgeführt werden. Die Reaktionszeit variiert in Abhängigkeit vom Maßstab der Reaktion und der Reaktionstemperatur, aber liegt im Allgemeinen zwischen einigen Minuten und 48 Stunden.

Alternativ kann eine Verbindung der Formel **(Va),** auch aus der entsprechenden Verbindung der Formel (**XVIIIb**) (wobei W² für Hydroxy steht) mit N,O-Dimethylhydroxamin, N,O-Dimethylhydroxamin-HCl Salz, Dialkylamin oder Dialkylamin-HCl Salz in Gegenwart eines Kupplungsreagenzes synthetisiert werden analog zu in der Literatur beschriebenen Vorschriften (z.B. Tetrahedron, 2005, 61, 10827, und darin zitierte Referenzen).

Geeignete Kupplungsreagenzien sind beispielsweise Peptidkupplungsreagenzien (zum Beispiel, N-(3-Dimethylaminopropyl)-N'-ethyl-carbodiimid gemischt mit 4-Dimethylamino-pyridin, N-(3-Dimethylaminopropyl)-N'-ethyl-carbodiimid gemischt mit 1-Hydroxy-benzotriazol, Bromtripyrrolidinophosphonium-hexafluorophosphat, O-(7-Azabenzotriazol-1-yl)-N,N,N',N'-tetramethyluroniumhexafluorophosphat, etc).

Gegebenenfalls kann eine Base, wie z.B. Triethylamin oder Hünig-Base in der Reaktion verwendet werden.

Die Reaktion wird bevorzugt bei Temperaturen von 0 °C bis 100 °C durchgeführt und besonders bevorzugt bei 0 °C bis 30 °C. Die Reaktionszeit variiert in Abhängigkeit vom Maßstab der Reaktion und der Re-aktionstemperatur, aber liegt im Allgemeinen zwischen einigen Minuten und 48 Stunden.

Das erfindungsgemäße Verfahren D wird vorzugsweise unter Verwendung eines oder mehrerer Verdünnungsmittel durchgeführt. Nach Beendigung der Reaktion, werden die Verbindungen (Va) von der Reaktionsmischung durch eine der üblichen Trenntechniken getrennt. Falls notwendig werden die Verbindungen durch Umkristallisation, Destillation oder Chromatographie gereinigt oder können gegebenenfalls auch im nächsten Schritt ohne vorhergehende Reinigung eingesetzt werden.

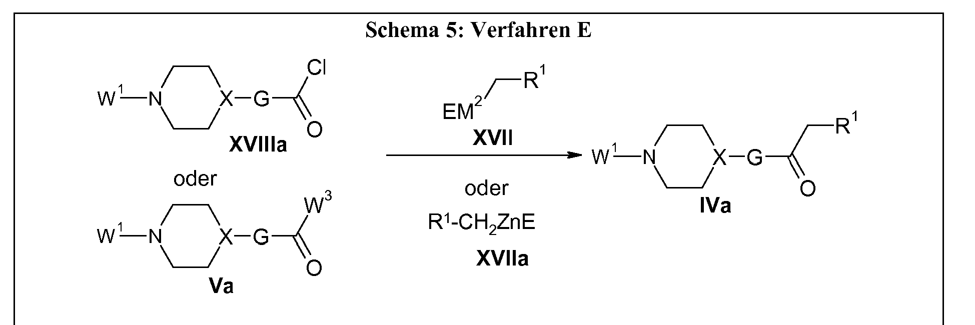

Eine Möglichkeit, Verbindungen der Formel (**IVa**) aus entsprechenden Verbindungen (**Va**) oder (**XVIIIa**) mit einem metallorganischen Reagenz (**XVII** oder **XVIIa**) herzustellen, ist in Schema 5 gezeigt.

Die Verbindungen der Formel (**IVa**) werden aus dem Amid **(Va),** wobei W³ für NMeOMe oder NMe₂ steht, durch Addition eines metallorganischen Reagenzes, R¹-CH₂M²E (**XVII**, M² = Mg oder Li) hergestellt. Bevorzugt wird die Reaktion in Tetrahydrofuran bei -78°C durchgeführt, und zwar unter Inertatmosphäre (s. z.B. J. Med. Chem. 2009, 52, 1701).

**(Va)** und das metallorganische Reagenz (mit M² = Mg oder Li) werden in äquimolaren Mengen eingesetzt, gegebenenfalls kann das metallorganische Reagenz aber auch im Überschuss verwendet werden. Die Reaktion wird vorzugsweise bei Raumtemperatur durchgeführt. Die Reaktionszeit variiert in Abhängigkeit vom Maßstab der Reaktion und der Reaktionstemperatur, liegt aber im Allgemeinen zwischen einigen Minuten und 48 Stunden.

Alternativ werden die Verbindungen der Formel (**IVa**) aus dem Chlorid (**XVIIIa**) durch Addition eines Organozink Reagenzes (**XVIIa**), R¹-CH₂ZnE (E = Cl, Br, oder I), gegebenenfalls in der Gegenwart eines Palladium Katalysators, hergestellt. Bevorzugt wird die Reaktion in Gegenwart von Pd(PPh₃)₄ oder PdCl₂ in Tetrahydrofuran bei Raumtemperatur, unter Inertatmosphäre (s. z.B. WO 2007/070818; Org. Lett., 2008, 10, 1107) durchgeführt. Die verwendete Katalysatormenge liegt bei 0,1-90 mol% bezogen auf das Edukt, vorzugsweise werden 1-30 mol% des Katalysators bezogen auf das Edukt verwendet.

(**XVIIIa**) und die Organozinkverbindung werden in äquimolaren Mengen eingesetzt. Die Organozinkverbindung kann gegebenenfalls aber auch im Überschuss verwendet werden. Die Reaktionszeit variiert in Abhängigkeit vom Maßstab der Reaktion und der Reaktionstemperatur, aber liegt im Allgemeinen zwischen einigen Minuten und 48 Stunden.

Das erfindungsgemäße Verfahren E wird vorzugsweise unter Verwendung eines oder mehrerer Verdünnungsmittel durchgeführt. Nach Beendigung der Reaktion, wird Verbindung (**IVa**) von der Reaktionsmischung durch eine der üblichen Trenntechniken getrennt. Falls notwendig werden die Verbindungen durch Umkristallisation, Destillation oder Chromatographie gereinigt oder können gegebenenfalls auch im nächsten Schritt ohne vorhergehende Reinigung eingesetzt werden.

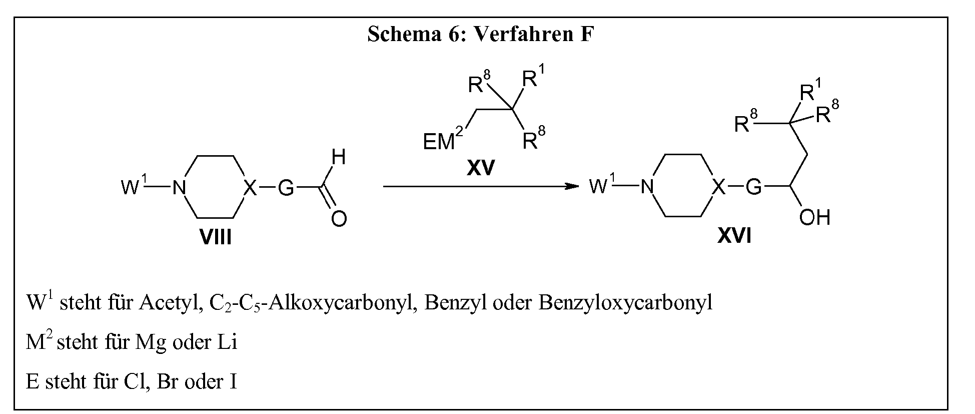

Eine Möglichkeit, Verbindungen der Formel (**XVI**) aus entsprechenden Verbindungen (**VIII**) mit einem metallorganischen Reagenz **(XV)** herzustellen, ist in Schema 6 gezeigt.

Verfahren F wird analog Verfahren B durchgeführt (s. oben).

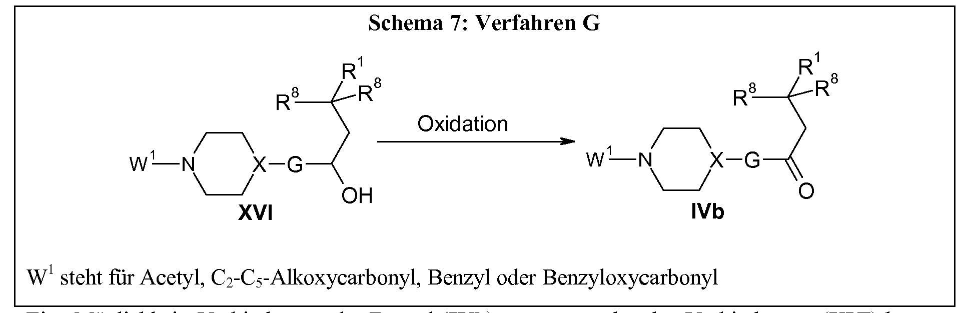

Eine Möglichkeit, Verbindungen der Formel (**IVb**) aus entsprechenden Verbindungen (**XVI**) herzustellen, ist in Schema 7 gezeigt.

Verfahren G wird analog Verfahren C durchgeführt (s. oben).

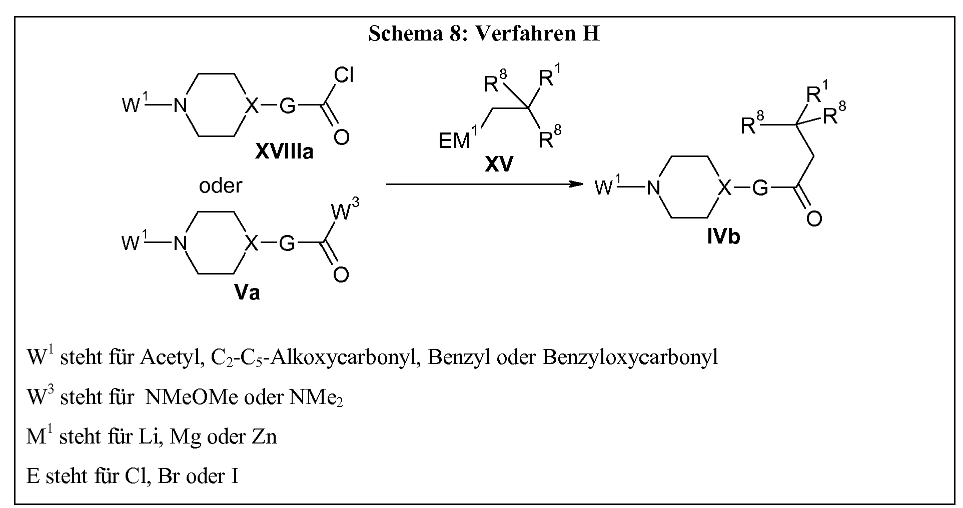

Eine Möglichkeit, Verbindungen der Formel (**IVb**) aus entsprechenden Verbindungen (**Va** bzw. **XVIIIa**) mit einem metallorganischen Reagenz (XV) herzustellen, ist in Schema 8 gezeigt.

Verfahren H wird analog Verfahren E durchgeführt (s. oben).

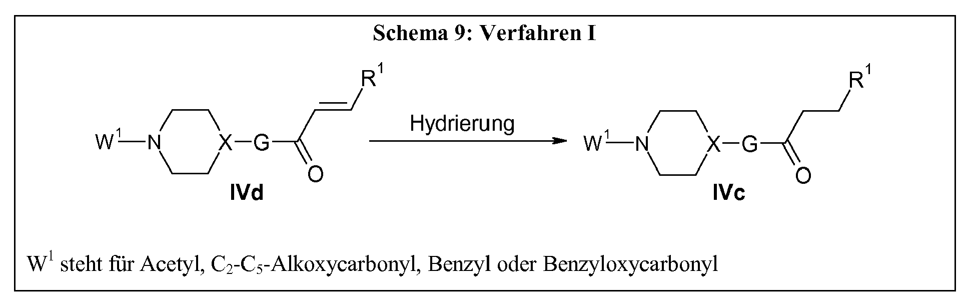

Eine Möglichkeit, das Zwischenprodukt (**IVc**) aus Verbindung (**IVd**) darzustellen, ist in Schema 9 gezeigt.

Eine Doppelbindung wird durch eine Hydrierung unter Verwendung eines geeigneten Katalysators in eine Einfachbindung umgewandelt. Der Katalysator für das Verfahren I wird aus den in der Literatur bekannten Katalysatoren zur Hydrierung ("Reductions in Organic Chemistry", Milos Hudlický, John Wiley & Sons, 1984**)** ausgewählt, wie z.B. Palladium auf Aktivkohle oder Pearlmans Katalysator (Pd(OH))₂ auf Aktivkohle).

Das erfindungsgemäße Verfahren I wird vorzugsweise unter Verwendung eines oder mehrerer Verdünnungsmittel durchgeführt.Bevorzugte Lösungsmittel sind *N,N-*Dimethylformamid, Ethylacetat und Ethanol.

Die verwendete Katalysatormenge liegt bei 0,1-90 mol% bezogen auf das Edukt, vorzugsweise werden 1-30 mol% des Katalysators bezogen auf das Edukt verwendet. Die Reaktion kann bei Überdruck (1-1000 bar) oder bevorzugt bei atmosphärischem Druck durchgeführt werden. Die Reaktion wird vorzugsweise bei Temperaturen von 0 °C - 150 °C durchgeführt und besonders bevorzugt bei Raumtemperatur. Die Reaktionszeit variiert in Abhängigkeit vom Maßstab der Reaktion und der Reaktionstemperatur, aber liegt im Allgemeinen zwischen einer halben Stunde und 72 Stunden.

Nach Beendigung der Reaktion, werden die Verbindungen **(IVc)** von der Reaktionsmischung durch eine der üblichen Trenntechniken getrennt. Falls notwendig werden die Verbindungen durch Umkristallisation, Destillation oder Chromatographie gereinigt oder können gegebenenfalls auch im nächsten Schritt ohne vorhergehende Reinigung eingesetzt werden.

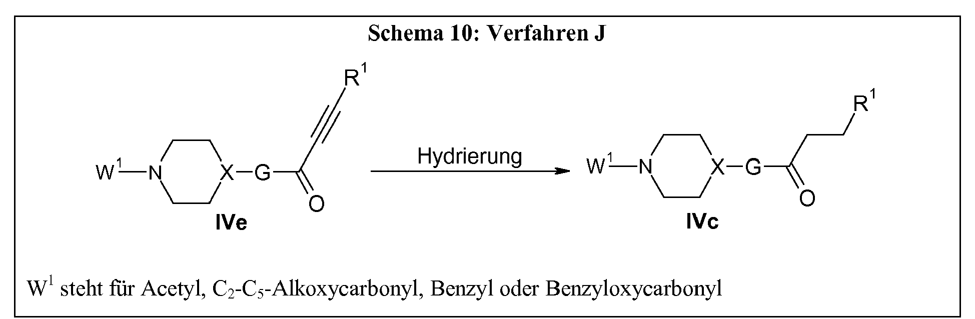

Eine Möglichkeit, Verbindungen der Formel (**IVc**) aus entsprechenden Verbindungen (**IVe**) herzustellen, ist in Schema 10 gezeigt.

Eine Dreifachbindung wird durch eine Hydrierung unter Verwendung eines geeigneten Katalysators in eine Einfachbindung umgewandelt

Es wird das gleiche Verfahren verwendet, dass bereits in Schema 9 (Verfahren I) beschrieben wurde.

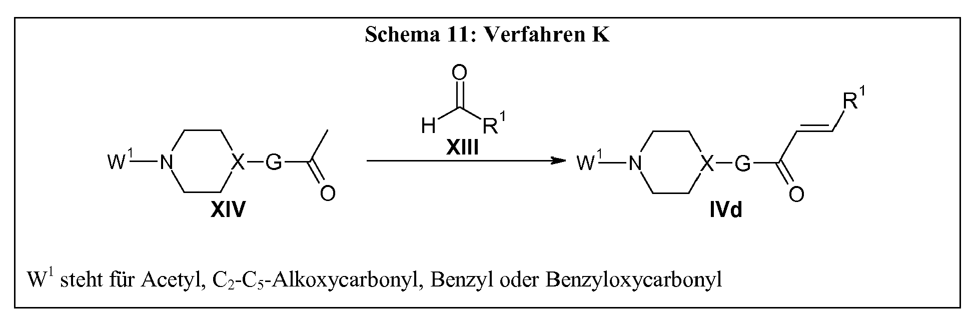

Eine Möglichkeit, Verbindungen der Formel (**IVd**) aus entsprechenden Verbindungen (**XIV**) mit einem Aldehyd (**XIII**) herzustellen, ist in Schema 11 gezeigt.

Verbindungen **(XIV)** können aus Verbindungen **(VIII)** synthetisiert werden, indem zunächst Verfahren B und dann Verfahren C auf Verbindungen der Formel (**XIII**) angewendet wird.

Verbindungen (**XIV**) können auch aus Verbindungen **(Va)** synthetisiert werden, indem Verfahren E auf Verbindungen der Formel **(Va)** angewendet wird.

Der Aldehyd **(XIII)** ist kommerziell verfügbar oder kann aus kommerziell erhältlichen Vorstufen nach literaturbeschriebenen Vorschriften hergestellt werden.

Eine Verbindung mit der allgemeinen Formel **(IVd)** kann durch eine Aldolkondensation einer Verbindung mit der entsprechenden allgemeinen Formel (**XIV**) mit einem Aldehyd **(XIII)** in der Gegenwart einer Säure oder Base synthetisiert werden (s. z.B. Synthetic Communications, 2009, 39, 2789; Organic Letters, 2009, 11, 3562; Pharmazie, 1988, 43, 82).

Geeignete Basen sind z.B. LiOH, NaOH oder KOH, z.B. in der Gegenwart von Wasser zusammen mit einem Cosolvens, bevorzugt THF, Toluen und/oder Methanol, um das Lösen des Esters zu vereinfachen. Das Startmaterial und die Base werden in equimolaren Mengen verwendet, aber die Base kann gegebenenfalls auch im Überschuss oder katalytisch verwendet werden.

Geeignete Säuren sind beispielsweise H₂SO₄ oder HCl, z.B. in der Gegenwart von Wasser zusammen mit einem Cosolvens, bevorzugt THF, Toluen und/oder Methanol, um das Lösen des Esters zu vereinfachen. Das Startmaterial und die Säuree werden in equimolaren Mengen verwendet, aber die Säure kann gegebenenfalls auch im Überschuss oder katalytisch verwendet werden.

(**XIV**) und **(XIII)** werden in äquimolaren Mengen eingesetzt. Der Aldehyd **(XIII)** kann gegebenenfalls aber auch im Überschuss verwendet werden. Die Reaktion wird vorzugsweise bei Temperaturen von 0 °C bis 100 °C durchgeführt und besonders bevorzugt bei Raumtemperatur. Die Reaktionszeit variiert in Abhängigkeit vom Maßstab der Reaktion und der Reaktionstemperatur, aber liegt im Allgemeinen zwischen einigen Minuten und 48 Stunden.

Das erfindungsgemäße Verfahren K wird vorzugsweise unter Verwendung eines oder mehrerer Verdünnungsmittel durchgeführt. Nach Beendigung der Reaktion wird Verbindung **(IVd)** von der Reaktionsmischung durch eine der üblichen Trenntechniken getrennt. Falls notwendig werden die Verbindungen durch Umkristallisation, Destillation oder Chromatographie gereinigt oder können gegebenenfalls auch im nächsten Schritt ohne vorhergehende Reinigung eingesetzt werden.

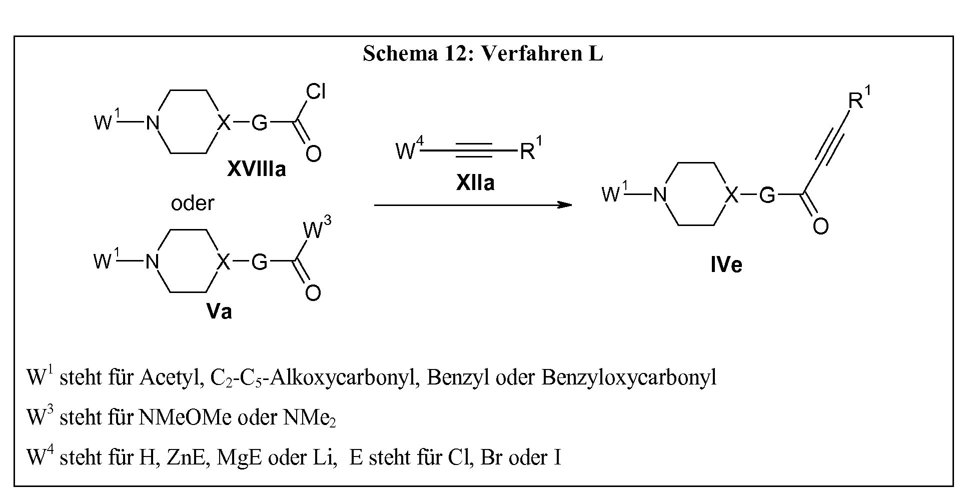

Eine Möglichkeit, Verbindungen der Formel (**IVe**) aus entsprechenden Verbindungen **(Va** oder **XVI-IIa)** mit einem Alkin der Formel **(XIIa)** herzustellen, ist in Schema 12 gezeigt.

Analog zu der zuvor beschriebenen Methode (Verfahren E) können mit einem metallorganischen Reagenz **(XIIa)**, wobei W⁴ für ZnE, MgE oder Li steht, die Verbindungen **(IVe)** aus Verbindungen **(Va)** synthetisiert werden.

Das metallorganische Reagenz **(XIIa)** ist kommerziell verfügbar oder kann aus kommerziell erhältlichen Vorstufen nach literaturbeschriebenen Vorschriften hergestellt werden (s. z.B. *"*Handbook of Functionalized Organometallics Vol. 1 and 2", Ed. P. Knochel, Weinheim, Wiley-VCH, 2005, und darin zitierte Referenzen).

Alternativ kann eine Verbindung der allgemeinen Formel **(IVe)** aus dem Edukt der Formel (**XVII-Ia)** durch eine Palladium-katalysierte Kreuzkupplung analog zu in der Literatur beschriebenen Vorschriften durchgeführt werden (siehe z.B. Synthesis, 2003, 2815). Bevorzugt wird **(XVIIIa)** zum Keton **(IVe)** durch Verwendung eines Alkins der Formel **(XIIa),** wobei W⁴ für H steht, in der Gegenwart eines Katalysators wie z.B. [(π-allyl)PdCl]₂, Pd(OAc)₂, PdCl₂(CH₃CN)₂, Pd(PPh₃)₄ oder PdCl₂(PPh₃)₂ sowie gegebenenfalls in Gegenwart weiterer Cokatalysatoren wie etwa CuI, Cs₂CO₃ und Triethylamin z.B. in einem Lösungsmittelgemisch von DMF oder THF bei 0-80 °C und zwar unter Inertatmosphäre umgesetzt. Die verwendete Katalysatormenge liegt bei 0,1-90 mol% bezogen auf das Edukt, vorzugsweise werden 0.5-30 mol% des Katalysators bezogen auf das Edukt verwendet.

Das Alkin **(XIIa)** ist kommerziell verfügbar oder kann aus kommerziell erhältlichen Vorstufen nach literaturbeschriebenen Vorschriften hergestellt werden.

Das erfindungsgemäße Verfahren L wird vorzugsweise unter Verwendung eines oder mehrerer Verdünnungsmittel durchgeführt. Nach Beendigung der Reaktion, wird Verbindung **(IVe)** von der Reaktionsmischung durch eine der üblichen Trenntechniken getrennt. Falls notwendig werden die Verbindungen durch Umkristallisation, Destillation oder Chromatographie gereinigt oder können gegebenenfalls auch im nächsten Schritt ohne vorhergehende Reinigung eingesetzt werden.

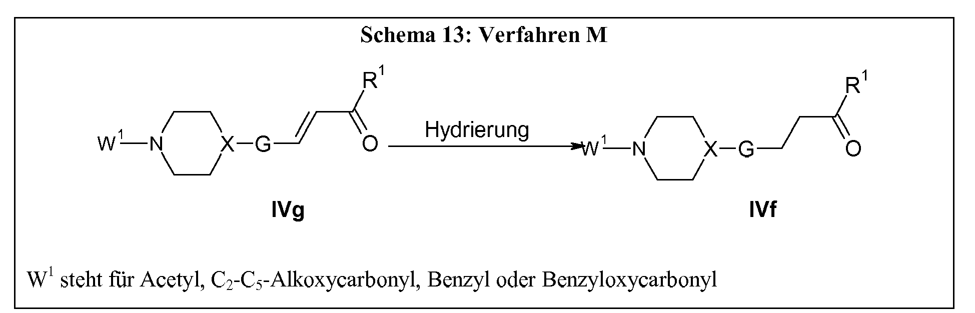

Eine Möglichkeit, Verbindungen der Formel (**IVf**) aus entsprechenden Verbindungen (**IVg**) herzustellen, ist in Schema 13 gezeigt.

Es wird das gleiche Verfahren verwendet, dass bereits in Schema 9 (Verfahren I) beschrieben wurde.

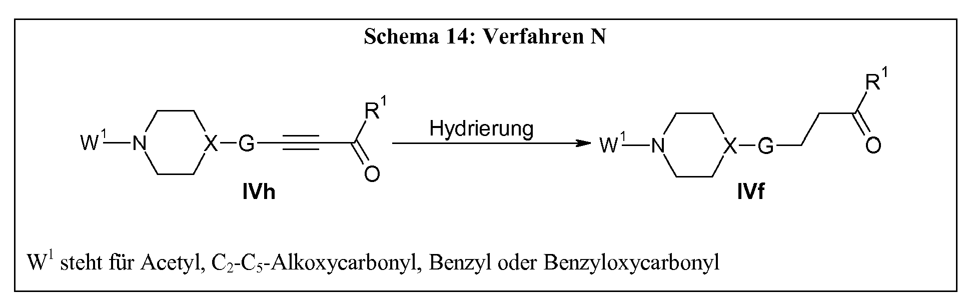

Eine Möglichkeit, Verbindungen der Formel **(IVf)** aus entsprechenden Verbindungen **(IVh)** herzustellen, ist in Schema 14 gezeigt.

Es wird das gleiche Verfahren verwendet, dass bereits in Schema 9 (Verfahren I) beschrieben wurde.

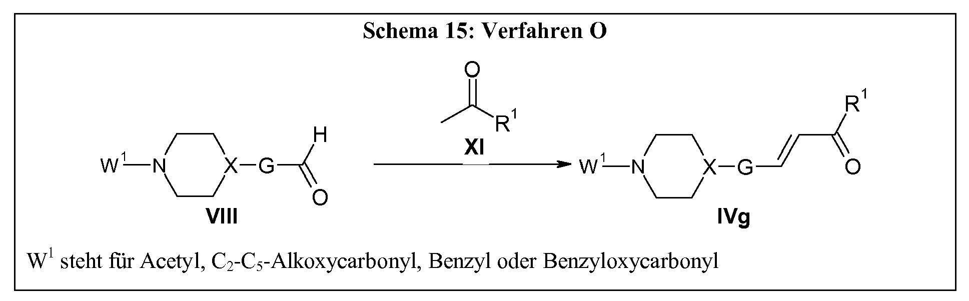

Eine Möglichkeit, Verbindungen der Formel **(IVg)** aus entsprechenden Verbindungen **(VIII)** mit einem Keton (**XI**) herzustellen, ist in Schema 15 gezeigt.

Verfahren O wird analog zu Verfahren K durchgeführt.

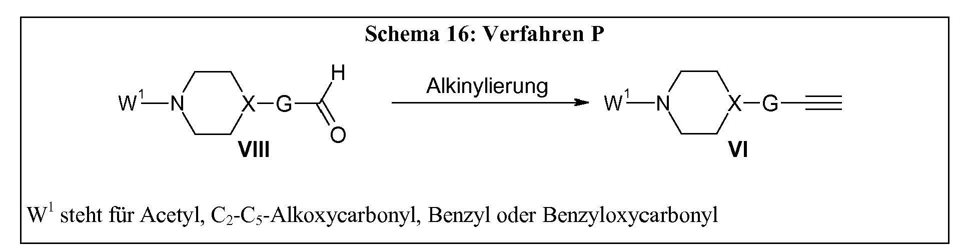

Eine Möglichkeit, das Zwischenprodukt (**VI**) aus Verbindung **(VIII)** herzustellen, ist in Schema 16 gezeigt.

In der Literatur ist bekannt, dass Alkinylierungen von Aldehyden durch eine Corey-Fuchs-Reaktion (Tetrahedron Lett., 1972, 36, 3769) oder eine Seyferth-Gilbert-Homologisierung (siehe z.B. J. Org. Chem., 1996, 61, 2540) erzielt werden kann. Alternativ kann das Alkin (**VI**) auch aus dem Aldehyd (**VIII**) mit Bestmann-Ohira's Reagenz analog der Literaturvorschriften hergestellt werden (s. z.B. Synthesis, 2004, 59). Bevorzugt werden Alkinylierung mit Bestmann-Ohira's Reagenz in Methanol oder Ethanol in der Gegenwert von Kaliumcarbonat oder Natriumcarbonat verwendet.

Das erfindungsgemäße Verfahren P wird vorzugsweise unter Verwendung eines oder mehrerer Verdünnungsmittel durchgeführt. In der Reaktion können alle bekannten Basen eingesetzt werden. Wenigstens ein Äquivalent der Base (z.B. Alklimetall- und Erdalkalimetalloxide, Hydroxide and Carbonate) muss zu dem Bestmann-Ohira's Reagenz zugegeben werden und gegebenenfalls kann sie im Überschuss verwendet werden.

Der Aldehyd **(VIII)** und das Alkinylierungsreagenz werden in äquimolaren Mengen eingesetzt, aber das Bestmann-Ohira's Reagenz kann gegebenenfalls im Überschuss verwendet werden. Die Reaktion wird vorzugsrweise bei Temperaturen von -100 °C bis 60 °C durchgeführt und besonders bevorzugt bei -78 °C bis 40 °C. Die Reaktionszeit variiert in Abhängigkeit vom Maßstab der Reaktion und der Reaktionstemperatur, aber liegt im Allgemeinen zwischen einigen Minuten und 48 Stunden.

Nach Beendigung der Reaktion, werden die Verbindungen (**VI**) von der Reaktionsmischung durch eine der üblichen Trenntechniken getrennt. Falls notwendig werden die Verbindungen durch Umkristallisation, Destillation oder Chromatographie gereinigt oder können gegebenenfalls auch im nächsten Schritt ohne vorhergehende Reinigung eingesetzt werden.

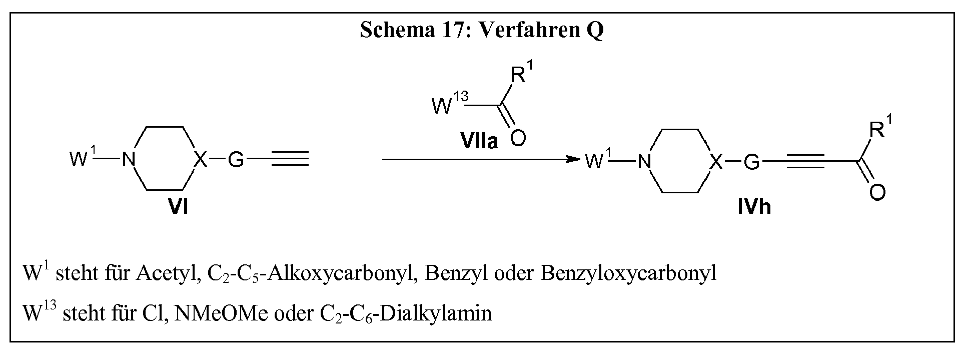

Eine Möglichkeit, Verbindungen der Formel (**IVh**) aus entsprechenden Verbindungen (**VI**) mit einer Verbindung der Formel **(VIIa)** herzustellen, ist in Schema 17 gezeigt.

Verfahren Q wird analog zu Verfahren L durchgeführt.

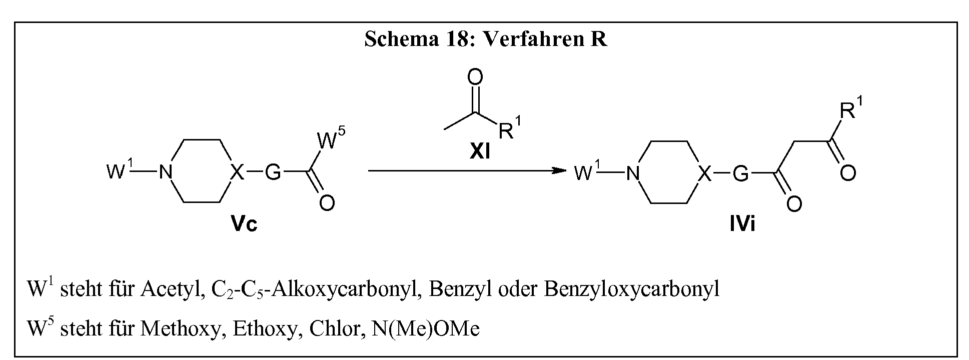

Eine Möglichkeit, Verbindungen der Formel (**IVi**) aus entsprechenden Verbindungen (**Vc**) mit einem Keton (**XI**) herzustellen, ist in Schema 18 gezeigt.

Eine Verbindung mit der allgemeinen Formel **(IVi)** kann durch die Reaktion einer Verbindung mit der entsprechenden allgemeinen Formel **(Vc)** mit einem Keton (**XI**) in der Gegenwart einer Base synthetisiert werden (s. z.B WO 2008/004100; Bioorganic & Medicinal Chemistry Letters, 2008, 18(17), 4859).

Es können alle bekannten geeigneten Basen eingesetzt werden. Bevorzugt werden für die Reaktion Lithium-, Natrium-, Kaliumhexamethyldisilazid, Natriummethoxid, Kaliummethoxid, Natriumethoxid, Kaliumethoxid und Lithiumdiisopropylamid verwendet. Bevorzugt muss wenigstens ein Äquivalent der Base zu dem Keton der allgemeinen Formel (**XI**) zugegeben werden, gegebenenfalls kann sie im Überschuss verwendet werden.

Das erfindungsgemäße Verfahren R wird vorzugsweise unter Verwendung eines oder mehrerer Verdünnungsmittel durchgeführt.

Die Verbindung (Vc) und das Keton (**XI**) werden in equimolaren Mengen eingesetzt. Die Reaktion wird vorzugsweise bei Temperaturen von -100 °C bis 100 °C durchgeführt und besonders bevorzugt se bei -78 °C bis 40 °C. Die Reaktionszeit variiert in Abhängigkeit vom Maßstab der Reaktion und der Reaktionstemperatur, liegt aber im Allgemeinen zwischen einigen Minuten und 48 Stunden.

Nach Beendigung der Reaktion, werden die Verbindungen (IVi) von der Reaktionsmischung durch eine der üblichen Trenntechniken getrennt. Falls notwendig werden die Verbindungen durch Umkristallisation, Destillation oder Chromatographie gereinigt oder können gegebenenfalls auch im nächsten Schritt ohne vorhergehende Reinigung eingesetzt werden.

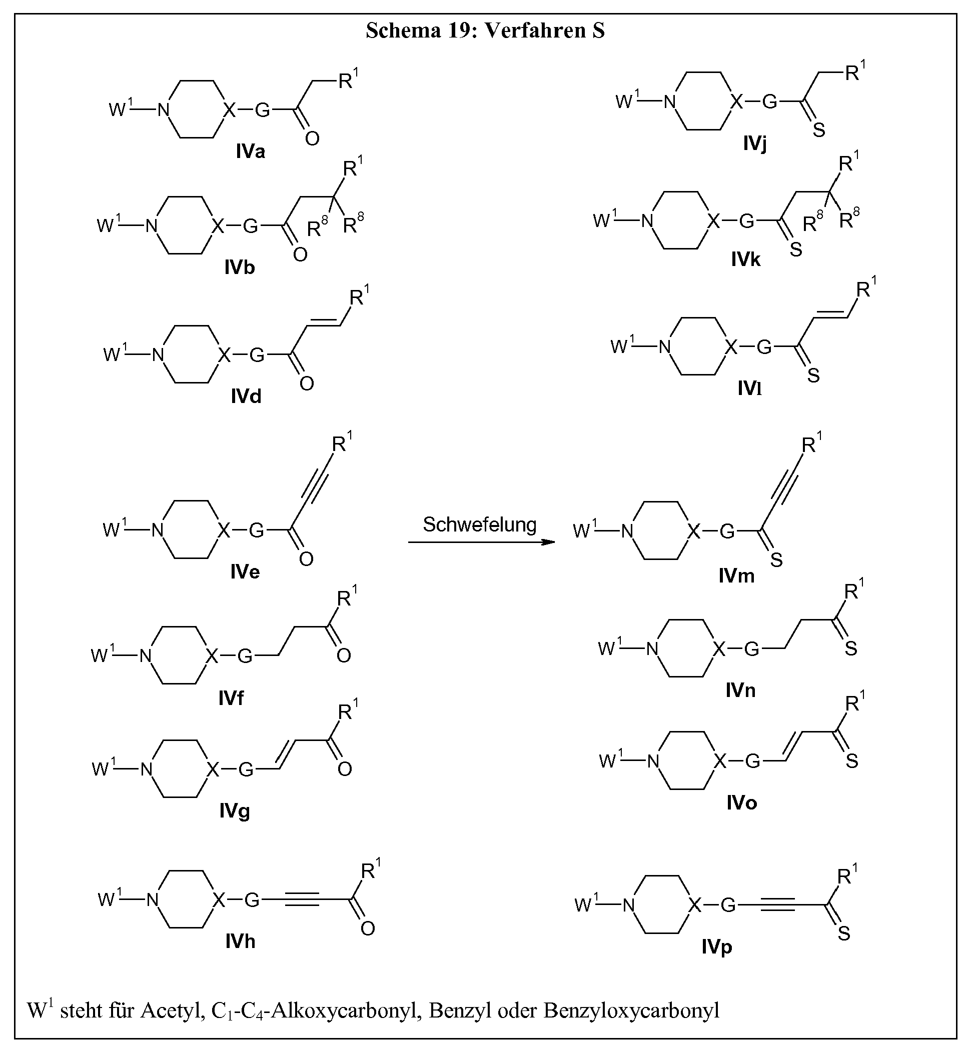

Eine Möglichkeit, Verbindungen der Formel (**IVj**)-(**IVp**) aus entsprechenden Verbindungen **(IVa), (IVb), (IVd)-(IVh)** darzustellen, ist in Schema 19 gezeigt.

Das erfindungsgemäße Verfahren S wird vorzugsweise unter Verwendung eines oder mehrerer Verdünnungsmittel durchgeführt. Die bevorzugten Lösungsmittel sind Chloroform und 1,2-Dimethoxyethan.

Geeignete Schwefelungsreagenzien sind beispielsweise Lawesson's Reagenz (s. Tetrahedron 1986, 42, 6555-6564, Tetrahedron Lett. 1993, 46, 7459-7462) und Phosphorpentasulfid. Das Startmaterial und das Schwefelungsreagenz werden in equimolaren Mengen verwendet, das Schwefelungsreagenz kann gegebenenfalls aber auch im Überschuss verwendet warden.

Die Reaktion wird vorzugsweise bei Temperaturen von 0 °C - 150 °C durchgeführt und besonders bevorzugt bei 0 °C - 100 °C. Die Reaktionszeit variiert in Abhängigkeit vom Maßstab der Reaktion und der Reaktionstemperatur, liegt aber im Allgemeinen zwischen einigen Minuten und 48 Stunden.

Nach Beendigung der Reaktion werden die Verbindungen **(IVj)-(IVp)** von der Reaktionsmischung durch eine der üblichen Trenntechniken getrennt. Falls notwendig werden die Verbindungen durch Umkristallisation, Destillation oder Chromatographie gereinigt.

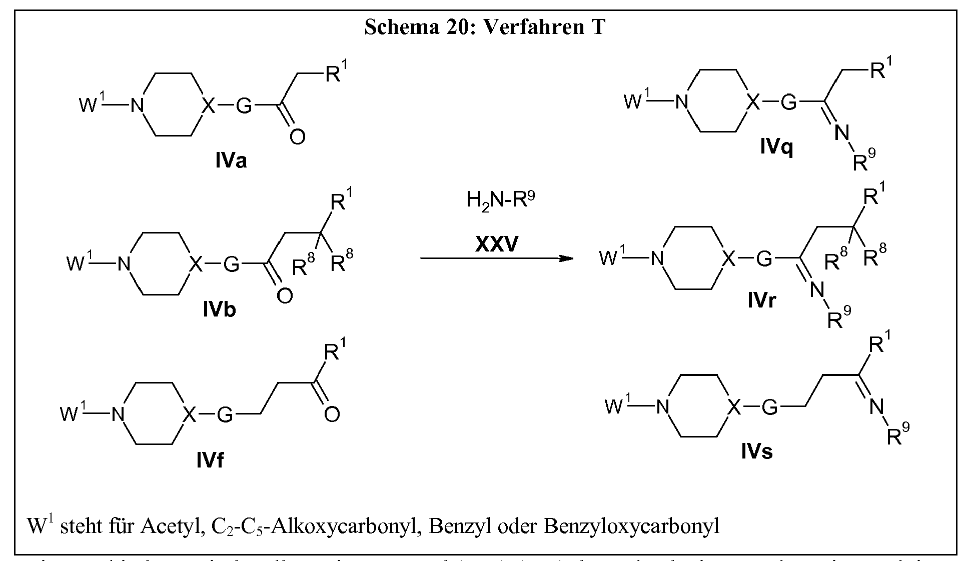

Eine Verbindung mit der allgemeinen Formel **(IVq)-(IVs),** kann durch eine Kondensationsreaktion einer Verbindung mit der entsprechenden allgemeinen Formel **(IVa), (IVb)** oder **(IVf)** mit einem Substrat der allgemeinen Formel **(XXV),** gegebenenfalls in der Gegenwart einer Säure, eines Säurefängers / Base oder eines basischen Ionentauschers synthetisiert werden.

Die Verbindung **(XXV)** oder die entsprechenden Salze der Salzsäure sind kommerziell erhältlich oder durch in der Literatur beschriebene Prozesse darstellbar (siehe z.B. Chem. Eur. J. 2005, 11, 6974-6981 und Chem. Soc. Rev., 2001, 30, 205-213).

Gegebenenfalls kann eine Säure wie z.B. Salzsäure oder eine Base wie z.B. Triethylamin, Hünig Base oder ein basischer Ionentauscher wie z.B. Amberlyst A21 in der Reaktion verwendet werden.

Das erfindungsgemäße Verfahren T wird vorzugsweise unter Verwendung eines oder mehrerer Verdünnungsmittel durchgeführt. Das bevorzugte Lösungsmittel ist Ethanol.

Die Reaktion wird vorzugsweise bei Temperaturen von 0 °C - 100 °C durchgeführt besonders bevorzugt bei 0 °C - 30 °C. Die Reaktionszeit variiert in Abhängigkeit vom Maßstab der Reaktion und der Reaktionstemperatur, liegt aber im Allgemeinen zwischen einigen Minuten und 48 Stunden.

Nach Beendigung der Reaktion werden die Verbindungen **(IVq)-(IVs)** von der Reaktionsmischung durch eine der üblichen Trenntechniken getrennt. Falls notwendig werden die Verbindungen durch Umkristallisation, Destillation oder Chromatographie gereinigt oder können gegebenenfalls auch im nächsten Schritt ohne vorhergehende Reinigung eingesetzt werden.

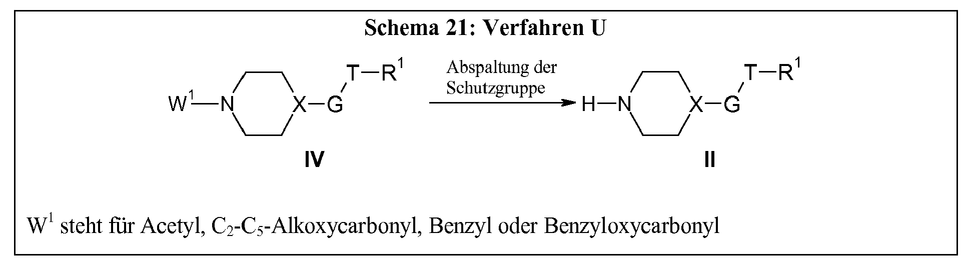

Eine Möglichkeit, Verbindungen der Formel **(**II**)** aus entsprechenden Verbindungen (**IV**) darzustellen, ist in Schema 21 gezeigt.

Eine Verbindung der Formel (**II**) wird in eine Verbindung der Formel **(IV)** durch geeignete Methoden zur Entfernung von Schutzgruppen, die in der Literatur beschrieben sind ("Protective Groups in Organic Synthesis"; Third Edition; 1999; 494, und dort zitierte Literatur), überführt.

tert-Butoxycarbonyl und Benzyloxycarbonyl Schutzgruppen können im sauren Medium entfernt werden (z.B mit Salzsäure oder der Trifluoressigsäure). Acetylschutzgruppen können unter basischen Bedingungen (z.B. mit Kaliumcarbonat oder Cäsiumcarbonat) entfernt werden. Benzylische Schutzgruppen können hydrogenolytisch mit Wasserstoff in Gegenwart eines Katalysators (z.B. Palladium auf Aktivkohle) entfernt werden.

Säuren, die für diese Reaktion, der Entschützung von t-Butoxycarbonyl und Benzyloxycarbonyl Gruppen verwendet werden können, sind z.B. Trifluoressigsäure, Salzsäure oder andere Säuren, wie sie in der Literatur beschrieben sind (z.B. "Protective Groups in Organic Synthesis"; Third Edition; 1999; S. 494).

Das erfindungsgemäße Verfahren U wird vorzugsweise unter Verwendung eines oder mehrerer Verdünnungsmittel durchgeführt. Die Reaktion wird vorzugsweise bei Temperaturen von 0° C bis +150 °C durchgeführt, besonders bevorzugt bei Raumtemperatur. Die Reaktionszeit variiert in Abhängigkeit vom Maßstab der Reaktion und der Reaktionstemperatur, liegt aber im Allgemeinen zwischen einer halben Stunde und 72 Stunden.

Nach Beendigung der Reaktion werden die Verbindungen **(II)** von der Reaktionsmischung durch eine der üblichen Trenntechniken getrennt. Falls notwendig werden die Verbindungen durch Umkristallisation, Destillation oder Chromatographie gereinigt oder können, wenn gewünscht, auch im nächsten Schritt ohne vorhergehende Reinigung eingesetzt werden. Es ist außerdem möglich, die Verbindung der allgemeinen Formel **(II)** als Salz zu isolieren, z.B. als Hydrochlorid oder Trifluoracetate.

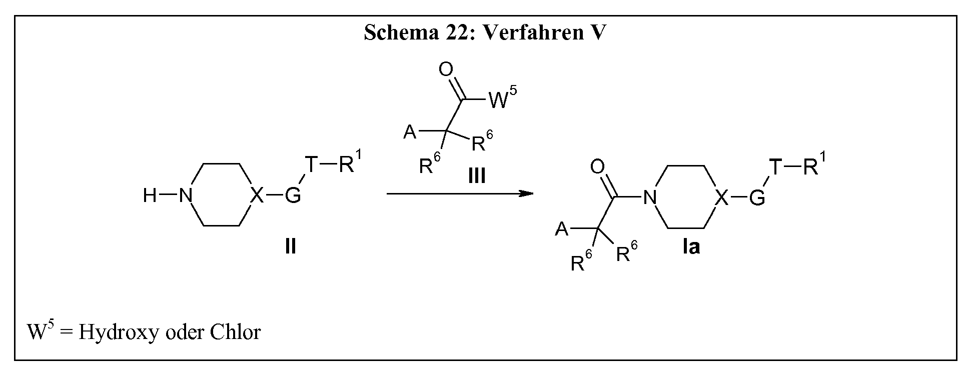

Eine Möglichkeit, Verbindungen der Formel **(Ia)** aus entsprechenden Verbindungen (**II**) mit der Verbindungen **(III)** darzustellen, ist in Schema 22 gezeigt

Verbindungen **(III)** sind durch in der Literatur beschriebene Prozesse darstellbar (s. z.B. WO 2008/013622 und WO 2008/013925).

Eine Verbindung mit der allgemeinen Formel **(Ia)** kann analog zu in der Literatur beschriebenen Vorschriften (s. z.B. WO 2007/147336) durch eine Kupplungsreaktion einer Verbindung mit der entsprechenden allgemeinen Formel (**II**) mit einem Substrat der allgemeinen Formel **(III),** wobei W⁵ für Chlor steht, gegebenenfalls in der Gegenwart eines Säurefängers / Base synthetisiert werden.

Wenigstens ein Äquivalent eines Säurefängers/einer Base (z.B. Hünig Base, Triethylamin oder kommerziell erhältliche polymere Säurefänger) wird im Verhältnis zum Startmaterial der allgemeinen Formel (**II**) verwendet. Ist das Startmaterial ein Salz, werden wenigstens zwei Äquivalente des Säurefängers benötigt.

Die Startmaterialien werden in equimolaren Mengen eingesetzt. Die Reaktion wird vorzugsweise bei Temperaturen von 0 °C bis 100 °C durchgeführt und besonders bevorzugt bei 20 °C bis 30 °C,. Die Reaktionszeit variiert in Abhängigkeit vom Maßstab der Reaktion und der Reaktionstemperatur, liegt aber im Allgemeinen zwischen einigen Minuten und 48 Stunden.

Alternativ kann eine Verbindung der Formel **(Ia),** auch aus der entsprechenden Verbindung der Formel (**II**) mit einem Substrat der Formel **(III),** wobei W⁵ für Hydroxy steht, in Gegenwart eines Kupplungsreagenzes analog zu in der Literatur beschriebenen Vorschriften synthetisiert werden (z.B. Tetrahedron 2005, 61, 10827-10852, und darin zitierte Referenzen).

Geeignete Kupplungsreagenzien sind beispielsweise Peptidkupplungsreagenzien zum Beispiel, N-(3-Dimethylaminopropyl)-N'-ethyl-carbodiimid gemischt mit 4-Dimethylamino-pyridin, N-(3-Dimethylaminopropyl)-N'-ethyl-carbodiimid gemischt mit 1-Hydroxy-benzotriazol, Bromtripyrrolidinophosphonium-hexafluorophosphat, O-(7-Azabenzotriazol-1-yl)-N,N,N',N'-tetramethyluroniumhexafluorophosphat, etc).

Die Reaktion wird vorzugsweise bei Temperaturen von 0 °C bis 100 °C durchgeführt, besonders bevorzugt bei 0 °C - 30 °C. Die Reaktionszeit variiert in Abhängigkeit vom Maßstab der Reaktion und der Re-aktionstemperatur, liegt aber im Allgemeinen zwischen einigen Minuten und 48 Stunden.

Das erfindungsgemäße Verfahren V wird vorzugsweise unter Verwendung eines oder mehrerer Verdünnungsmittel durchgeführt. Nach Beendigung der Reaktion, werden die Verbindungen (**Ia**) von der Reaktionsmischung durch eine der üblichen Trenntechniken getrennt. Falls notwendig werden die Verbindungen durch Umkristallisation, Destillation oder Chromatographie gereinigt.

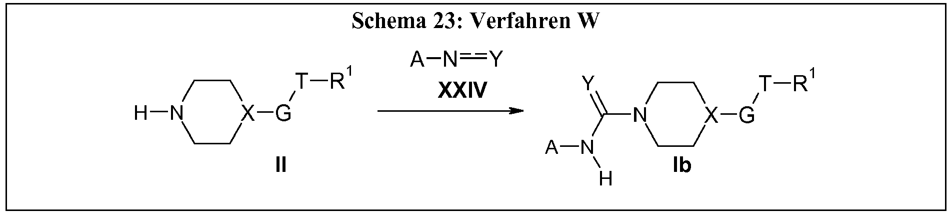

Eine Möglichkeit, Verbindungen der Formel (**Ib**) aus entsprechenden Verbindungen (**II**) mit der Verbindungen (**XXIV**) darzustellen, ist in Schema 23 gezeigt.

Eine Verbindung mit der allgemeinen Formel **(Ib** kann analog zu in der Literatur beschriebenen Vorschriften (s. z.B. WO 2009/055514) durch eine Kupplungsreaktion einer Verbindung mit der entsprechenden allgemeinen Formel (**II**) mit einem Substrat der allgemeinen Formel (**XXIV**), gegebenenfalls in der Gegenwart eines Säurefängers / Base, wie z.B. Triethylamin, 1,8-Diazabicyclo[5.4.0]undec-7-en oder Hünig-Base synthetisiert werden.

Das erfindungsgemäße Verfahren W wird vorzugsweise unter Verwendung eines oder mehrerer Verdünnungsmittel durchgeführt.

Die Startmaterialien werden in äquimolaren Mengen eingesetzt. Die Reaktion wird vorzugsweise bei Temperaturen von 0 °C bis 100 °C durchgeführt, besonders bevorzugt bei 20 °C bis 30 °C. Die Reaktionszeit variiert in Abhängigkeit vom Maßstab der Reaktion und der Reaktionstemperatur, liegt aber im Allgemeinen zwischen einigen Minuten und 48 Stunden.

Nach Beendigung der Reaktion, werden die Verbindungen (**Ib**) von der Reaktionsmischung durch eine der üblichen Trenntechniken getrennt. Falls notwendig werden die Verbindungen durch Umkristallisation, Destillation oder Chromatographie gereinigt.

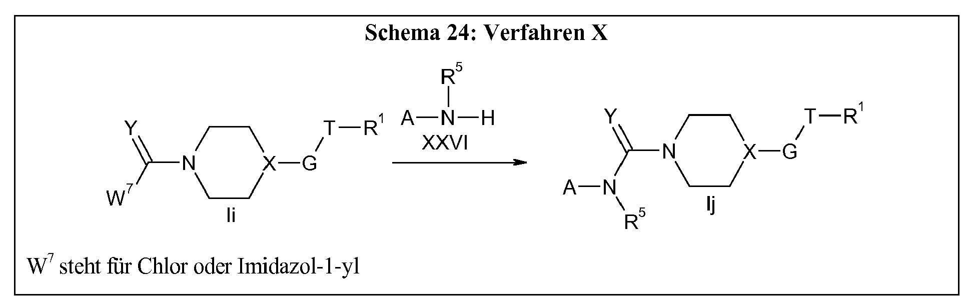

Die bei der Durchführung des erfindungsgemäßen Verfahrens als Ausgangstoffe benötigten Carbamoyl- und Thiocarbamoylchloride der Formel (**Ii**, W⁷ = Chlor) lassen sich mittels literaturbeschriebener Methoden herstellen (s. z.B. Tetrahedron, 2008, 7605; Journal of Organic Chemistry, 2004, 3787; Journal of Organic Chemistry, 1983, 4750; European Journal of Organic Chemistry, 2006, 1177). Typischerweise werden die Verbindungen der Formel (**Ii**, W⁷ = Chlor) ausgehend von Aminen der Formel (**II**) und Phosgen, Thiophosgen oder deren Äquivalenten hergestellt.
Die bei der Durchführung des erfindungsgemäßen Verfahren X als Ausgangstoffe benötigten Carbamoyl- und Thiocarbamoylimidazole der Formel (**Ii**, W⁷ = imidazol-1-yl) lassen sich mittels literaturbeschriebener Methoden herstellen (s. z.B. Tetrahedron Letters, 2008, 5279; Tetrahedron, 2005, 7153). Typischerweise werden die Verbindungen der Formel (**Ii**, W⁷ = imidazol-1-yl) ausgehend von Aminen der Formel (**II**) und 1,1'-Carbonyldiimidazole oder 1,1'-Thiocarbonyldimidazole hergestellt.
Das Verfahren X beschreibt die Herstellung von Verbindungen der Struktur (**Ij**) durch Reaktion von Verbindungen der Struktur (**Ii**, W⁷ = Chlor oder imidazol-1-yl) und Aminen (**XXVI**).
Das Verfahren X wird gegebenenfalls in Gegenwart eines geeigneten Säureakzeptors durchgeführt. Als solche kommen alle üblichen anorganischen oder organischen Basen infrage. Hierzu gehören vorzugsweise Erdalkalimetall- oder Alkalimetallhydride, -hydroxide, -amide, -alkoholate, -acetate, -carbonate oder -hydrogencarbonate, wie beispielsweise Natriumhydrid, Natriumamid, Lithiumdiisopropylamid, Natriummethylat, Natriumethylat, Kalium-tert.-butylat, Natriumhydroxid, Kaliumhydroxid, Natriumacetat, Natriumcarbonat, Kaliumcarbonat, Kaliumhydrogencarbonat, Natriumhydrogencarbonat oder Ammoniumcarbonat, sowie tertiäre Amine, wie Trimethylamin, Triethylamin, Tributylamin, N,N-Diisopropylethylamin, N,N-Dimethylanilin, N,N-Dimethyl-benzylamin, Pyridin, N-Methylpiperidin, N-Methylmorpholin, N,N-Dimethylaminopyridin, Diazabicyclooctan (DABCO), Diazabicyclononen (DBN) oder Diazabicycloundecen (DBU).
Die bei der Durchführung des erfindungsgemäßen Verfahren X erhaltenen Verbindungen (**Ij**) können alternativ teilweise auch ohne Verwendung eines Säureakzeptors als korrespondierende Säurechloride [(**Ij**)-HCl] (Edukt: W⁷ = Cl) erhalten werden. Bei Bedarf erfolgt die Freisetzung der Verbindungen **(Ij)** nach üblichen Methoden.

Das erfindungsgemäße Verfahren X wird vorzugsweise unter Verwendung eines oder mehrerer Verdünnungsmittel durchgeführt. Die Startmaterialien werden in equimolaren Mengen eingesetzt. Die Reaktion wird vorzugsweise bei Temperaturen von 0 °C bis 100 °C durchgeführt und besonders bevorzugt bei 20 °C bis 30 °C,. Die Reaktionszeit variiert in Abhängigkeit vom Maßstab der Reaktion und der Reaktionstemperatur, aber liegt im Allgemeinen zwischen einigen Minuten und 48 Stunden.

Nach Beendigung der Reaktion, werden die Verbindungen (**Ij**) von der Reaktionsmischung durch eine der üblichen Trenntechniken getrennt. Falls notwendig werden die Verbindungen durch Umkristallisation, Destillation oder Chromatographie gereinigt.

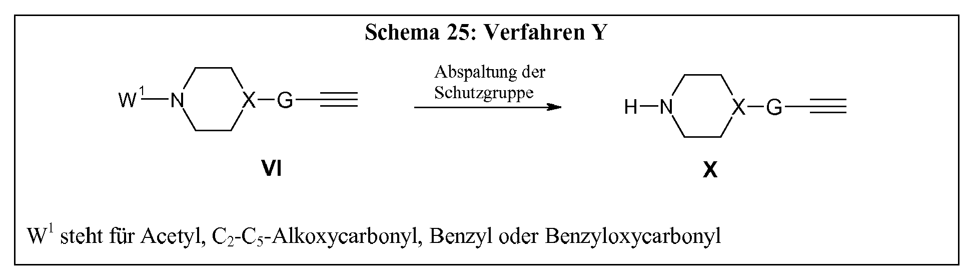

Eine Möglichkeit, Verbindungen der Formel **(X)** aus entsprechenden Verbindungen (**VI**) herzustellen, ist in Schema 25 gezeigt.

Verfahren Y wird analog Verfahren L durchgeführt.

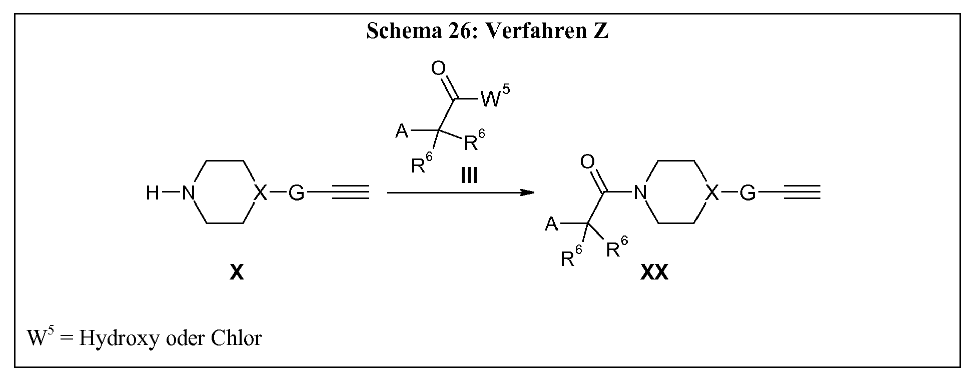

Eine Möglichkeit, Verbindungen der Formel **(XX)** aus entsprechenden Verbindungen (X) herzustellen, ist in Schema 26 gezeigt.

Es wird das gleiche Verfahren verwendet, dass bereits im Schema 22 (Verfahren V) beschrieben wurde.

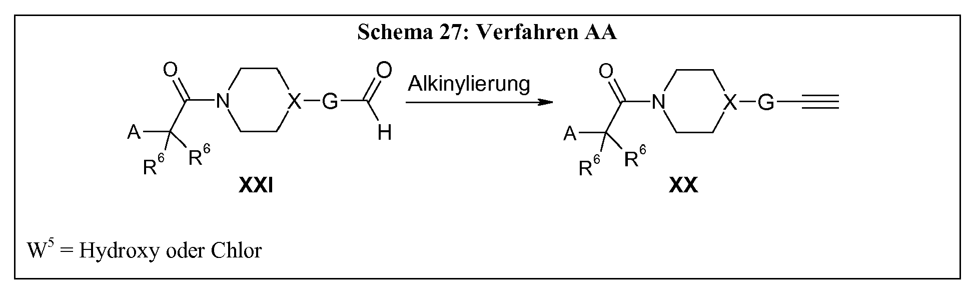

Eine Möglichkeit, Verbindungen der Formel **(XX)** aus entsprechenden Verbindungen (**XXI**) herzustellen, ist in Schema 27 gezeigt.

Es wird das gleiche Verfahren verwendet, dass bereits im Schema 16 (Verfahren P) beschrieben wurde.

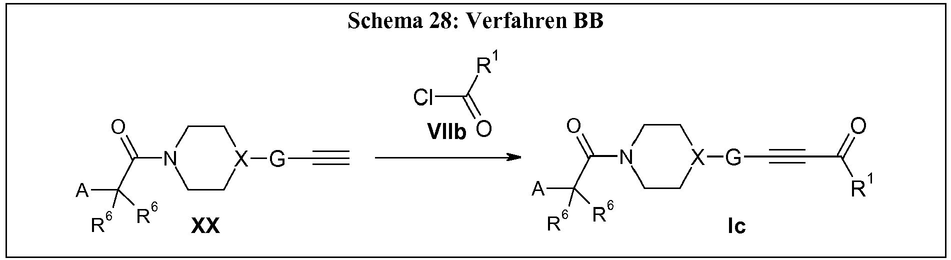

Eine Möglichkeit, Verbindungen der Formel (**Ic**) aus entsprechenden Verbindungen **(XX)** herzustellen, ist in Schema 28 gezeigt.

Es wird das gleiche Verfahren verwendet, dass bereits im Schema 12 (Verfahren L) beschrieben wurde.

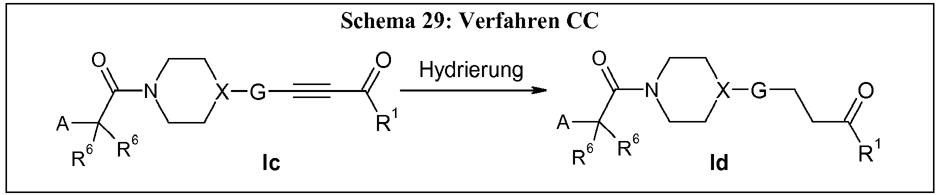

Eine Möglichkeit, Verbindungen der Formel (**Id**) aus entsprechenden Verbindungen (**Ic**) herzustellen, ist in Schema 29: Verfahren CC gezeigt.

Es wird das gleiche Verfahren verwendet, dass bereits im Schema 9 (Verfahren I) beschrieben wurde.

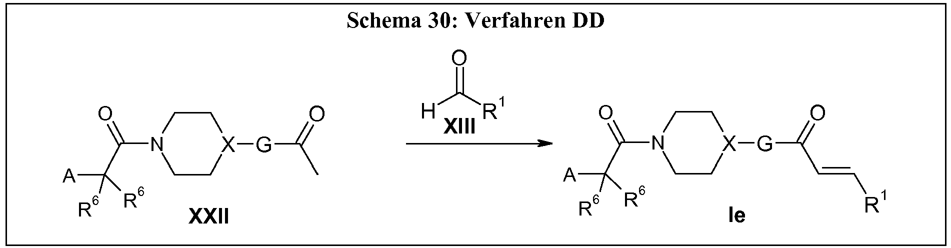

Eine Möglichkeit, Verbindungen der Formel (Ie) aus entsprechenden Verbindungen (**XXII**) mit einer Verbindung der Formel (**XIII**) herzustellen, ist in Schema 30 gezeigt.

Es wird das gleiche Verfahren verwendet, dass bereits im Schema 11 (Verfahren K) beschrieben wurde.

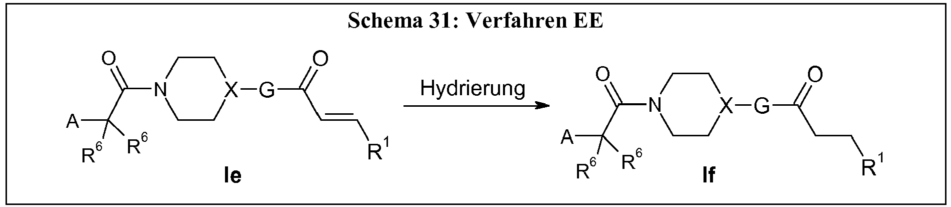

Eine Möglichkeit, Verbindungen der Formel **(If)** aus entsprechenden Verbindungen **(Ie)** herzustellen, ist in Schema 31 gezeigt.

Es wird das gleiche Verfahren verwendet, dass bereits im Schema 9 (Verfahren I) beschrieben wurde.

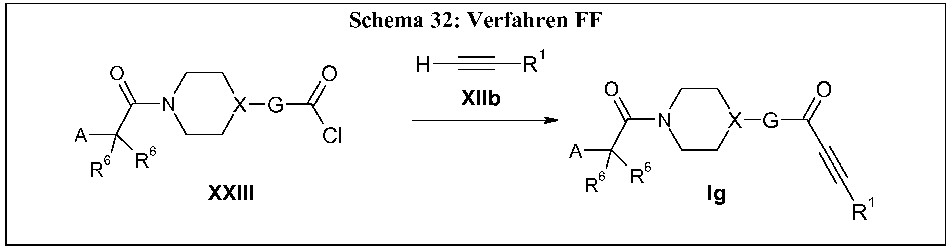

Eine Möglichkeit, Verbindungen der Formel **(Ig)** aus entsprechenden Verbindungen (**XXIII**) mit einer Verbindung der Formel **(XIIb)** herzustellen, ist in Schema 32 gezeigt.

Es wird das gleiche Verfahren verwendet, dass bereits im Schema 12 (Verfahren L) beschrieben wurde.

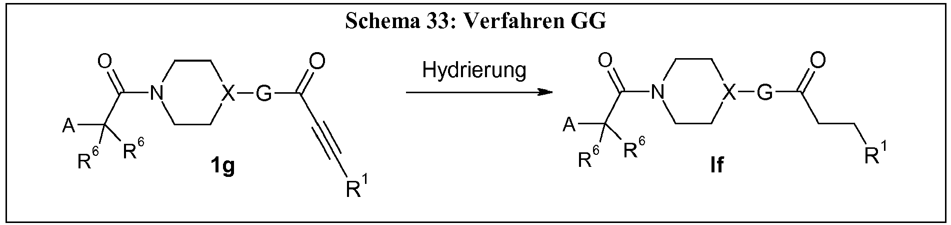

Eine Möglichkeit,Verbindungen der Formel **(If)** aus entsprechenden Verbindungen **(Ig)** herzustellen, ist in Schema 33 gezeigt.

Es wird das gleiche Verfahren verwendet, dass bereits im Schema 9 (Verfahren I) beschrieben wurde.

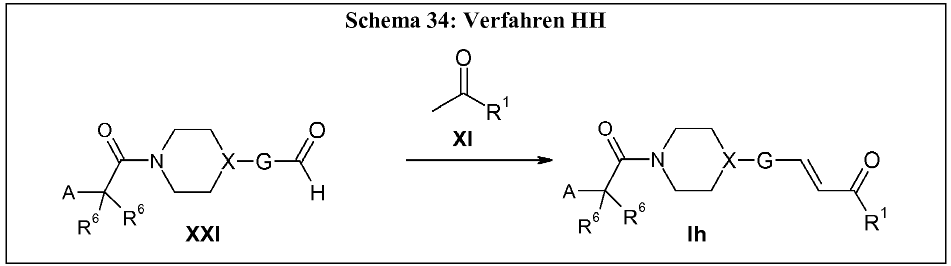

Eine Möglichkeit, Verbindungen der Formel (**Ih**) aus entsprechenden Verbindungen (**XXI**) mit einem Keton (**XI**) herzustellen, ist in Schema 34 gezeigt.

Es wird das gleiche Verfahren verwendet, dass bereits im Schema 11 (Verfahren K)beschrieben wurde.

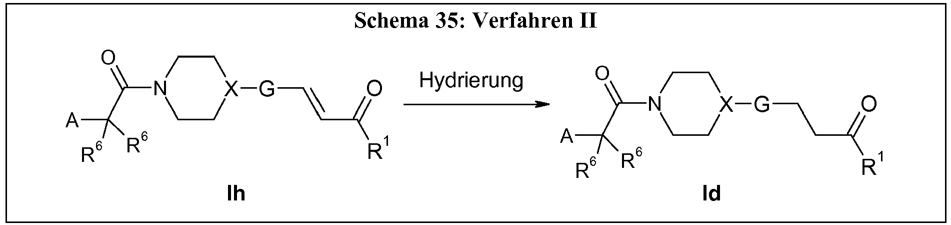

Eine Möglichkeit, Verbindungen der Formel (**Id**) aus entsprechenden Verbindungen (**Ih**) herzustellen, ist in Schema 35 gezeigt.

Es wird das gleiche Verfahren verwendet, dass bereits im Schema 9 (Verfahren I) beschrieben wurde.

Ein weiterer Gegenstand der Erfindung betrifft die nichtmedizinische Verwendung der erfindungsgemäßen Ketoheteroarylpiperidin und -piperazin Derivate zum Bekämpfen unerwünschter Mikroorganismen.

Die vorliegende Erfindung betrifft weiterhin ein Pflanzenschutzmittel zum Bekämpfen unerwünschter Pilze umfassend wenigstens eines der Ketoheteroarylpiperidin und -piperazin Derivate der Formel (I). Vorzugsweise handelt es sich um fungizide Mittel, welche landwirtschaftlich verwendbare Hilfsmittel, Solventien, Trägerstoffe, oberflächenaktive Stoffe oder Streckmittel enthalten.

Außerdem betrifft die Erfindung ein Verfahren zum Bekämpfen unerwünschter Mikroorganismen, dadurch gekennzeichnet, dass man erfindungsgemäß Ketoheteroarylpiperidin und -piperazin Derivate der Formel (I) auf die phytopathogenen Pilze und/oder deren Lebensraum ausbringt.

Erfindungsgemäß bedeutet Trägerstoff eine natürliche oder synthetische, organische oder anorganische Substanz, mit welchen die Wirkstoffe zur besseren Anwendbarkeit, v.a. zum Aufbringen auf Pflanzen oder Pflanzenteile oder Saatgut, gemischt oder verbunden sind. Der Trägerstoff, welcher fest oder flüssig sein kann, ist im Allgemeinen inert und sollte in der Landwirtschaft verwendbar sein.

Als feste oder flüssige Trägerstoffe kommen infrage: z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und natürliche oder synthetische Silikate, Harze, Wachse, feste Düngemittel, Wasser, Alkohole, besonders Butanol, organische Solventien, Mineral- und Pflanzenöle sowie Derivate hiervon. Mischungen solcher Trägerstoffe können ebenfalls verwendet werden. Als feste Trägerstoffe für Granulate kommen infrage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnussschalen, Maiskolben und Tabakstängel.

Als verflüssigte gasförmige Streckmittel oder Trägerstoffe kommen solche Flüssigkeiten infrage, welche bei normaler Temperatur und unter Normaldruck gasförmig sind, z.B. Aerosol-Treibgase, wie Halogenkohlenwasserstoffe, sowie Butan, Propan, Stickstoff und Kohlendioxid.

Es können in den Formulierungen Haftmittel wie Carboxymethylcellulose, natürliche und synthetische pulverige, körnige oder latexförmige Polymere verwendet werden, wie Gummiarabikum, Polyvinylalkohol, Polyvinylacetat, sowie natürliche Phospholipide, wie Kephaline und Lecithine, und synthetische Phospholipide. Weitere Additive können mineralische und vegetabile Öle sein.

Im Falle der Benutzung von Wasser als Streckmittel können z.B. auch organische Lösungsmittel als Hilfslösungsmittel verwendet werden. Als flüssige Lösungsmittel kommen im Wesentlichen infrage: Aromaten, wie Xylol, Toluol oder Alkylnaphthaline, chlorierte Aromaten oder chlorierte aliphatische Kohlenwasserstoffe, wie Chlorbenzole, Chlorethylene oder Dichlormethan, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone, wie Aceton, Methylethylketon, Methylisobutylketon oder Cyclohexanon, stark polare Lösungsmittel wie Dimethylformamid und Dimethylsulfoxid, sowie Wasser.

Die erfindungsgemäßen Mittel können zusätzlich weitere Bestandteile enthalten, wie z.B. oberflächenaktive Stoffe. Als oberflächenaktive Stoffe kommen Emulgier- und/oder Schaum erzeugende Mittel, Dispergiermittel oder Benetzungsmittel mit ionischen oder nicht-ionischen Eigenschaften oder Mischungen dieser oberflächenaktiven Stoffe infrage. Beispiele hierfür sind Salze von Polyacrylsäure, Salze von Lignosulphonsäure, Salze von Phenolsulphonsäure oder Naphthalinsulphonsäure, Polykondensate von Ethylenoxid mit Fettalkoholen oder mit Fettsäuren oder mit Fettaminen, substituierten Phenolen (vorzugsweise Alkylphenole oder Arylphenole), Salze von Sulphobernsteinsäureestern, Taurinderivate (vorzugsweise Alkyltaurate), Phosphorsäureester von polyethoxylierten Alkoholen oder Phenole, Fettsäureester von Polyolen, und Derivate der Verbindungen enthaltend Sulphate, Sulphonate und Phosphate, z.B. Alkylarylpolyglycolether, Alkylsulfonate, Alkylsulfate, Arylsulfonate, Eiweißhydrolysate, Lignin-Sulfitablaugen und Methylcellulose. Die Anwesenheit einer oberflächenaktiven Substanz ist notwendig, wenn einer der Wirkstoff und/oder einer der inerten Trägerstoffe nicht in Wasser löslich ist und wenn die Anwendung in Wasser erfolgt. Der Anteil an oberflächenaktiven Stoffen liegt zwischen 5 und 40 Gewichtsprozent des erfindungsgemäßen Mittels.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe, wie Salze von Eisen, Mangan, Bor, Kupfer, Kobalt, Molybdän und Zink verwendet werden.

Gegebenenfalls können auch andere zusätzliche Komponenten enthalten sein, z.B. schützende Kolloide, Bindemittel, Klebstoffe, Verdicker, thixotrope Stoffe, Penetrationsförderer, Stabilisatoren, Sequestiermittel, Komplexbildner. Im Allgemeinen können die Wirkstoffe mit jedem festen oder flüssigen Additiv, welches für Formulierungszwecke gewöhnlich verwendet wird, kombiniert werden.

Die Formulierungen enthalten im Allgemeinen zwischen 0,05 und 99 Gew.%, 0,01 und 98 Gew.-%, vorzugsweise zwischen 0,1 und 95 Gew.%, besonders bevorzugt zwischen 0,5 und 90 % Wirkstoff, ganz besonders bevorzugt zwischen 10 und 70 Gewichtsprozent.

Die erfindungsgemäßen Wirkstoffe bzw. Mittel können als solche oder in Abhängigkeit von ihren jeweiligen physikalischen und/oder chemischen Eigenschaften in Form ihrer Formulierungen oder den daraus bereiteten Anwendungsformen, wie Aerosole, Kapselsuspensionen, Kaltnebelkonzentrate, Heißnebelkonzentrate, verkapselte Granulate, Feingranulate, fließfähige Konzentrate für die Behandlung von Saatgut, gebrauchsfertige Lösungen, verstäubbare Pulver, emulgierbare Konzentrate, Öl-in-Wasser-Emulsionen, Wasser-in-Öl-Emulsionen, Makrogranulate, Mikrogranulate, Öl-dispergierbare Pulver, Öl-mischbare fließfähige Konzentrate, Öl-mischbare Flüssigkeiten, Schäume, Pasten, Pestizid-ummanteltes Saatgut, Suspensionskonzentrate, Suspensions-Emulsions-Konzentrate, lösliche Konzentrate, Suspensionen, Spritzpulver, lösliche Pulver, Stäubemittel und Granulate, wasserlösliche Granulate oder Tabletten, wasserlösliche Pulver für Saatgutbehandlung, benetzbare Pulver, Wirkstoff-imprägnierte Natur- und synthetische Stoffe sowie Feinstverkapselungen in polymeren Stoffen und in Hüllmassen für Saatgut, sowie ULV-Kalt- und Warmnebel-Formulierungen eingesetzt werden.

Die genannten Formulierungen können in an sich bekannter Weise hergestellt werden, z.B. durch Vermischen der Wirkstoffe mit mindestens einem üblichen Streckmittel, Lösungs- bzw. Verdünnungsmittel, Emulgator, Dispergier- und/oder Binde- oder Fixiermittels, Netzmittel, Wasser-Repellent, gegebenenfalls Sikkative und UV-Stabilisatoren und gegebenenfalls Farbstoffen und Pigmenten, Entschäumer, Konservierungsmittel, sekundäre Verdickungsmittel, Kleber, Gibberelline sowie weiteren Verarbeitungshilfsmitteln.

Die erfindungsgemäßen Mittel umfassen nicht nur Formulierungen, welche bereits anwendungsfertig sind und mit einer geeigneten Apparatur auf die Pflanze oder das Saatgut ausgebracht werden können, sondern auch kommerzielle Konzentrate, welche vor Gebrauch mit Wasser verdünnt werden müssen.

Die erfindungsgemäßen Wirkstoffe können als solche oder in ihren (handelsüblichen) Formulierungen sowie in den aus diesen Formulierungen bereiteten Anwendungsformen in Mischung mit anderen (bekannten) Wirkstoffen, wie Insektiziden, Lockstoffen, Sterilantien, Bakteriziden, Akariziden, Nematiziden, Fungiziden, Wachstumsregulatoren, Herbiziden, Düngemitteln, Safener bzw. Semiochemicals vorliegen.

Die erfindungsgemäße Behandlung der Pflanzen und Pflanzenteile mit den Wirkstoffen bzw. Mitteln erfolgt direkt oder durch Einwirkung auf deren Umgebung, Lebensraum oder Lagerraum nach den üblichen Behandlungsmethoden, z.B. durch Tauchen, (Ver-)Spritzen, (Ver-)Sprühen, Berieseln, Verdampfen, Zerstäuben, Vernebeln, (Ver-)Streuen, Verschäumen, Bestreichen, Verstreichen, Gießen (drenchen), Tröpfchenbewässerung und bei Vermehrungsmaterial, insbesondere bei Samen, weiterhin durch Trockenbeizen, Nassbeizen, Schlämmbeizen, Inkrustieren, ein- oder mehrschichtiges Umhüllen usw. Es ist ferner möglich, die Wirkstoffe nach dem Ultra-Low-Volume-Verfahren auszubringen oder die Wirkstoffzubereitung oder den Wirkstoff selbst in den Boden zu injizieren.

Die Erfindung umfasst weiterhin ein Verfahren zur Behandlung von Saatgut.

Die Erfindung betrifft weiterhin Saatgut, welches gemäß einem der im vorherigen Absatz beschriebenen Verfahren behandelt wurde. Die erfindungsgemäßen Saatgüter finden Anwendung in Verfahren zum Schutz von Saatgut vor unerwünschten Pilzen. Bei diesen wird ein mit wenigstens einem erfindungsgemäßen Wirkstoff behandeltes Saatgut verwendet.

Die erfindungsgemäßen Wirkstoffe bzw. Mittel sind auch geeignet für die Behandlung von Saatgut. Ein großer Teil des durch Schadorganismen hervorgerufenen Schadens an Kulturpflanzen wird durch den Befall des Saatguts während der Lagerung oder nach der Aussaat sowie während und nach der Keimung der Pflanze ausgelöst. Diese Phase ist besonders kritisch, weil die Wurzeln und Schösslinge der wachsenden Pflanze besonders empfindlich sind und auch nur eine kleine Schädigung zum Tod der Pflanze führen kann. Es besteht daher ein großes Interesse daran, das Saatgut und die keimende Pflanze durch Einsatz geeigneter Mittel zu schützen.

Die Bekämpfung von phytopathogenen Pilzen durch die Behandlung des Saatguts von Pflanzen ist seit langem bekannt und ist Gegenstand ständiger Verbesserungen. Dennoch ergeben sich bei der Behandlung von Saatgut eine Reihe von Problemen, die nicht immer zufrieden stellend gelöst werden können. So ist es erstrebenswert, Verfahren zum Schutz des Saatguts und der keimenden Pflanze zu entwickeln, die das zusätzliche Ausbringen von Pflanzenschutzmitteln nach der Saat oder nach dem Auflaufen der Pflanzen überflüssig machen oder zumindest deutlich verringern. Es ist weiterhin erstrebenswert, die Menge des eingesetzten Wirkstoffs dahingehend zu optimieren, dass das Saatgut und die keimende Pflanze vor dem Befall durch phytopathogene Pilze bestmöglich geschützt werden, ohne jedoch die Pflanze selbst durch den eingesetzten Wirkstoff zu schädigen. Insbesondere sollten Verfahren zur Behandlung von Saatgut auch die intrinsischen fungiziden Eigenschaften transgener Pflanzen einbeziehen, um einen optimalen Schutz des Saatguts und der keimenden Pflanze bei einem minimalen Aufwand an Pflanzenschutzmitteln zu erreichen.

Die vorliegende Erfindung bezieht sich daher auch auf ein Verfahren zum Schutz von Saatgut und keimenden Pflanzen vor dem Befall von phytopathogenen Pilzen, indem das Saatgut mit einem erfindungsgemäßen Mittel behandelt wird. Die Erfindung bezieht sich ebenfalls auf die Verwendung der erfindungsgemäßen Mittel zur Behandlung von Saatgut zum Schutz des Saatguts und der keimenden Pflanze vor phytopathogenen Pilzen. Weiterhin bezieht sich die Erfindung auf Saatgut, welches zum Schutz vor phytopathogenen Pilzen mit einem erfindungsgemäßen Mittel behandelt wurde.

Die Bekämpfung von phytopathogenen Pilzen, die Pflanzen nach dem Auflaufen schädigen, erfolgt in erster Linie durch die Behandlung des Bodens und der oberirdischen Pflanzenteile mit Pflanzenschutzmitteln. Aufgrund der Bedenken hinsichtlich eines möglichen Einflusses der Pflanzenschutzmittel auf die Umwelt und die Gesundheit von Menschen und Tieren gibt es Anstrengungen, die Menge der ausgebrachten Wirkstoffe zu vermindern.

Einer der Vorteile der vorliegenden Erfindung ist es, dass aufgrund der besonderen systemischen Eigenschaften der erfindungsgemäßen Wirkstoffe bzw. Mittel die Behandlung des Saatguts mit diesen Wirkstoffen bzw. Mitteln nicht nur das Saatgut selbst, sondern auch die daraus hervorgehenden Pflanzen nach dem Auflaufen vor phytopathogenen Pilzen schützt. Auf diese Weise kann die unmittelbare Behandlung der Kultur zum Zeitpunkt der Aussaat oder kurz danach entfallen.

Ebenso ist es als vorteilhaft anzusehen, dass die erfindungsgemäßen Wirkstoffe bzw. Mittel insbesondere auch bei transgenem Saatgut eingesetzt werden können, wobei die aus diesem Saatgut wachsende Pflanze in der Lage ist, ein Protein zu exprimieren, welches gegen Schädlinge wirkt. Durch die Behandlung solchen Saatguts mit den erfindungsgemäßen Wirkstoffen bzw. Mitteln können bereits durch die Expression des beispielsweise insektiziden Proteins bestimmte Schädlinge bekämpft werden. Überraschenderweise kann dabei ein weiterer synergistischer Effekt beobachtet werden, welcher zusätzlich die Effektivität zum Schutz gegen den Schädlingsbefall vergrößert.

Die erfindungsgemäßen Mittel eignen sich zum Schutz von Saatgut jeglicher Pflanzensorte, die in der Landwirtschaft, im Gewächshaus, in Forsten oder im Garten- und Weinbau eingesetzt wird. Insbesondere handelt es sich dabei um Saatgut von Getreide (wie Weizen, Gerste, Roggen, Triticale, Hirse und Hafer), Mais, Baumwolle, Soja, Reis, Kartoffeln, Sonnenblume, Bohne, Kaffee, Rübe (z.B. Zuckerrübe und Futterrübe), Erdnuss, Raps, Mohn, Olive, Kokosnuss, Kakao, Zuckerrohr, Tabak, Gemüse (wie Tomate, Gurke, Zwiebeln und Salat), Rasen und Zierpflanzen (siehe auch unten). Besondere Bedeutung kommt der Behandlung des Saatguts von Getreide (wie Weizen, Gerste, Roggen, Triticale und Hafer), Mais und Reis zu.

Wie auch weiter unten beschrieben, ist die Behandlung von transgenem Saatgut mit den erfindungsgemäßen Wirkstoffen bzw. Mitteln von besonderer Bedeutung. Dies betrifft das Saatgut von Pflanzen, die wenigstens ein heterologes Gen enthalten, das die Expression eines Polypeptids oder Proteins mit insektiziden Eigenschaften ermöglicht. Das heterologe Gen in transgenem Saatgut kann z.B. aus Mikroorganismen der Arten Bacillus, Rhizobium, Pseudomonas, Serratia, Trichoderma, Clavibacter, Glomus or Gliocladium stammen. Bevorzugt stammt dieses heterologe Gen aus Bacillus sp., wobei das Genprodukt eine Wirkung gegen den Maiszünsler (European corn borer) und/oder Western Corn Rootworm besitzt. Besonders bevorzugt stammt das heterologe Gen aus Bacillus thuringiensis.

Im Rahmen der vorliegenden Erfindung wird das erfindungsgemäße Mittel alleine oder in einer geeigneten Formulierung auf das Saatgut aufgebracht. Vorzugsweise wird das Saatgut in einem Zustand behandelt, in dem so stabil ist, dass keine Schäden bei der Behandlung auftreten. Im Allgemeinen kann die Behandlung des Saatguts zu jedem Zeitpunkt zwischen der Ernte und der Aussaat erfolgen. Üblicherweise wird Saatgut verwendet, das von der Pflanze getrennt und von Kolben, Schalen, Stängeln, Hülle, Wolle oder Fruchtfleisch befreit wurde. So kann zum Beispiel Saatgut verwendet werden, das geerntet, gereinigt und bis zu einem Feuchtigkeitsgehalt von unter 15 Gew.-% getrocknet wurde. Alternativ kann auch Saatgut verwendet werden, das nach dem Trocknen z.B. mit Wasser behandelt und dann erneut getrocknet wurde.

Im Allgemeinen muss bei der Behandlung des Saatguts darauf geachtet werden, dass die Menge des auf das Saatgut aufgebrachten erfindungsgemäßen Mittels und/oder weiterer Zusatzstoffe so gewählt wird, dass die Keimung des Saatguts nicht beeinträchtigt bzw. die daraus hervorgehende Pflanze nicht geschädigt wird. Dies ist vor allem bei Wirkstoffen zu beachten, die in bestimmten Aufwandmengen phytotoxische Effekte zeigen können.

Die erfindungsgemäßen Mittel können unmittelbar aufgebracht werden, also ohne weitere Komponenten zu enthalten und ohne verdünnt worden zu sein. In der Regel ist es vorzuziehen, die Mittel in Form einer geeigneten Formulierung auf das Saatgut aufzubringen. Geeignete Formulierungen und Verfahren für die Saatgutbehandlung sind dem Fachmann bekannt und werden z.B. in den folgenden Dokumenten beschrieben: US 4,272,417 A, US 4,245,432 A, US 4,808,430 A, US 5,876,739 A, US 2003/0176428 A1, WO 2002/080675 A1, WO 2002/028186 A2.

Die erfindungsgemäß verwendbaren Wirkstoffe können in die üblichen Beizmittel-Formulierungen überführt werden, wie Lösungen, Emulsionen, Suspensionen, Pulver, Schäume, Slurries oder andere Hüllmassen für Saatgut, sowie ULV-Formulierungen.

Diese Formulierungen werden in bekannter Weise hergestellt, indem man die Wirkstoffe mit üblichen Zusatzstoffen vermischt, wie zum Beispiel übliche Streckmittel sowie Lösungs- oder Verdünnungsmittel, Farbstoffe, Netzmittel, Dispergiermittel, Emulgatoren, Entschäumer, Konservierungsmittel, sekundäre Verdickungsmittel, Kleber, Gibberelline und auch Wasser.

Als Farbstoffe, die in den erfindungsgemäß verwendbaren Beizmittel-Formulierungen enthalten sein können, kommen alle für derartige Zwecke üblichen Farbstoffe in Betracht. Dabei sind sowohl in Wasser wenig lösliche Pigmente als auch in Wasser lösliche Farbstoffe verwendbar. Als Beispiele genannt seien die unter den Bezeichnungen Rhodamin B, C.I. Pigment Red 112 und C.I. Solvent Red 1 bekannten Farbstoffe.

Als Netzmittel, die in den erfindungsgemäß verwendbaren Beizmittel-Formulierungen enthalten sein können, kommen alle zur Formulierung von agrochemischen Wirkstoffen üblichen, die Benetzung fördernden Stoffe in Frage. Vorzugsweise verwendbar sind Alkylnaphthalin-Sulfonate, wie Diisopropyl- oder Diisobutyl-naphthalin-Sulfonate.

Als Dispergiermittel und/oder Emulgatoren, die in den erfindungsgemäß verwendbaren Beizmittel-Formulierungen enthalten sein können, kommen alle zur Formulierung von agrochemischen Wirkstoffen üblichen nichtionischen, anionischen und kationischen Dispergiermittel in Betracht. Vorzugsweise verwendbar sind nichtionische oder anionische Dispergiermittel oder Gemische von nichtionischen oder anionischen Dispergiermitteln. Als geeignete nichtionische Dispergiermittel sind insbesondere Ethylenoxid-Propylenoxid Blockpolymere, Alkylphenolpolyglykolether sowie Tristryrylphenolpolyglykolether und deren phosphatierte oder sulfatierte Derivate zu nennen. Geeignete anionische Dispergiermittel sind insbesondere Ligninsulfonate, Polyacrylsäuresalze und Arylsulfonat-Formaldehydkondensate.

Als Entschäumer können in den erfindungsgemäß verwendbaren Beizmittel-Formulierungen alle zur Formulierung von agrochemischen Wirkstoffen üblichen schaumhemmenden Stoffe enthalten sein. Vorzugsweise verwendbar sind Silikonentschäumer und Magnesiumstearat.

Als Konservierungsmittel können in den erfindungsgemäß verwendbaren Beizmittel-Formulierungen alle für derartige Zwecke in agrochemischen Mitteln einsetzbaren Stoffe vorhanden sein. Beispielhaft genannt seien Dichlorophen und Benzylalkoholhemiformal.

Als sekundäre Verdickungsmittel, die in den erfindungsgemäß verwendbaren Beizmittel-Formulierungen enthalten sein können, kommen alle für derartige Zwecke in agrochemischen Mitteln einsetzbaren Stoffe in Frage. Vorzugsweise in Betracht kommen Cellulosederivate, Acrylsäurederivate, Xanthan, modifizierte Tone und hochdisperse Kieselsäure.

Als Kleber, die in den erfindungsgemäß verwendbaren Beizmittel-Formulierungen enthalten sein können, kommen alle üblichen in Beizmitteln einsetzbaren Bindemittel in Frage. Vorzugsweise genannt seien Polyvinylpyrrolidon, Polyvinylacetat, Polyvinylalkohol und Tylose.

Als Gibberelline, die in den erfindungsgemäß verwendbaren Beizmittel-Formulierungen enthalten sein können, kommen vorzugsweise die Gibberelline A1, A3 (= Gibberellinsäure), A4 und A7 infrage, besonders bevorzugt verwendet man die Gibberellinsäure. Die Gibberelline sind bekannt (vgl. R. Wegler "Chemie der Pflanzenschutz- und Schädlingsbekämpfungsmittel", Bd. 2, Springer Verlag, 1970, S. 401-412).

Die erfindungsgemäß verwendbaren Beizmittel-Formulierungen können entweder direkt oder nach vorherigem Verdünnen mit Wasser zur Behandlung von Saatgut der verschiedensten Art, auch von Saatgut transgener Pflanzen, eingesetzt werden. Dabei können im Zusammenwirken mit den durch Expression gebildeten Substanzen auch zusätzliche synergistische Effekte auftreten.

Zur Behandlung von Saatgut mit den erfindungsgemäß verwendbaren Beizmittel-Formulierungen oder den daraus durch Zugabe von Wasser hergestellten Zubereitungen kommen alle üblicherweise für die Beizung einsetzbaren Mischgeräte in Betracht. Im einzelnen geht man bei der Beizung so vor, dass man das Saatgut in einen Mischer gibt, die jeweils gewünschte Menge an Beizmittel-Formulierungen entweder als solche oder nach vorherigem Verdünnen mit Wasser hinzufügt und bis zur gleichmäßigen Verteilung der Formulierung auf dem Saatgut mischt. Gegebenenfalls schließt sich ein Trocknungsvorgang an.

Die erfindungsgemäßen Wirkstoffe bzw. Mittel weisen eine starke fungizide Wirkung auf und können zur Bekämpfung von unerwünschten Pilzen im Pflanzenschutz und im Materialschutz eingesetzt werden.

Die erfindungsgemäßen Ketoheteroarylpipenidin und -piperazin Derivate lassen sich im Pflanzenschutz zur Bekämpfung von Plasmodiophoromycetes, Oomycetes, Chytridiomycetes, Zygomycetes, Ascomycetes, Basidiomycetes und Deuteromycetes einsetzen.

Die erfindungsgemäßen fungiziden Mittel können zur Bekämpfung von phytopathogenen Pilzen kurativ oder protektiv eingesetzt werden. Die Erfindung betrifft daher auch kurative und protektive Verfahren zum Bekämpfen von phytopathogenen Pilzen durch die Verwendung der erfindungsgemäßen Wirkstoffe oder Mittel, welche auf das Saatgut, die Pflanze oder Pflanzenteile, die Früchten oder den Boden, in welcher die Pflanzen wachsen, ausgebracht werden.

Die erfindungsgemäßen Mittel zum Bekämpfen von phytopathogenen Pilzen im Pflanzenschutz umfassen eine wirksame, aber nicht-phytotoxische Menge der erfindungsgemäßen Wirkstoffe. "Wirksame, aber nicht-phytotoxische Menge" bedeutet eine Menge des erfindungsgemäßen Mittels, die ausreichend ist, um die Pilzerkrankung der Pflanze ausreichend zu kontrollieren oder ganz abzutöten und die gleichzeitig keine nennenswerten Symptome von Phytotoxizität mit sich bringt. Diese Aufwandmenge kann im Allgemeinen in einem größeren Bereich variieren. Sie hängt von mehreren Faktoren ab, z.B. vom zu bekämpfenden Pilz, der Pflanze, den klimatischen Verhältnissen und den Inhaltsstoffen der erfindungsgemäßen Mittel.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Erfindungsgemäß können alle Pflanzen und Pflanzenteile behandelt werden. Unter Pflanzen werden hierbei alle Pflanzen und Pflanzenpopulationen verstanden, wie erwünschte und unerwünschte Wildpflanzen oder Kulturpflanzen (einschließlich natürlich vorkommender Kulturpflanzen). Kulturpflanzen können Pflanzen sein, die durch konventionelle Züchtungs- und Optimierungsmethoden oder durch biotechnologische und gentechnologische Methoden oder Kombinationen dieser Methoden erhalten werden können, einschließlich der transgenen Pflanzen und einschließlich der durch Sortenschutzrechte schützbaren oder nicht schützbaren Pflanzensorten. Unter Pflanzenteilen sollen alle oberirdischen und unterirdischen Teile und Organe der Pflanzen, wie Spross, Blatt, Blüte und Wurzel verstanden werden, wobei beispielhaft Blätter, Nadeln, Stängel, Stämme, Blüten, Fruchtkörper, Früchte und Samen sowie Wurzeln, Knollen und Rhizome aufgeführt werden. Zu den Pflanzenteilen gehört auch Erntegut sowie vegetatives und generatives Vermehrungsmaterial, beispielsweise Stecklinge, Knollen, Rhizome, Ableger und Samen.

Die erfindungsgemäßen Wirkstoffe eignen sich bei guter Pflanzenverträglichkeit, günstiger Warmblütertoxizität und guter Umweltverträglichkeit zum Schutz von Pflanzen und Pflanzenorganen, zur Steigerung der Ernteerträge, Verbesserung der Qualität des Erntegutes. Sie können vorzugsweise als Pflanzenschutzmittel eingesetzt werden. Sie sind gegen normal sensible und resistente Arten sowie gegen alle oder einzelne Entwicklungsstadien wirksam.

Als Pflanzen, welche erfindungsgemäß behandelt werden können, seien folgende erwähnt: Baumwolle, Flachs, Weinrebe, Obst, Gemüse, wie *Rosaceae sp.* (beispielsweise Kernfrüchte wie Apfel und Birne, aber auch Steinfrüchte wie Aprikosen, Kirschen, Mandeln und Pfirsiche und Beerenfrüchte wie Erdbeeren), *Ribesioidae sp., Juglandaceae sp., Betulaceae sp., Anacardiaceae sp., Fagaceae sp., Moraceae sp., Oleaceae sp., Actinidaceae sp., Lauraceae sp., Musaceae sp.* (beispielsweise Bananenbäume und - plantagen), *Rubiaceae sp.* (beispielsweise Kaffee), *Theaceae sp., Sterculiceae sp., Rutaceae sp.* (beispielsweise Zitronen, Organen und Grapefruit); *Solanaceae sp.* (beispielsweise Tomaten), *Liliaceae sp., Asteraceae sp.* (beispielsweise Salat), *Umbelliferae sp., Cruciferae sp., Chenopodiaceae sp., Cucurbitaceae sp.* (beispielsweise Gurke), *Alliaceae sp.* (beispielsweise Lauch, Zwiebel), *Papilionaceae sp.* (beispielsweise Erbsen); Hauptnutzpflanzen, wie *Gramineae sp.* (beispielsweise Mais, Rasen, Getreide wie Weizen, Roggen, Reis, Gerste, Hafer, Hirse und Triticale), *Poaceae sp.* (z.B. Zuckerrohr), *Asteraceae sp.* (beispielsweise Sonnenblume), *Brassicaceae sp.* (beispielsweise Weißkohl, Rotkohl, Brokkoli, Blumenkohl, Rosenkohl, Pak Choi, Kohlrabi, Radieschen sowie Raps, Senf, Meerrettich und Kresse), *Fabacae sp.* (beispielsweise Bohne, Erdnüsse), *Papilionaceae sp.* (beispielsweise Sojabohne), *Solanaceae sp.* (beispielsweise Kartoffeln), *Chenopodiaceae sp.* (beispielsweise Zuckerrübe, Futterrübe, Mangold, Rote Rübe); Nutzpflanzen und Zierpflanzen in Garten und Wald; sowie jeweils genetisch modifizierte Arten dieser Pflanzen.

Wie bereits oben erwähnt, können erfindungsgemäß alle Pflanzen und deren Teile behandelt werden. In einer bevorzugten Ausführungsform werden wild vorkommende oder durch konventionelle biologische Zuchtmethoden, wie Kreuzung oder Protoplastenfusion erhaltenen Pflanzenarten und Pflanzensorten sowie deren Teile behandelt. In einer weiteren bevorzugten Ausführungsform werden transgene Pflanzen und Pflanzensorten, die durch gentechnologische Methoden gegebenenfalls in Kombination mit konventionellen Methoden erhalten wurden (Genetically Modified Organisms) und deren Teile behandelt. Der Begriff "Teile" bzw. "Teile von Pflanzen" oder "Pflanzenteile" wurde oben erläutert. Besonders bevorzugt werden erfindungsgemäß Pflanzen der jeweils handelsüblichen oder in Gebrauch befindlichen Pflanzensorten behandelt. Unter Pflanzensorten versteht man Pflanzen mit neuen Eigenschaften ("Traits"), die sowohl durch konventionelle Züchtung, durch Mutagenese oder durch rekombinante DNA-Techniken gezüchtet worden sind. Dies können Sorten, Rassen, Bio- und Genotypen sein.

Das erfindungsgemäße Behandlungsverfahren kann für die Behandlung von genetisch modifizierten Organismen (GMOs), z. B. Pflanzen oder Samen, verwendet werden. Genetisch modifizierte Pflanzen (oder transgene Pflanzen) sind Pflanzen, bei denen ein heterologes Gen stabil in das Genom integriert worden ist. Der Begriff "heterologes Gen" bedeutet im wesentlichen ein Gen, das außerhalb der Pflanze bereitgestellt oder assembliert wird und das bei Einführung in das Zellkerngenom, das Chloroplastengenom oder das Mitochondriengenom der transformierten Pflanze dadurch neue oder verbesserte agronomische oder sonstige Eigenschaften verleiht, dass es ein interessierendes Protein oder Polypeptid exprimiert oder dass es ein anderes Gen, das in der Pflanze vorliegt bzw. andere Gene, die in der Pflanze vorliegen, herunterreguliert oder abschaltet (zum Beispiel mittels Antisense-Technologie, Cosuppressionstechnologie oder RNAi-Technologie [RNA Interference]). Ein heterologes Gen, das im Genom vorliegt, wird ebenfalls als Transgen bezeichnet. Ein Transgen, das durch sein spezifisches Vorliegen im Pflanzengenom definiert ist, wird als Transformations- bzw. transgenes Event bezeichnet.

In Abhängigkeit von den Pflanzenarten oder Pflanzensorten, ihrem Standort und ihren Wachstumsbedingungen (Böden, Klima, Vegetationsperiode, Ernährung) kann die erfindungsgemäße Behandlung auch zu überadditiven ("synergistischen") Effekten führen. So sind zum Beispiel die folgenden Effekte möglich, die über die eigentlich zu erwartenden Effekte hinausgehen: verringerte Aufwandmengen und/oder erweitertes Wirkungsspektrum und/oder erhöhte Wirksamkeit der Wirkstoffe und Zusammensetzungen, die erfindungsgemäß eingesetzt werden können, besseres Pflanzenwachstum, erhöhte Toleranz gegenüber hohen oder niedrigen Temperaturen, erhöhte Toleranz gegenüber Trockenheit oder Wasser- oder Bodensalzgehalt, erhöhte Blühleistung, Ernteerleichterung, Reifebeschleunigung, höhere Erträge, größere Früchte, größere Pflanzenhöhe, intensiver grüne Farbe des Blatts, frühere Blüte, höhere Qualität und/oder höherer Nährwert der Ernteprodukte, höhere Zuckerkonzentration in den Früchten, bessere Lagerfähigkeit und/oder Verarbeitbarkeit der Ernteprodukte.

In gewissen Aufwandmengen können die erfindungsgemäßen Wirkstoffkombinationen auch eine stärkende Wirkung auf Pflanzen ausüben. Sie eignen sich daher für die Mobilisierung des pflanzlichen Abwehrsystems gegen Angriff durch unerwünschte phytopathogene Pilze und/oder Mikroorganismen und/oder Viren. Dies kann gegebenenfalls einer der Gründe für die erhöhte Wirksamkeit der erfindungsgemäßen Kombinationen sein, zum Beispiel gegen Pilze. Pflanzenstärkende (resistenzinduzierende) Substanzen sollen im vorliegenden Zusammenhang auch solche Substanzen oder Substanzkombinationen bedeuten, die fähig sind, das pflanzliche Abwehrsystem so zu stimulieren, dass die behandelten Pflanzen, wenn sie im Anschluss daran mit unerwünschten phytopathogenen Pilzen inokuliert wurde, einen beträchtlichen Resistenzgrad gegen diese unerwünschten phytopathogenen Pilze aufweisen. Die erfindungsgemäßen Substanzen lassen sich daher zum Schutz von Pflanzen gegen Angriff durch die erwähnten Pathogene innerhalb eines gewissen Zeitraums nach der Behandlung einsetzen. Der Zeitraum, über den eine Schutzwirkung erzielt wird, erstreckt sich im allgemeinen von 1 bis 10 Tagen, vorzugsweise 1 bis 7 Tagen, nach der Behandlung der Pflanzen mit den Wirkstoffen.

Zu Pflanzen und Pflanzensorten, die vorzugsweise erfindungsgemäß behandelt werden, zählen alle Pflanzen, die über Erbgut verfügen, das diesen Pflanzen besonders vorteilhafte, nützliche Merkmale verleiht (egal, ob dies durch Züchtung und/oder Biotechnologie erzielt wurde).

Pflanzen und Pflanzensorten, die ebenfalls vorzugsweise erfindungsgemäß behandelt werden, sind gegen einen oder mehrere biotische Stressfaktoren resistent, d.h. diese Pflanzen weisen eine verbesserte Abwehr gegen tierische und mikrobielle Schädlinge wie Nematoden, Insekten, Milben, phytopathogene Pilze, Bakterien, Viren und/oder Viroide auf.

Pflanzen und Pflanzensorten, die ebenfalls erfindungsgemäß behandelt werden können, sind solche Pflanzen, die gegen einen oder mehrere abiotische Stressfaktoren resistent sind. Zu den abiotischen Stressbedingungen können zum Beispiel Dürre, Kälte- und Hitzebedingungen, osmotischer Stress, Staunässe, erhöhter Bodensalzgehalt, erhöhtes Ausgesetztsein an Mineralien, Ozonbedingungen, Starklichtbedingungen, beschränkte Verfügbarkeit von Stickstoffnährstoffen, beschränkte Verfügbarkeit von Phosphornährstoffen oder Vermeidung von Schatten zählen.

Pflanzen und Pflanzensorten, die ebenfalls erfindungsgemäß behandelt werden können, sind solche Pflanzen, die durch erhöhte Ertragseigenschaften gekennzeichnet sind. Ein erhöhter Ertrag kann bei diesen Pflanzen z. B. auf verbesserter Pflanzenphysiologie, verbessertem Pflanzenwuchs und verbesserter Pflanzenentwicklung, wie Wasserverwertungseffizienz, Wasserhalteeffizienz, verbesserter Stickstoffverwertung, erhöhter Kohlenstoffassimilation, verbesserter Photosynthese, verstärkter Keimkraft und beschleunigter Abreife beruhen. Der Ertrag kann weiterhin durch eine verbesserte Pflanzenarchitektur (unter Stress- und Nicht-Stress-Bedingungen) beeinflusst werden, darunter frühe Blüte, Kontrolle der Blüte für die Produktion von Hybridsaatgut, Keimpflanzenwüchsigkeit, Pflanzengröße, Internodienzahl und -abstand, Wurzelwachstum, Samengröße, Fruchtgröße, Schotengröße, Schoten- oder Ährenzahl, Anzahl der Samen pro Schote oder Ähre, Samenmasse, verstärkte Samenfüllung, verringerter Samenausfall, verringertes Schotenplatzen sowie Standfestigkeit. Zu weiteren Ertragsmerkmalen zählen Samenzusammensetzung wie Kohlenhydratgehalt, Proteingehalt, Ölgehalt und Ölzusammensetzung, Nährwert, Verringerung der nährwidrigen Verbindungen, verbesserte Verarbeitbarkeit und verbesserte Lagerfähigkeit.

Pflanzen, die erfindungsgemäß behandelt werden können, sind Hybridpflanzen, die bereits die Eigenschaften der Heterosis bzw. des Hybrideffekts exprimieren, was im allgemeinen zu höherem Ertrag, höherer Wüchsigkeit, besserer Gesundheit und besserer Resistenz gegen biotische und abiotische Stressfaktoren führt. Solche Pflanzen werden typischerweise dadurch erzeugt, dass man eine ingezüchtete pollensterile Elternlinie (den weiblichen Kreuzungspartner) mit einer anderen ingezüchteten pollenfertilen Elternlinie (dem männlichen Kreuzungspartner) kreuzt. Das Hybridsaatgut wird typischerweise von den pollensterilen Pflanzen geerntet und an Vermehrer verkauft. Pollensterile Pflanzen können manchmal (z. B. beim Mais) durch Entfahnen (d. h. mechanischem Entfernen der männlichen Geschlechtsorgane bzw. der männlichen Blüten), produziert werden; es ist jedoch üblicher, dass die Pollensterilität auf genetischen Determinanten im Pflanzengenom beruht. In diesem Fall, insbesondere dann, wenn es sich bei dem gewünschten Produkt, da man von den Hybridpflanzen ernten will, um die Samen handelt, ist es üblicherweise günstig, sicherzustellen, dass die Pollenfertilität in Hybridpflanzen, die die für die Pollensterilität verantwortlichen genetischen Determinanten enthalten, völlig restoriert wird. Dies kann erreicht werden, indem sichergestellt wird, dass die männlichen Kreuzungspartner entsprechende Fertilitätsrestorergene besitzen, die in der Lage sind, die Pollenfertilität in Hybridpflanzen, die die genetischen Determinanten, die für die Pollensterilität verantwortlich sind, enthalten, zu restorieren. Genetische Determinanten für Pollensterilität können im Cytoplasma lokalisiert sein. Beispiele für cytoplasmatische Pollensterilität (CMS) wurden zum Beispiel für Brassica-Arten beschrieben. Genetische Determinanten für Pollensterilität können jedoch auch im Zellkerngenom lokalisiert sein. Pollensterile Pflanzen können auch mit Methoden der pflanzlichen Biotechnologie, wie Gentechnik, erhalten werden. Ein besonders günstiges Mittel zur Erzeugung von pollensterilen Pflanzen ist in WO 89/10396 beschrieben, wobei zum Beispiel eine Ribonuklease wie eine Barnase selektiv in den Tapetumzellen in den Staubblättern exprimiert wird. Die Fertilität kann dann durch Expression eines Ribonukleasehemmers wie Barstar in den Tapetumzellen restoriert werden.

Pflanzen oder Pflanzensorten (die mit Methoden der Pflanzenbiotechnologie, wie der Gentechnik, erhalten werden), die erfindungsgemäß behandelt werden können, sind herbizidtolerante Pflanzen, d. h. Pflanzen, die gegenüber einem oder mehreren vorgegebenen Herbiziden tolerant gemacht worden sind. Solche Pflanzen können entweder durch genetische Transformation oder durch Selektion von Pflanzen, die eine Mutation enthalten, die solch eine Herbizidtoleranz verleiht, erhalten werden.

Herbizidtolerante Pflanzen sind zum Beispiel glyphosatetolerante Pflanzen, d. h. Pflanzen, die gegenüber dem Herbizid Glyphosate oder dessen Salzen tolerant gemacht worden sind. So können zum Beispiel glyphosatetolerante Pflanzen durch Transformation der Pflanze mit einem Gen, das für das Enzym 5-Enolpyruvylshikimat-3-phosphatsynthase (EPSPS) kodiert, erhalten werden. Beispiele für solche EPSPS-Gene sind das AroA-Gen (Mutante CT7) des Bakterium *Salmonella typhimurium,* das CP4-Gen des Bakteriums *Agrobacterium sp.,* die Gene, die für eine EPSPS aus der Petunie, für eine EPSPS aus der Tomate oder für eine EPSPS aus Eleusine kodieren. Es kann sich auch um eine mutierte EPSPS handeln. Glyphosatetolerante Pflanzen können auch dadurch erhalten werden, dass man ein Gen exprimiert, das für ein Glyphosate-Oxidoreduktase-Enzym kodiert. Glyphosatetolerante Pflanzen können auch dadurch erhalten werden, dass man ein Gen exprimiert, das für ein Glyphosate-acetyltransferase-Enzym kodiert. Glyphosatetolerante Pflanzen können auch dadurch erhalten werden, dass man Pflanzen, die natürlich vorkommende Mutationen der oben erwähnten Gene selektiert.

Sonstige herbizidresistente Pflanzen sind zum Beispiel Pflanzen, die gegenüber Herbiziden, die das Enzym Glutaminsynthase hemmen, wie Bialaphos, Phosphinotricin oder Glufosinate, tolerant gemacht worden sind. Solche Pflanzen können dadurch erhalten werden, dass man ein Enzym exprimiert, das das Herbizid oder eine Mutante des Enzyms Glutaminsynthase, das gegenüber Hemmung resistent ist, entgiftet. Solch ein wirksames entgiftendes Enzym ist zum Beispiel ein Enzym, das für ein Phosphinotricin-acetyltransferase kodiert (wie zum Beispiel das bar- oder pat-Protein aus Streptomyces-Arten). Pflanzen, die eine exogene Phosphinotricin-acetyltransferase exprimieren, sind beschrieben.

Weitere herbizidtolerante Pflanzen sind auch Pflanzen, die gegenüber den Herbiziden, die das Enzym Hydroxyphenylpyruvatdioxygenase (HPPD) hemmen, tolerant gemacht worden sind. Bei den Hydroxyphenylpyruvatdioxygenasen handelt es sich um Enzyme, die die Reaktion, in der para-Hydroxyphenylpyruvat (HPP) zu Homogentisat umgesetzt wird, katalysieren. Pflanzen, die gegenüber HPPD-Hemmern tolerant sind, können mit einem Gen, das für ein natürlich vorkommendes resistentes HPPD-Enzym kodiert, oder einem Gen, das für ein mutiertes HPPD-Enzym kodiert, transformiert werden. Eine Toleranz gegenüber HPPD-Hemmern kann auch dadurch erzielt werden, dass man Pflanzen mit Genen transformiert, die für gewisse Enzyme kodieren, die die Bildung von Homogentisat trotz Hemmung des nativen HPPD-Enzyms durch den HPPD-Hemmer ermöglichen. Die Toleranz von Pflanzen gegenüber HPPD-Hemmern kann auch dadurch verbessert werden, dass man Pflanzen zusätzlich zu einem Gen, das für ein HPPD-tolerantes Enzym kodiert, mit einem Gen transformiert, das für ein Prephenatdehydrogenase-Enzym kodiert.

Weitere herbizidresistente Pflanzen sind Pflanzen, die gegenüber Acetolactatsynthase (ALS)-Hemmern tolerant gemacht worden sind. Zu bekannten ALS-Hemmern zählen zum Beispiel Sulfonylharnstoff, Imidazolinon, Triazolopyrimidine, Pyrimidinyloxy(thio)benzoate und/oder Sulfonylaminocarbonyltriazolinon-Herbizide. Es ist bekannt, dass verschiedene Mutationen im Enzym ALS (auch als Acetohydroxysäure-Synthase, AHAS, bekannt) eine Toleranz gegenüber unterschiedlichen Herbiziden bzw. Gruppen von Herbiziden verleihen. Die Herstellung von sulfonylharnstofftoleranten Pflanzen und imidazolinontoleranten Pflanzen ist in der internationalen Veröffentlichung WO 1996/033270 beschrieben. Weitere sulfonylharnstoff- und imidazolinontolerante Pflanzen sind auch in z.B. WO 2007/024782 beschrieben.

Weitere Pflanzen, die gegenüber Imidazolinon und/oder Sulfonylharnstoff tolerant sind, können durch induzierte Mutagenese, Selektion in Zellkulturen in Gegenwart des Herbizids oder durch Mutationszüchtung erhalten werden.

Pflanzen oder Pflanzensorten (die nach Methoden der pflanzlichen Biotechnologie, wie der Gentechnik, erhalten wurden), die ebenfalls erfindungsgemäß behandelt werden können, sind insektenresistente transgene Pflanzen, d.h. Pflanzen, die gegen Befall mit gewissen Zielinsekten resistent gemacht wurden.

Solche Pflanzen können durch genetische Transformation oder durch Selektion von Pflanzen, die eine Mutation enthalten, die solch eine Insektenresistenz verleiht, erhalten werden.

Der Begriff "insektenresistente transgene Pflanze" umfasst im vorliegenden Zusammenhang jegliche Pflanze, die mindestens ein Transgen enthält, das eine Kodiersequenz umfasst, die für folgendes kodiert:
1) ein insektizides Kristallprotein aus *Bacillus thuringiensis* oder einen insektiziden Teil davon, wie die insektiziden Kristallproteine, die online bei:
   http://www.lifesci.sussex.ac.uk/Home/Neil_Crickmore/Bt/ beschrieben sind, zusammengestellt wurden, oder insektizide Teile davon, z.B. Proteine der Cry-Proteinklassen Cry1Ab, Cry1Ac, Cry1F, Cry2Ab, Cry3Ae oder Cry3Bb oder insektizide Teile davon; oder
2) ein Kristallprotein aus *Bacillus thuringiensis* oder einen Teil davon, der in Gegenwart eines zweiten, anderen Kristallproteins als *Bacillus thuringiensis* oder eines Teils davon insektizid wirkt, wie das binäre Toxin, das aus den Kristallproteinen Cy34 und Cy35 besteht; oder
3) ein insektizides Hybridprotein, das Teile von zwei unterschiedlichen insektiziden Kristallproteinen aus *Bacillus thuringiensis* umfasst, wie zum Beispiel ein Hybrid aus den Proteinen von 1) oben oder ein Hybrid aus den Proteinen von 2) oben, z. B. das Protein Cry1A.105, das von dem Mais-Event MON98034 produziert wird (WO 2007/027777); oder
4) ein Protein gemäß einem der Punkte 1) bis 3) oben, in dem einige, insbesondere 1 bis 10, Aminosäuren durch eine andere Aminosäure ersetzt wurden, um eine höhere insektizide Wirksamkeit gegenüber einer Zielinsektenart zu erzielen und/oder um das Spektrum der entsprechenden Zielinsektenarten zu erweitern und/oder wegen Veränderungen, die in die Kodier- DNA während der Klonierung oder Transformation induziert wurden, wie das Protein Cry3Bb1 in Mais-Events MON863 oder MON88017 oder das Protein Cry3A im Mais-Event MIR 604;
5) ein insektizides sezerniertes Protein aus *Bacillus thuringiensis* oder *Bacillus cereus* oder einen insektiziden Teil davon, wie die vegetativ wirkenden insektentoxischen Proteine (vegetative insekticidal proteins, VIP), die unter http://www.lifesci.sussex.ac.uk/Home/Neil_Crickmore/Bt/vip.html angeführt sind, z.B. Proteine der Proteinklasse VIP3Aa; oder
6) ein sezerniertes Protein aus *Bacillus thuringiensis* oder *Bacillus cereus,* das in Gegenwart eines zweiten sezernierten Proteins aus *Bacillus thuringiensis* oder *B. cereus* insektizid wirkt, wie das binäre Toxin, das aus den Proteinen VIP1A und VIP2A besteht.
7) ein insektizides Hybridprotein, das Teile von verschiedenen sezernierten Proteinen von *Bacillus thuringiensis* oder *Bacillus cereus* umfasst, wie ein Hybrid der Proteine von 1) oder ein Hybrid der Proteine von 2) oben; oder
8) ein Protein gemäß einem der Punkte 1) bis 3) oben, in dem einige, insbesondere 1 bis 10, Aminosäuren durch eine andere Aminosäure ersetzt wurden, um eine höhere insektizide Wirksamkeit gegenüber einer Zielinsektenart zu erzielen und/oder um das Spektrum der entsprechenden Zielinsektenarten zu erweitern und/oder wegen Veränderungen, die in die Kodier- DNA während der Klonierung oder Transformation induziert wurden (wobei die Kodierung für ein insektizides Protein erhalten bleibt), wie das Protein VIP3Aa im Baumwoll-Event COT 102.

Natürlich zählt zu den insektenresistenten transgenen Pflanzen im vorliegenden Zusammenhang auch jegliche Pflanze, die eine Kombination von Genen umfasst, die für die Proteine von einer der oben genannten Klassen 1 bis 8 kodieren. In einer Ausführungsform enthält eine insektenresistente Pflanze mehr als ein Transgen, das für ein Protein nach einer der oben genannten 1 bis 8 kodiert, um das Spektrum der entsprechenden Zielinsektenarten zu erweitern oder um die Entwicklung einer Resistenz der Insekten gegen die Pflanzen dadurch hinauszuzögern, dass man verschiedene Proteine einsetzt, die für dieselbe Zielinsektenart insektizid sind, jedoch eine unterschiedliche Wirkungsweise, wie Bindung an unterschiedliche Rezeptorbindungsstellen im Insekt, aufweisen.

Pflanzen oder Pflanzensorten (die nach Methoden der pflanzlichen Biotechnologie, wie der Gentechnik, erhalten wurden), die ebenfalls erfindungsgemäß behandelt werden können, sind gegenüber abiotischen Stressfaktoren tolerant. Solche Pflanzen können durch genetische Transformation oder durch Selektion von Pflanzen, die eine Mutation enthalten, die solch eine Stressresistenz verleiht, erhalten werden. Zu besonders nützlichen Pflanzen mit Stresstoleranz zählen folgende:
a. Pflanzen, die ein Transgen enthalten, das die Expression und/oder Aktivität des Gens für die Poly(ADP-ribose)polymerase (PARP) in den Pflanzenzellen oder Pflanzen zu reduzieren vermag.
b. Pflanzen, die ein stresstoleranzförderndes Transgen enthalten, das die Expression und/oder Aktivität der für PARG kodierenden Gene der Pflanzen oder Pflanzenzellen zu reduzieren vermag;
c. Pflanzen, die ein stresstoleranzförderndes Transgen enthalten, das für ein in Pflanzen funktionelles Enzym des Nicotinamidadenindinukleotid-Salvage-Biosynthesewegs kodiert, darunter Nicotinamidase, Nicotinatphosphoribosyltransferase, Nicotinsäuremononukleotidadenyltransferase, Nicotinamidadenindinukleotidsynthetase oder Nicotinamidphosphoribosyltransferase.

Pflanzen oder Pflanzensorten (die nach Methoden der pflanzlichen Biotechnologie, wie der Gentechnik, erhalten wurden), die ebenfalls erfindungsgemäß behandelt werden können, weisen eine veränderte Menge, Qualität und/oder Lagerfähigkeit des Ernteprodukts und/oder veränderte Eigenschaften von bestimmten Bestandteilen des Ernteprodukts auf, wie zum Beispiel:
1) Transgene Pflanzen, die eine modifizierte Stärke synthetisieren, die bezüglich ihrer chemischphysikalischen Eigenschaften, insbesondere des Amylosegehalts oder des Amylose/Amylopektin-Verhältnisses, des Verzweigungsgrads, der durchschnittlichen Kettenlänge, der Verteilung der Seitenketten, des Viskositätsverhaltens, der Gelfestigkeit, der Stärkekorngröße und/oder Stärkekornmorphologie im Vergleich mit der synthetisierten Stärke in Wildtyppflanzenzellen oder -pflanzen verändert ist, so dass sich diese modifizierte Stärke besser für bestimmte Anwendungen eignet.
2) Transgene Pflanzen, die Nichtstärkekohlenhydratpolymere synthetisieren, oder Nichtstärkekohlenhydratpolymere, deren Eigenschaften im Vergleich zu Wildtyppflanzen ohne genetische Modifikation verändert sind. Beispiele sind Pflanzen, die Polyfructose, insbesondere des Inulin- und Levantyps, produzieren, Pflanzen, die alpha-1,4-Glucane produzieren, Pflanzen, die alpha-1,6-verzweigte alpha-1,4-Glucane produzieren und Pflanzen, die Alternan produzieren.
3) Transgene Pflanzen, die Hyaluronan produzieren.

Pflanzen oder Pflanzensorten (die nach Methoden der pflanzlichen Biotechnologie, wie der Gentechnik, erhalten wurden), die ebenfalls erfindungsgemäß behandelt werden können, sind Pflanzen wie Baumwollpflanzen mit veränderten Fasereigenschaften. Solche Pflanzen können durch genetische Transformation oder durch Selektion von Pflanzen, die eine Mutation enthalten, die solche veränderten Fasereigenschaften verleiht, erhalten werden; dazu zählen:
a) Pflanzen wie Baumwollpflanzen, die eine veränderte Form von Cellulosesynthasegenen enthalten,
b) Pflanzen wie Baumwollpflanzen, die eine veränderte Form von rsw2- oder rsw3-homologen Nukleinsäuren enthalten;
c) Pflanzen wie Baumwollpflanzen mit einer erhöhten Expression der Saccharosephosphatsynthase;
d) Pflanzen wie Baumwollpflanzen mit einer erhöhten Expression der Saccharosesynthase;
e) Pflanzen wie Baumwollpflanzen bei denen der Zeitpunkt der Durchlaßsteuerung der Plasmodesmen an der Basis der Faserzelle verändert ist, z. B. durch Herunterregulieren der faserselektiven β-1,3-Glucanase;
f) Pflanzen wie Baumwollpflanzen mit Fasern mit veränderter Reaktivität, z. B. durch Expression des N-Acetylglucosamintransferasegens, darunter auch nodC, und von Chitinsynthasegenen.

Pflanzen oder Pflanzensorten (die nach Methoden der pflanzlichen Biotechnologie, wie der Gentechnik, erhalten wurden), die ebenfalls erfindungsgemäß behandelt werden können, sind Pflanzen wie Raps oder verwandte Brassica-Pflanzen mit veränderten Eigenschaften der Ölzusammensetzung. Solche Pflanzen können durch genetische Transformation oder durch Selektion von Pflanzen, die eine Mutation enthalten, die solche veränderten Öleigenschaften verleiht, erhalten werden; dazu zählen:
a) Pflanzen wie Rapspflanzen, die Öl mit einem hohen Ölsäuregehalt produziere;
b) Pflanzen wie Rapspflanzen, die Öl mit einem niedrigen Linolensäuregehalt produzieren.
c) Pflanzen wie Rapspflanzen, die Öl mit einem niedrigen gesättigten Fettsäuregehalt produzieren.

Besonders nützliche transgene Pflanzen, die erfindungsgemäß behandelt werden können, sind Pflanzen mit einem oder mehreren Genen, die für ein oder mehrere Toxine kodieren, sind die transgenen Pflanzen, die unter den folgenden Handelsbezeichnungen angeboten werden: YIELD GARD® (zum B eispiel Mais, Baumwolle, Sojabohnen), KnockOut® (zum Beispiel Mais), BiteGard® (zum Beispiel Mais), BT-Xtra® (zum Beispiel Mais), StarLink® (zum Beispiel Mais), Bollgard® (Baumwolle), Nucotn® (Baumwolle), Nucotn 33B® (Baumwolle), NatureGard® (zum Beispiel Mais), Protecta® und NewLeaf® (Kartoffel). Herbizidtolerante Pflanzen, die zu erwähnen sind, sind zum Beispiel Maissorten, Baumwollsorten und Sojabohnensorten, die unter den folgenden Handelsbezeichnungen angeboten werden: Roundup Ready® (Glyphosatetoleranz, zum Beispiel Mais, Baumwolle, Sojabohne), Liberty Link® (Phosphinotricintoleranz, zum Beispiel Raps), IMI® (Imidazolinontoleranz) und SCS® (Sylfonylharnstofftoleranz), zum Beispiel Mais. Zu den herbizidresistenten Pflanzen (traditionell auf Herbizidtoleranz gezüchtete Pflanzen), die zu erwähnen sind, zählen die unter der Bezeichnung Clearfield® angebotenen Sorten (zum Beispiel Mais).

Besonders nützliche transgene Pflanzen, die erfindungsgemäß behandelt werden können, sind Pflanzen, die Transformations-Events, oder eine Kombination von Transformations-Events, enthalten und die zum Beispiel in den Dateien von verschiedenen nationalen oder regionalen Behörden angeführt sind (siehe zum Beispiel http://gmoinfo.jrc.it/gmp_browse.aspx und http://www.agbios.com/dbase.php).

Die erfindungsgemäßen Wirkstoffe bzw. Mittel können außerdem im Materialschutz zum Schutz von technischen Materialien gegen Befall und Zerstörung durch unerwünschte Mikroorganismen, wie z.B. Pilzen, eingesetzt werden.

Unter technischen Materialien sind im vorliegenden Zusammenhang nichtlebende Materialien zu verstehen, die für die Verwendung in der Technik zubereitet worden sind. Beispielsweise können technische Materialien, die durch erfindungsgemäße Wirkstoffe vor pilzlicher Veränderung oder Zerstörung geschützt werden sollen, Klebstoffe, Leime, Papier, Wandpappe und Karton, Textilien, Teppiche, Leder, Holz, Anstrichmittel und Kunststoffartikel, Kühlschmierstoffe und andere Materialien sein, die von Mikroorganismen befallen oder zersetzt werden können. Im Rahmen der zu schützenden Materialien seien auch Teile von Produktionsanlagen und Gebäuden, z.B. Kühlwasserkreisläufe, Kühl- und Heizsysteme und Belüftungs- und Klimaanlagen, genannt, die durch Vermehrung von Mikroorganismen beeinträchtigt werden können. Im Rahmen der vorliegenden Erfindung seien als technische Materialien vorzugsweise Klebstoffe, Leime, Papiere und Kartone, Leder, Holz, Anstrichmittel, Kühlschmiermittel und Wärmeübertragungsflüssigkeiten genannt, besonders bevorzugt Holz. Die erfindungsgemäßen Wirkstoffe bzw. Mittel können nachteilige Effekte wie Vermodern, Verfall, Ver-, Entfärbung oder Verschimmeln verhindern. Außerdem können die erfindungsgemäßen Verbindungen zum Schutz vor Bewuchs von Gegenständen, insbesondere von Schiffskörpern, Sieben, Netzen, Bauwerken, Kaianlagen und Signalanlagen, welche mit See-oder Brackwasser in Verbindung kommen, eingesetzt werden.

Das erfindungsgemäße Verfahren zum Bekämpfen von unerwünschten Pilzen kann auch zum Schutz von so genannten Storage Goods verwendet werden. Unter "Storage Goods" werden dabei natürliche Substanzen pflanzlichen oder tierischen Ursprungs oder deren Verarbeitungsprodukte, welche der Natur entnommen wurden und für die Langzeitschutz gewünscht ist, verstanden. Storage Goods pflanzlichen Ursprungs, wie z.B. Pflanzen oder Pflanzenteile, wie Stiele, Blätter, Knollen, Samen, Früchte, Körner, können in frisch geerntetem Zustand oder nach Verarbeitung durch (Vor-)Trocknen, Befeuchten, Zerkleinern, Mahlen, Pressen oder Rösten, geschützt werden. Storage Goods umfasst auch Nutzholz, sei es unverarbeitet, wie Bauholz, Stromleitungsmasten und Schranken, oder in Form fertiger Produkte, wie Möbel. Storage Goods tierischen Ursprungs sind beispielsweise Felle, Leder, Pelze und Haare. Die erfindungsgemäßen Wirkstoffe können nachteilige Effekte wie Vermodern, Verfall, Ver-, Entfärbung oder Verschimmeln verhindern.

Beispielhaft, aber nicht begrenzend, seien einige Erreger von pilzlichen Erkrankungen, die erfindungsgemäß behandelt werden können, genannt:
Erkrankungen, hervorgerufen durch Erreger des Echten Mehltaus wie z.B. Blumeria-Arten, wie beispielsweise Blumeria graminis; Podosphaera-Arten, wie beispielsweise Podosphaera leucotricha; Sphaerotheca-Arten, wie beispielsweise Sphaerotheca fuliginea; Uncinula-Arten, wie beispielsweise Uncinula necator;
Erkrankungen, hervorgerufen durch Erreger von Rostkrankheiten wie z.B. Gymnosporangium-Arten, wie beispielsweise Gymnosporangium sabinae; Hemileia-Arten, wie beispielsweise Hemileia vastatrix; Phakopsora-Arten, wie beispielsweise Phakopsora pachyrhizi und Phakopsora meibomiae; Puccinia-Arten, wie beispielsweise Puccinia recondita oder Puccinia triticina; Uromyces-Arten, wie beispielsweise Uromyces appendiculatus;
Erkrankungen, hervorgerufen durch Erreger der Gruppe der Oomyceten wie z.B. Bremia-Arten, wie beispielsweise Bremia lactucae; Peronospora-Arten, wie beispielsweise Peronospora pisi oder P. brassicae; Phytophthora-Arten, wie beispielsweise Phytophthora infestans; Plasmopara-Arten, wie beispielsweise Plasmopara viticola; Pseudoperonospora-Arten, wie beispielsweise Pseudoperonospora humuli oder Pseudoperonospora cubensis; Pythium-Arten, wie beispielsweise Pythium ultimum;
Blattfleckenkrankheiten und Blattwelken, hervorgerufen durch z.B. Alternaria-Arten, wie beispielsweise Alternaria solani; Cercospora-Arten, wie beispielsweise Cercospora beticola; Cladiosporum-Arten, wie beispielsweise Cladiosporium cucumerinum; Cochliobolus-Arten, wie beispielsweise Cochliobolus sativus (Konidienform: Drechslera, Syn: Helminthosporium); Colletotrichum-Arten, wie beispielsweise Colletotrichum lindemuthanium; Cycloconium-Arten, wie beispielsweise Cycloconium oleaginum; Diaporthe-Arten, wie beispielsweise Diaporthe citri; Elsinoe-Arten, wie beispielsweise Elsinoe fawcettii; Gloeosporium-Arten, wie beispielsweise Gloeosporium laeticolor; Glomerella-Arten, wie beispielsweise Glomerella cingulata; Guignardia-Arten, wie beispielsweise Guignardia bidwelli; Leptosphaeria-Arten, wie beispielsweise Leptosphaeria maculans; Magnaporthe-Arten, wie beispielsweise Magnaporthe grisea; Microdochium-Arten, wie beispielsweise Microdochium nivale; Mycosphaerella-Arten, wie beispielsweise Mycosphaerella graminicola und M. fijiensis; Phaeosphaeria-Arten, wie beispielsweise Phaeosphaeria nodorum; Pyrenophora-Arten, wie beispielsweise Pyrenophora teres; Ramularia-Arten, wie beispielsweise Ramularia collo-cygni; Rhynchosporium-Arten, wie beispielsweise Rhynchosporium secalis; Septoria-Arten, wie beispielsweise Septoria apii; Typhula-Arten, wie beispielsweise Typhula incarnata; Venturia-Arten, wie beispielsweise Venturia inaequalis;
Wurzel- und Stängelkrankheiten, hervorgerufen durch z.B. Corticium-Arten, wie beispielsweise Corticium graminearum; Fusarium-Arten, wie beispielsweise Fusarium oxysporum; Gaeumannomyces-Arten, wie beispielsweise Gaeumannomyces graminis; Rhizoctonia-Arten, wie beispielsweise Rhizoctonia solani; Tapesia-Arten, wie beispielsweise Tapesia acuformis; Thielaviopsis-Arten, wie beispielsweise Thielaviopsis basicola;
Ähren- und Rispenerkrankungen (inklusive Maiskolben), hervorgerufen durch z.B. Alternaria-Arten, wie beispielsweise Alternaria spp.; Aspergillus-Arten, wie beispielsweise Aspergillus flavus; Cladosporium-Arten, wie beispielsweise Cladosporium cladosporioides; Claviceps-Arten, wie beispielsweise Claviceps purpurea; Fusarium-Arten, wie beispielsweise Fusarium culmorum; Gibberella-Arten, wie beispielsweise Gibberella zeae; Monographella-Arten, wie beispielsweise Monographella nivalis; Septoria-Arten, wie beispielsweise Septoria nodorum;
Erkrankungen, hervorgerufen durch Brandpilze wie z.B. Sphacelotheca-Arten, wie beispielsweise Sphacelotheca reiliana; Tilletia-Arten, wie beispielsweise Tilletia caries, T. controversa; Urocystis-Arten, wie beispielsweise Urocystis occulta; Ustilago-Arten, wie beispielsweise Ustilago nuda, U. nuda tritici;
Fruchtfäule hervorgerufen durch z.B. Aspergillus-Arten, wie beispielsweise Aspergillus flavus; Botrytis-Arten, wie beispielsweise Botrytis cinerea; Penicillium-Arten, wie beispielsweise Penicillium expansum und P. purpurogenum; Sclerotinia-Arten, wie beispielsweise Sclerotinia sclerotiorum;
Verticilium-Arten, wie beispielsweise Verticilium alboatrum;
Samen- und bodenbürtige Fäulen und Welken, sowie Sämlingserkrankungen, hervorgerufen durch z.B. Fusarium-Arten, wie beispielsweise Fusarium culmorum; Phytophthora Arten, wie beispielsweise Phytophthora cactorum; Pythium-Arten, wie beispielsweise Pythium ultimum; Rhizoctonia-Arten, wie beispielsweise Rhizoctonia solani; Sclerotium-Arten, wie beispielsweise Sclerotium rolfsii;
Krebserkrankungen, Gallen und Hexenbesen, hervorgerufen durch z.B. Nectria-Arten, wie beispielsweise Nectria galligena;
Welkeerkrankungen hervorgerufen durch z.B. Monilinia-Arten, wie beispielsweise Monilinia laxa;
Deformationen von Blättern, Blüten und Früchten, hervorgerufen durch z.B. Taphrina-Arten, wie beispielsweise Taphrina deformans;
Degenerationserkrankungen holziger Pflanzen, hervorgerufen durch z.B. Esca-Arten, wie beispielsweise Phaemoniella clamydospora und Phaeoacremonium aleophilum und Fomitiporia mediterranea;
Blüten- und Samenerkrankungen, hervorgerufen durch z.B. Botrytis-Arten, wie beispielsweise Botrytis cinerea;
Erkrankungen von Pflanzenknollen, hervorgerufen durch z.B. Rhizoctonia-Arten, wie beispielsweise Rhizoctonia solani; Helminthosporium-Arten, wie beispielsweise Helminthosporium solani;
Erkrankungen, hervorgerufen durch bakterielle Erreger wie z.B. Xanthomonas-Arten, wie beispielsweise Xanthomonas campestris pv. oryzae; Pseudomonas-Arten, wie beispielsweise Pseudomonas syringae pv. lachrymans; Erwinia-Arten, wie beispielsweise Erwinia amylovora.

Bevorzugt können die folgenden Krankheiten von Soja-Bohnen bekämpft werden:

Pilzkrankheiten an Blättern, Stängeln, Schoten und Samen verursacht durch z.B. Alternaria leaf spot (Alternaria spec. atrans tenuissima), Anthracnose (Colletotrichum gloeosporoides dematium var. truncatum), Brown spot (Septoria glycines), Cercospora leaf spot and blight (Cercospora kikuchii), Choanephora leaf blight (Choanephora infundibulifera trispora (Syn.)), Dactuliophora leaf spot (Dactuliophora glycines), Downy Mildew (Peronospora manshurica), Drechslera blight (Drechslera glycini), Frogeye Leaf spot (Cercospora sojina), Leptosphaerulina Leaf Spot (Leptosphaerulina trifolii), Phyllostica Leaf Spot (Phyllosticta sojaecola), Pod and Stem Blight (Phomopsis sojae), Powdery Mildew (Microsphaera diffusa), Pyrenochaeta Leaf Spot (Pyrenochaeta glycines), Rhizoctonia Aerial, Foliage, and Web Blight (Rhizoctonia solani), Rust (Phakopsora pachyrhizi, Phakopsora meibomiae), Scab (Sphaceloma glycines), Stemphylium Leaf Blight (Stemphylium botryosum), Target Spot (Corynespora cassiicola).

Pilzkrankheiten an Wurzeln und der Stängelbasis verursacht durch z.B. Black Root Rot (Calonectria crotalariae), Charcoal Rot (Macrophomina phaseolina), Fusarium Blight or Wilt, Root Rot, and Pod and Collar Rot (Fusarium oxysporum, Fusarium orthoceras, Fusarium semitectum, Fusarium equiseti), Mycoleptodiscus Root Rot (Mycoleptodiscus terrestris), Neocosmospora (Neocosmopspora vasinfecta), Pod and Stem Blight (Diaporthe phaseolorum), Stem Canker (Diaporthe phaseolorum var. caulivora), Phytophthora Rot (Phytophthora megasperma), Brown Stem Rot (Phialophora gregata), Pythium Rot (Pythium aphanidermatum, Pythium irregulare, Pythium debaryanum, Pythium myriotylum, Pythium ultimum), Rhizoctonia Root Rot, Stem Decay, and Damping-Off (Rhizoctonia solani), Sclerotinia Stem Decay (Sclerotinia sclerotiorum), Sclerotinia Southern Blight (Sclerotinia rolfsii), Thielaviopsis Root Rot (Thielaviopsis basicola).

Als Organismen, die einen Abbau oder eine Veränderung der technischen Materialien bewirken können, seien Pilze genannt. Vorzugsweise wirken die erfindungsgemäßen Wirkstoffe gegen Pilze, insbesondere Schimmelpilze, Holz verfärbende und Holz zerstörende Pilze (Basidiomyceten). Es seien beispielsweise Pilze der folgenden Gattungen genannt: Alternaria, wie Alternaria tenuis; Aspergillus, wie Aspergillus niger; Chaetomium, wie Chaetomium globosum; Coniophora, wie Coniophora puetana; Lentinus, wie Lentinus tigrinus; Penicillium, wie Penicillium glaucum; Polyporus, wie Polyporus versicolor; Aureobasidium, wie Aureobasidium pullulans; Sclerophoma, wie Sclerophoma pityophila; Trichoderma, wie Trichoderma viride.

Darüber hinaus weisen die erfindungsgemäßen Wirkstoffe auch sehr gute antimykotische Wirkungen auf. Sie besitzen ein sehr breites antimykotisches Wirkungsspektrum, insbesondere gegen Dermatophyten und Sprosspilze, Schimmel und diphasische Pilze (z.B. gegen Candida-Spezies wie Candida albicans, Candida glabrata) sowie Epidermophyton floccosum, Aspergillus-Spezies wie Aspergillus niger und Aspergillus fumigatus, Trichophyton-Spezies wie Trichophyton mentagrophytes, Microsporon-Spezies wie Microsporon canis und audouinii. Die Aufzählung dieser Pilze stellt keinesfalls eine Beschränkung des erfassbaren mykotischen Spektrums dar, sondern hat nur erläuternden Charakter.

Beim Einsatz der erfindungsgemäßen Wirkstoffe als Fungizide können die Aufwandmengen je nach Applikationsart innerhalb eines größeren Bereiches variiert werden. Die Aufwandmenge der erfindungsgemäßen Wirkstoffe beträgt
bei der Behandlung von Pflanzenteilen, z.B. Blättern: von 0,1 bis 10 000 g/ha, bevorzugt von 10 bis 1 000 g/ha, besonders bevorzugt von 50 bis 300g/ha (bei Anwendung durch Gießen oder Tropfen kann die Aufwandmenge sogar verringert werden, vor allem wenn inerte Substrate wie Steinwolle oder Perlit verwendet werden);
bei der Saatgutbehandlung: von 2 bis 200 g pro 100 kg Saatgut, bevorzugt von 3 bis 150 g pro 100 kg Saatgut, besonders bevorzugt von 2,5 bis 25 g pro 100 kg Saatgut, ganz besonders bevorzugt von 2,5 bis 12,5 g pro 100 kg Saatgut;
bei der Bodenbehandlung: von 0,1 bis 10 000 g/ha, bevorzugt von 1 bis 5 000 g/ha.

Diese Aufwandmengen seien nur beispielhaft und nicht limitierend im Sinne der Erfindung genannt.

Die erfindungsgemäßen Wirkstoffe bzw. Mittel können also eingesetzt werden, um Pflanzen innerhalb eines gewissen Zeitraumes nach der Behandlung gegen den Befall durch die genannten Schaderreger zu schützen. Der Zeitraum, innerhalb dessen Schutz herbeigeführt wird, erstreckt sich im Allgemeinen auf 1 bis 28 Tage, bevorzugt auf 1 bis 14 Tage, besonders bevorzugt auf 1 bis 10 Tage, ganz besonders bevorzugt auf 1 bis 7 Tage nach der Behandlung der Pflanzen mit den Wirkstoffen bzw. auf bis zu 200 Tage nach einer Saatgutbehandlung.

Darüber hinaus kann durch die erfindungsgemäße Behandlung der Mykotoxingehalt im Erntegut und den daraus hergestellten Nahrungs- und Futtermitteln verringert werden. Besonders, aber nicht ausschließlich sind hierbei folgende Mykotoxine zu nennen: Deoxynivalenol (DON), Nivalenol, 15-Ac-DON, 3-Ac-DON, T2- und HT2- Toxin, Fumonisine, Zearalenon, Moniliformin, Fusarin, Diaceotoxyscirpenol (DAS), Beauvericin, Enniatin, Fusaroproliferin, Fusarenol, Ochratoxine, Patulin, Mutterkorn-alkaloide und Aflatoxine, die beispielsweise von den folgenden Pilzen verursacht werden können: Fusarium spec., wie Fusarium acuminatum, F. avenaceum, F. crookwellense, F. culmorum, F. graminearum (Gibberella zeae), F. equiseti, F. fujikoroi, F. musarum, F. oxysporum, F. proliferatum, F. poae, F. pseudograminearum, F. sambucinum, F. scirpi, F. semitectum, F. solani, F. sporotrichoides, F. langsethiae, F. subglutinans, F. tricinctum, F. verticillioides u.a. sowie auch von Aspergillus spec., Penicillium spec., Claviceps purpurea, Stachybotrys spec. u.a.

Die aufgeführten Pflanzen können besonders vorteilhaft erfindungsgemäß mit den Ketoheteroarylpiperidin und -piperazin Derivaten der Formel (I) oder den erfindungsgemäßen Mitteln behandelt werden. Die bei den Wirkstoffen bzw. Mitteln oben angegebenen Vorzugsbereiche gelten auch für die Behandlung dieser Pflanzen. Besonders hervorgehoben sei die Pflanzenbehandlung mit den im vorliegenden Text speziell aufgeführten Verbindungen bzw. Mitteln.

Die Herstellung und die Verwendung der erfindungsgemäßen Wirkstoffe der Formeln (I) geht aus den folgenden Beispielen hervor. Die Erfindung ist jedoch nicht auf diese Beispiele beschränkt.

Allgemeines: Wenn nicht anders angegeben, werden alle chromatografischen Reinigungs- bzw. Trennungsschritte an Kieselgel und mit einem Lösungsmittelgradienten von 0:100 Essigsäure-ethylester/Cyclohexan zu 100:0 Essigsäure-ethylester/Cyclohexan durchgeführt.

### Beispiele:

### Herstellung von (I-24):

### Schritt 1

### tert-Butyl-4-{4-[methoxy(methyl)carbamoyl]-1,3-thiazol-2-yl}piperidin-1-carboxylat (V-1)

Zu einer Suspension von 2-[1-(*tert*-Butoxycarbonyl)piperidin-4-yl]-1,3-thiazol-4-carbonsäure (1.0 g) in Dichlormethan (30 mL) wurde bei Raumtemperatur Triethylamin (324 mg) gegeben. Nach zehnminütigem Rühren wurden Methoxy(methyl)ammoniumchlorid (312 mg), 4-Dimethylaminopyridin (39 mg) und 1-Ethyl-3-(3'-dimethylaminopropyl)carbodiimide-HCl Salz (675 mg) dazu gegeben. Das Gemisch wurde über Nacht bei Raumtemperatur gerührt und dann mit Wasser versetzt. Die wässrige Phase wurde abgetrennt und mit Ethylacetat extrahiert. Die vereinigten organischen Phasen werden über Natriumsulfat getrocknet und unter vermindertem Druck eingeengt. Der Rückstand wurde chromatographisch gereinigt. Man erhielt *tert*-Butyl-4-{4-[methoxy(methyl)carbamoyl]-1,3-thiazol-2-yl}piperidin-1-carboxylat (1.0 g).
logP (pH2.7): 2.40
¹H NMR (DMSO-d₆, 400 MHz): δₚₚₘ: 1.41 (s, 9H), 1.52-1.63 (m, 2H), 2.02-2.08 (m, 2H), 2.90 (bs, 2H), 3.19-3.30 (1H), 3.29 (s, 3H), 3.32 (s, 3H), 3.96-4.04 (m, 2H), 8.09 (s, 1H)
MS (ESI): 356 ([M+H]⁺)

### Schritt 2

### tert-Butyl-4-[4-(cyclohexylacetyl)-1,3-thiazol-2-yl]piperidin-1-carboxylat (IV-1)

Zu einer Lösung von *tert*-Butyl-4-{4-[methoxy(methyl)carbamoyl]-1,3-thiazol-2-yl}piperidin-1-carboxylat (480 mg) in Tetrahydrofuran (5 mL) wurde Cyclohexylmethylmagnesiumchlorid 0.5M in Diethylether, (2.7 mL) bei -78 °C unter Argon getropft. Das Reaktionsgemisch wurde eine Stunde bei -78 °C gerührt. Das Reaktionsgemisch wurde dann eine Stunde bei Raumtemperatur gerührt. Das Reaktionsgemisch wurde anschließend mit gesättigter Ammoniumchloridlösung versetzt und die wässrige Phase abgetrennt. Nach Extraktion der wässrigen Phase mit Essigsäureethylester wurden die vereinigten organischen Phasen über Natriumsulfat getrocknet und das Lösungsmittel unter vermindertem Druck entfernt. Man erhielt *tert*-Butyl-4-[4-(cyclohexylacetyl)-1,3-thiazol-2-yl]piperidin-1-carboxylat (335 mg).
logP (pH2.7): 5.43
¹H NMR (DMSO-d₆, 400 MHz): δₚₚₘ: 0.98-1.05 (m, 2H), 1.14-1.22 (m, 1H), 1.22-1.31 (m, 2H), 1.44 (s, 9H), 1.61-1.76 (m, 8H), 2.05-2.10 (m, 2H). 2.87 (d, 2H), 2.90 (bs, 2H), 3.18-3.26 (m, 1H), 4.07-4.15 (m, 2H), 8.10 (s, 1H)
MS (ESI): 337 ([M-(CH₃)₃C+H]⁺)

### Schritt 3

### 4-[4-(Cyclohexylacetyl)-1,3-thiazol-2-yl]piperidiniumchlorid (II-1)

Zu einer Suspension von *tert*-Butyl-4-[4-(cyclohexylacetyl)-1,3-thiazol-2-yl]piperidin-1-carboxylat (5.0 g) in Diethylether (1 mL) wurde unter Argon und bei 0 °C eine Lösung von Chlorwasserstoff in Dioxan (4M, 1 mL) gegeben. Das Gemisch wurde bei 0 °C gerührt und danach langsam auf Raumtemperatur erwärmt. Nach Rühren über Nacht wurden die überschüssige Säure und das Lösungsmittel unter vermindertem Druck entfernt. Man erhielt 4-[4-(Cyclohexylacetyl)-1,3-thiazol-2-yl]piperidiniumchlorid (315 mg).
logP (pH2.7): 1.35
MS (ESI): 293 ([M-Cl]⁺)

### Schritt 4

### 2-[3,5-Bis(difluormethyl)-1H-pyrazol-1-yl]-1-{4-[4-(cyclohexylacetyl)-1,3-thiazol-2-yl]piperidin-1-yl}ethanon (I-24)

Zu einer Lösung von [3,5-(Difluormethyl)-1H-pyrazol-1-yl]essigsäure (69 mg) in Dichlormethan (5 mL) wurde Oxalylchlorid (116 mg) und ein Tropfen N,N-Dimethylformamid gegeben. Dann wurde das Reaktionsgemisch für 30 Minuten gerührt. Dann wurde der Überschuss Oxalylchlorid unter vermindertem Druck entfernt und der Rückstand wurde wieder in Dichlormethan (2 mL) gelöst. Die Lösung wurde dann zu einer Lösung aus 4-[4-(Cyclohexylacetyl)-1,3-thiazol-2-yl]piperidiniumchlorid (100 mg) in Dichlormethan (5 mL) und Triethylamin (92 mg) gegeben Die Reaktionsmischung wurde 30 Minuten gerührt. Das Lösungsmittel und Triethylamin wurden unter vermindertem Druck entfernt. Nach säulenchromatographischer Reinigung des Rückstandes, erhielt **man** 2-[3,5-Bis(difluormethyl)-1H-pyrazol-1-yl]-1-{4-[4-(cyclohexylacetyl)-1,3-thiazol-2-yl]piperidin-1-yl}ethanon (105 mg).
logP (pH2.7): 4.02
¹H NMR (DMSO-d₆, 400 MHz): δₚₚₘ: 0.95-1.02 (m, 2H), 1.10-1.26 (m, 3H), 1.54-1.69 (m, 6H), 1.77-1.91 (m, 2H), 2.06-2.15 (m, 2H), 2.82-2.87 (m, 1H), 2.89 (d, 2H), 3.25-3.30 (m, 1H), 3.35-3.41 (m, 1H), 3.94-3.99 (m, 1H), 4.33-4.37 (m, 1H), 5.36 (d, 1H), 5.45 (d, 1H), 6.91 (s, 1H), 7.04 (t, 1H), 7.18 (t, 1H), 8.44 (s, 1H)
MS (ESI): 501 ([M+H]⁺)

### Herstellung von Verbindung (I-25)

### Schritt 1

### N-(5-Chlor-2-methylphenyl)-4-[4-(cyclohexylacetyl)-1,3-thiazol-2-yl]piperidin-1-carboxamid (I-25)

Zu einer Mischung von 4-[4-(Cyclohexylacetyl)-1,3-thiazol-2-yl]piperidiniumchlorid (100 mg) in Dichlormethan (1 mL) wurde Triethylamin (0.047 mL) gegeben. Nachdem der Feststoff vollständig in Lösung gegangen war, wurden 4-Chlor-2-isocyanato-1-methylbenzol (51 mg) und ein Tropfen 1,8-Diazabicyclo[5.4.0]undec-7-en dazu gegeben. Dann wurde das Reaktionsgemisch für 5 Minuten gerührt. Das Lösungsmittel und Triethylamin wurden unter vermindertem Druck entfernt. Nach säulenchromatographischer Reinigung des Rückstandes, erhielt man N-(5-Chlor-2-methylphenyl)-4-[4-(cyclohexylacetyl)-1,3-thiazol-2-yl]piperidin-1-carboxamid (128 mg).
logP (pH2.7): 4.51
1H NMR (DMSO-d₆, 400 MHz): δₚₚₘ: 0.94-1.05 (m, 2H), 1.10-1.26 (m, 4H), 1.56-1.73 (m, 6H), 1.83-1.92 (m, 1H), 2.06-2.13 (m, 2H), 2.16 (s, 3H), 2.88 (d, 2H), 2.98-3.06 (m, 2H), 3.30-3.38 (m, 1H), 4.11-4.18 (m, 2H), 7.07 (dd, 1H), 7.19 (d, 1H), 7.33 (d, 1H), 8.12 (s, 1H), 8.04 (s, 1H)
MS (ESI): 460 ([M+H]⁺)

### Herstellung von Verbindung (I-58)

### Schritt 1

### 1-[2-(1-{2-[3,5-Bis(difluormethyl)-1H-pyrazol-1-yl]ethanthioyl}piperidin-4-yl)-1,3-thiazol-4-yl]-2-cyclohexylethanon (I-58)

Zu einer Lösung von 2-[3,5-Bis(difluormethyl)-1H-pyrazol-1-yl]-1-{4-[4-(cyclohexylacetyl)-1,3-thiazol-2-yl]piperidin-1-yl}ethanon (200 mg) in Toluen (2 mL), wurde bei Raumtemperatur 2,4-Bis(4-methoxyphenyl)-1,3,2,4-dithiadiphosphetan-2,4-disulfid (194 mg) gegeben. Die Reaktionsmischung wurde 2 Stunden bei 60 °C gerührt. Nach Entfernen des Lösungsmittels unter vermindertem Druck wurde der Rückstand chromatographisch gereinigt. Man erhielt t 1-[2-(1-{2-[3,5-Bis(difluormethyl)-1H-pyrazol-1-yl]ethanthioyl}piperidin-4-yl)-1,3-thiazol-4-yl]-2-cyclohexylethanon (101 mg, 49 %).
logP (pH2.7): 4.63
1H NMR (DMSO-d₆, 400 MHz): δₚₚₘ: 0.94-1.05 (m, 2H), 1.10-1.30 (m, 4H), 1.56-1.80 (m, 6H), 1.83-1.95 (m, 1H), 2.06-2.15 (m, 2H), 2.88 (d, 2H), 3.30-3.42 (m, 1H), 3.51-3.61 (m, 2H), 4.42-4.49 (m, 1H), 5.26-5.33 (m, 1H), 5.55 (d, 1H), 5.59 (d, 1H), 6.89 (s, 1H), 7.02 (t, 1H), 7.20 (t, 1H), 8.04 (s, 1H)
MS (ESI): 517 ([M+H]⁺)

### Herstellung von Verbindung (I-60)

### Schritt 1

### tert-Butyl-4-[4-(2-cyclohexyl-N-methoxyethanimidoyl)-1,3-thiazol-2-yl]piperidin-1-carboxylat

Zu einer Lösung von *tert*-Butyl-4-{4-[methoxy(methyl)carbamoyl]-1,3-thiazol-2-yl}piperidin-1-carboxylat (403 mg) in Ethanol (2 mL) wurde Methoxyammoniumchlorid (171 mg) bei Raumtemperatur gegeben. Die Reaktionsmischung wurde bei 50 °C für 24 Stunden gerührt dann wurde mit Wasser versetzt. Die wässrige Phase wurde abgetrennt und mit Ethylacetat extrahiert. Die vereinigten organischen Phasen werden über Natriumsulfat getrocknet und unter vermindertem Druck eingeengt. Der Rückstand wurde chromatographisch gereinigt. Man erhielt *tert*-Butyl-4-[4-(2-cyclohexyl-N-methoxyethanimidoyl)-1,3-thiazol-2-yl]piperidin-1-carboxylat (361 mg).

### Schritt 2

### 2-[3,5-Bis(difluormethyl)-1H-pyrazol-1-yl]-1-{4-[4-(2-cyclohexyl-N-methoxyethanimidoyl)-1,3-thiazol-2-yl]piperidin-1-yl}ethanon (I-60)

Zu einer Suspension von *tert*-Butyl-4-[4-(2-cyclohexyl-N-methoxyethanimidoyl)-1,3-thiazol-2-yl]piperidin-1-carboxylat (287 mg) wurde unter Argon und bei 0 °C eine Lösung von Chlorwasserstoff in Dioxan (4M, 2.6 mL) gegeben. Das Gemisch wurde bei 0 °C gerührt und danach langsam auf Raumtemperatur erwärmt. Nach Rühren über Nacht wurden die überschüssige Säure und das Lösungsmittel unter vermindertem Druck entfernt. Man erhielt 4-[4-(2-Cyclohexyl-N-methoxyethanimidoyl)-1,3-thiazol-2-yl]piperidiniumchlorid.

Zu **einer Lösung aus** 4-[4-(2-Cyclohexyl-N-methoxyethanimidoyl)-1,3-thiazol-2-yl]piperidiniumchlorid (357 mg) in Dichlormethan (3 mL) und Triethylamin (101 mg) wurde bei Raumtemperatur [3,5-(Difluormethyl)-1H-pyrazol-1-yl]essigsäure (225 mg), Dimethylaminopyridin (12 mg) und 1-Ethyl-3-(3'-dimethylaminopropyl)carbodiimide (89 mg) gegeben. Das Gemisch wurde für 3 Stunden gerührt und dann mit Wasser versetzt. Die wässrige Phase wurde abgetrennt und mit Ethylacetat extrahiert. Die vereinigten organischen Phasen wurden mit Natriumsulfat getrocknet. Der Feststoff wurde abfiltriert und das Lösungsmittel abdestilliert. Der Rückstand wird chromatographisch gereinigt. Man erhielt 2-[3,5-Bis(difluormethyl)-1H-pyrazol-1-yl]-1-{4-[4-(2-cyclohexyl-N-methoxyethanimidoyl)-1,3-thiazol-2-yl]piperidin-1-yl}ethanon (182 mg, 70%).
logP (pH2.7): 4.80
MS (ESI): 530 ([M+H]⁺)

### Herstellung von Verbindung (I-14)

### Schritt 1

### tert-Butyl-4-[4-(1-hydroxy-3-phenylpropyl)-1,3-thiazol-2-yl]piperidin-1-carboxylat (XVI-1)

Zu einer Lösung von *tert*-Butyl-4-(4-formyl-1,3-thiazol-2-yl)piperidin-1-carboxylat (1.0 g) in Tetrahydrofuran (10 mL) wurde bei -78 °C unter Argonatmosphäre Chlor(2-phenylethyl)magnesium (1M in Diethylether, 3.77 mL) getropft. Das Reaktionsgemisch wurde eine Stunde bei -78 °C gerührt und dann wurde zusätzlich Chlor(2-phenylethyl)magnesium (3M in Diethylether, 0.20 mL) zugetropft. Das Reaktionsgemisch wurde dann für 20 Minuten gerührt. Das Reaktionsgemisch wurde anschließend mit gesättigter Ammoniumchloridlösung versetzt und die wässrige Phase abgetrennt. Nach Extraktion der wässrigen Phase mit Essigsäureethylester wurden die vereinigten organischen Phasen über Natriumsulfat getrocknet und das Lösungsmittel unter vermindertem Druck eingeengt. Man erhielt *tert*-Butyl-4-[4-(1-hydroxy-3-phenylpropyl)-1,3-thiazol-2-yl]piperidin-1-carboxylat (790 mg).
logP (pH2.7): 3.79
¹H NMR (DMSO-d₆, 400 MHz): δₚₚₘ: 1.41 (s, 9H), 1.48-1.61 (m, 2H), 1.90-2.11 (4H), 2.62-2.67 (m, 2H), 2.87-2.95 (m, 2H), 3.15-3.20 (m, 1H), 3.94-4.00 (2H), 4.62-4.67 (m, 1H), 5.09-5.12 (m, 1H), 7.11-7.18 (m, 3H), 7.22-7.27 (m, 3H)
MS (ESI): 347 ([M-C(CH₃)₃+2H]⁺)

### Schritt 2

### tert-Butyl-4-[4-(3-phenylpropanoyl)-1,3-thiazol-2-yl]piperidin-1-carboxylat (IV-2)

Zu einer Lösung von *tert*-Butyl-4-[4-(1-hydroxy-3-phenylpropyl)-1,3-thiazol-2-yl]piperidin-1-carboxylat (790 mg) in Dichlormethan (7.9 mL) wurde bei Raumtemperatur 1,1,1-triacetoxy-1,1-dihydro-1,2-benziodoxol-3(1H)-one (15%ige) Lösung in Dichlormethan, 8.3 g) getropft. Das Reaktionsgemisch wurde über Nacht bei Raumtemperatur gerührt. Dann wurde 5 g Silicagel zugegeben und das Lösungsmittel unter Vakuum entfernt. Der Rückstand wurde chromatographisch gereinigt. Man erhielt *tert*-Butyl-4-[4-(3-phenylpropanoyl)-1,3-thiazol-2-yl]piperidin-1-carboxylat (545 mg).
logP (pH2.7): 4.54
¹H NMR (DMSO-d₆, 400 MHz): δₚₚₘ: 1.41 (s, 9H), 1.54-1.65 (m, 2H), 2.00-2.08 (m, 2H), 2.88-2.97 (m, 4H), 3.20-3.30 (m, 1H), 3.31 (t, 2H), 3.96-4.02 (m, 2H), 7.12-7.18 (m, 1H), 7.21-7.28 (m, 4H), 8.35 (s, 1H)
MS (ESI): 345 ([M-C(CH₃)₃+2H]⁺)

### Schritt 3

### 4-[4-(3-Phenylpropanoyl)-1,3-thiazol-2-yl]piperidiniumchlorid (II-2)

*tert*-Butyl-4-[4-(3-phenylpropanoyl)-1,3-thiazol-2-yl]piperidin-1-carboxylat (550 mg) wurde analog zu **II-1 (Schritt 3)** mit Chlorwasserstoff in Diethylether (2M, 11 mL) umgesetzt. Man erhielt 4-[4-(3-Phenylpropanoyl)-1,3-thiazol-2-yl]piperidiniumchlorid (500 mg).
logP (pH2.7): 1.19
¹H NMR (DMSO-d₆, 400 MHz): δₚₚₘ: 1.95-2.05 (m, 2H), 2.18-2.25 (m, 2H), 2.96 (t, 2H), 3.00-3.08 (m, 2H), 3.29-3.36 (m, 4H), 3.36-3.46 (m, 1H), 7.13-7.18 (m, 1H), 7.22-7.30 (m, 4H), 8.40 (s, 1H), 8.99 (bs, 1H), 9.22 (bs, 1H)
MS (ESI): 301 ([M-C1]⁺)

### Schritt 4

### 1-[2-(1-{[5-Methyl-3-(trifluormethyl)-1H-pyrazol-1-yl]acetyl}piperidin-4-yl)-1,3-thiazol-4-yl]-3-phenylpropan-1-on

4-[4-(3-Phenylpropanoyl)-1,3-thiazol-2-yl]piperidiniumchlorid (502 mg) wurde analog zu **I-24** (**Schritt 4**) mit [5-Methyl-3-(trifluormethyl)-1H-pyrazol-1-yl]essigsäure (310 mg) umgesetzt. Man erhielt 1-[2-(1-{[5-Methyl-3-(trifluormethyl)-1H-pyrazol-1-yl]acetyl}piperidin-4-yl)-1,3-thiazol-4-yl]-3-phenylpropan-1-on (495 mg).
logP (pH2.7): 3.64
¹H NMR (DMSO-d₆, 400 MHz): δₚₚₘ: 1.65 (bs, 1H), 1.80 (bs, 1H), 2.08-2.17 (m, 2H), 2.22 (s, 3H), 2.90 (bs, 1H), 2.96 (t, 2H), 3.20-3.42 (m, 2H), 3.33 (t, 2H), 3.99 (bs, 1H), 4.33 (bs, 1H), 5.15-5.25 (m, 2H), 6.44 (s, 1H), 7.14-7.18 (m, 1H), 7.21-7.30 (m, 4H), 8.37 (s, 1H)
MS (ESI): 491 ([M+H]⁺)

### Herstellung von Verbindung (I-12)

### Schritt 1

### 1-[2-(Piperidin-4-yl)-1,3-thiazol-4-yl]ethanonhydrochlorid

Zu einer Lösung von *tert*-Butyl-4-(4-acetyl-1,3-thiazol-2-yl)piperidin-1-carboxylat (920 mg) in Diethylether (2 mL), wurde tropfenweise unter Argonatmosphäre bei 0 °C Salzsäure (2 M in Diethylether, 23 mL) gegeben. Die Reaktionsmischung wurde 24 Stunden lang gerührt. Das Lösungsmittel und die überschüssige Säure wurden unter vermindertem Druck entfernt. Man erhielt 1-[2-(Piperidin-4-yl)-1,3-thiazol-4-yl]ethanonhydrochlorid (1.05 g) als einen weißen sehr hygrospkopischen Feststoff, der sofort weiterverarbeitet wurde.
¹H NMR (DMSO-d₆, 400 MHz): δₚₚₘ : 2.01 (qd, 2H), 2.28-2.20 (m, 2H), 2.55 (s, 3H), 3.02 (q, 2H), 3.38-3.27 (m, 2H), 3.42 (m, 1H), 8.39 (s, 1H), 9.06 (bs, 1H), 9.25 (bs, 1H)
MS (ESI): 211 ([M+H-Cl]⁺)

### Schritt 2

### 1-[4-(4-Acetyl-1,3-thiazol-2-yl)piperidin-1-yl]-2-[5-methyl-3-(trifluormethyl)-1H-pyrazol-1-yl]ethanon (XXII-1)

Zu einer Lösung von [5-Methyl-3-(trifluormethyl)-1H-pyrazol-1-yl]essigsäure (1.00 g) in Dichlormethan (10 mL) wurde Oxalylchlorid (1.74 g) und ein Tropfen N,N-Dimethylformamid gegeben. Dann wurde das Reaktionsgemisch für 24 Stunden gerührt. Dann wurde der Überschuss Oxalylchlorid unter vermindertem Druck entfernt und der Rückstand wurde wieder in Dichlormethan (10 mL) gelöst. Die Lösung wurde dann unter Kühlung mit einem Eisbad zu einer Suspension aus 1-[2-(Piperidin-4-yl)-1,3-thiazol-4-yl]ethanonhydrochlorid (1.13 g) in Dichlormethan (10 mL) und N,N-Diisopropylethylamin (1.77 g) gegeben. Die Reaktionsmischung ließ man dann auf Raumtemperatur erwärmen und rührte weitere 2 Stunden. Dann wurde gesättigte, wässrige Ammoniumchlorid-Lösung (5 mL) zu dem Reaktionsgemisch gegeben. Die wässrige Phase wurde abgetrennt und mit Dichlormethan extrahiert. Die gesamten organischen Phasen wurden vereint und mit wasserfreiem Natriumsulfat getrocknet. Anschließend wurde der Feststoff abfiltriert und das Lösungsmittel unter reduziertem Druck entfernt. Nach säulenchromatographischer Reinigung (Kieselgel, Ethylacetate: Hexan 0% - 100% Elutionsgradient) erhielt man1-[4-(4-Acetyl-1,3-thiazol-2-yl)piperidin-1-yl]-2-[5-methyl-3-(trifluormethyl)-1H-pyrazol-1-yl]ethanon (1.00 g)
logP (pH2.7): 2.25
¹H NMR (DMSO-d₆, 400 MHz): δₚₚₘ : 1.65 (bs, 1H), 1.80 (bs, 1H), 2.18-2.11 (m, 2H), 2.23 (s, 3H), 2.55 (s, 3H), 2.90 (bs, 1H), 3.28 (bs, 1H), 3.39 (m, 1H), 4.00 (bs, 1H), 4.33 (bs, 1H), 5.22 (bs, 2H), 6.45 (s, 1H), 8.36 (s, 1H)
MS (ESI): 401 ([M+H]⁺)

### Schritt 3

### (2E)-3-(2,6-Difluorphenyl)-1-[2-(1-{[5-methyl-3-(trifluormethyl)-1H-pyrazol-1-yl]acetyl}piperidin-4-yl)-1,3-thiazol-4-yl]prop-2-en-1-on (I-12)

**Zu einer Lösung von** 1-[4-(4-Acetyl-1,3-thiazol-2-yl)piperidin-1-yl]-2-[5-methyl-3-(trifluormethyl)-1H-pyrazol-1-yl]ethanon (190 mg) und 2,6-Difluorbenzaldehyd (67 mg) in Methanol (0.12 mL) wurde einer Lösung von Natriumhydroxid (19 mg) in Methanol (0.25 mL) und Wasser (0.05 mL) bei Raumtemperatur gegeben. Das Reaktionsgemisch wurde über Nacht bei Raumtemperatur gerührt. Nach wässriger Aufarbeitung wurde mit Essigsäureethylester extrahiert, die Extrakte mit Natriumsulfat getrocknet und unter vermindertem Druck eingeengt. Der Rückstand wurde chromatografisch gereinigt. Man erhielt (2E)-3-(2,6-Difluorphenyl)-1-[2-(1-{[5-methyl-3-(trifluormethyl)-1H-pyrazol-1-yl]acetyl}piperidin-4-yl)-1,3-thiazol-4-yl]prop-2-en-1-on (160 mg).
logP (pH2.7): 3.83
¹H NMR (DMSO-d₆, 400 MHz): δₚₚₘ: 1.56-1.68 (m, 1H), 1.78-1.91 (m, 1H), 2.10-2.21 (m, 2H), 2.22 (s, 3H), 2.84-2.92 (m, 1H), 3.20-3.50 (m, 2H), 3.95-4.05 (m, 1H), 4.36-4.92 (m, 1H), 5.23 (d, 1H), 5.34 (d, 1H), 6.50 (s, 1H), 7.26 (d, 1H), 7.28 (d, 1H), 7.58 (m, 1H), 7.81 (d, 1H), 8.07 (d, 1H), 8.626 (s, 1H)
MS (ESI): 525 ([M+H]⁺)

### Herstellung von Verbindung (I-11)

### Schritt 1

### tert-Butyl-4-(4-ethinyl-1,3-thiazol-2-yl)piperidin-1-carboxylat (VI-1)

*tert*-Butyl-4-(4-formyl-1,3-thiazol-2-yl)piperidin-1-carboxylat (600 mg) wurde unter Argon in Methanol gelöst und mit Kaliumcarbonat (839 mg) und Dimethyl-1-diazo-2-oxopropylphosphonat (786 mg) versetzt. Das Gemisch wurde bei Raumtemperatur für 3 Stunden gerührt. Nach wässriger Aufarbeitung wuirde mit Essigsäure-ethylester extrahiert, die Extrakte mit Natriumsulfat getrocknet und unter vermindertem Druck das Lösungsmittel eingeengt. Der Rückstand wurde chromatografisch gereinigt. Man erhielt *tert*-Butyl-4-(4-ethinyl-1,3-thiazol-2-yl)piperidin-1-carboxylat (493 mg).
logP (pH2.7): 3.10
¹H NMR (CD₃CN): δ 7.51 (s, 1H), 4.07 (d, 2H), 3.36 (s, 1H), 3.16 (m, 1H), 2.90 (t, 2H), 2.10-2.00 (m, 2H), 1.65 (qd, 2H), 1.43 (s, 9H) ppm
MS (ESI): 237 ([M+2H-C(CH₃)₃]⁺)

### Schritt 2

### 1-[4-(4-Ethinyl-1,3-thiazol-2-yl)piperidin-1-yl]-2-[5-methyl-3-(trifluormethyl)-1H-pyrazol-1-yl]ethanon (XX-1)

Zu *tert*-Butyl-4-(4-ethinyl-1,3-thiazol-2-yl)piperidin-1-carboxylat (100 mg) wurde eine Lösung von Trifluoressigsäure (30% in Dichlormethan, 2 mL) bei Raumtemperatur gegeben. Nach 30 minütigem Rühren wurde das Reaktionsgemisch mit Triethylamin (2 mL) versetzt.

Zu einer Lösung von [5-Methyl-3-(trifluormethyl)-1H-pyrazol-1-yl]essigsäure (71 mg) in Dichlormethan (2 mL) wurde Oxalylchlorid (130 mg) und ein Tropfen N,N-Dimethylformamid gegeben. Dann wurde das Reaktionsgemisch für 30 Minuten gerührt. Dann wurde der Überschuss Oxalylchlorid unter vermindertem Druck entfernt und der Rückstand wurde wieder in Dichlormethan (2 mL) gelöst. Die Lösung wurde dann zu der ersteren Lösung gegeben. Die Reaktionsmischung wurde für 1 Stunde gerührt. Das Lösungsmittel und Triethylamin wurden unter vermindertem Druck entfernt und danach der Rückstand chromatographisch gereinigt wird. Man erhielt 1-[4-(4-Ethinyl-1,3-thiazol-2-yl)piperidin-1-yl]-2-[5-methyl-3-(trifluormethyl)-1H-pyrazol-1-yl]ethanon (130 mg).
logP (pH2.7): 2.61
¹H NMR (DMSO-d₆, 400 MHz): δₚₚₘ: 1.60 (bs, 1H), 1.76 (bs, 1H), 2.05-2.12 (m, 2H), 2.22 (s, 3H), 2.88 (bs, 1H), 3.20-3.35 (m, 2H), 3.98 (bs, 1H), 4.08 (s, 1H), 4.32 (bs, 1H), 5.20 (bs, 2H), 6.45 (s, 1H), 7.86 (s, 1H)
MS (ESI): 383 ([M+H]⁺)

### Schritt 3

### 1-(2-Ethoxyphenyl)-3-[2-(1-{[5-methyl-3-(trifluormethyl)-1H-pyrazol-1-yl]acetyl}piperidin-4-yl)-1,3-thiazol-4-yl]prop-2-in-1-on (I-11)

**Zu einer Lösung von** 1-[4-(4-Ethinyl-1,3-thiazol-2-yl)piperidin-1-yl]-2-[5-methyl-3-(trifluormethy1)-1H-pyrazol-1-yl]ethanon (250 mg) und 2-Ethoxybenzoylchlorid in Tetrahydrofuran wuirde Palladium(II)-chlorid (5.8 mg) und Kupfer(I)-iodid bei Raumtemperatur unter Argonatmosphäre gegeben. Das Gemisch wurde bei Raumtemperatur für 1 Minute gerührt, bevor Triethylamin (83 mg) dazu gegeben wurde. Das Reaktionsgemisch wurde über Nacht bei Raumtemperatur gerührt. Dann wurden 5 g Silicagel zugegeben und das Lösungsmittel unter Vakuum entfernt. Der Rückstand wurde säulenchromatographisch gereinigt. Man erhielt 1-(2-Ethoxyphenyl)-3-[2-(1-{[5-methyl-3-(trifluormethyl)-1H-pyrazol-1-yl]acetyl}piperidin-4-yl)-1,3-thiazol-4-yl]prop-2-in-1-on (244 mg).
logP (pH2.7): 3.78
¹H NMR (CD₃CN, 400 MHz): δₚₚₘ: 1.44 (t, 3H), 1.65-1.95 (m, 2H), 2.12-2.21 (m, 2H), 2.24 (s, 3H), 2.90 (bs, 1H), 3.25-3.38 (m, 2H), 3.95 (bs, 1H), 4.22 (q, 2H), 4.43 (1H), 5.04 (bs, 2H), 6.37 (s, 1H), 7.02-7.18 (m, 2H), 7.54-7.60 (m, 1H), 7.87 (dd, 1H), 7.89 (s, 1H)
MS (ESI): 531 ([M+H]⁺)

### Herstellung von Verbindung (I-20)

### Schritt 1

### 1-(2-Ethoxyphenyl)-3-[2-(1-{[5-methyl-3-(trifluormethyl)-1H-pyrazol-1-yl]acetyl}piperidin-4-yl)-1,3-thiazol-4-yl]propan-1-on (I-20)

1-(2-Ethoxyphenyl)-3-[2-(1-{[5-methyl-3-(trifluormethyl)-1H-pyrazol-1-yl]acetyl}piperidin-4-yl)-1,3-thiazol-4-yl]prop-2-in-1-on (130 mg) wurde in Methanol gelöst und bei 40 °C, einem Druck von 10 bar H₂ und in Anwesenheit von Pd/C (10%) hydriert. Nach Filtration und Entfernung des Lösungsmittels unter vermindertem Druck erhielt man 1-(2-Ethoxyphenyl)-3-[2-(1-{[5-methyl-3-(trifluormethyl)-1H-pyrazol-1-yl]acetyl}piperidin-4-yl)-1,3-thiazol-4-yl]propan-1-on (40 mg).
logP (pH2.7): 3.70
1H NMR (CD₃CN, 400 MHz): δₚₚₘ: 1.44 (t, 3H), 1.58-1.70 (m, 1H), 1.75-1.86 (m, 1H), 2.06-2.18 (m, 2H), 2.25 (s, 3H), 2.82-2.90 (m, 1H), 3.09 (t, 2H), 3.20-3.32 (m, 2H), 3.42 (t, 2H), 3.88-3.95 (m, 1H), 4.16 (q, 2H), 4.42-4.47 (m, 1H), 5.04 (d, 1H), 5.11 (d, 1H), 6.42 (s, 1H), 6.95 (s, 1H), 7.01 (t, 1H), 7.09 (d, 1H), 7.47-7.53 (m, 1H), 5.59 (dd, 1H)
MS (ESI): 535 ([M+H]⁺)

### Herstellung von Verbindung (I-22)

### Schritt 1

### tert-Butyl-4-{4-[(1E)-3-(2,6-difluorphenyl)-3-oxoprop-1-en-1-yl]-1,3-thiazol-2-yl}piperidin-1-carboxylat (IV-3)

*tert*-Butyl-4-(4-formyl-1,3-thiazol-2-yl)piperidin-1-carboxylat (500 mg) wurde analog zu **I-12** (**Schritt 3**) mit 1-(2,6-Difluorphenyl)ethanon (263 mg) umgesetzt. Man erhielt *tert*-Butyl-4-{4-[(1E)-3-(2,6-difluorphenyl)-3-oxoprop-1-en-1-yl]-1,3-thiazol-2-yl}piperidin-1-carboxylat (720 mg).
logP (pH2.7): 4.30
¹H NMR (DMSO-d₆, 400 MHz): δₚₚₘ:1.41 (s, 9H), 1.51-1.61 (m, 2H), 2.00-2.08 (m, 2H), 2.90 (bs, 2H), 3.80-4.04 (m, 3H), 7.15 (d, 1H), 7.27 (t, 2H), 7.47 (d, 1H), 7.60-7.70 (m, 1H), 8.16 (s, 1H)
MS (ESI): 335 ([M-C=OOC(CH₃)₃+2H]⁺)

### Schritt 2

### (2E)-3-[2-(1-{[3,5-Bis(difluormethyl)-1H-pyrazol-1-yl]acetyl}piperidin-4-yl)-1,3-thiazol-4-yl]-1-(2,6-difluorphenyl)prop-2-en-1-on (I-22)

*tert*-Butyl-4-{4-[(1E)-3-(2,6-difluorphenyl)-3-oxoprop-1-en-1-yl]-1,3-thiazol-2-yl}piperidin-1-carboxylat (720 mg) wurde analog zu **II-1** (**Schritt 3**) umgesetzt. Man erhielt 4-{4-[(1E)-3-(2,6-Difluorphenyl)-3-oxoprop-1-en-1-yl]-1,3-thiazol-2-yl}piperidiniumchlorid (800 mg).

4-{4-[(1E)-3-(2,6-Difluorphenyl)-3-oxoprop-1-en-1-yl]-1,3-thiazol-2-yl}piperidiniumchlorid (300 mg) wurde analog zu **I-24** (**Schritt 4**) mit [3,5-Bis(difluormethyl)-1H-pyrazol-1-yl]essigsäure (183 mg) umgesetzt. Man erhielt (2E)-3-[2-(1-{[3,5-Bis(difluormethyl)-1H-pyrazol-1-yl]acetyl}piperidin-4-yl)-1,3-thiazol-4-yl]-1-(2,6-difluorphenyl)prop-2-en-1-on (100 mg).
logP (pH2.7): 3.35
¹H NMR (DMSO-d₆, 400 MHz): δₚₚₘ: 1.51-1.63 (m, 1H), 1.75-1.88 (m, 1H), 2.05-2.16 (m, 2H), 2.80-2.88 (m, 1H), 3.20-3.42 (m, 2H), 3.92-4.01 (m, 1H), 4.30-4.46 (m, 1H), 5.35 (d, 1H), 5.44 (d. 1H), 6.88 (s, 1H), 7.04 (t, 1H), 7.15-7.20 (m, 1H), 7.18 (t, 1H), 7.24-7.32 (m, 2H), 7.49 (d, 1H), 7.60-7.70 (m, 1H), 8.17 (s, 1H)
MS (ESI): 543 ([M-H]⁺)

Analog zu den zuvor angegebenen Methoden können die in der nachstehenden Tabelle 1 aufgeführten Verbindungen der Formel (I) erhalten werden:

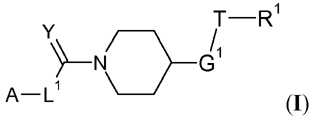

Für alle Beispiele von Tabelle 1 ist G¹ = 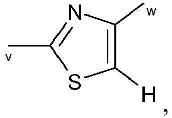 wobei die Bindung, die mit "v" identifiziert ist, direkt an den Piperidin-Ring gebunden ist, und wobei die Bindung, die mit "w" identifiziert ist, direkt an T gebunden ist. Die mit "*" identifizierte Bindung von T ist an G¹ gebunden, die mit "#" identifizierte Bindung ist an R¹ gebunden.

**Tabelle 1:**

| Bsp. | A | L¹ | Y | T | R¹ | logp |
|---|---|---|---|---|---|---|
| 1-1 | 5-Methyl-3-(trifluormethyl)-1H-pyrazol-1-yl | CH₂ | O | *-C≡C-CO-# | Phenyl | 3.58^{[a]} |
| I-2 | 5-Methyl-3-(trifluormethyl)-1H-pyrazol-1-yl | CH₂ | O | *-C≡C-CO-# | 2-Methoxyphenyl | 3.35^{[a]}; 3.36^{[b]} |
| I-3 | 5-Methyl-3-(trifluormethyl)-1H-pyrazol-1-yl | CH₂ | O | *-C≡C-CO-# | Naphthalen-1-yl | 4.25^{[a]} |
| I-4 | 5-Methyl-3-(trifluormethyl)-1H-pyrazol-1-yl | CH₂ | O | *-C≡C-CO-# | Cyclohexyl | 4.15^{[a]} |
| I-5 | 5-Methyl-3-(trifluormethyl)-1H-pyrazol-1-yl | CH₂ | O | *-C≡C-CO-# | 2-Chlorphenyl | 3.79^{[a]} |
| I-6 | 5-Methyl-3-(trifluormethyl)-1H-pyrazol-1-yl | CH₂ | O | *-C≡C-CO-# | 2,4-Dichlorphenyl | 4.31^{[a]} |
| I-7 | 5-Methyl-3-(trifluormethyl)-1H-pyrazol-1-yl | CH₂ | O | *-C≡C-CO-# | 4-Methoxyphenyl | 3.54^{[a]} |
| I-8 | 5-Methyl-3-(trifluormethyl)-1H-pyrazol-1-yl | CH₂ | O | *-C≡C-CO-# | tert-Butyl | 3.68^{[a]} |
| I-9 | 5-Methyl-3-(trifluormethyl)-1H-pyrazol-1-yl | CH₂ | O | *-C≡C-CO-# | Thiophen-2-yl | 3.29^{[a]} |
| I-10 | 5-Methyl-3-(trifluormethyl)-1H-pyrazol-1-yl | CH₂ | O | *-C≡C-CO-# | Furan-2-yl | 2.92^{[a]} |
| I-11 | 5-Methyl-3-(trifluormethyl)-1H-pyrazol-1-yl | CH₂ | O | *-C≡C-CO-# | 2-Ethoxyphenyl | 3.76^{[a]} |
| I-12 | 5-Methyl-3-(trifluormethyl)-1H-pyrazol-1-yl | CH₂ | O | *-CO-CH=CH-[E]-# | 2,6-Difluorphenyl | 3.83^{[a]} |
| I-13 | 5-Methyl-3-(trifluormethyl)-1H-pyrazol-1-yl | CH₂ | O | *-CO-CH₂-# | 2-Bromphenyl | 3.67^{[a]} 3.71^{[b]} |
| I-14 | 5-Methyl-3-(trifluormethyl)-1H-pyrazol-1-yl | CH₂ | O | *-COCH₂CH₂-# | Phenyl | 3.64^{[a]} |
| I-15 | 5-Methyl-3-(trifluormethyl)-1H-pyrazol-1-yl | CH₂ | O | *-CO-CH₂-C(CH₃)₂-# | Phenyl | 4.08^{[a]} |
| I-16 | 5-Methyl-3-(trifluormethyl)-1H-pyrazol-1-yl | CH₂ | O | *-CO-CH₂-# | Phenyl | 3.36^{[a]} |
| I-17 | 5-Methyl-3-(trifluormethyl)-1H-pyrazol-1-yl | CH₂ | O | *-CO-CH₂-# | Cyclohexyl | 4.13^{[a]}; 4.24^{[b]} |
| I-18 | 5-Methyl-3-(trifluormethyl)-1H-pyrazol-1-yl | CH₂ | O | *-CO-CH₂-# | Naphthalen-1-yl | 3.87^{[a]} |
| I-19 | 5-Methyl-3-(trifluormethyl)-1H-pyrazol-1-yl | CH₂ | O | *-CH₂CH₂-CO-# | 2-Methoxyphenyl | 3.34^{[a]} |
| I-20 | 5-Methyl-3-(trifluormethyl)-1H-pyrazol-1-yl | CH₂ | O | *-CH₂CH₂-CO-# | 2-Ethoxyphenyl | 3.7^{[a]}; 3.68^{[b]} |
| I-21 | 5-Methyl-3-(trifluormethyl)-1H-pyrazol-1-yl | CH₂ | O | *-CH₂CH₂-CO-# | 2-Chlorphenyl | 3.6^{[a]}; 3.63^{[b]} |
| I-22 | 3,5-Bis(difluormethyl)-1H-pyrazol-1-yl | CH₂ | O | *-CH=CH-CO-[E]-# | 2,6-Difluorphenyl | 3.35^{[a]} |
| I-23 | 2,5-Dimethylphenyl | NH | O | *-CH=CH-CO-[E]-# | 2,6-Difluorphenyl | 3.56^{[a]} |
| I-24 | 3,5-Bis(difluormethyl)-1H-pyrazol-1-yl | CH₂ | O | *-CO-CH₂-# | Cyclohexyl | 4.02^{[a]}; 4.09^{[b]} |
| I-25 | 5-Chlor-2-methylphenyl | NH | O | *-CO-CH₂-# | Cyclohexyl | 4.51^{[a]}; 4.55^{[b]} |
| I-26 | 5-Methyl-3-(trifluormethyl)-1H-pyrazol-1-yl | CH₂ | O | *-C(Cl)=CHCO-[E and/or Z]-# | Phenyl | 4^{[a]} |
| I-29 | 3,5-Bis(difluormethyl)-1H-pyrazol-1-yl | CH₂ | O | *-CH=CHCO-[E]-# | 2-Bromphenyl | 3.51^{[a]}; 3.49^{[b]} |
| I-30 | 2,5-Bis(difluormethyl)phenyl | -NH- | O | *-COCH2-# | Cyclohexyl | 4.3^{[a]}; 4.3^{[b]} |
| I-33 | 3,5-Bis(difluormethyl)-1H-pyrazol-1-yl | CH₂ | O | *-CH=CHCO-[E]-# | 2-Iodphenyl | 3.65^{[a]}; 3.64^{[b]} |
| I-34 | 3,5-Bis(difluormethyl)-1H-pyrazol-1-yl | CH₂ | O | *-CH=CHCO-[E]-# | 2-Brom-4-fluorphenyl | 3.7^{[a]}; 3.68^{[b]} |
| I-35 | 3,5-Bis(difluormethyl)-1H-pyrazol-1-yl | CH₂ | O | *-CH=CHCO-[E]-# | 2-Chlor-5-fluorphenyl | 3.64^{[a]}; 3.6^{[b]} |
| I-36 | 3,5-Bis(difluormethyl)-1H-pyrazol-1-yl | CH₂ | O | *-CH=CHCO-[E]-# | 2-Chlor-6-fluorphenyl | 3.54^{[a]}; 3.51^{[b]} |
| I-37 | 3,5-Bis(difluormethyl)-1H-pyrazol-1-yl | CH₂ | O | *-CH=CHCO-[E]-# | Phenyl | 3.31^{[a]}; 3.31^{[b]} |
| I-38 | 3,5-Bis(difluormethyl)-1H-pyrazol-1-yl | CH₂ | O | *-CH=CHCO-[E]-# | 3-Methylphenyl | 3.64^{[a]}; 3.63^{[b]} |
| I-39 | 3,5-Bis(difluormethyl)-1H-pyrazol-1-yl | CH₂ | O | *-CH=CHCO-[E]-# | 2,6-Dimethoxyphenyl | 3.04^{[a]} |
| I-40 | 3,5-Bis(difluormethyl)-1H-pyrazol-1-yl | CH₂ | O | *-CH=CHCO-[E]-# | 4-fluorophenyl | 3.43^{[a]} |
| I-41 | 3,5-Bis(difluormethyl)-1H-pyrazol-1-yl | CH₂ | O | *-CH=CHCO-[E]-# | 3-Fluorphenyl | 3.48^{[a]} |
| I-42 | 3,5-Bis(difluormethyl)-1H-pyrazol-1-yl | CH₂ | O | *-CH=CHCO-[E]-# | 2-Chlorphenyl | 3.51^{[a]} |
| I-43 | 3,5-Bis(difluormethyl)-1H-pyrazol-1-yl | CH₂ | O | *-CH=CHCO-[E]-# | 2,6-Dichlorphenyl | 3.7^{[a]} |
| I-44 | 3,5-Bis(difluormethyl)-1H-pyrazol-1-yl | CH₂ | O | *-CH=CHCO-[E]-# | Cyclohexyl | 3.89^{[a]} |
| I-45 | 3,5-Bis(difluormethyl)-1H-pyrazol-1-yl | CH₂ | O | *-CH=CHCO-[E]-# | 2-Methylphenyl | 3.56^{[a]} |
| I-46 | 3,5-Bis(difluormethyl)-1H-pyrazol-1-yl | CH₂ | O | *-CH=CHCO-[E]-# | Naphthalen-1-yl | 2.84^{[a]} |
| I-47 | 3,5-Bis(difluormethyl)-1H-pyrazol-1-yl | CH₂ | O | *-CH=CHCO-[E]-# | tert-Butyl | 3.45^{[a]} |
| I-48 | 3,5-Bis(difluormethyl)-1H-pyrazol-1-yl | CH₂ | O | *-CH=CHCO-[E]-# | 2-Fluor-4-methoxyphenyl | 3.45^{[a]} |
| I-49 | 3,5-Bis(difluormethyl)-1H-pyrazol-1-yl | CH₂ | O | *-CH=CHCO-[E]-# | Cyclopentyl | 3.52^{[a]} |
| I-50 | 3,5-Bis(difluormethyl)-1H-pyrazol-1-yl | CH₂ | O | *-CH=CHCO-[E]-# | 2-Methylcyclohexyl | 4.11^{[a]} |
| I-51 | 3,5-Bis(difluormethyl)-1H-pyrazol-1-yl | CH₂ | O | *-CH(CH3)CH2CO-# | 2,6-Difluorphenyl | |
| I-52 | 3,5-Bis(difluormethyl)-1H-pyrazol-1-yl | CH₂ | O | *-CH=CHCO-[E]-# | 2-Fluorphenyl | 3.35^{[a]} |
| I-55 | 3,5-Bis(difluormethyl)-1H-pyrazol-1-yl | CH₂ | O | *-CH=CHCO-[E]-# | 2-(Prop-2-yn-1-yloxy)phenyl | 3.28^{[a]} |
| I-57 | 3,5-Bis(difluormethyl)-1H-pyrazol-1-yl | CH₂ | O | *-CH=CHCO-[E]-# | 2,4,6-Trifluorphenyl | 3.51^{[a]}; 3.5^{[b]} |
| I-58 | 3,5-Bis(Difluormethyl)-1H-pyrazol-1-yl | CH₂ | s | *-COCH2-# | Cyclohexyl | 4.63^{[a]}; 4.56^{[b]} |
| I-59 | 3,5-Bis(difluormethyl)-1H-pyrazol-1-yl | CH₂ | O | *-COCH2-# | 2,3-Dimethylphenyl | 3.71^{[a]} |
| I-60 | 3,5-Bis(difluormethyl)-1H-pyrazol-1-yl | CH₂ | O | *-C(=NOCH3)CH2-[Z]-# | Cyclohexyl | 4.8^{[a]} |
| I-61 | 3,5-Bis(difluormethyl)-1H-pyrazol-1-yl | CH₂ | O | *-C(=NOCH3)CH2-[Z]-# | 2,3-Dimethylphenyl | 4.33^{[a]} |
| I-62 | 3,5-Bis(difluormethyl)-1H-pyrazol-1-yl | CH₂ | O | *-COCH=CH-[E]-# | 2,6-Difluorphenyl | 3.68^{[a]}; 3.59^{[b]} |
| I-63 | 3,5-Bis(difluormethyl)-1H-pyrazol-1-yl | CH₂ | O | *-CH=CHCO-[E]-# | 2,6-Dimethylphenyl | 3.65^{[a]}; 3.64^{[b]} |
| I-64 | 3,5-Bis(difluormethyl)-1H-pyrazol-1-yl | CH₂ | O | *-CH=CHC(=NOH)-[E,E]-# | 2-(Prop-2-yn-1-yloxy)phenyl | 3.08^{[a][d]}; 3.30^{[a][e]} |
| I-65 | 5-Methyl-3-(trifluormethyl)-1H-pyrazol-1-yl | CH₂ | O | *-C(Cl)=CHCO-[E and/or Z]-# | 2-Methoxyphenyl | 3.96^{[a][d]}; 3.79^{[a][e]} |

| | | | | | | |
|---|---|---|---|---|---|---|
| ^{[d]} major isomer; ^{[e]} minor isomer | | | | | | |

Die Bestimmung der logP Werte erfolgte gemäß EEC Directive 79/831 Annex V.A8 durch HPLC (High Performance Liquid Chromatography) an reversed-phase Säulen (C 18), mit nachfolgenden Methoden:
[a] Die Bestimmung mit der LC-MS im sauren Bereich erfolgt bei pH 2,7 mit 0,1 % wässriger Ameisensäure und Acetonitril (enthält 0,1% Ameisensäure) als Eluenten; linearer Gradient von 10% Acetonitril bis 95% Acetonitril
[b] Die Bestimmung mit der LC-MS im neutralen Bereich erfolgt bei pH 7.8 mit 0,001 molarer wässriger Ammoniumhydrogencarbonat-Lösung und Acetonitril als Eluenten; linearer Gradient von 10 % Acetonitril bis 95 % Acetonitril.

Die Eichung erfolgt mit unverzweigten Alkan-2-onen (mit 3 bis 16 Kohlenstoffatomen), deren logP-Werte bekannt sind (Bestimmung der logP-Werte anhand der Retentionszeiten durch lineare Interpolation zwischen zwei aufeinander folgenden Alkanonen).

Die lambda-maX Werte wurden an Hand der UV-Spektren von 200 nm bis 400 nm in den Maxima der chromatographischen Signale ermittelt.

### NMR-Daten ausgewählter Beispiele

| **Bsp**. | **NMR-Daten** |
|---|---|
| 1-1 | ¹H NMR (CD₃CN, 400 MHz): δₚₚₘ : 1.65-1.76 (m, 1H), 1.83-1.95 (m, 1H), 2.10-2.21 (m, 2H), 2.23 (s, 3H), 2.82-2.91 (m, 1H), 3.25-3.40 (m, 2H), 3.90-3.98 (m, 1H), 4.45-4.53 (m, 1H), 5.04 (d, 1H), 5.11 (d, 1H), 6.40 (s, 1H), 7.56-7.63 (m, 2H), 7.70-7.75 (m, 1H), 8.08 (s, 1H), 8.18-8.22 (m, 2H) |
| I-2 | 1H NMR (CD₃CN, 400 MHz): δₚₚₘ : 1.65-1.76 (m, 1H), 1.83-1.95 (m, 1H), 2.10-2.20 (m, 2H), 2.23 (s, 3H), 2.82-2.91 (m, 1H), 3.25-3.39 (m, 2H), 3.90-3.97 (m, 1H), 3.95 (s, 3H), 4.45-4.51 (m, 1H), 5.04 (d, 1H), 5.11 (d, 1H), 6.39 (s, 1H), 7.08 (t, 1H), 7.17 (d, 1H), 7.60-7.65 (m, 1H), 7.97 (s, 1H), 7.97-8.00 (m, 1H) |
| I-3 | ¹H NMR (CD₃CN, 400 MHz): δₚₚₘ : 1.65-1.78 (m, 1H), 1.82-1.95 (m, 1H), 2.13-2.20 (m, 2H), 2.23 (s, 3H), 2.80-2.90 (m, 1H), 3.25-3.40 (m, 2H), 3.90-3.98 (m, 1H), 4.45-4.53 (m, 1H), 5.04 (d, 1H), 5.11 (d, 1H), 6.40 (s, 1H), 7.10-7.25 (m, 3H), 8.02 (d, 1H), 8.06 (s, 1H), 8.23 (d, 1H), 8.69 (d, 1H), 9.15 (d, 1H) |
| I-4 | ¹H NMR (DMSO-d₆, 400 MHz): δₚₚₘ : 1.15-1.48 (m, 5H), 1.50-1.88 (m, 5H), 1.92-1.99 (m, 2H), 2.07-2.15 (m, 2H), 2.22 (s, 3H), 2.50-2.59 (m, 1H), 2.88 (bs, 1H), 3.20-3.41 (m, 2H), 3.99 (bs, 1H), 4.32 (bs, 1H), 5.21 (bs, 2H), 6.45 (s, 1H), 8.31 (a, 1H) |
| I-5 | ¹H NMR (CD₃CN, 400 MHz): δₚₚₘ : 1.70-1.95 (m, 2H), 2.18-2.25 (m, 2H), 2.29 (s, 3H), 2.97 (bs, 1H), 3.25-3.44 (m, 2H), 4.01 (bs, 1H), 4.50 (bs, 1H), 5.10 (bs, 2H), 6.42 (s, 1H), 7.52-7.65 (m, 3H), 8.05 (s, 1H), 8.16 (dd, 1H) |
| I-6 | ¹H NMR (CD₃CN, 400 MHz): δₚₚₘ : 1.64-1.74 (m, 1H), 1.80-1.91 (m, 1H), 2.10-2.23 (m, 2H), 2.23 (s, 3H), 2.80-2.89 (m, 1H), 3.25-3.38 (m, 2H), 3.89-3.95 (m, 1H), 4.45-4.51 (m, 1H), 5.04 (d, 1H), 5.11 (d, 1H), 6.39 (s, 1H), 7.53 (dd, 1H), 7.63 (d, 1H), 8.06 (s, 1H), 8.14 (d, 1H) |
| I-7 | ¹H NMR (CD₃CN, 400 MHz): δₚₚₘ : 1.71-2.05 (m, 2H), 2.11-2.21 (m, 2H), 2.24 (s, 3H), 2.90 (bs, 1H), 3.24-3.40 (m, 2H), 3.90 (s, 3H), 3.98 (bs, 1H), 4.45 (bs, 1H), 5.05 (bs, 2H), 6.37 (s, 1H), 7.05-7.95 (m, 2H), 7.99 (s, 1H), 8.13-8.18 (m, 2H) |
| I-8 | ¹H NMR (CD₃CN, 400 MHz): δₚₚₘ : 1.25 (s, 9H), 1.62-1.90 (m, 2H), 2.10-2.19 (m, 2H), 2.24 (s, 3H), 2.89 (bs, 1H), 3.20-3.37 (m, 2H), 3.95 (bs, 1H), 4.44 (bs, 1H), 5.04 (bs, 2H), 6.36 (s, 1H), 7.89 (s, 1H) |
| I-9 | ¹H NMR (CD₃CN, 400 MHz): δₚₚₘ : 1.70-1.95 (m, 2H), 2.13-2.22 (m, 2H), 2.24 (s, 3H), 2.91 (bs, 1H), 3.23-3.40 (m, 2H), 3.95 (bs, 1H), 4.44 (bs, 1H), 5.05 (bs, 2H), 6.37 (s, 1H), 7-26 (dd, 1H), 7.90 (dd, 1H), 8.01 (s, 1H), 8.06 (dd, 1H) |
| I-10 | ¹H NMR (CD₃CN, 400 MHz): δₚₚₘ : 1.68-1.95 (m, 2H), 2.10-2.20 (m, 2H), 2.24 (s, 3H), 2.92 (bs, 1H), 3.22-3.40 (m, 2H), 3.98 (bs, 1H), 4.46 (bs, 1H), 5.05 (bs, 2H), 6.37 (s, 1H), 6.69 (dd, 1H), 7.52 (d, 1H), 7.82 (d, 1H), 7.99 (s, 1H) |
| I-11 | ¹H NMR (CD₃CN, 400 MHz): δₚₚₘ : 1.44 (t, 3H), 1.65-1.95 (m, 2H), 2.12-2.21 (m, 2H), 2.24 (s, 3H), 2.90 (bs, 1H), 3.25-3.38 (m, 2H), 3.95 |
| | (bs, 1H), 4.22 (q, 2H), 4.43 (1H), 5.04 (bs, 2H), 6.37 (s, 1H), 7.02-7.18 (m, 2H), 7.54-7.60 (m, 1H), 7.87 (dd, 1H), 7.89 (s, 1H) |
| I-12 | ¹H NMR (DMSO-d₆, 400 MHz): δₚₚₘ: 1.56-1.68 (m, 1H), 1.78-1.91 (m, 1H), 2.10-2.21 (m, 2H), 2.22 (s, 3H), 2.84-2.92 (m, 1H), 3.20-3.50 (m, 2H), 3.95-4.05 (m, 1H), 4.36-4.92 (m, 1H), 5.23 (d, 1H), 5.34 (d, 1H), 6.50 (s, 1H), 7.26 (d, 1H), 7.28 (d, 1H), 7.58 (m, 1H), 7.81 (d, 1H), 8.07 (d, 1H), 8.626 (s, 1H) |
| I-13 | ¹H NMR (DMSO-d₆, 400 MHz): δₚₚₘ : 1.56-1.68 (m, 1H), 1.80-1.91 (m, 1H), 2.10-2.22 (m, 2H), 2.22 (s, 3H), 2.83-2.92 (m, 1H), 3.25-3.35 (m, 1H), 3.40-3.48 (m, 1H), 3.96-4.03 (m, 1H), 4.35-4.42 (m, 1H), 4.57 (s, 2H), 5.24 (d, 1H), 5.33 (d, 1H), 6.50 (s, 1H), 7.23 (td, 1H), 7.33-7.42 (m, 2H), 7.62 (dd, 1H), 8.54 (s, 1H) |
| I-14 | ¹H NMR (DMSO-d₆, 400 MHz): δₚₚₘ : 1.41 (s, 9H), 1.48-1.61 (m, 2H), 1.90-2.11 (4H), 2.62-2.67 (m, 2H), 2.87-2.95 (m, 2H), 3.15-3.20 (m, 1H), 3.94-4.00 (2H), 4.62-4.67 (m, 1H), 5.09-5.12 (m, 1H), 7.11-7.18 (m, 3H), 7.22-7.27 (m, 3H) |
| I-15 | ¹H NMR (DMSO-d₆, 400 MHz): δₚₚₘ : 1.41 (s, 6H), 1.52-1.85 (m, 2H), 2.08-2.15 (m, 2H), 2.23 (s, 3H), 2.90 (bs, 1H), 3.20-3.40 (m, 2H), 3.41 (s, 2H), 3.99 (bs, 1H), 4.34 (bs, 1H), 5.22 (bs, 2H), 6.45 (s, 1H), 7.08-7.13 (m, 1H), 7.21-7.26 (m, 2H), 7.35-7.39 (m, 2H), 8.22 (s, 1H) |
| I-16 | ¹H NMR (DMSO-d₆, 400 MHz): δₚₚₘ : 1.58-1.90 (m, 2H), 2.12-2.19 (m, 2H), 2.23 (s, 3H), 2.93 (bs, 1H), 3.31 (bs, 1H), 3.36-3.47 (m, 1H), 4.00 (bs, 1H), 4.32 (bs, 1H), 4.33 (s, 2H), 5.17-5.27 (m, 2H), 6.45 (s, 1H), 7.18-7.32 (m, 5H), 8.43 (s, 1H) |
| I-17 | ¹H NMR (DMSO-d₆, 400 MHz): δₚₚₘ : 0.94-1.05 (m, 2H), 1.10-1.29 (m, 3H), 1.54-1.71 (m, 6H), 1.75-1.93 (m, 2H), 2.06-2.18 (m, 2H), 2.21 (s, 3H), 2.81-2.89 (m, 1H), 2.89 (d, 2H), 3.22-3.45 (m, 2H), 3.94-4.01 (m, 1H), 4.33-4.40 (m, 1H), 5.22 (d, 1H), 5.33 (d, 1H), 6.50 (s, 1H), 8.42 (s, 1H) |
| I-18 | ¹H NMR (DMSO-d₆, 400 MHz): δₚₚₘ : 1.60-1.91 (m, 2H), 2.11-2.21 (m, 2H), 2.23 (s, 3H), 2.92 (bs, 1H), 3.31 (bs, 1H), 3.40-3.49 (m, 1H), 4.02 (bs, 1H), 4.34 (bs, 1H), 4.83 (s, 2H), 5.22 (bs, 2H), 6.45 (s, 1H), 7.43-7.52 (m, 3H), 7.79-7.82 (m, 2H), 7,88-7.94 (m, 1H), 7.97-8.01 (m, 1H), 8.46 (s, 1H) |
| I-19 | ¹H NMR (CD₃CN, 400 MHz): δₚₚₘ : 1.55-1.67 (m, 1H), 1.72-1.83 (m, 1H), 2.03-2.14 (m, 2H), 2.22 (s, 3H), 2.79-2.87 (m, 1H), 3.06 (t, 2H), 3.19-3.35 (m, 2H), 3.34 (t, 2H), 3.85-3.92 (m, 1H), 3.88 (s, 3H), 4.40-4.46 (m, 1H), 5.03 (d, 1H), 5.08 (d, 1H), 6.39 (s, 1H), 6.98 (s, 1H), 7.00 (t, 1H), 7.09 (d, 1H), 7.45-7.56 (m, 2H) |
| I-20 | ¹H NMR (CD₃CN, 400 MHz): δₚₚₘ : 1.44 (t, 3H), 1.58-1.70 (m, 1H), 1.75-1.86 (m, 1H), 2.06-2.18 (m, 2H), 2.25 (s, 3H), 2.82-2.90 (m, 1H), 3.09 (t, 2H), 3.20-3.32 (m, 2H), 3.42 (t, 2H), 3.88-3.95 (m, 1H), 4.16 (q, 2H), 4.42-4.47 (m, 1H), 5.04 (d, 1H), 5.11 (d, 1H), 6.42 (s, 1H), 6.95 (s, 1H), 7.01 (t, 1H), 7.09 (d, 1H), 7.47-7.53 (m, 1H), 5.59 (dd, 1H) |
| I-21 | ¹H NMR (CD₃CN, 400 MHz): δₚₚₘ : 1.55-1.66 (m, 1H), 1.71-1.82 (m, 1H), 2.02-2.15 (m, 2H), 2.22 (s, 3H), 2.78-2.86 (m, 1H), 3.08 (t, 2H), |
| | 3.18-3.33 (m, 2H), 3.32 (t, 2H), 3.85-3.92 (m, 1H). 4.39-4.46 (m, 1H), 5.02 (d, 1H), 5.09 (d, 1H), 6.39 (s, 1H), 6.96 (s, 1H), 7.35-7.52 (m, 4H) |
| I-22 | ¹H NMR (DMSO-d₆, 400 MHz): δₚₚₘ : 1.51-1.63 (m, 1H), 1.75-1.88 (m, 1H), 2.05-2.16 (m, 2H), 2.80-2.88 (m, 1H), 3.20-3.42 (m, 2H), 3.92-4.01 (m, 1H), 4.30-4.46 (m, 1H), 5.35 (d, 1H), 5.44 (d. 1H), 6.88 (s, 1H), 7.04 (t, 1H), 7.15-7.20 (m, 1H), 7.18 (t, 1H), 7.24-7.32 (m, 2H), 7.49 (d, 1H), 7.60-7.70 (m, 1H), 8.17 (s, 1H) |
| I-23 | ¹H NMR (DMSO-d₆, 400 MHz): δₚₚₘ : 1.62-1.69 (m, 2H), 2.05-2.09 (m, 2H), 2.11 (s, 3H), 2.23 (s, 3H), 2.95-3.01 (m, 2H), 3.29-3.35 (m, 1H), 4.13-4.17 (m, 2H), 6.86 (d, 1H), 6.99 (s, 1H), 7.04 (d, 1H), 7.17 (d, 1H), 7.27-7.32 (m, 2H), 7.49 (d, 1H), 7.63-7.68 (m, 1H), 8.03 (s, 1H), 8.18 (s, 1H) |
| I-24 | ¹H NMR (DMSO-d₆, 400 MHz): δₚₚₘ : 0.95-1.02 (m, 2H), 1.10-1.26 (m, 3H), 1.54-1.69 (m, 6H), 1.77-1.91 (m, 2H), 2.06-2.15 (m, 2H), 2.82-2.87 (m, 1H), 2.89 (d, 2H), 3.25-3.30 (m, 1H), 3.35-3.41 (m, 1H), 3.94-3.99 (m, 1H), 4.33-4.37 (m, 1H), 5.36 (d, 1H), 5.45 (d, 1H), 6.91 (s, 1H), 7.04 (t, 1H), 7.18 (t, 1H), 8.44 (s, 1H) |
| I-25 | ¹H NMR (DMSO-d₆, 400 MHz): δₚₚₘ : 0.94-1.05 (m, 2H), 1.10-1.26 (m, 4H), 1.56-1.73 (m, 6H), 1.83-1.92 (m, 1H), 2.06-2.13 (m, 2H), 2.16 (s, 3H), 2.88 (d, 2H), 2.98-3.06 (m, 2H), 3.30-3.38 (m, 1H), 4.11-4.18 (m, 2H), 7.07 (dd, 1H), 7.19 (d, 1H), 7.33 (d, 1H), 8.12 (s, 1H), 8.04 (s, 1H) |

Die 1H-NMR-Daten ausgewählter Beispiele sind in Form von 1H-NMR-Peaklisten notiert. Zu jedem Signalpeak sind der δ-Wert in ppm und die Signalintensität in Klammern aufgeführt:

| **Bsp.** | **NMR-Peaklisten-Daten** |
|---|---|
| I-26 | [DMSO-D₆] 8,1231 6,35;8,0482 7,38;7,9751 0,56;7,9599 2,84;7,9569 3,62;7,9443 1,14;7,9392 4,50;7,9357 3,58;7,8555 0,74;7,8509 1,42;7,8379 0,77;7,8343 0,72;7,6973 0,46;7,6939 0,91;7,6907 0,51;7,6806 0,67;7,6755 2,23;7,6706 0,80;7,6604 1,05;7,6571 1,85;7,6537 0,93;7,5924 3,06;7,5886 1,33;7,5760 2,41;7,5729 4,24;7,5589 1,05;7,5547 2,02;7,5528 1,33;7,5417 0,38;7,4696 0,59;7,4502 0,78;7,4321 0,37;7,0164 1,35;6,4681 0,77;6,4582 0,72;6,4485 3,44;5,6885 5,31;5,2322 0,85;5,2161 0,86;5,1375 1,62;4,9763 2,78;4,0644 0,46;4,0468 1,31;4,0289 1,42;4,0112 0,61;3,5676 0,65;3,4437 0,34;3,4342 0,61;3,4244 0,38;3,4159 0,68;3,4063 1,22;3,3965 0,76;3,3876 0,47;3,3782 0,74;3,3689 0,42;3,2752 0,36;3,2544 0,34;3,1201 134,75;3,0183 0,50;2,9006 0,33;2,6626 0,42;2,6579 0,59;2,6533 0,42;2,5272 0,49;2,5111 1,30;2,5064 1,84;2,4982 32,10;2,4935 66,61;2,4888 94,30;2,4841 66,08;2,4794 31,81;2,3203 0,38;2,3157 0,60;2,3109 0,44;2,2578 3,63;2,2563 3,71;2,2265 15,73;2,2250 16,00;2,1931 0,80;2,1744 0,92;2,1442 1,08;2,0406 0,95;1,9742 5,50;1,9015 0,40;1,6943 0,40;1,6725 0,38;1,6649 0,36;1,6591 0,40;1,6396 0,35;1,2449 0,45;1,1952 1,59;1,1774 3,19;1,1597 1,59;-0,0001 2,84 |
| I-29 | [DMSO-D₆] 8,1441 11,91;7,7596 3,68;7,7418 3,77;7,7397 4,01;7,6802 0,35;7,6610 0,38;7,5576 1,03;7,5549 1,06;7,5382 3,23;7,5215 4,67;7,5188 4,35;7,5112 3,71;7,5060 6,34;7,4917 5,35;7,4861 3,10;7,4745 2,90;7,4721 3,56;7,4689 2,31;7,4665 2,27;7,4549 1,87;7,4492 1,33;7,4147 0,37;7,4013 0,34;7,3191 4,88;7,3060 2,61;7,2797 8,40;7,1972 0,86;7,1779 8,71;7,1727 6,14;7,1580 2,85;7,1386 4,51;7,0394 2,75;7,0219 6,38;6,8997 |
| | 5,57;6,8860 3,13;5,4484 1,02;5,4057 4,32;5,3696 4,24;5,3445 0,38;5,3267 1,03;4,3555 1,27;4,3222 1,34;4,0571 1,33;4,0393 3,75;4,0215 3,79;4,0038 1,44;3,9800 1,26;3,9453 1,36;3,4781 0,43;3,4577 0,49;3,4356 0,72;3,4109 1,52;3,4017 1,30;3,3918 1,86;3,3825 2,70;3,3729 2,13;3,3631 2,09;3,3537 2,84;3,3057 573,68;3,2826 9,11;3,2429 1,20;2,8901 1,16;2,8789 0,91;2,8508 1,60;2,8206 0,91;2,7321 0,68;2,6740 0,89;2,6695 1,12;2,6648 0,82;2,5394 2,52;2,5091 62,88;2,5048 112,86;2,5004 143,95;2,4960 99,46;2,4917 47,75;2,3315 0,71;2,3271 0,91;2,3225 0,67;2,1468 1,23;2,1107 2,33;2,0697 1,63;1,9869 16,00;1,8609 0,43;1,8514 0,48;1,8237 1,11;1,8017 0,98;1,7928 0,89;1,7702 0,38;1,6262 0,43;1,6163 0,48;1,5961 1,00;1,5879 1,06;1,5650 0,98;1,5573 0,90;1,5367 0,40;1,2363 0,48;1,1928 4,35;1,1750 8,73;1,1572 4,22;0,9264 0,37;0,0080 0,67;-0,0002 11,85;-0,0084 0,46 |
| I-30 | [DMSO-D₆] 8,6206 4,81;8,4136 16,00;7,7324 0,47;7,7047 2,72;7,6850 3,18;7,5317 4,01;7,4853 2,48;7,4658 2,02;7,2201 2,26;7,2139 1,78;7,0810 4,92;7,0761 4,14;6,9500 0,37;6,9418 2,61;4,2512 0,33;4,2282 0,34;4,1744 2,50;4,1403 2,58;4,0571 0,57;4,0393 1,54;4,0215 1,61;4,0036 0,60;3,7222 1,42;3,7090 0,46;3,6975 0,44;3,5947 0,39;3,5678 1,69;3,4175 0,41;3,4044 0,54;3,3904 1,05;3,3810 1,53;3,3707 1,63;3,3619 2,31;3,3517 3,59;3,3135 2176,06;3,2900 33,84;3,2738 1,64;3,2531 0,61;3,0807 1,68;3,0522 3,15;3,0228 1,72;2,8922 9,42;2,8752 9,74;2,6790 0,68;2,6744 1,35;2,6699 1,88;2,6651 1,34;2,6608 0,69;2,5398 3,16;2,5231 5,65;2,5184 8,38;2,5097 103,40;2,5053 202,26;2,5008 272,55;2,4963 187,40;2,4918 88,41;2,3320 1,34;2,3275 1,94;2,3229 1,39;2,3182 0,64;2,1176 2,00;2,0914 2,31;2,0856 2,24;2,0691 6,17;2,0494 0,37;1,9869 6,91;1,9197 0,44;1,9097 0,65;1,9003 0,94;1,8916 0,94;1,8828 1,19;1,8743 1,04;1,8649 0,76;1,8555 0,77;1,8460 0,49;1,8393 0,33;1,7467 0,73;1,7363 0,91;1,7146 1,85;1,7063 2,08;1,6841 4,03;1,6751 4,16;1,6565 4,64;1,6501 3,99;1,6325 2,59;1,6263 2,30;1,5853 1,04;1,3985 0,45;1,2841 0,36;1,2718 0,58;1,2411 1,68;1,2180 1,77;1,2099 2,18;1,1928 2,46;1,1874 1,53;1,1751 5,58;1,1572 2,51;1,1491 1,32;1,1195 0,86;1,0386 0,94;1,0324 1,09;1,0041 2,29;0,9743 1,82;0,9499 0,64;0,9395 0,49;-0,0001 5,55 |
| I-3 | [DMSO-D₆] 8,1416 7,23;7,9858 2,56;7,9664 2,64;7,5640 1,19;7,5462 2,69;7,5281 1,81;7,4712 0,37;7,4521 2,56;7,4481 2,76;7,4331 2,10;7,4292 1,92;7,4220 0,39;7,3303 0,72;7,3051 1,95;7,2949 4,15;7,2793 2,46;7,2753 2,39;7,2557 6,06;7,2010 0,33;7,1713 4,28;7,1679 5,64;7,1572 1,93;7,1284 2,66;7,0388 1,80;7,0211 3,80;6,9590 0,32;6,9000 3,83;6,8854 1,90;5,4476 0,81;5,4045 2,98;5,3871 0,76;5,3690 2,96;5,3273 0,85;4,3570 1,12;4,3185 1,12;4,2594 0,41;4,2556 0,36;4,1721 0,48;4,1607 0,41;4,1086 0,44;4,0905 0,48;4,0727 0,51;4,0568 1,63;4,0392 4,13;4,0215 4,29;4,0034 1,80;3,9821 1,27;3,9679 1,10;3,9456 1,34;3,9094 0,67;3,8857 0,67;3,8808 0,78;3,8652 0,75;3,8537 0,73;3,7222 1,37;3,6832 1,62;3,6610 1,85;3,6523 1,86;3,6335 2,02;3,6298 1,99;3,5555 2,99;3,4880 4,86;3,3256 3385,14;3,2484 4,44;3,1974 2,05;3,1152 1,41;3,1033 1,39;3,0967 1,29;3,0836 1,22;3,0670 1,02;3,0343 1,05;3,0223 0,96;2,9873 0,87;2,9446 0,88;2,9150 0,86;2,9014 0,97;2,8821 1,42;2,8499 2,05;2,8220 1,49;2,7990 1,00;2,7843 1,05;2,7708 1,02;2,7565 1,08;2,7296 1,25;2,6955 2,61;2,6702 3,41;2,6661 2,97;2,5403 11,32;2,5057 258,17;2,5014 323,27;2,4973 233,05;2,4329 1,69;2,4245 1,31;2,4158 1,07;2,3762 0,73;2,3676 0,70;2,3320 2,03;2,3281 2,50;2,2801 0,54;2,2681 0,50;2,2450 0,46;2,2164 0,51;2,1967 0,60;2,1900 0,52;2,1779 0,78;2,1570 1,27;2,1516 1,30;2,1150 2,00;2,0844 1,70;2,0686 2,66;2,0489 0,68;2,0398 0,47;2,0179 0,55;1,9867 16,00;1,9595 0,42;1,9414 0,36;1,9203 0,46;1,9076 0,60;1,9035 0,58;1,8815 0,49;1,8643 0,62;1,8534 0,78;1,8276 1,06;1,8055 0,93;1,7744 0,56;1,7633 0,52;1,7524 0,36;1,7370 0,40;1,7172 0,32;1,6846 0,33;1,6321 0,58;1,6218 0,57;1,5907 1,01;1,5674 0,96;1,5634 0,92;1,4347 0,33;1,3982 1,38;1,2917 0,36;1,2355 0,75;1,2291 0,51;1,2133 0,47;1,1929 4,61;1,1751 8,54;1,1574 4,31;1,0366 0,33;-0,0002 5,53 |
| I-34 | [DMSO-D₆] 8,1326 4,77;7,7556 1,35;7,7495 1,36;7,7338 1,36;7,7277 1,30;7,6227 1,19;7,6075 1,32;7,6013 1,54;7,5861 1,39;7,4392 0,85;7,4330 0,81;7,4179 1,53;7,4117 1,40;7,3966 0,72;7,3904 0,66;7,3518 2,08;7,3126 3,32;7,3064 1,15;7,1775 3,36;7,1732 2,50;7,1580 1,13;7,1384 1,91;7,0399 1,10;7,0219 2,47;6,9006 2,17;6,8860 1,21;5,4501 0,42;5,4064 1,72;5,3694 1,67;5,3271 0,42;4,3574 0,53;4,3232 0,53;4,0571 1,27;4,0392 3,71;4,0214 3,73;4,0036 1,33;3,9819 0,51;3,9477 0,55;3,4127 0,72;3,4027 0,68;3,3938 0,92;3,3840 1,30;3,3745 1,13;3,3083 410,62;3,2447 0,51;2,8802 0,36;2,8502 0,67;2,8210 0,37;2,6741 0,43;2,6694 0,57;2,6647 0,42;2,5396 1,36;2,5093 31,74;2,5049 57,40;2,5005 73,67;2,4961 51,08;2,4917 24,52;2,3320 0,35;2,3272 0,46;2,3227 0,32;2,1467 0,53;2,1121 0,92;2,0693 0,85;1,9868 16,00;1,8299 0,41;1,8237 0,46;1,7994 0,38;1,7922 0,36;1,5982 0,41;1,5894 0,42;1,5672 0,38;1,5578 0,36;1,1928 4,47;1,1750 8,78;1,1572 4,31;-0,0002 2,18 |
| I-35 | [DMSO-D₆] 8,1524 10,14;7,6638 2,28;7,6518 2,45;7,6417 2,83;7,6297 2,73;7,5019 2,25;7,4944 3,06;7,4809 2,39;7,4733 3,03;7,4636 2,19;7,4559 1,54;7,4427 2,99;7,4346 2,07;7,4209 1,82;7,4132 1,23;7,3880 4,78;7,3486 6,57;7,3068 2,09;7,2890 0,40;7,2189 0,39;7,1709 7,64;7,1580 2,65;7,1313 4,26;7,0462 0,74;7,0403 2,41;7,0220 5,38;6,9590 0,34;6,9009 4,78;6,8861 2,72;5,7461 16,00;5,4494 0,94;5,4074 3,67;5,3893 0,62;5,3699 3,71;5,3271 0,90;5,2962 0,85;4,3562 1,10;4,3249 1,13;4,2641 0,33;4,2522 0,39;4,2400 0,34;4,0572 0,84;4,0395 2,42;4,0217 2,47;4,0039 0,95;3,9826 1,07;3,9495 1,17;3,6968 0,35;3,6930 0,36;3,6843 0,55;3,6729 0,75;3,6637 0,58;3,6512 0,38;3,5681 0,54;3,4416 0,48;3,4149 1,26;3,4056 1,13;3,3956 1,61;3,3864 2,39;3,3769 1,98;3,3671 2,06;3,3138 679,74;3,2457 1,26;3,0376 0,32;2,8818 0,78;2,8506 1,55;2,8225 0,79;2,6748 0,58;2,6701 0,76;2,6656 0,56;2,5401 1,68;2,5098 43,12;2,5055 78,38;2,5011 100,99;2,4967 70,61;2,4924 34,59;2,3325 0,54;2,3278 0,69;2,3232 0,54;2,1470 1,05;2,1123 2,00;2,0694 1,35;1,9871 10,39;1,8628 0,39;1,8583 0,40;1,8521 0,45;1,8320 0,89;1,8239 0,98;1,8014 0,84;1,7715 0,36;1,7633 0,32;1,6205 0,46;1,5984 0,89;1,5901 0,95;1,5688 0,87;1,5608 0,83;1,5391 0,36;1,2359 0,41;1,1930 2,90;1,1753 5,73;1,1574 2,83;-0,0002 3,83 |
| I-36 | [DMSO-D₆] 8,1773 7,83;7,6322 0,91;7,6164 1,11;7,6117 2,06;7,5960 2,17;7,5909 1,51;7,5752 1,34;7,4931 3,38;7,4729 2,41;7,4336 1,63;7,4113 2,75;7,3872 3,63;7,3470 4,08;7,3062 1,64;7,1729 3,65;7,1584 1,82;7,1246 4,22;7,0852 3,07;7,0397 1,80;7,0223 4,02;6,9007 3,62;6,8864 2,01;5,4484 0,70;5,4053 2,95;5,3701 2,86;5,3281 0,70;4,3546 0,89;4,3227 0,91;4,0572 1,28;4,0395 3,75;4,0217 3,79;4,0039 1,37;3,9818 0,86;3,9475 0,93;3,4377 0,56;3,4221 0,84;3,4139 1,17;3,4041 1,11;3,3951 1,53;3,3857 2,19;3,3762 1,98;3,3136 766,08;3,2443 1,08;2,8810 0,62;2,8506 1,18;2,8241 0,66;2,6747 0,64;2,6701 0,82;2,6654 0,62;2,5399 1,92;2,5097 49,29;2,5055 88,69;2,5011 113,23;2,4967 79,02;2,4926 38,38;2,3323 0,57;2,3278 0,74;2,3229 0,57;2,1466 0,85;2,1100 1,57;2,0693 1,39;1,9871 16,00;1,8543 0,33;1,8317 0,68;1,8244 0,76;1,8027 0,69;1,6207 0,34;1,6005 0,67;1,5914 0,71;1,5697 0,67;1,5619 0,63;1,4039 0,53;1,1930 4,42;1,1752 8,75;1,1574 4,28;-0,0002 2,58 |
| I-37 | [DMSO-D₆] 8,1616 16,00;8,0401 8,89;8,0224 10,20;8,0188 8,00;7,9030 0,42;7,8856 0,55;7,8818 0,43;7,8365 5,64;7,7983 10,55;7,7190 11,16;7,7010 2,08;7,6979 1,48;7,6813 9,55;7,6672 2,70;7,6642 4,06;7,6611 2,34;7,6069 7,16;7,5875 10,45;7,5693 4,54;7,4781 0,36;7,4590 0,50;7,3184 3,26;7,1851 7,42;7,1645 3,65;7,0519 3,67;7,0285 8,29;6,9891 0,40;6,9572 0,46;6,9076 7,49;6,8926 4,22;6,6263 0,45;6,5951 0,37;5,4668 1,50;5,4242 5,66;5,3824 5,56;5,3398 1,53;5,3181 0,75;4,3941 1,69;4,3606 1,77;4,0571 1,03;4,0393 3,16;4,0215 3,78;4,0038 2,55;3,9740 1,79;3,5680 0,97;3,4493 0,98;3,4400 1,67;3,4303 1,35;3,4209 2,07;3,4113 3,29;3,4018 2,38;3,3922 2,10;3,3823 2,85;3,3729 2,70;3,3125 1921,95;3,1672 0,70;3,1034 0,49;3,0697 0,43;3,0466 0,38;3,0374 0,40;2,8905 1,40;2,8613 2,26;2,8325 1,34;2,6954 0,60;2,6790 0,84;2,6745 1,47;2,6699 1,90;2,6652 1,43;2,5399 4,06;2,5229 9,24;2,5095 110,27;2,5053 201,65;2,5008 260,70;2,4965 183,97;2,4922 90,87;2,3322 1,53;2,3276 1,98;2,3230 1,51;2,1793 1,79;2,1479 3,46;2,1144 1,92;2,0693 0,89;1,9869 13,51;1,9082 0,69;1,8916 0,66;1,8836 0,82;1,8623 1,46;1,8530 1,55;1,8305 1,46;1,8238 1,42;1,8005 0,68;1,6775 0,67;1,6666 0,80;1,6450 1,52;1,6365 1,61;1,6143 1,47;1,6060 1,34;1,5960 0,64;1,5853 0,63;1,5746 0,53;1,3983 1,42;1,2358 0,74;1,1929 3,79;1,1751 7,42;1,1573 3,71;0,8902 0,34;-0,0002 2,24 |
| I-38 | [DMSO-D₆] 8,1561 7,74;7,8566 0,37;7,8334 5,18;7,8152 3,93;7,7767 5,16;7,7497 0,35;7,7080 5,45;7,6698 2,67;7,5071 0,70;7,5026 0,68;7,4865 5,18;7,4671 2,46;7,4480 0,82;7,3179 1,59;7,1846 3,56;7,1638 1,91;7,0513 1,77;7,0278 4,10;6,9068 3,59;6,8920 2,12;5,4675 0,71;5,4248 2,68;5,3818 2,65;5,3389 0,85;4,3976 0,82;4,3643 0,84;4,0571 1,06;4,0393 3,04;4,0215 3,33;4,0038 1,68;3,9757 0,84;3,5679 0,65;3,4497 0,44;3,4407 0,74;3,4310 0,63;3,4213 0,94;3,4119 1,51;3,4029 1,09;3,3922 0,91;3,3827 1,29;3,3734 1,16;3,3106 847,89;3,2606 1,74;2,8865 0,63;2,8590 1,06;2,8271 0,65;2,6744 0,68;2,6697 0,90;2,6652 0,67;2,6607 0,39;2,5765 0,35;2,5608 1,10;2,5398 1,95;2,5228 4,26;2,5095 50,87;2,5051 93,57;2,5007 121,23;2,4963 85,30;2,4920 42,05;2,4215 16,00;2,3809 0,65;2,3656 1,42;2,3319 0,79;2,3272 1,23;2,1795 0,84;2,1466 1,68;2,1134 0,93;2,0693 0,42;1,9869 12,96;1,8882 0,34;1,8796 0,38;1,8582 0,69;1,8502 0,73;1,8273 0,71;1,8240 0,69;1,7982 0,33;1,6711 0,33;1,6606 0,39;1,6385 0,68;1,6312 0,76;1,6095 0,70;1,5999 0,65;1,3984 0,70;1,2360 0,33;1,1929 3,60;1,1751 7,09;1,1573 3,53;-0,0002 0,86 |
| I-39 | [DMSO-D₆] 8,0529 2,63;7,4083 0,69;7,3873 1,39;7,3663 0,80;7,3046 0,54;7,1713 1,24;7,1572 0,83;7,1539 1,29;7,1145 1,75;7,0380 0,63;7,0216 1,35;6,9734 1,68;6,9340 1,07;6,8995 1,24;6,8857 0,67;6,7675 2,92;6,7464 2,73;5,7462 2,13;5,4023 0,98;5,3669 |
| | 0,97;5,2961 0,37;3,7483 0,73;3,7297 0,96;3,7183 16,00;3,6726 0,38;3,3900 0,39;3,3710 0,55;3,3621 0,81;3,3520 0,80;3,3120 148,95;3,2739 0,52;3,2692 0,52;3,1774 0,33;2,8482 0,36;2,5397 0,32;2,5093 9,70;2,5051 17,38;2,5007 22,12;2,4964 15,46;2,0966 0,55;2,0691 0,42;1,9869 0,66;1,1751 0,38 |
| I-40 | [DMSO-D₆] 8,1658 16,00;8,1537 1,38;8,1463 6,35;8,1411 3,10;8,1324 7,18;8,1242 7,06;8,1155 3,20;8,1103 6,18;7,8314 5,38;7,7934 10,54;7,7232 11,07;7,6852 5,42;7,4246 6,41;7,4198 2,50;7,4025 12,01;7,3853 2,51;7,3804 5,93;7,3287 0,58;7,3200 3,34;7,1867 7,49;7,1659 3,73;7,0534 3,64;7,0299 8,05;6,9096 7,63;6,8940 4,13;5,7478 8,22;5,4673 1,53;5,4248 5,92;5,3843 5,83;5,3421 1,61;4,3962 1,81;4,3639 1,89;4,0579 0,37;4,0400 1,00;4,0221 1,56;4,0047 1,88;3,9742 1,86;3,4467 0,76;3,4379 1,37;3,4280 1,08;3,4184 1,72;3,4091 2,88;3,3999 1,80;3,3901 1,27;3,3804 1,69;3,3714 1,12;3,3059 258,95;3,2617 1,92;2,8898 1,27;2,8608 2,24;2,8319 1,28;2,6751 0,45;2,6705 0,58;2,6661 0,43;2,5404 1,00;2,5099 34,49;2,5058 61,68;2,5014 78,48;2,4972 55,77;2,3326 0,48;2,3283 0,60;2,3235 0,46;2,1782 1,69;2,1462 3,51;2,1120 1,96;2,0707 0,36;1,9876 3,68;1,8915 0,62;1,8809 0,72;1,8599 1,47;1,8518 1,55;1,8298 1,47;1,8221 1,38;1,7999 0,62;1,7897 0,51;1,6799 0,62;1,6710 0,77;1,6497 1,51;1,6409 1,60;1,6192 1,46;1,6106 1,39;1,5891 0,59;1,5794 0,50;1,1934 1,02;1,1756 1,98;1,1578 1,00;-0,0002 2,56 |
| I-41 | [DMSO-D₆] 8,1966 16,00;7,9032 4,15;7,8838 4,79;7,8736 0,65;7,8076 3,98;7,7992 2,84;7,7930 3,32;7,7893 2,92;7,7694 15,05;7,7407 13,03;7,7026 3,85;7,6729 1,85;7,6584 2,23;7,6527 3,53;7,6382 3,57;7,6332 2,61;7,6186 2,20;7,5794 0,35;7,5584 2,16;7,5536 1,96;7,5374 3,17;7,5320 2,93;7,5178 1,45;7,5159 1,46;7,5112 1,36;7,3183 3,17;7,2152 0,48;7,1850 7,11;7,1646 3,72;7,0517 3,54;7,0286 7,98;6,9075 7,40;6,8927 4,12;5,7472 7,53;5,4663 1,46;5,4241 5,62;5,3831 5,56;5,3407 1,65;5,3275 0,53;4,3943 1,71;4,3616 1,80;4,0575 0,36;4,0398 0,94;4,0219 1,45;4,0045 1,76;3,9740 1,76;3,4478 0,76;3,4389 1,51;3,4291 1,10;3,4199 1,75;3,4103 2,74;3,4010 1,80;3,3909 1,32;3,3815 1,84;3,3721 1,26;3,3093 504,59;3,2858 10,43;3,2613 2,21;3,1909 0,40;3,1817 0,36;2,8901 1,23;2,8603 2,16;2,8324 1,25;2,6750 0,52;2,6704 0,69;2,6659 0,54;2,5403 1,13;2,5099 40,78;2,5057 74,07;2,5013 95,31;2,4970 68,09;2,3327 0,58;2,3282 0,75;2,3233 0,58;2,1812 1,62;2,1474 3,34;2,1138 1,88;2,0702 0,38;1,9874 3,52;1,8930 0,64;1,8834 0,72;1,8625 1,42;1,8542 1,49;1,8315 1,41;1,8243 1,34;1,8023 0,63;1,7924 0,52;1,6773 0,63;1,6680 0,75;1,6468 1,48;1,6378 1,54;1,6161 1,44;1,6072 1,35;1,5864 0,60;1,5775 0,48;1,2348 0,40;1,1933 0,98;1,1755 1,92;1,1577 0,98;-0,0002 2,25 |
| I-42 | [DMSO-D₆] 9,6022 0,51;8,1426 16,00;8,1000 0,44;7,6087 1,85;7,6058 2,17;7,5886 7,11;7,5868 6,55;7,5851 6,19;7,5820 4,73;7,5772 4,59;7,5655 4,35;7,5606 5,97;7,5567 3,74;7,5517 3,37;7,5462 1,73;7,5411 4,80;7,5359 7,56;7,5327 4,36;7,5119 5,22;7,5077 5,25;7,4955 3,89;7,4925 4,83;7,4883 2,58;7,4762 1,72;7,4724 1,65;7,4214 0,33;7,3567 6,58;7,3442 0,48;7,3174 11,38;7,3054 3,64;7,2919 0,47;7,2126 10,79;7,1726 12,70;7,1575 4,07;7,0464 0,85;7,0388 3,87;7,0214 8,53;6,9989 0,46;6,9656 0,43;6,8997 7,57;6,8856 4,42;6,2908 0,40;6,2754 0,37;5,7463 7,66;5,4482 1,44;5,4054 5,92;5,3888 1,38;5,3685 5,82;5,3261 1,46;5,2949 0,54;5,2788 0,55;4,3552 1,70;4,3220 1,80;4,0392 0,32;4,0215 0,39;3,9786 1,58;3,9462 1,77;3,6839 0,32;3,6720 0,36;3,5679 0,70;3,4728 0,35;3,4485 0,47;3,4115 1,95;3,4019 1,88;3,3925 2,79;3,3835 4,22;3,3733 3,67;3,3107 2339,33;3,2435 3,77;3,1939 1,38;3,1526 0,83;3,1441 0,81;3,0746 0,52;3,0209 0,43;2,9962 0,35;2,9745 0,36;2,9605 0,33;2,9565 0,34;2,9265 0,35;2,9100 0,35;2,8784 1,39;2,8524 2,31;2,8212 1,38;2,7681 0,33;2,6955 0,42;2,6742 1,96;2,6697 2,54;2,6651 1,88;2,6192 0,39;2,5396 4,00;2,5226 12,06;2,5093 145,42;2,5050 264,34;2,5006 339,77;2,4962 237,87;2,4920 117,25;2,3747 0,67;2,3716 0,59;2,3579 0,58;2,3497 0,55;2,3366 1,23;2,3318 2,03;2,3273 2,66;2,3228 2,02;2,3019 0,47;2,2849 0,41;2,2255 0,33;2,2202 0,34;2,2162 0,33;2,1927 0,34;2,1429 1,76;2,1103 3,38;2,0692 3,06;1,9869 1,67;1,9078 0,46;1,8600 0,75;1,8515 0,87;1,8217 1,66;1,7989 1,50;1,7925 1,40;1,7706 0,70;1,7493 0,32;1,6417 0,36;1,6269 0,71;1,6175 0,84;1,5959 1,49;1,5872 1,59;1,5658 1,48;1,5573 1,41;1,5359 0,68;1,3983 0,38;1,2361 1,25;1,1928 0,61;1,1749 1,07;1,1572 0,59;1,0271 0,58;0,8902 0,42;-0,0002 4,66 |
| I-43 | [DMSO-D₆] 8,1720 14,54;7,6305 6,14;7,6255 7,71;7,6087 16,00;7,6074 16,00;7,5745 8,52;7,5580 5,31;7,5511 3,66;7,5394 0,60;7,5347 2,63;7,3074 9,53;7,2679 9,62;7,1737 6,57;7,1590 3,22;7,0746 8,73;7,0404 3,64;7,0351 6,55;7,0230 7,51;6,9008 6,24;6,8871 3,63;5,7468 9,42;5,4475 1,13;5,4051 5,10;5,3719 5,08;5,3295 1,10;4,3546 1,43;4,3212 1,50;3,9811 1,35;3,9467 1,46;3,4212 0,76;3,4118 1,24;3,4020 1,00;3,3927 1,54;3,3834 2,49;3,3739 1,67;3,3640 1,31;3,3550 1,83;3,3451 1,62;3,3057 532,18;3,2430 1,10;2,8798 |
| | 0,99;2,8505 1,73;2,8229 0,98;2,6743 0,57;2,6697 0,76;2,6652 0,57;2,5397 1,04;2,5228 3,13;2,5095 43,31;2,5051 80,87;2,5006 105,64;2,4962 72,60;2,4918 34,34;2,3319 0,51;2,3274 0,70;2,3228 0,49;2,1460 1,23;2,1109 2,57;2,0761 1,48;2,0697 2,08;1,9871 0,34;1,8677 0,43;1,8576 0,52;1,8371 1,09;1,8282 1,16;1,8061 1,08;1,7981 1,00;1,7764 0,44;1,7668 0,35;1,6318 0,42;1,6227 0,53;1,6019 1,10;1,5928 1,18;1,5713 1,11;1,5628 1,06;1,5420 0,45;1,5330 0,36;0,0080 0,91;-0,0002 21,13;-0,0084 0,80 |
| I-44 | [DMSO-D₆] 8,0076 16,00;7,5620 7,74;7,5229 8,94;7,3109 3,30;7,1776 7,54;7,1606 3,72;7,0444 3,71;7,0246 8,50;7,0016 8,77;6,9624 7,56;6,9033 7,09;6,8887 4,24;5,7458 7,11;5,4559 1,43;5,4133 5,52;5,3717 5,46;5,3291 1,43;4,3700 1,59;4,3368 1,68;4,0395 0,48;4,0217 0,54;3,9873 1,51;3,9526 1,65;3,4072 0,82;3,3978 1,47;3,3885 1,21;3,3787 1,90;3,3692 3,11;3,3597 2,31;3,3494 2,32;3,3398 3,75;3,3125 811,08;3,2891 6,55;3,2722 3,23;3,2425 1,55;2,8735 1,16;2,8462 2,03;2,8160 1,18;2,7890 0,76;2,7808 1,11;2,7718 0,85;2,7617 1,77;2,7536 2,28;2,7453 1,29;2,7344 1,26;2,7266 1,30;2,6792 0,32;2,6744 0,58;2,6700 0,77;2,6654 0,59;2,5400 1,06;2,5231 2,82;2,5098 41,65;2,5054 78,84;2,5009 103,87;2,4965 72,41;2,4921 35,05;2,3323 0,58;2,3276 0,74;2,3230 0,55;2,1412 1,46;2,1087 3,04;2,0691 2,21;1,9869 2,02;1,8576 0,66;1,8486 0,85;1,8145 4,71;1,7863 4,49;1,7557 2,72;1,7487 3,00;1,7410 2,68;1,7240 3,48;1,7174 4,43;1,6634 1,69;1,6318 2,31;1,6232 1,88;1,6021 1,41;1,5927 1,46;1,5714 1,36;1,5628 1,29;1,5420 0,57;1,5316 0,47;1,3981 0,51;1,3911 0,60;1,3835 0,81;1,3603 1,87;1,3529 2,68;1,3209 4,92;1,3078 3,17;1,2903 6,56;1,2644 2,70;1,2579 2,99;1,2346 1,37;1,2280 1,35;1,2204 1,12;1,2119 0,95;1,1929 1,76;1,1898 1,76;1,1817 1,38;1,1753 1,84;1,1682 0,93;1,1576 1,58;1,1517 0,93;1,1384 0,38;1,1303 0,44;-0,0002 1,84 |
| I-45 | [DMSO-D₆] 8,1080 7,57;7,8502 0,34;7,5392 1,54;7,5353 1,71;7,5296 0,62;7,5187 2,18;7,5165 2,17;7,4670 0,76;7,4637 0,76;7,4483 1,94;7,4450 1,65;7,4296 1,63;7,4262 1,46;7,3967 2,30;7,3577 7,53;7,3514 2,76;7,3416 2,36;7,3319 1,81;7,3232 1,13;7,3115 6,23;7,2725 2,19;7,1752 3,35;7,1676 0,47;7,1588 1,71;7,0420 1,66;7,0304 0,42;7,0227 3,82;6,9019 3,16;6,8868 1,93;5,7458 2,90;5,4523 0,59;5,4097 2,46;5,3896 0,33;5,3711 2,44;5,3292 0,70;4,3626 0,70;4,3298 0,73;3,9844 0,65;3,9506 0,72;3,4132 0,57;3,4042 0,46;3,3943 0,75;3,3848 1,24;3,3755 0,91;3,3651 0,81;3,3563 1,21;3,3172 394,60;3,2752 1,65;3,2468 0,83;2,8817 0,52;2,8509 0,96;2,8238 0,52;2,6703 0,35;2,5759 0,78;2,5429 0,67;2,5404 0,53;2,5236 1,24;2,5102 19,67;2,5058 37,43;2,5013 49,46;2,4968 34,40;2,4924 16,69;2,4237 0,37;2,3502 16,00;2,3327 0,50;2,3280 0,51;2,3236 0,39;2,1503 0,67;2,1166 1,34;2,0824 0,76;2,0693 0,65;1,9871 0,62;1,8345 0,60;1,8276 0,64;1,8041 0,60;1,7975 0,55;1,6048 0,57;1,5958 0,61;1,5740 0,57;1,5651 0,55;1,1753 0,35;-0,0002 5,31 |
| I-46 | [DMSO-D₆] 8,2538 0,55;8,2464 2,60;8,2378 2,12;8,2347 2,34;8,2281 1,60;8,2220 2,83;8,1638 3,93;8,1432 4,27;8,1115 16,00;8,0617 2,86;8,0565 2,00;8,0492 2,57;8,0456 2,60;8,0379 3,08;8,0297 0,58;7,8844 3,94;7,8817 4,05;7,8667 4,91;7,8639 4,50;7,7642 0,76;7,6727 4,14;7,6642 0,56;7,6547 4,45;7,6523 4,57;7,6423 0,70;7,6345 4,12;7,6246 6,02;7,6190 4,20;7,6137 4,57;7,6106 4,56;7,6058 4,05;7,6002 6,58;7,5888 1,05;7,5827 0,40;7,5410 1,71;7,5020 14,99;7,4927 14,47;7,4782 0,38;7,4538 1,63;7,3082 2,72;7,1749 6,26;7,1590 3,10;7,1437 0,43;7,0417 3,11;7,0325 0,86;7,0230 7,09;6,9977 0,40;6,9665 0,52;6,9025 6,29;6,8871 3,50;6,7280 0,46;6,6967 0,38;5,7462 12,28;5,4531 1,14;5,4104 4,61;5,3909 0,50;5,3709 4,52;5,3284 1,15;5,2700 0,36;5,2559 0,36;4,3684 1,31;4,3351 1,40;4,0578 0,48;4,0400 1,47;4,0222 1,52;4,0043 0,79;3,9874 1,31;3,9536 1,42;3,4289 0,57;3,4196 1,07;3,4100 0,87;3,4001 1,34;3,3910 2,26;3,3817 1,55;3,3715 1,30;3,3622 1,96;3,3196 789,08;3,2752 2,90;3,2467 1,47;3,0381 0,49;2,8795 1,00;2,8512 2,01;2,8221 0,98;2,6753 0,48;2,6709 0,62;2,6664 0,48;2,5409 0,77;2,5241 2,11;2,5107 33,80;2,5063 64,26;2,5019 84,75;2,4974 59,16;2,4930 28,67;2,3332 0,51;2,3285 0,65;2,3239 0,49;2,1541 1,23;2,1216 2,54;2,0873 1,44;2,0699 0,84;1,9875 6,45;1,8702 0,47;1,8618 0,54;1,8393 1,10;1,8316 1,17;1,8097 1,10;1,8017 1,02;1,7795 0,47;1,7697 0,41;1,6407 0,45;1,6312 0,58;1,6099 1,07;1,6009 1,16;1,5794 1,10;1,5706 1,03;1,5491 0,48;1,5397 0,39;1,2345 0,60;1,1933 1,90;1,1755 3,61;1,1577 1,79;0,0081 0,37;-0,0002 9,08;-0,0083 0,39 |
| I-47 | [DMSO-D₆] 8,0468 1,47;7,5409 0,62;7,5028 1,01;7,3802 0,96;7,3422 0,60;7,1786 0,70;7,1604 0,36;7,0454 0,35;7,0244 0,79;6,9036 0,67;6,8885 0,41;5,4185 0,50;5,3705 0,50;3,3180 74,17;2,5103 3,27;2,5059 6,15;2,5014 8,08;2,4970 5,64;2,4927 2,73;1,1568 16,00;1,0780 0,44 |
| I-48 | [DMSO-D₆] 8,1047 5,08;7,8248 1,17;7,8028 2,14;7,7814 0,98;7,6546 0,54;7,6500 0,56;7,6164 2,14;7,6120 2,06;7,5928 1,97;7,5860 1,94;7,5547 0,48;7,5478 0,50;7,3152 |
| | 0,97;7,1819 2,21;7,1620 1,12;7,0487 1,09;7,0259 2,52;6,9976 1,12;6,9916 1,33;6,9637 0,87;6,9571 2,99;6,9508 0,98;6,9354 1,52;6,9293 1,17;6,9059 2,12;6,8901 1,28;5,7462 1,77;5,4651 0,46;5,4226 1,58;5,3754 1,60;5,3330 0,45;4,3811 0,48;4,3467 0,50;4,0039 0,41;3,9974 0,47;3,9625 0,51;3,8751 16,00;3,8388 1,04;3,5688 0,58;3,4328 0,49;3,4228 0,42;3,4136 0,63;3,4043 0,94;3,3950 0,66;3,3853 0,56;3,3754 0,74;3,3659 0,69;3,3209 214,30;3,2901 0,87;3,2858 0,87;3,2541 0,41;2,8861 0,33;2,8554 0,58;2,8286 0,34;2,5413 0,33;2,5245 0,85;2,5112 10,46;2,5069 19,22;2,5024 24,80;2,4980 16,91;2,4936 7,87;2,1657 0,42;2,1299 0,85;2,0948 0,47;1,9877 0,79;1,8415 0,38;1,8332 0,39;1,8105 0,37;1,8037 0,34;1,6143 0,38;1,6061 0,40;1,5846 0,38;1,5756 0,35;1,1758 0,42;-0,0002 0,46 |
| I-49 | [DMSO-D₆] 9,6003 0,61;8,0177 16,00;7,5816 8,06;7,5556 8,77;7,3632 0,40;7,3605 0,34;7,2989 0,38;7,2743 2,73;7,2673 0,33;7,2175 0,34;7,1857 6,39;7,1784 0,61;7,1287 3,26;7,0972 3,11;7,0605 0,61;7,0383 7,66;6,9762 0,37;6,9703 0,58;6,9520 8,89;6,9480 3,85;6,9259 7,98;6,9154 7,06;6,3327 0,60;6,3226 0,62;5,4579 2,40;5,4294 5,41;5,4039 0,99;5,3792 5,39;5,3644 0,41;5,3509 2,44;5,3087 0,77;5,2972 0,39;5,2893 0,33;4,3668 1,60;4,3487 1,64;4,3448 1,67;4,1728 0,35;4,1637 0,34;3,9801 1,48;3,9568 1,62;3,6698 0,48;3,6598 0,47;3,4003 0,60;3,3941 1,23;3,3879 0,77;3,3811 1,49;3,3749 2,61;3,3685 1,68;3,3618 1,37;3,3438 592,30;3,3202 11,51;3,3150 1,54;3,3028 2,20;3,3004 2,26;3,2884 4,89;3,2764 2,49;3,2740 2,60;3,2656 2,49;3,2624 2,60;3,2465 1,40;3,2426 1,12;2,8581 1,03;2,8539 1,46;2,8363 1,99;2,8332 2,06;2,8159 1,22;2,8119 1,02;2,6205 0,45;2,6176 0,98;2,6146 1,40;2,6116 1,01;2,5423 0,52;2,5239 2,52;2,5208 3,20;2,5177 3,15;2,5088 75,70;2,5058 163,26;2,5028 224,58;2,4998 163,62;2,4968 76,90;2,3929 0,53;2,3900 1,06;2,3870 1,47;2,3840 1,07;2,3811 0,53;2,1352 1,38;2,1152 1,65;2,0948 1,52;2,0768 2,25;1,9558 2,53;1,8583 0,73;1,8509 1,97;1,8417 2,34;1,8364 3,21;1,8317 2,66;1,8276 2,78;1,8206 3,17;1,8150 3,13;1,8054 1,50;1,7998 1,58;1,7937 1,41;1,7799 0,71;1,7732 0,63;1,7219 0,79;1,7097 2,70;1,6972 3,10;1,6888 2,85;1,6846 2,05;1,6766 2,32;1,6662 0,84;1,6638 0,94;1,6199 0,33;1,6008 2,09;1,5913 6,28;1,5845 8,13;1,5782 10,69;1,5734 5,83;1,5679 4,51;1,5590 2,66;1,5460 0,99;1,5386 0,78;1,4972 0,40;1,4097 1,92;1,3893 0,44;1,2336 0,96;1,1708 0,45;1,0236 0,38;0,0052 0,81;-0,0002 27,57;-0,0057 0,89 |
| I-50 | [DMSO-D₆] 8,0244 14,36;7,9983 1,29;7,6093 6,63;7,5701 7,63;7,5310 0,62;7,4919 0,67;7,3112 3,18;7,1779 7,28;7,1611 3,67;7,0446 3,64;7,0354 0,96;7,0251 8,22;6,9964 0,61;6,9733 7,58;6,9341 6,68;6,9039 7,11;6,8892 4,39;5,7457 4,24;5,4559 1,35;5,4133 5,41;5,3722 5,38;5,3298 1,43;5,2957 0,59;4,3705 1,56;4,3368 1,70;4,0396 0,55;4,0218 0,62;3,9878 1,49;3,9532 1,65;3,6730 0,43;3,4016 1,37;3,3915 1,09;3,3821 1,76;3,3728 2,86;3,3631 2,32;3,3168 815,40;3,2783 3,69;3,2444 1,84;2,9482 0,33;2,9225 0,37;2,8758 1,20;2,8455 2,05;2,8176 1,15;2,6750 0,59;2,6704 0,77;2,6661 0,56;2,5493 1,43;2,5405 1,78;2,5102 39,56;2,5058 72,67;2,5013 94,78;2,4969 66,70;2,4926 32,43;2,3324 0,52;2,3280 0,73;2,3235 0,53;2,1415 1,50;2,1088 3,06;2,0693 2,13;1,9871 2,49;1,8476 0,74;1,8264 1,44;1,8184 1,52;1,7952 1,51;1,7875 1,53;1,7662 2,67;1,7362 3,74;1,7218 4,03;1,6837 4,30;1,6677 2,83;1,6409 1,91;1,6328 1,82;1,6242 1,83;1,6056 1,81;1,5722 1,42;1,5641 1,28;1,5417 0,57;1,4071 0,41;1,2632 3,22;1,2549 3,25;1,2314 1,99;1,2254 1,94;1,1932 1,69;1,1753 1,64;1,1576 0,83;1,0719 0,61;1,0447 1,44;1,0189 1,19;0,9883 0,45;0,7780 15,77;0,7621 16,00;0,7414 1,58;-0,0002 1,35 |
| I-51 | [DMSO-D₆] 7,6117 0,46;7,5934 1,32;7,5740 1,93;7,5557 1,42;7,5381 0,48;7,3069 2,68;7,2107 1,34;7,2040 5,75;7,1829 11,79;7,1792 12,89;7,1776 12,89;7,1738 8,13;7,1617 5,80;7,0403 2,98;7,0230 6,62;6,9007 5,78;6,8871 3,34;5,4383 1,13;5,3952 4,33;5,3542 4,38;5,3115 1,20;5,2976 0,36;4,3064 1,34;4,2744 1,38;3,9328 1,27;3,8993 1,37;3,4838 1,18;3,4666 2,41;3,4496 2,62;3,4325 1,53;3,4159 0,53;3,3792 2,18;3,3621 1,74;3,3374 3,86;3,3116 292,13;3,2880 5,08;3,2679 1,61;3,2598 1,71;3,2488 1,23;3,2391 2,49;3,2309 3,43;3,2215 1,64;3,2024 1,86;3,1296 1,93;3,1120 1,79;3,0883 1,45;3,0706 1,29;2,8495 0,87;2,8420 0,93;2,8150 1,62;2,7851 0,91;2,5102 13,21;2,5059 24,79;2,5014 32,48;2,4970 22,75;2,4927 11,05;2,0699 0,41;2,0440 1,16;2,0066 1,78;1,9635 1,32;1,7392 0,47;1,7097 1,07;1,6864 1,00;1,6802 0,96;1,6566 0,40;1,6493 0,34;1,5146 0,48;1,5036 0,45;1,4845 1,10;1,4540 1,05;1,4237 0,42;1,2883 16,00;1,2712 15,74;-0,0002 3,98 |
| I-52 | [DMSO-D₆] 8,1450 16,00;7,9241 0,88;7,8342 0,33;7,8301 0,34;7,7728 2,13;7,7685 2,39;7,7542 4,11;7,7497 4,60;7,7345 2,55;7,7303 2,48;7,7035 1,30;7,6987 1,27;7,6901 1,60;7,6853 2,71;7,6823 2,22;7,6714 2,25;7,6674 2,23;7,6644 2,75;7,6595 1,60;7,6510 1,63;7,6463 1,35;7,6221 3,53;7,6190 3,50;7,5836 6,54;7,5806 6,29;7,4983 5,45;7,4922 5,33;7,4598 2,94;7,4539 2,87;7,4088 3,30;7,3968 3,95;7,3944 4,12;7,3875 3,63;7,3830 |
| | 3,87;7,3780 6,81;7,3593 5,87;7,3104 3,30;7,2799 0,41;7,1772 7,65;7,1594 3,67;7,0440 3,77;7,0234 8,21;6,9281 0,61;6,9027 7,22;6,8875 4,01;5,7458 4,96;5,4582 1,60;5,4166 5,55;5,3715 5,47;5,3481 0,78;5,3291 1,57;4,3715 1,80;4,3388 1,84;4,1303 0,35;4,0392 0,39;4,0202 0,43;3,9930 1,81;3,9598 1,88;3,8840 0,33;3,8496 0,33;3,8245 0,39;3,7895 0,39;3,7752 0,35;3,7579 0,37;3,7289 0,37;3,7233 0,37;3,6965 0,42;3,6610 0,45;3,6217 0,54;3,5969 0,60;3,5520 0,72;3,4287 3,21;3,4096 4,17;3,3998 5,68;3,3903 5,36;3,3708 7,58;3,3190 2661,98;3,2498 2,21;3,2196 0,57;3,2014 0,46;3,1642 0,33;2,8811 1,22;2,8518 2,09;2,8243 1,26;2,6750 1,07;2,6704 1,30;2,6657 1,03;2,5943 0,50;2,5402 2,29;2,5101 76,99;2,5057 139,24;2,5013 178,61;2,4969 123,40;2,4926 59,64;2,4440 0,40;2,3322 0,85;2,3282 1,16;2,3235 0,84;2,1585 1,58;2,1250 3,07;2,0852 2,03;2,0692 1,31;1,9868 0,46;1,8602 0,71;1,8300 1,49;1,8091 1,34;1,8014 1,27;1,7781 0,60;1,7688 0,55;1,6302 0,69;1,6093 1,37;1,6001 1,42;1,5787 1,36;1,5704 1,30;1,5477 0,56;1,5370 0,50;1,2365 0,54;-0,0002 12,15 |
| I-55 | [DMSO-D₆] 8,2161 0,55;8,0448 12,22;8,0245 0,94;8,0222 1,08;7,5743 1,29;7,5700 1,58;7,5519 2,58;7,5491 2,43;7,5351 1,79;7,5306 2,10;7,4889 2,95;7,4846 3,06;7,4699 3,52;7,4657 3,40;7,4413 0,38;7,4237 15,38;7,4226 16,00;7,4168 2,28;7,4129 1,63;7,4071 1,54;7,3832 0,47;7,3043 2,07;7,2886 0,59;7,2762 3,88;7,2556 3,38;7,2039 0,40;7,1915 0,41;7,1822 0,70;7,1709 4,73;7,1574 2,58;7,1299 2,24;7,1113 3,88;7,0945 2,00;7,0927 2,00;7,0766 0,42;7,0550 0,57;7,0464 0,76;7,0377 2,42;7,0213 5,38;6,9783 0,33;6,9548 0,49;6,9007 4,86;6,8854 3,08;6,8539 0,53;6,7244 0,41;5,7466 1,57;5,4440 0,74;5,4010 3,72;5,3720 3,77;5,3580 0,73;5,3465 0,34;5,3300 0,85;5,3056 0,36;5,2957 0,98;4,9216 9,22;4,9158 10,09;4,8789 0,73;4,8732 0,75;4,8116 0,32;4,8058 0,34;4,3462 1,07;4,3106 1,10;4,2628 0,35;4,2512 0,38;4,2396 0,39;4,0218 0,33;3,9780 1,07;3,9416 1,11;3,6973 0,44;3,6931 0,44;3,6841 0,58;3,6723 0,76;3,6630 0,54;3,6508 0,35;3,5793 0,49;3,5560 2,51;3,5501 5,43;3,5442 2,70;3,5373 0,69;3,4073 0,79;3,3931 1,05;3,3883 1,12;3,3794 1,79;3,3697 1,27;3,3585 1,26;3,3498 1,63;3,3074 734,77;3,2863 8,37;3,2551 2,26;3,1387 0,42;3,0369 0,63;2,9014 0,88;2,8763 1,52;2,8492 0,96;2,8150 0,33;2,6740 0,51;2,6692 0,61;2,6649 0,47;2,5744 0,55;2,5394 0,75;2,5090 34,19;2,5047 64,65;2,5002 85,50;2,4958 60,67;2,4915 30,30;2,3315 0,56;2,3269 0,72;2,3224 0,56;2,2033 0,50;2,1583 1,06;2,1246 1,99;2,0894 1,22;2,0693 0,92;2,0330 0,41;1,9867 0,54;1,8534 0,51;1,8246 0,97;1,8018 0,91;1,7728 0,47;1,6391 0,61;1,6185 1,06;1,6096 1,07;1,5879 1,10;1,5802 0,95;1,5579 0,63;1,4151 1,04;1,4082 6,37;1,3933 0,47;1,3889 0,41;1,3846 0,34;1,2654 0,63;1,2499 0,74;1,2358 1,21;1,1822 0,32;1,1747 0,38;1,1085 0,55;1,0909 1,03;1,0735 0,51;0,0079 0,32;-0,0002 5,93 |
| I-57 | [DMSO-D₆] 8,1634 13,11;8,1472 0,76;7,5710 4,48;7,5319 5,69;7,5073 0,36;7,4373 0,57;7,4241 3,82;7,4040 5,44;7,4009 5,75;7,3808 3,84;7,3282 0,89;7,3111 3,27;7,3060 1,88;7,2835 0,77;7,1776 11,64;7,1653 3,74;7,1379 3,93;7,0447 3,27;7,0293 7,89;7,0152 0,64;6,9934 0,35;6,9096 7,23;6,8935 4,18;5,4580 1,46;5,4154 5,09;5,3913 0,87;5,3745 5,11;5,3554 0,72;5,3319 1,52;4,3627 1,46;4,3299 1,58;4,0564 1,19;4,0386 3,60;4,0208 3,65;4,0030 1,43;3,9843 1,37;3,9494 1,66;3,9175 0,40;3,8629 2,49;3,8203 0,37;3,8073 0,48;3,5491 0,33;3,3691 454,41;3,3559 688,63;3,2715 3,26;3,2420 1,92;3,1943 0,69;3,1851 0,69;3,1660 1,21;3,1448 0,46;2,8753 1,09;2,8446 1,91;2,8179 1,18;2,6822 0,40;2,6779 0,81;2,6733 1,07;2,6688 0,80;2,6643 0,39;2,5434 1,87;2,5264 3,05;2,5133 58,45;2,5088 117,23;2,5042 155,51;2,4996 111,90;2,4951 53,73;2,3401 0,37;2,3356 0,77;2,3309 1,04;2,3263 0,76;2,1489 1,33;2,1120 2,64;2,0867 1,53;2,0729 3,43;1,9891 16,00;1,8606 0,49;1,8514 0,59;1,8303 1,16;1,8217 1,27;1,7998 1,17;1,7909 1,10;1,7697 0,49;1,7602 0,45;1,6289 0,55;1,6179 0,63;1,5974 1,18;1,5885 1,27;1,5667 1,18;1,5584 1,13;1,5367 0,51;1,5266 0,44;1,3105 0,53;1,2439 1,83;1,2350 1,03;1,2166 2,36;1,1930 4,45;1,1752 8,86;1,1574 4,35;-0,0002 1,64 |
| I-59 | [DMSO-D₆] 8,4936 9,05;7,3144 1,47;7,1811 3,27;7,1608 1,62;7,0612 1,55;7,0551 1,88;7,0476 2,64;7,0404 6,28;7,0247 4,40;7,0209 3,64;7,0058 1,19;6,9989 0,84;6,9846 0,66;6,9041 3,26;6,8889 1,86;5,7472 1,75;5,4671 0,69;5,4239 2,46;5,3787 2,48;5,3356 0,70;4,5903 0,34;4,4152 10,64;4,3748 0,80;4,3413 0,86;4,0390 0,34;4,0212 0,44;4,0039 0,78;3,9676 0,78;3,7096 0,47;3,7057 0,36;3,6980 0,55;3,6960 0,58;3,6810 0,60;3,6715 0,37;3,6677 0,41;3,4979 0,45;3,4861 0,42;3,4746 0,85;3,4639 1,03;3,4561 0,61;3,4462 0,82;3,4364 1,23;3,4268 0,75;3,4179 0,57;3,4087 0,72;3,3982 0,47;3,3009 297,63;3,2772 7,56;2,9051 0,55;2,8768 0,95;2,8471 0,57;2,6732 0,49;2,6686 0,69;2,6639 0,51;2,5386 1,04;2,5084 39,69;2,5041 72,43;2,4997 93,44;2,4953 65,59;2,4910 32,05;2,3452 0,39;2,3310 |
| | 0,53;2,3263 0,69;2,3219 0,51;2,2393 15,38;2,2068 0,36;2,1885 0,79;2,1850 0,78;2,1726 0,67;2,1483 1,40;2,1128 1,01;2,0768 16,00;1,9866 1,34;1,8627 0,63;1,8541 0,66;1,8330 0,61;1,8258 0,57;1,6385 0,63;1,6303 0,65;1,6093 0,60;1,5996 0,58;1,2368 0,78;1,1925 0,38;1,1747 0,68;1,1570 0,36;0,0079 0,62;-0,0002 11,73;-0,0085 0,51 |
| I-60 | [DMSO-D₆] 8,3299 0,40;7,7755 6,73;7,6623 0,33;7,3056 1,04;7,2679 0,47;7,1722 2,36;7,1569 1,27;7,0388 1,16;7,0317 0,56;7,0211 2,58;6,8993 2,66;6,8854 1,32;5,7474 1,35;5,4566 0,56;5,4132 1,63;5,3601 1,71;5,3176 0,60;5,2956 0,47;5,2746 0,36;4,3544 0,44;4,3432 0,59;4,3127 0,63;3,9748 0,57;3,9354 0,78;3,9197 0,40;3,8887 1,04;3,8676 16,00;3,6860 0,35;3,6721 0,45;3,6627 0,36;3,4247 0,45;3,3792 1,14;3,3008 1113,92;3,2777 19,21;3,2076 0,63;3,1878 0,59;3,1744 0,49;3,1672 0,43;3,1486 0,39;2,8868 0,54;2,8592 0,74;2,8383 0,35;2,8211 0,50;2,6925 2,76;2,6736 4,08;2,6688 3,15;2,6642 2,17;2,6598 1,18;2,6255 0,37;2,6134 0,41;2,6090 0,44;2,5388 4,71;2,5085 159,43;2,5042 286,60;2,4997 366,70;2,4953 252,97;2,4909 121,25;2,4240 0,76;2,3732 0,38;2,3676 0,37;2,3563 0,40;2,3312 2,00;2,3265 2,52;2,3218 1,83;2,1289 0,65;2,1226 0,64;2,0845 4,97;2,0694 4,57;1,9864 0,54;1,7881 0,59;1,7521 0,51;1,5951 2,86;1,5634 2,25;1,3991 0,42;1,2368 0,73;1,1747 0,51;1,1577 0,50;1,1150 2,15;1,0951 1,38;1,0060 0,57;0,9835 0,98;0,9616 0,73;0,9555 0,77;0,8901 0,49;0,0080 2,80;-0,0002 51,71;-0,0084 2,10;-0,1497 0,32 |
| I-61 | [DMSO-D₆] 7,7676 6,50;7,3003 0,96;7,1671 2,26;7,1545 1,10;7,0337 1,12;7,0185 2,56;6,9655 0,92;6,9483 1,70;6,9263 1,19;6,9067 2,05;6,8964 2,35;6,8827 1,36;6,8246 1,41;6,8057 0,97;5,4374 0,51;5,3940 1,76;5,3536 1,74;5,3104 0,49;4,3147 0,63;4,2815 0,61;4,0985 4,92;4,0568 0,38;4,0389 0,97;4,0211 1,02;4,0034 0,36;3,9443 0,64;3,9025 16,00;3,7095 0,35;3,6962 0,36;3,6808 0,38;3,4982 0,39;3,4752 0,58;3,4640 0,53;3,4527 0,53;3,4443 0,52;3,4166 0,67;3,4003 0,89;3,3047 1218,33;3,2814 18,99;3,2131 0,65;3,0832 0,49;2,8555 0,43;2,8231 0,72;2,7949 0,49;2,6736 1,46;2,6690 1,91;2,6643 1,44;2,6602 0,80;2,6131 0,39;2,5390 3,14;2,5087 109,72;2,5044 200,01;2,4999 258,25;2,4955 180,12;2,4912 86,91;2,3311 1,32;2,3265 1,73;2,3220 1,17;2,3174 0,62;2,2191 10,31;2,1997 10,49;2,0828 0,52;2,0691 1,44;2,0478 0,92;2,0120 0,61;1,9867 4,02;1,7607 0,47;1,7301 0,42;1,5373 0,48;1,5301 0,45;1,5074 0,45;1,3985 1,31;1,2364 0,52;1,1926 1,17;1,1748 2,17;1,1570 1,12;0,8903 0,37;0,0079 1,13;-0,0002 20,73;-0,0084 0,91 |
| I-62 | [DMSO-D₆] 8,6244 16,00;8,4920 0,52;8,0955 4,59;8,0545 6,27;7,8260 6,63;7,7849 4,95;7,6205 0,66;7,6038 1,48;7,5993 1,46;7,5828 2,83;7,5663 1,51;7,5618 1,74;7,5454 0,79;7,3139 2,67;7,2904 4,80;7,2679 7,01;7,2461 3,91;7,1806 5,94;7,1610 2,97;7,0782 0,35;7,0473 3,04;7,0250 6,55;6,9047 5,84;6,8891 3,33;5,4687 1,21;5,4263 4,20;5,3779 4,28;5,3351 1,26;5,2959 0,43;4,3852 1,32;4,3523 1,40;4,0572 1,04;4,0394 3,11;4,0216 3,52;4,0039 2,14;3,9745 1,38;3,5682 0,36;3,4991 0,69;3,4899 1,12;3,4799 0,94;3,4707 1,40;3,4614 2,23;3,4520 1,49;3,4426 1,16;3,4330 1,50;3,4252 1,10;3,4115 0,90;3,3151 1941,32;3,1796 0,72;3,1408 0,67;2,9072 1,09;2,8782 1,77;2,8506 1,14;2,8302 0,33;2,6956 0,39;2,6795 0,76;2,6749 1,36;2,6702 1,76;2,6658 1,33;2,6612 0,76;2,5402 3,49;2,5233 8,22;2,5099 97,98;2,5056 183,21;2,5011 241,25;2,4967 173,04;2,4923 86,93;2,3804 0,38;2,3491 0,35;2,3323 1,45;2,3278 1,79;2,3233 1,42;2,1993 1,36;2,1620 2,48;2,1238 1,57;2,0851 0,33;2,0694 2,27;1,9871 13,50;1,9085 0,86;1,8950 0,53;1,8854 0,62;1,8636 1,17;1,8565 1,25;1,8330 1,17;1,8256 1,14;1,8047 0,55;1,7953 0,50;1,6675 0,61;1,6469 1,16;1,6376 1,25;1,6157 1,15;1,6072 1,11;1,5860 0,52;1,5764 0,47;1,3984 0,42;1,2355 0,71;1,1930 3,81;1,1753 7,51;1,1575 3,69;0,0079 0,46;-0,0002 9,48;-0,0085 0,45 |
| I-63 | [DMSO-D₆] 8,1046 3,68;7,3058 0,73;7,2811 0,75;7,2622 1,16;7,2431 1,07;7,1725 1,68;7,1582 0,89;7,1374 2,66;7,1301 1,34;7,1186 1,96;7,0904 3,04;7,0561 2,91;7,0392 0,87;7,0221 1,91;7,0166 1,02;6,9007 1,65;6,8863 0,91;5,4029 1,36;5,3696 1,32;4,3529 0,40;4,3211 0,42;4,0573 0,42;4,0395 1,20;4,0217 1,22;4,0038 0,46;3,9768 0,38;3,9445 0,42;3,3995 0,60;3,3902 0,62;3,3808 0,83;3,3713 1,19;3,3608 1,19;3,3132 179,92;3,2393 0,40;2,8479 0,49;2,5398 0,71;2,5096 16,90;2,5053 30,21;2,5009 38,39;2,4966 26,71;2,4923 12,94;2,1841 0,47;2,1360 16,00;2,1043 0,77;2,0695 0,51;1,9870 5,09;1,8196 0,33;1,5817 0,32;1,3982 0,54;1,1929 1,40;1,1752 2,76;1,1573 1,35;-0,0002 0,40 |
| I-64 | [DMSO-D₆] 11,4444 4,39;11,1901 0,81;9,8995 0,61;9,0748 0,34;8,6602 0,56;7,9819 0,34;7,8239 1,45;7,7835 1,56;7,6641 0,87;7,6598 0,95;7,6444 1,02;7,6404 0,97;7,6203 2,89;7,6118 0,37;7,6004 2,97;7,5921 0,47;7,5795 0,71;7,5283 0,70;7,4820 0,52;7,4651 1,27;7,4613 1,12;7,4428 1,44;7,4257 0,56;7,4216 0,56;7,3257 0,38;7,3187 0,66;7,3051 0,81;7,2482 0,43;7,2196 1,03;7,2109 2,00;7,1990 1,13;7,1919 2,15;7,1884 2,39;7,1714 2,69;7,1462 0,37;7,0881 0,41;7,0802 1,14;7,0690 0,56;7,0608 2,03;7,0526 1,09;7,0426 |
| | 1,41;7,0388 1,54;7,0349 2,49;7,0276 1,94;6,9091 2,84;6,8913 0,96;6,3120 1,53;6,2723 1,45;6,1958 0,37;6,1870 0,76;6,1688 0,61;5,7851 0,68;5,7646 0,79;5,7573 1,82;5,7375 0,50;5,4713 0,46;5,4445 0,43;5,4276 1,10;5,4027 1,31;5,3905 0,78;5,3807 1,73;5,3669 1,21;5,3376 0,51;5,3260 0,44;5,0197 0,55;5,0141 0,57;4,9042 2,67;4,8986 2,60;4,8296 0,71;4,8208 0,86;4,8045 2,17;4,7984 2,25;4,7687 0,39;4,7639 0,42;4,3678 0,52;4,3440 0,75;4,3274 0,56;4,0553 0,78;4,0374 2,23;4,0197 2,26;4,0018 0,77;3,9550 0,68;3,8317 0,39;3,8045 0,41;3,7879 0,78;3,7614 0,68;3,7015 0,68;3,6923 0,45;3,6806 0,63;3,6583 0,34;3,6384 0,38;3,6124 0,86;3,6067 2,11;3,6010 0,87;3,5675 0,69;3,5118 1,03;3,5061 2,16;3,5001 0,92;3,4144 0,39;3,4015 0,59;3,3913 0,99;3,3795 1,66;3,3470 1964,81;3,3250 5,70;3,2972 1,40;3,2599 1,04;3,2431 0,82;3,2282 0,61;3,2018 0,33;2,8662 0,42;2,8307 0,70;2,8039 0,50;2,6768 1,01;2,6718 1,53;2,6673 1,15;2,5421 0,78;2,5251 2,65;2,5206 3,84;2,5112 86,20;2,5071 166,76;2,5028 232,18;2,4986 160,26;2,4944 80,12;2,4562 0,37;2,3342 1,12;2,3295 1,39;2,3251 1,04;2,1407 0,58;2,1029 1,02;2,0746 16,00;2,0457 0,53;1,9892 10,59;1,8258 0,58;1,7937 0,54;1,7670 0,43;1,7581 0,43;1,5938 0,34;1,5805 0,39;1,5736 0,52;1,5644 0,52;1,5448 0,62;1,2346 0,73;1,1922 2,79;1,1744 5,96;1,1567 2,76;0,0079 0,59;-0,0002 20,96;-0,0087 0,56 |
| I-65 | [CD₃CN] 8,0859 4,04;7,8317 3,92;7,8312 3,99;7,6910 0,33;7,6866 0,33;7,6476 1,02;7,6434 1,12;7,6283 1,07;7,6241 1,11;7,5577 0,89;7,5556 1,56;7,5534 0,79;7,5395 0,82;7,5368 0,92;7,5350 0,83;7,5323 0,74;7,5185 0,85;7,5139 0,73;7,1323 1,29;7,1122 1,16;7,0765 0,89;7,0741 0,85;7,0575 1,35;7,0557 1,19;7,0390 0,79;7,0366 0,71;7,0124 0,43;7,0047 0,33;6,9912 0,42;6,9858 0,49;6,9674 0,35;6,9022 1,35;6,3609 2,00;5,4215 10,07;5,0391 1,26;5,0021 0,57;3,9498 0,56;3,8868 16,00;3,8073 4,63;3,7942 1,21;3,3556 0,49;3,3456 0,35;3,3381 0,57;3,3281 0,99;3,3181 0,58;3,3108 0,41;3,3009 0,58;3,2899 0,44;2,2310 12,74;2,2294 10,23;2,1955 0,52;2,1638 0,59;2,0857 0,36;1,9912 41,03;1,9596 1,75;1,9524 1,81;1,9428 7,37;1,9367 4,93;1,9307 19,09;1,9246 33,26;1,9184 43,17;1,9123 29,21;1,9061 14,83;1,7469 0,34;1,2802 0,39;1,2203 0,34;1,2026 0,74;1,1847 0,39;0,9161 0,44;-0,0001 4,75 |

Die Intensität scharfer Signale korreliert mit der Höhe der Signale in einem gedruckten Beispiel eines NMR-Spektrums in cm und zeigt die wirklichen Verhältnisse der Signalintensitäten. Bei breiten Signalen können mehrere Peaks oder die Mitte des Signals und ihre relative Intensität im Vergleich zum intensivsten Signal im Spektrum gezeigt werden.

Die Listen der 1 H-NMR-Peaks sind ähnlich den klassischen 1 H-NMR-Ausdrucken und enthalten somit gewöhnlich alle Peaks, die bei einer klassischen NMR-Interpretation aufgeführt werden.

Darüber hinaus können sie wie klassische 1 H-NMR-Ausdrucke Lösungsmittelsignale, Signale von Stereoisomeren der Zielverbindungen, die ebenfalls Gegenstand der Erfindung sind, und/oder Peaks von Verunreinigungen zeigen.

Bei der Angabe von Verbindungssignalen im delta-Bereich von Lösungsmitteln und/oder Wasser sind in unseren Listen von 1 H-NMR-Peaks die gewöhnlichen Lösungsmittelpeaks, zum Beispiel Peaks von DMSO in DMSO-d6 und der Peak von Wasser, gezeigt, die gewöhnlich im Durchschnitt eine hohe Intensität aufweisen.

Die Peaks von Stereoisomeren der Targetverbindungen und/oder Peaks von Verunreinigungen haben gewöhnlich im Durchschnitt eine geringere Intensität als die Peaks der Zielverbindungen (zum Beispiel mit einer Reinheit von >90%).

Solche Stereoisomere und/oder Verunreinigungen können typisch für das jeweilige Herstellungsverfahren sein. Ihre Peaks können somit dabei helfen, die Reproduktion unseres Herstellungsverfahrens anhand von "Nebenprodukt-Fingerabdrücken" zu erkennen.

Einem Experten, der die Peaks der Zielverbindungen mit bekannten Verfahren (MestreC, ACD-Simulation, aber auch mit empirisch ausgewerteten Erwartungswerten) berechnet, kann je nach Bedarf die Peaks der Zielverbindungen isolieren, wobei gegebenenfalls zusätzliche Intensitätsfilter eingesetzt werden. Diese Isolierung wäre ähnlich dem betreffenden Peak-Picking bei der klassischen 1H-NMR-Interpretation.

### Verwendungsbeispiele

### Beispiel A

### Phytophthora-Test (Tomate) / protektiv

| | | |
|---|---|---|
| Lösungsmittel: | 49 | Gewichtsteile N, N - Dimethylformamid |
| Emulgator: | 1 | Gewichtsteil Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Tomatenpflanzen mit der Wirkstoffzubereitung in der angegebenen Aufwandmenge besprüht. 1 Tag nach der Behandlung werden die Pflanzen mit einer Sporensuspension von ***Phytophthora infestans*** inokuliert und stehen dann 24h bei 100 rel. Feuchte und 22°C. Anschließend werden die Pflanzen in einer Klimazelle bei ca. 96% relativer Luftfeuchtigkeit und einer Temperatur von ca. 20°C aufgestellt.

7 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, daß kein Befall beobachtet wird.

In diesem Test zeigen die nachfolgenden erfindungsgemäßen Verbindungen, I-2, I-3, I-4, I-5, I-6, I-7, I-8, I-11, I-13, I-14, I-15, I-17, I-18, I-19, I-20 und I-21, bei einer Konzentration an Wirkstoff von 500ppm einen Wirkungsgrad von 70% oder mehr.

Ferner zeigen in diesem Test die nachfolgenden Verbindungen bei einer Konzentration an Wirkstoff von 500ppm einen Wirkungsgrad von 70% oder mehr, wobei im einzelnen die in Klammern berichteteten Wirkungsgrade beobachtet werden können: I-16 (80%), I-22 (100%), I-23 (70%), I-24 (94%), I-25 (100%), I-29 (100%), I-30 (95%), I-33 (95%), I-34 (95%), I-35 (95%), I-36 (100%), I-37 (95%), I-38 (95%), I-39 (90%), I-41 (95%), I-42 (100%), I-43 (90%), I-44 (95%), I-45 (95%), I-46 (95%), I-47 (94%), I-48 (94%), I-49 (90%), I-50 (95%), I-51 (98%), I-52 (98%), I-53 (90%), I-54 (85%), I-55 (98%), I-57 (98%), I-60 (95%), I-61 (95%), I-65 (93%).

### Beispiel B

### Plasmopara-Test (Rebe) / protektiv

| | | |
|---|---|---|
| Lösungsmittel : | 24,5 | Gewichtsteile Aceton |
| | 24,5 | Gewichtsteile Dimethylacetamid |
| Emulgator: | 1 | Gewichtsteil Alkyl-Aryl-Polyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung in der angegebenen Aufwandmenge besprüht. Nach Antrocknen des Spritzbelages werden die Pflanzen mit einer wäßrigen Sporensuspension von ***Plasmopara viticola*** inokuliert und verbleiben dann 1 Tag in einer Inkubationskabine bei ca. 20°C und 100% relativer Luftfeuchtigkeit. Anschließend werden die Pflanzen 4 Tage im Gewächshaus bei ca. 21°C und ca. 90% Luftfeuchtigkeit aufgestellt. Die Pflanzen werden dann angefeuchtet und 1 Tag in eine Inkubationskabine gestellt.

6 Tage nach der Inokulation erfolgt die Auswertung. Dabei bedeutet 0% ein Wirkungsgrad, der demjenigen der Kontrolle entspricht, während ein Wirkungsgrad von 100% bedeutet, daß kein Befall beobachtet wird.

In diesem Test zeigen die nachfolgenden erfindungsgemäßen Verbindungen, I-3, I-11, I-13, I-14, I-16, I-17, I-18, I-19, I-20, I-21, bei einer Konzentration an Wirkstoff von 100ppm einen Wirkungsgrad von 70% oder mehr.

Ferner zeigen in diesem Test die nachfolgenden Verbindungen bei einer Konzentration an Wirkstoff von 500ppm einen Wirkungsgrad von 70% oder mehr, wobei im einzelnen die in Klammern berichteteten Wirkungsgrade beobachtet werden können: I-22 (100%), I-24 (100%), I-25 (96%), I-29 (95%), I-30 (85%), I-33 (89%), I-34 (84%), I-35 (89%), I-36 (96%), I-42 (90%), I-43 (100%), I-44 (74%), I-45 (100%), I-46 (100%), I-49 (79%), I-50 (93%), I-51 (100%), I-52 (94%), I-53 (76%), I-55 (100%), I-57 (91%).

## Patentansprüche

1. Verbindungen der Formel (**I**), 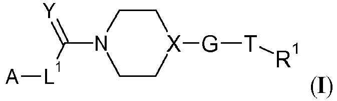 in welcher die Symbole folgende Bedeutungen haben:
A steht für Phenyl, das bis zu drei Substituenten enthalten kann,
wobei die Substituenten unabhängig voneinander aus R² ausgewählt sind,
oder
A' steht für ein gegebenenfalls benzokondensiertes, unsubstituiertes oder substituiertes 5- oder 6-gliedriges Heteroaryl, wobei die Substituenten am Kohlenstoff unabhängig voneinander aus R³ und die Substituenten am Stickstoff unabhängig voneinander aus R⁴ ausgewählt sind,
L¹ steht für NR⁵ oder C(R⁶)₂,
X steht für CH oder Stickstoff,
Y steht für Schwefel oder Sauerstoff,
G steht für: 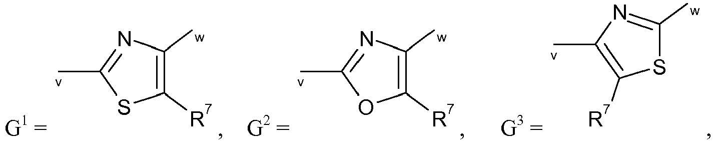 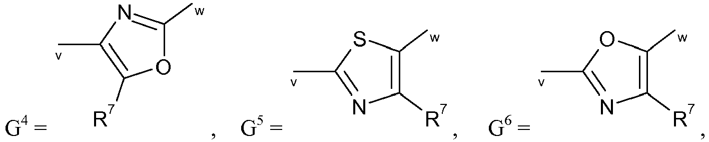 wobei die Bindung, die mit "v" identifiziert ist, direkt an X gebunden ist, und wobei die Bindung, die mit "w" identifiziert ist, direkt an T gebunden ist,
T steht für *-C(=O)CH₂-#, *-C(=O)CH₂C(R⁸)₂-#, *-C(=O)CH=CH-#, *-C(=O)C=C-#, *-C(=O)CH₂C(=O)-#, *-C≡CC(=O)-#, *-CH=CHC(=O)-#, *-CH₂CH₂C(=O)-#, *-C(=S)CH₂-#, *-C(=S)CH₂C(R⁸)₂-#, *-C(=S)CH=CH-#, *-C(=S)C=C-#, *-C=CC(=S)-#, *-CH=CHC(=S)-#, *-CH₂CH₂C(=S)-#, *-C(=NR⁹)CH₂-#, *-C(=NR⁹)CH₂C(R⁸)₂-# oder *-CH₂CH₂C(=NR⁹)- #, *-C(=NR⁹)CH=CH- #, *-CH=CHC(=NR⁹)- #, *-CH=CHC(NOH)-#, *-CH=CHC(NO-C₁-C₄-Alkyl)CH₂-#,
wobei die Bindung, die mit * identifiziert ist, direkt an G gebunden ist, und wobei die Bindung, die mit # identifiziert ist, direkt an R¹ gebunden ist,
R¹ ausgewählt ist aus der Gruppe enthaltend R¹ₐ, R¹_{b}, R¹_{c}, R¹_{d}, R¹ₑ, R¹_{f}, R¹_{g} oder R¹ₙ, wobei
R¹ₐ steht für C₁-C₆-Alkyl, C₁-C₆-Halogenalkyl, C₂-C₆-Alkenyl, C₂-C₆-Alkinyl, C₂-C₈-Alkoxyalkyl oder C₅-C₉-Cycloalkoxyalkyl oder
R¹_{b} steht für unsubstituiertes oder substituiertes C₃-C₁₀-Cycloalkyl,
wobei die Substituenten unabhängig voneinander aus -Q oder R¹⁰ ausgewählt sind oder
R¹_{c} steht für unsubstituiertes oder substituiertes C₅-C₁₀-Cycloalkenyl,
wobei die Substituenten unabhängig voneinander aus R¹¹ ausgewählt sind oder
R¹_{d} steht für unsubstituiertes oder substituiertes Phenyl,
wobei die Substituenten unabhängig voneinander aus -L²Q oder R¹² ausgewählt sind oder
R¹ₑ steht für unsubstituiertes oder substituiertes, Naphthalen-1-yl, Naphthalen-2-yl, 1,2,3,4-Tetrahydronaphthalen-l-yl, 1,2,3,4-Tetrahydronaphthalen-2-yl, 5,6,7,8-Tetrahydronaphthalen-1-yl, 5,6,7,8-Tetrahydronaphthalen-2-yl, Decalin-1-yl, De-calin-2-yl, 1H-Inden-1-yl, 1H-Inden-2-yl, 1H-Inden-3-yl, 1H-Inden-4-yl, 1H-Inden-5-yl, 1H-Inden-6-yl, 1H-Inden-7-yl, Indan-1-yl, Indan-2-yl, Indan-3-yl, In-dan-4-yl oder Indan-5-yl,
wobei die Substituenten unabhängig voneinander aus R¹³ ausgewählt sind oder
R¹_{f} steht für einen unsubstituierten oder substituierten 5- oder 6-gliedrigen Heteroaryl-rest, wobei die Substituenten am Kohlenstoff unabhängig voneinander aus -L²Q oder R¹⁴ ausgewählt sind, und die Substituenten am Stickstoff unabhängig voneinander aus -L³Q oder R¹⁵ ausgewählt sind oder
R¹_{g} steht für benzokondensiertes unsubstituiertes oder substituiertes 5- oder 6- gliedriges Heteroaryl, wobei die Substituenten am Kohlenstoff unabhängig voneinander aus R¹⁶ ausgewählt sind, und die Substituenten am Stickstoff unabhängig voneinander aus R¹⁷ ausgewählt sind oder
R¹ₕ steht für unsubstituiertes oder substituiertes C₅-C₁₅-Heterocyclyl, wobei die Substituenten am Kohlenstoff unabhängig voneinander ausgewählt aus R¹⁸ sind, und die Substituenten am Stickstoff unabhängig voneinander ausgewählt aus R¹⁹ sind,
L² steht für eine direkte Bindung, -O-, -C(R²⁰)₂- oder -NR²¹-,
L³ steht für eine direkte Bindung oder -C(R²⁰)₂-,
Q ist ausgewählt aus der Gruppe enthaltend Q', Q" und Q"', wobei
Q' steht für ein Phenyl, das bis zu zwei Substituenten enthalten kann, wobei die Substituenten unabhängig voneinander aus R²² ausgewählt sind oder
Q" steht für einen 5- oder 6-gliedrigen Heteroarylrest, der bis zu zwei Substituenten enthalten kann, wobei die Substituenten am Kohlenstoff unabhängig voneinander aus R²³ ausgewählt sind und die Substituenten am Stickstoff unabhängig voneinander aus R²⁴ ausgewählt sind oder
Q"' steht für gesättigtes oder teilweise ungesättigtes C₃-C₁₀-Cycloalkyl,
R², R¹² und R²² stehen unabhängig voneinander für Halogen, Cyano, Hydroxy, SH, Amino, Nitro, Phenyl, C(=O)H, C(=O)OH, CONR²⁵R²⁶ NR²⁵R²⁶, C₁-C₆-Alkyl, C₂-C₆-Alkenyl, C₂-C₆-Alkinyl, C₁-C₆-Halogenalkyl, C₂-C₆-Halogenalkenyl, C₂-C₆-Halogenalkinyl, C₃-C₈-Cycloalkyl, C₃-C₈-Halogencycloalkyl, C₄-C₁₀-Alkyl-cycloalkyl, C₄-C₁₀-Cycloalkylalkyl, C₆-C₁₄-Cycloalkylcycloalkyl, C₄-C₁₀-Halogencycloalkylalkyl, C₅-C₁₀-Alkylcycloalkylalkyl, C₃-C₈-Cycloalkenyl, C₃-C₈-Halogencycloalkenyl, C₂-C₆-Alkoxyalkyl, C₄-C₁₀-Cycloalkoxyalkyl, C₃-C₈-Alkoxyalkoxyalkyl, C₂-C₆-Alkylthioalkyl, C₂-C₆-Alkylsulfinylalkyl, C₂-C₆-Alkylsulfonylalkyl, C₂-C₆-Alkylaminoalkyl, C₃-C₈-Dialkylaminoalkyl, C₂-C₆-Halogenalkylaminoalkyl, C₄-C₁₀-Cycloalkylaminoalkyl, C₂-C₆-Alkylcarbonyl, C₂-C₆-Halogenalkylcarbonyl, C₄-C₈-Cycloalkylcarbonyl, C₂-C₆-Alkoxycarbonyl, C₄-C₈-Cycloalkoxycarbonyl, C₅-C₁₀-Cycloalkylalkoxycarbonyl, C₂-C₆-Alkylaminocarbonyl, C₃-C₈-Dialkylaminocarbonyl, C₄-C₈-Cycloalkylaminocarbonyl, C₂-C₆-Halogenalkoxyalkyl, C₁-C₆-Hydroxyalkyl, C₁-C₆-Alkoxy, C₁-C₆-Halogenalkoxy, C₃-C₈-Cycloalkoxy, C₃-C₈-Halogencycloalkoxy, C₄-C₁₀-Cycloalkylalkoxy, C₃-C₆-Alkenyloxy, C₃-C₆-Halogenalkenyloxy, C₃-C₆-Alkinyloxy, C₃-C₆-Halogenalkinyloxy, C₂-C₆-Alkoxyalkoxy, C₂-C₆-Alkylcarbonyloxy, C₂-C₆-Halogenalkylcarbonyloxy, C₄-C₈-Cycloalkylcarbonyloxy, C₃-C₆-Alkylcarbonylalkoxy, C₁-C₆-Alkylthio, C₁-C₆-Halogenalkylthio, C₃-C₆-Cycloalkylthio, C₁-C₆-Alkylsulfinyl, C₁-C₆-Halogenalkylsulfinyl, C₁-C₆-Alkylsulfonyl, C₁-C₆-Halogenalkylsulfonyl, C₃-C₈-Cycloalkylsulfonyl, Tri(C₁-C₄-alkyl)silyl, C₁-C₆-Alkylsulfonylamino oder C₁-C₆-Halogenalkylsulfonylamino,
R³, R¹⁴, R¹⁶, R¹⁸ und R²³ stehen unabhängig voneinander für Halogen, Cyano, Hydroxy, SH, Amino, Nitro, NR²⁵R²⁶, C₁-C₆-Alkyl, C₂-C₆-Alkenyl, C₂-C₆-Alkinyl, C₁-C₆-Halogenalkyl, C₂-C₆-Halogenalkenyl, C₂-C₆-Halogenalkinyl, C₃-C₆-Cycloalkyl, C₃-C₆-Halogencycloalkyl, C₄-C₁₀-Alkylcycloalkyl, C₄-C₁₀-Cycloalkylalkyl, C₆-C₁₄-Cycloalkylcycloalkyl, C₅-C₁₀-Alkylcycloalkylalkyl, C₂-C₄-Alkoxyalkyl, C₂-C₄-Alkylcarbonyl, C₂-C₆-Alkoxycarbonyl, C₂-C₆-Alkylaminocarbonyl, C₃-C₈-Dialkylaminocarbonyl, C₁-C₄-Hydroxyalkyl, C₁-C₄-Alkoxy, C₃-C₆-Alkenyloxy, C₃-C₆-Alkinyloxy, C₁-C₄-Halogenalkoxy, C₁-C₆-Alkylcarbonyloxy, C₂-C₆-Alkylcarbonylthio, C₁-C₄-Alkylthio, C₁-C₄-Halogenalkylthio, C₁-C₄-Alkylsulfinyl, C₁-C₄-Halogenalkylsulfinyl, C₁-C₄-Alkylsulfonyl, C₁-C₄-Halogenalkylsulfonyl, Tri(C₁-C₄-alkyl)silyl oder Phenyl,
R⁴, R¹⁵, R¹⁷, R¹⁹ und R²⁴ stehen unabhängig voneinander für C₁-C₆-Alkyl, C₂-C₆-Alkenyl, C₂-C₆-Alkinyl, C₁-C₆-Halogenalkyl, C₃-C₆-Halogenalkenyl, C₃-C₆-Halogenalkinyl, C₃-C₆-Cycloalkyl, C₃-C₆-Halogencycloalkyl, C₄-C₁₀-Alkylcycloalkyl, C₄-C₁₀-Cycloalkylalkyl, Phenyl, Benzyl, C₁-C₄-Alkylsulfonyl, C(=O)H, C₂-C₄-Alkylcarbonyloxy, C₂-C₅-Alkoxycarbony, C₂-C₅-oder Alkylcarbonyl,
R⁵ steht für Wasserstoff oder C₁-C₄-Alkyl,
R⁶ steht unabhängig voneinander für Wasserstoff, C₁-C₄-Alkyl, C₁-C₄-Halogenalkyl, Cyclopropyl, Halogen,
oder die beiden Reste R⁶ bilden zusammen mit dem Kohlenstoffatom, an das sie gebunden sind, einen Cyclopropylring,
R⁷ steht für Wasserstoff, C₁-C₃-Alkyl, C₁-C₃-Halogenalkyl, C₁-C₄-Alkoxy, C₂-C₅-Alkoxycarbonyl oder Halogen,
R⁸ steht unabhängig voneinander für Wasserstoff oder C₁-C₄-Alkyl,
R⁹ steht für OH, Nitro, Cyano, C₁-C₄-Alkyl, C₁-C₄-Alkylamino, C₂-C₄-Dialkylamino, C₁-C₄-Alkoxy, C₁-C₄-Halogenalkoxy oder Cyclopropyloxy
R¹⁰und R¹¹ stehen unabhängig voneinander für Cyano, Halogen, C₁-C₆-Alkyl, C₁-C₆-Halogenalkyl, C₃-C₆-Cycloalkyl, C₂-C₆-Alkenyl, C₂-C₆-Halogenalkenyl, C₂-C₆-Alkinyl, C₂-C₆-Halogenalkinyl, Tri(C₁-C₄-alkyl)silyl, Phenyl, Hydroxy, Oxo, C₁-C₆-Alkoxy, C₁-C₆-Halogenalkoxy, C₃-C₆-Alkenyloxy, C₃-C₆-Alkinyloxy, C₁-C₆-Alkylthio oder C₁-C₆-Halogenalkylthio,
R¹³ steht unabhängig voneinander für Cyano, Nitro, Halogen, C₁-C₆-Alkyl, C₁-C₄-Halogenalkyl, C₃-C₆-Cycloalkyl, C₂-C₆-Alkenyl, C₂-C₆-Halogenalkenyl, C₂-C₆-Alkinyl, C₁-C₆-Halogenalkinyl, Tri(C₁-C₄-alkyl)silyl, Benzyl, Phenyl, Hydroxy, SH, C₁-C₆-Alkoxy, C₁-C₆-Halogenalkoxy, C₃-C₆-Alkenyloxy, C₃-C₆-Alkinyloxy, C₁-C₆-Alkylthio oder C₁-C₆-Halogenalkylthio,
R²⁰ steht unabhängig voneinander für Wasserstoff, Halogen, C₁-C₄-Alkyl oder C₁-C₄-Halogenalkyl,
R²¹ steht für C₁-C₆-Alkyl, C₁-C₆-Halogenalkyl, C₃-C₈-Cycloalkyl, C₂-C₆-Alkylcarbonyl, C₂-C₆-Halogenalkylcarbonyl, C₂-C₆-Alkoxycarbonyl oder C₂-C₆-Halogenalkoxycarbonyl,
R²⁵ und R²⁶ stehen unabhängig voneinander für Wasserstoff, C₁-C₄-Alkyl, C₂-C₆-Alkenyl, C₂-C₆-Alkinyl, C₁-C₃-Halogenalkyl, C₃-C₆-Cycloalkyl, Benzyl oder Phenyl,
sowie agrochemisch wirksame Salze davon.

2. Verbindungen der Formel (I) nach Anspruch 1,
in welcher die Symbole folgende Bedeutungen haben,
A steht für Phenyl, das bis zu zwei Substituenten enthalten kann, wobei die Substituenten unabhängig voneinander aus R² Liste ausgewählt sind und R² steht für:
Halogen, Cyano, Hydroxy, Amino, Nitro, C₁-C₆-Alkyl, C₂-C₆-Alkenyl, C₂-C₆-Alkinyl, C₃-C₈-Cycloalkyl, C₁-C₆-Halogenalkyl, C₂-C₆-Halogenalkenyl, C₂-C₆-Halogenalkinyl, C₃-C₆-Halogencycloalkyl, C₁-C₄-Alkoxy, C₁-C₄-Halogenalkoxy, C₁-C₄-Alkylthio, C₁-C₄-Alkylsulfonyl, C₁-C₄-Halogenalkylthio, C₁-C₄-Halogenalkylsulfonyl, C₂-C₄-Alkoxyalkyl, C₂-C₅-Alkylcarbonyl, C₂-C₆-Alkoxycarbonyl, C₂-C₆-Alkylcarbonyloxy oder C(=O)H,
A' steht für einen heteroaromatischen Rest ausgewählt aus folgender Gruppe: Furan-2-yl, Furan-3-yl, Thiophen-2-yl, Thiophen-3-yl, Isoxazol-3-yl, Isoxazol-4-yl, Isoxazol-5-yl, Pyrrol-1-yl, Pyrrol-2-yl, Pyrrol-3-yl, Oxazol-2-yl, Oxazol-4-yl, Oxazol-5-yl, Thiazol-2-yl, Thiazol-4-yl, Thiazol-5-yl, Isothiazol-3-yl, Isothiazol-4-yl, Isothiazol-5-yl, Pyrazol-1-yl, Pyrazol-3-yl, Pyrazol-4-yl, Imidazol-1-yl, Imidazol-2-yl, Imidazol-4-yl, 1,2,3-Triazol-1-yl, 1,2,4-Triazol-1-yl, Pyridin-2-yl, Pyridin-3-yl, Pyridin-4-yl, Pyridazin-3-yl, Pyridazin-4-yl, Pyrazin-2-yl, Pyrazin-3-yl, Pyrimidin-2-yl, Pyrimidin-4-yl oder Pyrimidin-5-yl, der bis zu zwei Substituenten enthalten kann, wobei die Substituenten unabhängig voneinander aus R³ und R⁴ ausgewählt sind und
R³ steht für einen Substituenten am Kohlenstoff:
Halogen, Cyano, Hydroxy, Amino, Nitro, C₁-C₆-Alkyl, C₂-C₆-Alkenyl, C₂-C₆-Alkinyl, C₃-C₈-Cycloalkyl, C₁-C₆-Halogenalkyl, C₂-C₆-Halogenalkenyl, C₂-C₆-Halogenalkinyl, C₃-C₆-Halogencycloalkyl, C₁-C₄-Alkoxy, C₁-C₄-Halogenalkoxy, C₁-C₄-Alkylthio, C₁-C₄-Alkylsulfonyl, C₁-C₄-Halogenalkylthio, C₁-C₄-Halogenalkylsulfonyl, C₂-C₄-Alkoxyalkyl, C₂-C₆-Alkoxycarbonyl, C₂-C₆-Alkylcarbonyloxy, C(=O)H' oder Phenyl
R⁴ steht für einen Substituenten am Stickstoff:
C₁-C₆-Alkyl, C₁-C₄-Halogenalkyl, C₃-C₆-Alkenyl, C₃-C₆-Halogenalkenyl, C₃-C₆-Alkinyl oder C₃-C₆-Halogenalkinyl.
G steht für G¹ oder G²,
T steht für *-C(=O)CH₂-# *-C(=O)CH₂C(R⁸)₂-#, *-C(=O)CH=CH-#, *-C(=O)C=C-#, *-C=CC(=O)-#, *-CH=CHC(=O)-#, *-CH₂CH₂C(=O)-#, *-C(=S)CH₂-#, *-C(=S)CH₂C(R⁸)₂-#, *-CH₂CH₂C(=S)- #, *-C(=NR⁹)CH₂-# *-C(=NR⁹)CH=CH-#, *-CH=CHC(=NR⁹)-#, *-CH=CHC(NOH)-# oder *-CH=CHC(NO-C₁-C₄-Alkyl)CH₂-#,
L¹ für CH₂ oder NR⁵,
X steht für CH,
Y steht für Sauerstoff,
R¹ₐ steht für C₁-C₆-Alkyl, C₁-C₆-Halogenalkyl, C₂-C₆-Alkenyl oder C₂-C₆-Alkinyl, C₂-C₆-Alkoxyalkyl,
R¹_{b} steht für unsubstituiertes oder substituiertes C₃-C₁₀-Cycloalkyl, wobei die Substituenten unabhängig voneinander aus -Q oder R¹⁰ ausgewählt sind und R¹⁰ steht für:
Cyano, Halogen, C₁-C₆-Alkyl, C₁-C₆-Halogenalkyl, C₃-C₆-Cycloalkyl, C₂-C₆-Alkenyl, C₂-C₆-Halogenalkenyl, C₂-C₆-Alkinyl, C₂-C₆-Halogenalkinyl, Tri(C₁-C₄-alkyl)silyl, Hydroxy, Oxo, C₁-C₆-Alkoxy, C₁-C₆-Halogenalkoxy, C₃-C₆-Alkenyloxy, C₃-C₆-Alkinyloxy, C₁-C₆-Alkylthio, C₁-C₆-Halogenalkylthio,
R¹_{c} steht für unsubstituiertes oder substituiertes C₅-C₁₀-Cycloalkenyl, wobei die Substituenten unabhängig voneinander aus R¹¹ ausgewählt sind und R¹¹ steht für:
Cyano, Chlor, Fluor, C₁-C₆-Alkyl, C₁-C₆-Halogenalkyl, C₃-C₆-Cycloalkyl, C₂-C₆-Alkenyl, C₂-C₆-Halogenalkenyl, C₂-C₆-Alkinyl, C₂-C₆-Halogenalkinyl, Phenyl, Oxo, C₁-C₆-Alkoxy, C₁-C₆-Halogenalkoxy, C₃-C₆-Alkenyloxy, C₃-C₆-Alkinyloxy, C₁-C₆-Alkylthio oder C₁-C₆-Halogenalkylthio,
R¹_{d} steht für unsubstituiertes oder substituiertes Phenyl, wobei die Substituenten unabhängig voneinander aus -L²Q oder R¹² ausgewählt sind und R¹² steht für:
Halogen, Cyano, Hydroxy, SH Amino, Nitro, C(=O)H, C(=O)OH, CONR²⁵R²⁶, NR²⁵R²⁶, C₁-C₆-Alkyl, C₂-C₆-Alkenyl, C₂-C₆-Alkinyl, C₁-C₆-Halogenalkyl, C₂-C₆-Halogenalkenyl, C₂-C₆-Halogenalkinyl, C₃-C₈-Cycloalkyl, C₃-C₈-Halogen-cycloalkyl, C₄-C₁₀-Alkylcycloalkyl, C₄-C₁₀-Cycloalkylalkyl, C₆-C₁₄-Cycloalkylcycloalkyl, C₄-C₁₀-Halogencycloalkylalkyl, C₅-C₁₀-Alkylcycloalkylalkyl, C₃-C₈-Cycloalkenyl, C₃-C₈-Halogencycloalkenyl, C₂-C₆-Alkoxyalkyl, C₄-C₁₀-Cycloalkoxyalkyl, C₃-C₈-Alkoxyalkoxyalkyl, C₂-C₆-Alkylthioalkyl, C₂-C₆-Alkylsulfinylalkyl, C₂-C₆-Alkylsulfonylalkyl, C₂-C₆-Alkylaminoalkyl, C₃-C₈-Dialkylaminoalkyl, C₂-C₆-Halogenalkylaminoalkyl, C₄-C₁₀-Cycloalkylaminoalkyl, C₂-C₆-Alkylcarbonyl, C₂-C₆-Halogenalkylcarbonyl, C₄-C₈-Cycloalkylcarbonyl, C₂-C₆-Alkoxycarbonyl, C₄-C₈-Cycloalkoxycarbonyl, C₅-C₁₀-Cycloalkylalkoxycarbonyl, C₂-C₆-Alkylaminocarbonyl, C₃-C₈-Dialkylaminocarbonyl, C₄-C₈-Cycloalkylaminocarbonyl, C₂-C₆-Halogenalkoxyalkyl, C₁-C₆-Hydroxyalkyl, C₁-C₆-Alkoxy, C₁-C₆-Halogenalkoxy, C₃-C₈-Cycloalkoxy, C₃-C₈-Halogencycloalkoxy, C₄-C₁₀-Cycloalkylalkoxy, C₃-C₆-Alkenyloxy, C₃-C₆-Halogenalkenyloxy, C₃-C₆-Alkinyloxy, C₃-C₆-Halogenalkinyloxy, C₂-C₆-Alkoxyalkoxy, C₂-C₆-Alkylcarbonyloxy, C₂-C₆-Halogenalkylcarbonyloxy, C₄-C₈-Cycloalkylcarbonyloxy, C₃-C₆-Alkylcarbonylalkoxy, C₁-C₆-Alkylthio, C₁-C₆-Halogenalkylthio, C₃-C₆-Cycloalkylthio, C₁-C₆-Alkylsulfinyl, C₁-C₆-Halogenalkylsulfinyl, C₁-C₆-Alkyl-sulfonyl, C₁-C₆-Halogenalkylsulfonyl, C₃-C₈-Cycloalkylsulfonyl, Tri(C₁-C₄-alkyl)silyl, C₁-C₆-Alkylsulfonylamino, C₁-C₆-Halogenalkylsulfonylamino,
R¹ₑ steht für unsubstituiertes oder substituietes, Naphthalen-1-yl, Naphthalen-2-yl, 1,2,3,4-Tetrahydronaphthalen-1-yl, 1,2,3,4-Tetrahydronaphthalen-2-yl, 5,6,7,8-Tetrahydronaphthalen-1-yl, 5,6,7,8-Tetrahydronaphthalen-2-yl, Decalin-1-yl, Decalin-2-yl, 1H-Inden-1-yl, 1H-Inden-2-yl, 1H-Inden-3-yl, 1H-Inden-4-yl, 1H-Inden-5-yl, 1H-Inden-6-yl, 1H-Inden-7-yl, Indan-1-yl, Indan-2-yl, Indan-3-yl, Indan-4-yl oder Indan-5-yl, wobei die Substituenten unabhängig voneinander aus R¹³ ausgewählt sind und R¹³ steht für:
Cyano, Halogen, C₁-C₆-Alkyl, C₁-C₄-Halogenalkyl, C₂-C₆-Alkenyl, C₂-C₆-Alkinyl, Benzyl, Phenyl, C₁-C₆-Alkoxy, C₁-C₆-Halogenalkoxy, C₃-C₆-Alkenyloxy, C₃-C₆-Alkinyloxy oder C₁-C₆-Alkylthio,
R¹_{f} steht für einen unsubstituierten oder substituierten 5- oder 6-gliedrigen Heteroarylrest, wobei die Substituenten unabhängig voneinander aus -L²Q oder R¹⁴ und -L³Q oder R¹⁵ ausgewählt sind und R¹⁴ steht für einen Substituenten am Kohlenstoff:
Halogen, Cyano, Hydroxy, SH Amino, Nitro, NR²⁵R²⁶, C₁-C₆-Alkyl, C₂-C₆-Alkenyl, C₂-C₆-Alkinyl, C₁-C₆-Halogenalkyl, C₂-C₆-Halogenalkenyl, C₂-C₆-Halogenalkinyl, C₃-C₆-Cycloalkyl, C₃-C₆-Halogencycloalkyl, C₄-C₁₀-Alkyl-cycloalkyl, C₄-C₁₀-Cycloalkylalkyl, C₆-C₁₄-Cycloalkylcycloalkyl, C₅-C₁₀-Alkylcycloalkylalkyl, C₂-C₄-Alkoxyalkyl, C₃-C₆-Alkenyloxy, C₃-C₆-Alkinyloxy, C₂-C₄-Alkylcarbonyl, C₂-C₆-Alkoxycarbonyl, C₂-C₆-Alkylaminocarbonyl, C₃-C₈-Dialkylaminocarbonyl, C₁-C₄-Hydroxyalkyl, C₁-C₄-Alkoxy, C₁-C₄-Halogenalkoxy, C₂-C₆-Alkylcarbonyloxy, C₂-C₆-Alkylcarbonylthio, C₁-C₄-Alkylthio, C₁-C₄-Halogenalkylthio, C₁-C₄-Alkylsulfinyl, C₁-C₄-Halogenalkylsulfinyl, C₁-C₄-Alkyl-sulfonyl, C₁-C₄-Halogenalkylsulfonyl, Tri(C₁-C₄-alkyl)silyl,
R¹⁵ steht für einen Substituenten am Stickstoff:
C₁-C₆-Alkyl, C₁-C₆-Alkenyl, C₃-C₆-Alkinyl, C₁-C₆-Halogenalkyl, C₃-C₆-Halogen-alkenyl, C₃-C₆-Halogenalkinyl, C₃-C₆-Cycloalkyl, C₃-C₆-Halogencycloalkyl, C₄-C₁₀-Alkylcycloalkyl, C₄-C₁₀-Cycloalkylalkyl, Phenyl,
R¹_{g} steht für benzokondensiertes unsubstituiertes oder substituiertes 5- oder 6- gliedriges Heteroaryl, wobei die Substituenten unabhängig voneinander ausgewählt sind aus R¹⁶ und R¹⁷ und R¹⁶ steht für einen Substituenten am Kohlenstoff:
Halogen, Cyano, Hydroxy, SH, Amino, Nitro, NR²⁵R²⁶, C₁-C₆-Alkyl, C₂-C₆-Alkenyl, C₂-C₆-Alkinyl, C₁-C₆-Halogenalkyl, C₂-C₆-Halogenalkenyl, C₂-C₆-Halogenalkinyl, C₃-C₆-Cycloalkyl, C₃-C₆-Halogencycloalkyl, C₄-C₁₀-Alkyl-cycloalkyl, C₄-C₁₀-Cycloalkylalkyl, C₆-C₁₄-Cycloalkylcycloalkyl, C₅-C₁₀-Alkylcycloalkylalkyl, C₂-C₄-Alkoxyalkyl, C₂-C₄-Alkylcarbonyl, C₂-C₆-Alkoxycarbonyl, C₂-C₆-Alkylaminocarbonyl, C₃-C₈-Dialkylaminocarbonyl, C₁-C₄-Hydroxyalkyl, C₁-C₄-Alkoxy, C₁-C₄-Halogenalkoxy, C₂-C₆-Alkylcarbonyloxy, C₂-C₆-Alkylcarbonylthio, C₁-C₄-Alkylthio, C₁-C₄-Halogenalkylthio, C₁-C₄-Alkylsulfinyl, C₁-C₄-Halogenalkylsulfinyl, C₁-C₄-Alkylsulfonyl, C₁-C₄-Halogenalkylsulfonyl, Tri(C₁-C₄-alkyl)silyl oder Phenyl,
R¹⁷ steht für einen Substituenten am Stickstoff:
C₁-C₆-Alkyl, C₃-C₆-Alkenyl, C₃-C₆-Alkinyl, C₁-C₆-Halogenalkyl, C₃-C₆-Halogen-alkenyl, C₃-C₆-Halogenalkinyl, C₃-C₆-Cycloalkyl, C₃-C₆-Halogencycloalkyl, C₄-C₁₀-Alkylcycloalkyl, C₄-C₁₀-Cycloalkylalkyl oder Phenyl,
R¹ₕ steht für unsubstituiertes oder substituiertes C₅-C₁₅-Heterocyclyl, wobei mögliche Substituenten unabhängig voneinander ausgewählt sind aus R¹⁸ und R¹⁹ und
R¹⁸ steht für einen Substituenten am Kohlenstoff:
Halogen, Cyano, Hydroxy, SH, Amino, Nitro, NR²⁵R²⁶, C₁-C₆-Alkyₗ, C₂-C₆-Alkenyl, C₂-C₆-Alkinyl, C₁-C₆-Halogenalkyl, C₂-C₆-Halogenalkenyl, C₂-C₆-Halogenalkinyl, C₃-C₆-Cycloalkyl, C₃-C₆-Halogencycloalkyl, C₄-C₁₀-Alkyl-cycloalkyl, C₄-C₁₀-Cycloalkylalkyl, C₆-C₁₄-Cycloalkylcycloalkyl, C₅-C₁₀-Alkylcycloalkylalkyl, C₂-C₄-Alkoxyalkyl, C₂-C₄-Alkylcarbonyl, C₂-C₆-Alkoxycarbonyl, C₂-C₆-Alkylaminocarbonyl, C₃-C₈-Dialkylanünocarbonyl, C₁-C₄-Hydroxyalkyl, C₁-C₄-Alkoxy, C₁-C₄-Halogenalkoxy, C₂-C₆-Alkylcarbonyloxy, C₂-C₆-Alkylcarbonylthio, C₁-C₄-Alkylthio, C₁-C₄-Halogenalkylthio, C₁-C₄-Alkylsulfinyl, C₁-C₄-Halogenalkylsulfmyl, C₁-C₄-Alkylsulfonyl, C₁-C₄-Halogenalkylsulfonyl, Tri(C₁-C₄-alkyl)silyl oder Phenyl,
R¹⁹ steht für einen Substituenten am Stickstoff:
C₁-C₆-Alkyl, C₃-C₆-Alkenyl, C₃-C₆-Alkinyl, C₁₀-C₆-Halogenalkyl, C₃-C₆-Halogenalkenyl, C₃-C₆-Halogenalkinyl, C₃-C₆-Cycloalkyl, C₃-C₆-Halogencycloalkyl, C₄-C₁₀-Alkylcycloalkyl, C₄-C₁₀-Cycloalkylalkyl oder Phenyl,
L² steht für eine direkt Bindung oder - O -,
L³ steht für eine direkt Bindung,
Q' steht für Phenyl, das bis zu zwei Substituenten enthalten kann, wobei die Substituenten unabhängig voneinander aus R²² ausgewählt sind und R²² steht für:
Fluor, Chlor, Brom, Iod, Cyano, Hydroxy, SH, Nitro, C₁-C₆-Alkyl, C₁-C₆-Halogenalkyl, C₃-C₆-Cycloalkyl, C₁-C₄-Alkoxy, C₁-C₄-Halogenalkoxy, C₁-C₄-Alkylthio oder C₁-C₄-Halogenalkylthio,
Q" steht für Furan-2-yl, Furan-3-yl, Thiophen-2-yl, Thiophen-3-yl, Isoxazol-3-yl, Isoxazol-4-yl, Isoxazol-5-yl, Pyrrol-1-yl, Pyrrol-2-yl, Pyrrol-3-yl, Oxazol-2-yl, Oxazol-4-yl, Oxazol-5-yl, Thiazol-2-yl, Thiazol-4-yl, Thiazol-5-yl, Isothiazol-3-yl, Isothiazol-4-yl, Isothiazol-5-yl, Pyrazol-1-yl, Pyrazol-3-yl, Pyrazol-4-yl, Imidazol-1-yl, Imidazol-2-yl, Imidazol-4-yl, 1,2,4-Oxadiazol-3-yl, 1,2,4-Oxadiazol-5-yl, 1,3,4-Oxadiazol-2-yl, Tetrazol-5-yl, 1,2,4-Thiadiazol-3-yl, 1,2,4-Thiadiazol-5-yl, 1,3,4-Thiadiazol-2-yl, 1,2,3-Triazol-1-yl, 1,2,3-Triazol-2-yl, 1,2,3-Triazol-4-yl, 1,2,4-Triazol-1-yl, 1,2,4-Triazol-3-yl, 1,2,4-Triazol-4-yl, Tetrazol-5-yl, Pyridin-2-yl, Pyridin-3-yl, Pyridin-4-yl, Pyridazin-3-yl, Pyridazin-4-yl, Pyrimidin-2-yl, Pyrimidin-4-yl, Pyrimidin-5-yl, Pyrazin-2-yl, 1,3,5-Triazin-2-yl oder 1,2,4-Triazin-3-yl, welche jeweils bis zu zwei Substituenten enthalten können, wobei die Substituenten unabhängig voneinander aus R²³ und R²⁴ ausgewählt sind und R²³ steht für einen Substituenten am Kohlenstoff:
Fluor, Chlor, Brom, Iod, Cyano, Hydroxy, SH, Nitro, C₁-C₆-Alkyl, C₁-C₆-Halogenalkyl, C₃-C₆-Cycloalkyl, C₁-C₄-Alkoxy, C₁-C₄-Halogenalkoxy, C₁-C₄-Alkylthio oder C₁-C₄-Halogenalkylthio,
R²⁴ steht für einen Substituenten am Stickstoff:
C₁-C₆-Alkyl, C₃-C₆-Alkenyl, C₃-C₆-Alkinyl, C₃-C₆-Cycloalkyl oder Phenyl,
R⁵ steht für Wasserstoff oder Methyl, Ethyl, Propyl, *iso*-Propyl, Butyl, *iso*-Butyl oder *tert-*Butyl*,*
R⁷ steht für Wasserstoff,
R⁸ steht unabhängig voneinander für Wasserstoff, Methyl oder Ethyl,
R²⁵ und R²⁶ stehen unabhängig voneinander für Wasserstoff, Methyl, Ethyl, Propyl, *iso*-Propyl, Butyl, *iso*-Butyl oder *tert-*Butyl*,*
sowie agrochemisch wirksame Salze davon.

3. Verbindungen der Formel **(I)** nach einem oder mehreren der Ansprüche 1 bis 2, in welcher die Symbole folgende Bedeutungen haben,
A steht für Phenyl, das bis zu zwei Substituenten enthalten kann, wobei die Substituenten unabhängig voneinander ausgewählt sind aus R² und R² steht für:
Fluor, Brom, Iod, Chlor, Cyano, Nitro, Methyl, Ethyl, Propyl, 1-Methylethyl, 1,1-Dimethylethyl, Chlorfluormethyl, Dichlormethyl, Dichlorfluormethyl, Difluormethyl, Trichlormethyl, Trifluormethyl, Cyclopropyl, Ethoxy, 1-Methylethoxy, Propoxy, Methoxy, Trifluormethoxy, Difluormethoxy, 1-Methylethylthio, Methylthio, Ethylthio, Propylthio, Difluormethylthio oder Trifluormethylthio,
A' steht für einen heteroaromatischen Rest ausgewählt aus folgender Gruppe: Furan-2-yl, Furan-3-yl, Thiophen-2-yl, Thiophen-3-yl, Isoxazol-3-yl, Isoxazol-4-yl, Isoxazol-5-yl, Pyrrol-1-yl, Pyrrol-2-yl, Pyrrol-3-yl, Oxazol-2-yl, Oxazol-4-yl, Oxazol-5-yl, Thiazol-2-yl, Thiazol-4-yl, Thiazol-5-yl, Isothiazol-3-yl, Isothiazol-4-yl, Isothiazol-5-yl, Pyrazol-1-yl, Pyrazol-3-yl, Pyrazol-4-yl, Imidazol-1-yl, Imidazol-2-yl, Imidazol-4-yl, 1,2,3-Triazol-1-yl, 1,2,4-Triazol-1-yl, Pyridin-2-yl, Pyridin-3-yl, Pyridin-4-yl, Pyridazin-3-yl, Pyridazin-4-yl, Pyrimidin-2-yl, Pyrimidin-4-yl oder Pyrimidin-5-yl, der bis zu zwei Substituenten enthalten kann, wobei die Substituenten unabhängig voneinander ausgewählt sind aus aus R³ und R⁴ und
R³ steht für einen Substituenten am Kohlenstoff:
Fluor, Chlor, Brom, Iod, Cyano, Nitro, Methyl, Ethyl, Propyl, 1-Methylethyl, 1,1-Dimethylethyl, Chlorfluormethyl, Dichlormethyl, Dichlorfluormethyl, Difluormethyl, Trichlormethyl, Trifluormethyl, Cyclopropyl, Ethoxy, 1-Methylethoxy, Propoxy, Methoxy, Trifluormethoxy, Difluormethoxy, 1-Methylethylthio, Methylthio, Ethylthio, Propylthio, Difluormethylthio Trifluormethylthio oder Phenyl,
R⁴ steht für einen Substituenten am Stickstoff:
Methyl, Ethyl, Propyl, 1-Methylethyl, 2,2-Trifluorethyl, 2,2-Difluorethyl, 2,2-Dichlor-2-fluorethyl, 2-Chlor-2-difluorethyl oder 2-Chlor-2-fluorethyl,
G steht für G¹,
T *-C(=O)CH₂-#. *-C(=O)CH₂C(R⁸)₂-#, *-C(=O)CH=CH-#, *-C=CC(=O)-#, *-CH=CHC(=O)-#, *-CH₂CH₂C(=O)-#, *-C(=S)CH2-#, *-C(=S)CH₂C(R⁸)₂-#, *-CH₂CH₂C(=S)-#, *-C(=NR⁹)CH₂-# *-C(=NR⁹)CH=CH-#, *-CH=CHC(-NOH)-# oder *-CH=CHC(N-O-C₁-C₄-Alkyl)CH₂-#,
L¹ steht für CH₂,
X steht für CH,
Y steht für Sauerstoff,
R¹ₐ steht für 1,1-Dimethylethyl, 3,3-Dimethylbutyl, 2,2-Dimethylpropyl, 1,2,2-Trimethylpropyl, Pentyl, 1-Ethylpropyl, Butyl, 2-Methylpropyl, 1-Methylethyl, Ethyl, Propyl, 4-Methylpentyl oder Hexyl,
R¹_{b} steht für Cyclopentyl, Cyclohexyl oder Cycloheptyl, welche jeweils bis zu zwei Substituenten enthalten können, wobei die Substituenten unabhängig voneinander aus R¹⁰ ausgewählt sind und R¹⁰ steht für: Cyano, Chlor, Fluor, Brom, Iod, Methyl, Ethyl, Propyl, 1-Methylethyl, 1,1-Dimethylethyl, Ethenyl, Phenyl, Methoxy, Ethoxy, Propyloxy, Trifluormethoxy, Ethinyl, 2-Propinyloxy, Methylthio, Ethylthio oder Trifluomethylthio,
R¹_{c} steht für Cyclopentenyl, Cyclohexenyl oder Cycloheptenyl, welche jeweils bis zu zwei Substituenten enthalten können, wobei die Substituenten unabhängig voneinander aus R¹¹ ausgewählt sind und R¹¹ steht für:
Methyl, Ethyl, Methoxy, Ethoxy, Trifluormethoxy, Ethinyl, 2-Propinyloxy, Methylthio, Ethylthio oder Trifluormethylthio,
R¹_{d} steht außerdem für Phenyl, das bis zu drei Substituenten enthalten kann, wobei die Substituenten unabhängig voneinander aus -L²Q' oder R¹² ausgewählt sind und R¹² steht für:
Fluor, Chlor, Brom, Iod, Cyano, Nitro, Methyl, Ethyl, Propyl, 1-Methylethyl, Butyl, 1,1-Dimethylethyl, 1,2-Dimethylethyl, Ethenyl, Ethinyl, Trifluoromethyl, Difluormethyl, Trichlormethyl, Dichlormethyl, Cyclopropyl, Methoxy, Ethoxy, Propoxy, 1-Methylethoxyl, 1,1-Dimethylethoxyl, Methylcarbonyl, Ethylcarbonyl, Trifluormethylcarbonyl, Methoxycarbonyl, Ethoxycarbonyl, Propoxycarbonyl, 1-Methylethoxycarbonyl, 1,1-Dimethylethoxycarbonyl, 2-Propenyloxy, 2-Propinyloxy, Methylthio, Ethylthio, Methylsulfinyl oder Methylsulfonyl,
R¹ₑ steht für Naphthalen-1-yl, Naphthalen-2-yl, 1,2,3,4-Tetrahydronaphthalen-1-yl, 1,2,3,4-Tetrahydronaphthalen-2-yl, 5,6,7,8-Tetrahydronaphthalen-1-yl, 5,6,7,8-Tetrahydronaphthalen-2-yl, Decalin-1-yl, Decalin-2-yl, 1H-Inden-1-yl, 1H-Inden-2-yl, 1H-Inden-3-yl, 1H-Inden-4-yl, 1H-Inden-5-yl, 1H-Inden-6-yl, 1H-Inden-7-yl, Indan-1-yl, Indan-2-yl, Indan-3-yl, Indan-4-yl oder In-dan-5-yl, das bis zu drei Substituenten enthalten kann, wobei die Substituenten unabhängig voneinander aus R¹³ ausgewählt sind und R¹³ steht für:
Methyl, Methoxy, Cyano, Fluor, Chlor, Brom, Iod,
R¹_{f} steht für Furan-2-yl, Furan-3-yl, Thiophen-2-yl, Thiophen-3-yl, Isoxazol-3-yl, Isoxazol-4-yl, Isoxazol-5-yl, Pyrrol-1-yl, Pyrrol-2-yl, Pyrrol-3-yl, Oxazol-2-yl, Oxazol-4-yl, Oxazol-5-yl, Thiazol-2-yl, Thiazol-4-yl, Thiazol-5-yl, Isothiazol-3-yl, Isothiazol-4-yl, Isothiazol-5-yl, Pyrazol-1-yl, Pyrazol-3-yl, Pyrazol-4-yl, Imidazol-1-yl, Imidazol-2-yl, Imidazol-4-yl, 1,2,4-Oxadiazol-3-yl, 1,2,4-Oxadiazol-5-yl, 1,3,4-Oxadiazol-2-yl, 1,2,4-Thiadiazol-3-yl, 1,2,4-Thiadiazol-5-yl, 1,3,4-Thiadiazol-2-yl, 1,2,3-Triazol-1-yl, 1,2,3-Triazol-2-yl, 1,2,3-Triazol-4-yl, 1,2,4-Triazol-1-yl, 1,2,4-Triazol-3-yl, 1,2,4-Triazol-4-yl, Pyridin-2-yl, Pyridin-3-yl, Pyridin-4-yl, Pyridazin-3-yl, Pyridazin-4-yl, Pyrimidin-2-yl, Pyrimidin-4-yl, Pyrimidin-5-yl oder Pyrazin-2-yl, welche jeweils bis zu zwei Substituenten enthalten können, wobei die Substituenten unabhängig voneinander aus R¹⁴ und R¹⁵ ausgewählt sind und
R¹⁴ steht für einen Substituenten am Kohlenstoff:
Chlor, Fluor, Brom, Iod, Cyano, Nitro, Methyl, Ethyl, Propyl, 1-Methylethyl, Butyl, 1,1-Dimethylethyl, Ethenyl, Ethinyl, Trifluormethyl, Difluormethyl, Cyclopropyl, Cyclopentyl, Cyclohexyl, Methylcarbonyl, Ethylcarbonyl, Methoxylcarbonyl, Ethoxycarbonyl, Methoxy, Ethoxy, Propoxy, 1-Methylethoxy, 2-Propinyoxy, Trifluormethoxyl, Methylcabonyloxy, Methylcarbonylthio, Methylthio, Ethylthio, Trifluomethylthio, Methylsulfinyl, Ethylsulfinyl, Trifluoromethylsulfinyl, Methylsulfonyl, Ethylsulfonyl, oder Trifluormethylsulfonyl,
R¹⁵ steht für einen Substituenten am Stickstoff:
Methyl, Ethyl, Propyl, Cyclopropyl, Cyclohexyl, Phenyl oder 2-Propinyl,
R¹_{g} steht für Indol-1-yl, Indol-2-yl, Indol-3-yl, Indol-4-yl, Indol-5-yl, Indol-6-yl, Indol-7-yl, Benzimidazol-1-yl, Benzimidazol-2-yl, Benzimidazol-4-yl, Benzimidazol-5-yl, Indazol-1-yl, Indazol-3-yl, Indazol-4-yl, Indazol-5-yl, Indazol-6-yl, Indazol-7-yl, Indazol-2-yl, 1-Benzofuran-2-yl, 1-Benzofuran-3-yl, 1-Benzofuran-4-yl, 1-Benzofuran-5-yl, 1-Benzofuran-6-yl, 1-Benzofuran-7-yl, 1-Benzothiophen-2-yl, 1-Benzothiophen-3-yl, 1-Benzothiophen-4-yl, 1-Benzothiophen-5-yl, 1-Benzothiophen-6-yl, 1-Benzothiophen-7-yl, 1,3-Benzothiazol-2-yl, 1,3-Benzothiazol-4-yl, 1 , 3-Benzothiazol-5-yl, 1,3-Benzothiazol-6-yl, 1,3-Benzothiazol-7-yl, 1,3-Benzoxazol-2-yl, 1,3-Benzoxazol-4-yl, 1,3-Benzoxazol-5-yl, 1,3-Benzoxazol-6-yl, 1,3-Benzoxazol-7-yl, Chinolin-2-yl, Chinolin-3-yl, Chinolin-4-yl, Chinolin-5-yl, Chinolin-6-yl, Chinolin-7-yl, Chinolin-8-yl, Isochinolin-1-yl, Isochinolin-3-yl, Isochinolin-4-yl, Isochinolin-5-yl, Isochinolin-6-yl, Isochinolin-7-yl oder Isochinolin-8-yl, welche jeweils bis zu zwei Substituenten enthalten können, wobei die Substituenten unabhängig voneinander aus R¹⁶ und R¹⁷ ausgewählt sind und
R¹⁶ steht für einen Substituenten am Kohlenstoff:
Fluor, Chlor, Brom, Iod, Methyl, Methoxy, 2-Propinyloxy, 2-Propenyloxy,
R¹⁷ steht für einen Substituenten am Stickstoff:
Methyl, Ethyl, Propyl, Cyclopropyl, Cyclohexyl, Phenyl oder 2-Propinyl,
L² steht für eine direkt Bindung,
Q' steht für Phenyl,
R⁵ steht für Wasserstoff,
R⁷ steht für Wasserstoff,
R⁸ steht für Wasserstoff, Methyl,
sowie agrochemisch wirksame Salze davon.

4. Verfahren zur Bekämpfung von pflanzenpathogenen Schadpilzen, **dadurch gekennzeichnet, dass** man Verbindungen der Formel **(I)** gemäß einem oder mehreren der Ansprüche 1 bis 3, auf die pflanzenpathogenen Schadpilze und/oder deren Lebensraum ausbringt.

5. Mittel zur Bekämpfung von pflanzenpathogenen Schadpilzen, **gekennzeichnet durch** einen Gehalt an mindestens einer Verbindung der Formel **(I)** gemäß einem oder mehreren der Ansprüche 1 bis 3, neben Streckmitteln und/oder oberflächenaktiven Stoffen.

6. Verwendung von Ketoheteroarylpiperidin und -piperazin Derivaten der Formel **(I),** gemäß einem oder mehreren der Ansprüche 1 bis 3 zur Bekämpfung von pflanzenpathogenen Schadpilzen.

7. Verfahren zur Herstellung von Mitteln zur Bekämpfung von pflanzenpathogenen Schadpilzen, **dadurch gekennzeichnet, dass** man Ketoheteroarylpiperidin und -piperazin Derivate der Formel **(I)** gemäß einem oder mehreren der Ansprüche 1 bis 3 mit Streckmitteln und/oder oberflächenaktiven Stoffen vermischt.

8. Verwendung von Verbindungen der Formel **(I)** gemäß den Ansprüchen 1 bis 3 zur Behandlung von Saatgut.

9. Verwendung von Verbindungen der Formel **(I)** gemäß den Ansprüchen 1 bis 3 zur Behandlung von transgenen Pflanzen.

10. Verwendung von Verbindungen der Formel **(I)** gemäß den Ansprüchen 1 bis 3 zur Behanlung von transgenem Saatgut.

## Claims

1. Compounds of the formula (I) 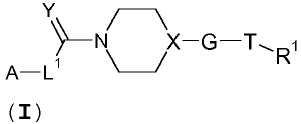 in which the symbols have the following meanings:
A represents phenyl which may contain up to three substituents,
where the substituents independently of one another are selected from R²,
or
A' represents optionally benzo-fused unsubstituted or substituted 5- or 6-membered heteroaryl, where the substituents at carbon are independently of one another selected from R³ and the substituents at nitrogen are independently of one another selected from R⁴,
L¹ represents NR⁵ or C(R⁶)₂,
X represents CH or nitrogen,
Y represents sulphur or oxygen,
G represents: 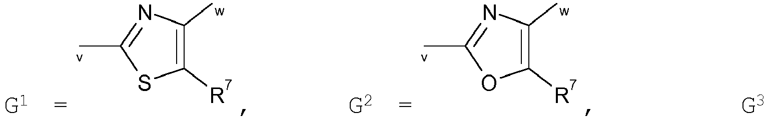 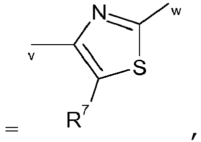 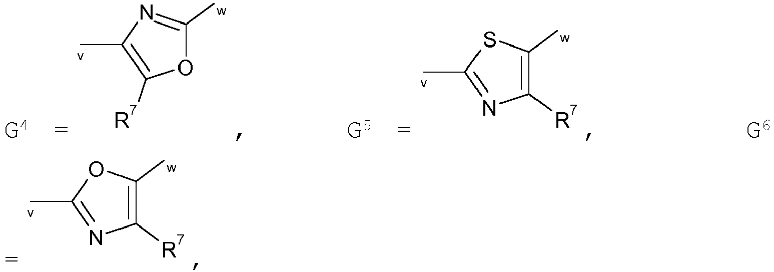
where the bond identified by "v" is attached directly to X and where the bond identified by "w" is attached directly to T,
T represents *-C(=O)CH₂-#, *-C(=O)CH₂C(R⁸)₂-#, *-C(=O)CH=CH-#, *-C(=O)C=C-#, *-C(=O)CH₂C(=O)-#, *-C=CC(=O)-#, *-CH=CHC(=O)-#, *-CH₂CH₂C(=O)-#, *-C(=S)CH2-#, *-C(=S)CH2C(R₈)₂-#, *-C(=S)CH=CH-#, *-C(=S)C=C-#, *-C=CC(=S)-#, *-CH=CHC(=S)-#, *-CH₂CH₂C(=S)-#, *-C(=NR⁹)CH₂-#, *-C(=NR⁹)CH₂C(R⁸)₂-# or *-CH₂CH₂C(=NR⁹)-#, *-C(=NR⁹)CH=CH-#, *-CH=CHC(=NR⁹)-#, *-CH=CHC(NOH)-#, *-CH=CHC(NO-C₁-C₄-alkyl)CH₂-#, where the bond identified by * is attached directly to G and where the bond identified by # is attached directly to R¹,
R¹ is selected from the group containing R¹ₐ, R¹_{b}, R¹_{c}, R¹_{d}, R¹ₑ, R¹_{f}, R¹_{g} or R¹ₕ, where
R¹ₐ represents C₁-C₆-alkyl, C₁-C₆-haloalkyl, C₂-C₆-alkenyl, C₂-C₆-alkynyl, C₂-C₈-alkoxyalkyl or C₅-C₉-cycloalkoxyalkyl or
R¹_{b}, represents unsubstituted or substituted C₃-C₁₀-cycloalkyl,
where the substituents independently of one another are selected from -Q or R¹⁰ or
R¹_{c} represents unsubstituted or substituted C₅-C₁₀-cycloalkenyl,
where the substituents independently of one another are selected from R¹¹ or
R¹_{d} represents unsubstituted or substituted phenyl,
where the substituents independently of one another are selected from -L²Q or R¹² or
R¹ₑ represents unsubstituted or substituted naphthalen-1-yl, naphthalen-2-yl, 1,2,3,4-tetrahydronaphthalen-1-yl, 1,2,3,4-tetrahydronaphthalen-2-yl, 5,6,7,8-tetrahydronaphthalen-1-yl, 5,6,7,8-tetrahydronaphthalen-2-yl, decalin-1-yl, decalin-2-yl, 1H-inden-1-yl, 1H-inden-2-yl, 1H-inden-3-yl, 1H-inden-4-yl, 1H-inden-5-yl, 1H-inden-6-yl, 1H-inden-7-yl, indan-1-yl, indan-2-yl, indan-3-yl, indan-4-yl or indan-5-yl,
where the substituents independently of one another are selected from R¹³ or
R¹_{f} represents an unsubstituted or substituted 5- or 6-membered heteroaryl radical, where the substituents at carbon independently of one another are selected from -L²Q or R¹⁴, and the substituents at nitrogen independently of one another are selected from -L³Q or R¹⁵ or
R¹_{g} represents benzo-fused unsubstituted or substituted 5- or 6-membered heteroaryl, where the substituents at carbon independently of one another are selected from R¹⁶ and the substituents at nitrogen independently of one another are selected from R¹⁷ or
R¹ₕ represents unsubstituted or substituted C₅-C₁₅-heterocyclyl, where the substituents at carbon independently of one another are selected from R¹⁸ and the substituents at nitrogen independently of one another are selected from R¹⁹,
L² represents a direct bond, -O-, -C(R²⁰)₂- or - NR²¹-,
L³ represents a direct bond or -C(R²⁰)₂-,
Q is selected from the group containing Q', Q" and Q''', where
Q' represents phenyl which may contain up to two substituents, where the substituents independently of one another are selected from R²² or
Q'' represents a 5- or 6-membered heteroaryl radical which may contain up to two substituents, where the substituents at carbon independently of one another are selected from R²³ and the substituents at nitrogen independently of one another are selected from R²⁴ or
Q''' represents saturated or partially unsaturated C₃-C₁₀-cycloalkyl,
R², R¹² and R²² independently of one another represent halogen, cyano, hydroxyl, SH, amino, nitro, phenyl, C(=O)H, C(=O)OH, CONR²⁵R²⁶, NR²⁵R²⁶, C₁-C₆-alkyl, C₂-C₆-alkenyl, C₂-C₆-alkynyl, C₁-C₆-haloalkyl, C₂-C₆-haloalkenyl, C₂-C₆-haloalkynyl, C₃-C₈-cycloalkyl, C₃-C₈-halocycloalkyl, C₄-C₁₀-alkylcycloalkyl, C₄-C₁₀-cycloalkylalkyl, C₆-C₁₄-cycloalkylcycloalkyl, C₄-C₁₀-halocycloalkylalkyl, C₅-C₁₀-alkylcycloalkylalkyl, C₃-C₈-cycloalkenyl, C₃-C₈-halocycloalkenyl, C₂-C₆-alkoxyalkyl, C₄-C₁₀-cycloalkoxyalkyl, C₃-C₈-alkoxyalkoxyalkyl, C₂-C₆-alkylthioalkyl, C₂-C₆-alkylsulphinylalkyl, C₂-C₆-alkylsulphonylalkyl, C₂-C₆-alkylaminoalkyl, C₃-C₈-dialkylaminoalkyl, C₂-C₆-haloalkylaminoalkyl, C₄-C₁₀-cycloalkylaminoalkyl, C₂-C₆-alkylcarbonyl, C₂-C₆-haloalkylcarbonyl, C₄-C₈-cycloalkylcarbonyl, C₂-C₆-alkoxycarbonyl, C₄-C₈-cycloalkoxycarbonyl, C₅-C₁₀-cycloalkylalkoxycarbonyl, C₂-C₆-alkylaminocarbonyl, C₃-C₈-dialkylaminocarbonyl, C₄-C₈-cycloalkylaminocarbonyl, C₂-C₆-haloalkoxyalkyl, C₁-C₆-hydroxyalkyl, C₁-C₆-alkoxy, C₁-C₆-haloalkoxy, C₃-C₈-cycloalkoxy, C₃-C₈-halocycloalkoxy, C₄-C₁₀-cycloalkylalkoxy, C₃-C₆-alkenyloxy, C₃-C₆-haloalkenyloxy, C₃-C₆-alkynyloxy, C₃-C₆-haloalkynyloxy, C₂-C₆-alkoxyalkoxy, C₂-C₆-alkylcarbonyloxy, C₂-C₆-haloalkylcarbonyloxy, C₄-C₈-cycloalkylcarbonyloxy, C₃-C₆-alkylcarbonylalkoxy, C₁-C₆-alkylthio, C₁-C₆-haloalkylthio, C₃-C₆-cycloalkylthio, C₁-C₆-alkylsulphinyl, C₁-C₆-haloalkylsulphinyl, C₁-C₆-alkylsulphonyl, C₁-C₆-haloalkylsulphonyl, C₃-C₈-cycloalkylsulphonyl, tri(C₁-C₄-alkyl)silyl, C₁-C₆-alkylsulphonylamino or C₁-C₆-haloalkylsulphonylamino,
R³, R¹⁴, R¹⁶, R¹⁸ and R²³ independently of one another represent halogen, cyano, hydroxyl, SH, amino, nitro, NR²⁵R²⁶, C₁-C₆-alkyl, C₂-C₆-alkenyl, C₂-C₆-alkynyl, C₁-C₆-haloalkyl, C₂-C₆-haloalkenyl, C₂-C₆-haloalkynyl, C₃-C₆-cycloalkyl, C₃-C₆-halocycloalkyl, C₄-C₁₀-alkyl-cycloalkyl, C₄-C₁₀-cycloalkylalkyl, C₆-C₁₄-cycloalkylcycloalkyl, C₅-C₁₀-alkylcycloalkylalkyl, C₂-C₄-alkoxyalkyl, C₂-C₄-alkylcarbonyl, C₂-C₆-alkoxycarbonyl, C₂-C₆-alkylaminocarbonyl, C₃-C₈-dialkylaminocarbonyl, C₁-C₄-hydroxyalkyl, C₁-C₄-alkoxy, C₃-C₆-alkenyloxy, C₃-C₆-alkynyloxy, C₁-C₄-haloalkoxy, C₂-C₆-alkylcarbonyloxy, C₂-C₆-alkylcarbonylthio, C₁-C₄-alkylthio, C₁-C₄-haloalkylthio, C₁-C₄-alkylsulphinyl, C₁-C₄-haloalkylsulphinyl, C₁-C₄-alkylsulphonyl, C₁-C₄-haloalkylsulphonyl, tri(C₁-C₄-alkyl)silyl or phenyl,
R⁴, R¹⁵, R¹⁷, R¹⁹ and R²⁴ independently of one another represent C₁-C₆-alkyl, C₂-C₆-alkenyl, C₂-C₆-alkynyl, C₁-C₆-haloalkyl, C₃-C₆-haloalkenyl, C₃-C₆-haloalkynyl, C₃-C₆-cycloalkyl, C₃-C₆-halocycloalkyl, C₄-C₁₀-alkylcycloalkyl, C₄-C₁₀-cycloalkylalkyl, phenyl, benzyl, C₁-C₄-alkylsulphonyl, C(=O)H, C₂-C₄-alkylcarbonyloxy, C₂-C₅-alkoxycarbonyl or C₂-C₅-alkylcarbonyl,
R⁵ represents hydrogen or C₁-C₄-alkyl,
R⁶ independently of one another represent hydrogen, C₁-C₄-alkyl, C₁-C₄-haloalkyl, cyclopropyl, halogen,
or the two radicals R⁶ together with the carbon atom to which they are attached form a cyclopropyl ring,
R⁷ represents hydrogen, C₁-C₃-alkyl, C₁-C₃-haloalkyl, C₁-C₄-alkoxy, C₂-C₅-alkoxycarbonyl or halogen,
R⁸ independently of one another represent hydrogen or C₁-C₄-alkyl,
R⁹ represents OH, nitro, cyano, C₁-C₄-alkyl, C₁-C₄-alkylamino, C₂-C₄-dialkylamino, C₁-C₄-alkoxy, C₁-C₄-haloalkoxy or cyclopropyloxy
R¹⁰ and R¹¹ independently of one another represent cyano, halogen, C₁-C₆-alkyl, C₁-C₆-haloalkyl, C₃-C₆-cycloalkyl, C₂-C₆-alkenyl, C₂-C₆-haloalkenyl, C₂-C₆-alkynyl, C₂-C₆-haloalkynyl, tri(C₁-C₄-alkyl)silyl, phenyl, hydroxyl, oxo, C₁-C₆-alkoxy, C₁-C₆-haloalkoxy, C₃-C₆-alkenyloxy, C₃-C₆-alkynyloxy, C₁-C₆-alkylthio or C₁-C₆-haloalkylthio,
R¹³ independently of one another represent cyano, nitro, halogen, C₁-C₆-alkyl, C₁-C₄-haloalkyl, C₃-C₆-cycloalkyl, C₂-C₆-alkenyl, C₂-C₆-haloalkenyl, C₂-C₆-alkynyl, C₂-C₆-haloalkynyl, tri (C₁-C₄-alkyl) silyl, benzyl, phenyl, hydroxyl, SH, C₁-C₆-alkoxy, C₁-C₆-haloalkoxy, C₃-C₆-alkenyloxy, C₃-C₆-alkynyloxy, C₁-C₆-alkylthio or C₁-C₆-haloalkylthio,
R²⁰ independently of one another represent hydrogen, halogen, C₁-C₄-alkyl or C₁-C₄-haloalkyl,
R²¹ represents C₁-C₆-alkyl, C₁-C₆-haloalkyl, C₃-C₈-cycloalkyl, C₂-C₆-alkylcarbonyl, C₂-C₆-haloalkylcarbonyl, C₂-C₆-alkoxycarbonyl or C₂-C₆-haloalkoxycarbonyl,
R²⁵ and R²⁶ independently of one another represent hydrogen, C₁-C₄-alkyl, C₂-C₆-alkenyl, C₂-C₆-alkynyl, C₁-C₃-haloalkyl, C₃-C₆-cycloalkyl, benzyl or phenyl,
and also agrochemically active salts thereof.

2. Compounds of the formula (I) according to Claim 1, in which the symbols have the following meanings:
A represents phenyl which may contain up to two substituents, where the substituents independently of one another are selected from R² list and R² represents:
halogen, cyano, hydroxyl, amino, nitro, C₁-C₆-alkyl, C₂-C₆-alkenyl, C₂-C₆-alkynyl, C₃-C₈-cycloalkyl, C₁-C₆-haloalkyl, C₂-C₆-haloalkenyl, C₂-C₆-haloalkynyl, C₃-C₆-halocycloalkyl, C₁-C₄-alkoxy, C₁-C₄-haloalkoxy, C₁-C₄-alkylthio, C₁-C₄-alkylsulphonyl, C₁-C₄-haloalkylthio, C₁-C₄-haloalkylsulphonyl, C₂-C₄-alkoxyalkyl, C₂-C₅-alkylcarbonyl, C₂-C₆-alkoxycarbonyl, C₂-C₆-alkylcarbonyloxy or C(=O)H,
A' represents a heteroaromatic radical selected from the group below: furan-2-yl, furan-3-yl, thiophen-2-yl, thiophen-3-yl, isoxazol-3-yl, isoxazol-4-yl, isoxazol-5-yl, pyrrol-1-yl, pyrrol-2-yl, pyrrol-3-yl, oxazol-2-yl, oxazol-4-yl, oxazol-5-yl, thiazol-2-yl, thiazol-4-yl, thiazol-5-yl, isothiazol-3-yl, isothiazol-4-yl, isothiazol-5-yl, pyrazol-1-yl, pyrazol-3-yl, pyrazol-4-yl, imidazol-1-yl, imidazol-2-yl, imidazol-4-yl, 1,2,3-triazol-1-yl, 1,2,4-triazol-1-yl, pyridin-2-yl, pyridin-3-yl, pyridin-4-yl, pyridazin-3-yl, pyridazin-4-yl, pyrazin-2-yl, pyrazin-3-yl, pyrimidin-2-yl, pyrimidin-4-yl or pyrimidin-5-yl which may contain up to two substituents, where the substituents independently of one another are selected from R³ and R⁴ and
R³ represents a substituent at carbon:
halogen, cyano, hydroxyl, amino, nitro, C₁-C₆-alkyl, C₂-C₆-alkenyl, C₂-C₆-alkynyl, C₃-C₈-cycloalkyl, C₁-C₆-haloalkyl, C₂-C₆-haloalkenyl, C₂-C₆-haloalkynyl, C₃-C₆-halocycloalkyl, C₁-C₄-alkoxy, C₁-C₄-haloalkoxy, C₁-C₄-alkylthio, C₁-C₄-alkylsulphonyl, C₁-C₄-haloalkylthio, C₁-C₄-haloalkylsulphonyl, C₂-C₄-alkoxyalkyl, C₂-C₆-alkoxycarbonyl, C₂-C₆-alkylcarbonyloxy, C(=O)H or phenyl
R⁴ represents a substituent at nitrogen: C₁-C₆-alkyl, C₁-C₄-haloalkyl, C₃-C₆-alkenyl, C₃-C₆-haloalkenyl, C₃-C₆-alkynyl or C₃-C₆-haloalkynyl
G represents G¹ or G²,
T represents *-C(=O)CH₂-#, *-C(=O)CH₂C(R⁸)₂-#, *-C(=O)CH=CH-#, *-C(=O)C≡C-#, *-C=CC(=O)-#, *-CH=CHC(=O)-#, *-CH₂CH₂C(=O)-#, *-C(=S)CH2-#, *-C(=S)CH₂C(R⁸)2-#_{,} *-CH₂CH₂C(=S)-#, *-C(=NR⁹)CH₂-#, *-C(=NR₉)CH=CH-#, *-CH=CHC(=NR⁹)-#, *-CH=CHC(NOH)-# or *-CH=CHC(NO-C₁-C₄-Alkyl)CH₂-#,
L¹ represents CH₂ or NR⁵,
X represents CH,
Y represents oxygen,
R¹ₐ represents C₁-C₆-alkyl, C₁-C₆-haloalkyl, C₂-C₆-alkenyl or C₂-C₆-alkynyl, C₂-C₆-alkoxyalkyl,
R¹_{b}, represents unsubstituted or substituted C₃-C₁₀-cycloalkyl, where the substituents independently of one another are selected from -Q or R¹⁰ and R¹⁰ represents:
cyano, halogen, C₁-C₆-alkyl, C₁-C₆-haloalkyl, C₃-C₆-cycloalkyl, C₂-C₆-alkenyl, C₂-C₆-haloalkenyl, C₂-C₆-alkynyl, C₂-C₆-haloalkynyl, tri (C₁-C₄-alkyl) silyl, hydroxyl, oxo, C₁-C₆-alkoxy, C₁-C₆-haloalkoxy, C₃-C₆-alkenyloxy, C₃-C₆-alkynyloxy, C₁-C₆-alkylthio, C₁-C₆-haloalkylthio,
R¹_{c} represents unsubstituted or substituted C₅-C₁₀-cycloalkenyl, where the substituents independently of one another are selected from R¹¹ and R¹¹ represents:
cyano, chlorine, fluorine, C₁-C₆-alkyl, C₁-C₆-haloalkyl, C₃-C₆-cycloalkyl, C₂-C₆-alkenyl, C₂-C₆-haloalkenyl, C₂-C₆-alkynyl, C₂-C₆-haloalkynyl, phenyl, oxo, C₁-C₆-alkoxy, C₁-C₆-haloalkoxy, C₃-C₆-alkenyloxy, C₃-C₆-alkynyloxy, C₁-C₆-alkylthio or C₁-C₆-haloalkylthio,
R¹_{d} represents unsubstituted or substituted phenyl, where the substituents independently of one another are selected from -L²Q or R¹² and R¹² represents:
halogen, cyano, hydroxyl, SH, amino, nitro, C(=O)H, C(=O)OH, CONR²⁵R²⁶, NR²⁵R²⁶, C₁-C₆-alkyl, C₂-C₆-alkenyl, C₂-C₆-alkynyl, C₁-C₆-haloalkyl, C₂-C₆-haloalkenyl, C₂-C₆-haloalkynyl, C₃-C₈-cycloalkyl, C₃-C₈-halocycloalkyl, C₄-C₁₀-alkyl-cycloalkyl, C₄-C₁₀-cycloalkylalkyl, C₆-C₁₄-cycloalkylcycloalkyl, C₄-C₁₀-halocycloalkylalkyl, C₅-C₁₀-alkylcycloalkylalkyl, C₃-C₈-cycloalkenyl, C₃-C₈-halocycloalkenyl, C₂-C₆-alkoxyalkyl, C₄-C₁₀-cycloalkoxyalkyl, C₃-C₈-alkoxyalkoxyalkyl, C₂-C₆-alkylthioalkyl, C₂-C₆-alkylsulphinylalkyl, C₂-C₆-alkylsulphonylalkyl, C₂-C₆-alkylaminoalkyl, C₃-C₈-dialkylaminoalkyl, C₂-C₆-haloalkylaminoalkyl, C₄-C₁₀-cycloalkylaminoalkyl, C₂-C₆-alkylcarbonyl, C₂-C₆-haloalkylcarbonyl, C₄-C₈-cycloalkylcarbonyl, C₂-C₆-alkoxycarbonyl, C₄-C₈-cycloalkoxycarbonyl, C₅-C₁₀-cycloalkylalkoxycarbonyl, C₂-C₆-alkylaminocarbonyl, C₃-C₈-dialkylaminocarbonyl, C₄-C₈-cycloalkylaminocarbonyl, C₂-C₆-haloalkoxyalkyl, C₁-C₆-hydroxyalkyl, C₁-C₆-alkoxy, C₁-C₆-haloalkoxy, C₃-C₈-cycloalkoxy, C₃-C₈-halocycloalkoxy, C₄-C₁₀-cycloalkylalkoxy, C₃-C₆-alkenyloxy, C₃-C₆-haloalkenyloxy, C₃-C₆-alkynyloxy, C₃-C₆-haloalkynyloxy, C₂-C₆-alkoxyalkoxy, C₂-C₆-alkylcarbonyloxy, C₂-C₆-haloalkylcarbonyloxy, C₄-C₈-cycloalkylcarbonyloxy, C₃-C₆-alkylcarbonylalkoxy, C₁-C₆-alkylthio, C₁-C₆-haloalkylthio, C₃-C₆-cycloalkylthio, C₁-C₆-alkylsulphinyl, C₁-C₆-haloalkylsulphinyl, C₁-C₆-alkylsulphonyl, C₁-C₆-haloalkylsulphonyl, C₃-C₈-cycloalkylsulphonyl, tri(C₁-C₄-alkyl)silyl, C₁-C₆-alkylsulphonylamino, C₁-C₆-haloalkylsulphonylamino,
R¹ₑ represents unsubstituted or substituted naphthalen-1-yl, naphthalen-2-yl, 1,2,3,4-tetrahydronaphthalen-1-yl, 1,2,3,4-tetrahydronaphthalen-2-yl, 5,6,7,8-tetrahydronaphthalen-1-yl, 5,6,7,8-tetrahydronaphthalen-2-yl, decalin-1-yl, decalin-2-yl, 1H-inden-1-yl, 1H-inden-2-yl, 1H-inden-3-yl, 1H-inden-4-yl, 1H-inden-5-yl, 1H-inden-6-yl, 1H-inden-7-yl, indan-1-yl, indan-2-yl, indan-3-yl, indan-4-yl or indan-5-yl, where the substituents independently of one another are selected from R¹³ and R¹³ represents:
cyano, halogen, C₁-C₆-alkyl, C₁-C₄-haloalkyl, C₂-C₆-alkenyl, C₂-C₆-alkynyl, benzyl, phenyl, C₁-C₆-alkoxy, C₁-C₆-haloalkoxy, C₃-C₆-alkenyloxy, C₃-C₆-alkynyloxy or C₁-C₆-alkylthio,
R¹_{f} represents an unsubstituted or substituted 5- or 6-membered heteroaryl radical, where the substituents independently of one another are selected from -L²Q or R¹⁴ and -L³Q or R¹⁵ and R¹⁴ represents a substituent at carbon:
halogen, cyano, hydroxyl, SH, amino, nitro, NR²⁵R²⁶, C₁-C₆-alkyl, C₂-C₆-alkenyl, C₂-C₆-alkynyl, C₁-C₆-haloalkyl, C₂-C₆-haloalkenyl, C₂-C₆-haloalkynyl, C₃-C₆-cycloalkyl, C₃-C₆-halocycloalkyl, C₄-C₁₀-alkylcycloalkyl, C₄-C₁₀-cycloalkylalkyl, C₆-C₁₄-cycloalkylcycloalkyl, C₅-C₁₀-alkylcycloalkylalkyl, C₂-C₄-alkoxyalkyl, C₃-C₆-alkenyloxy, C₃-C₆-alkynyloxy, C₂-C₄-alkylcarbonyl, C₂-C₆-alkoxycarbonyl, C₂-C₆-alkylaminocarbonyl, C₃-C₈-dialkylaminocarbonyl, C₁-C₄-hydroxyalkyl, C₁-C₄-alkoxy, C₁-C₄-haloalkoxy, C₂-C₆-alkylcarbonyloxy, C₂-C₆-alkylcarbonylthio, C₁-C₄-alkylthio, C₁-C₄-haloalkylthio, C₁-C₄-alkylsulphinyl, C₁-C₄-haloalkylsulphinyl, C₁-C₄-alkylsulphonyl, C₁-C₄-haloalkylsulphonyl, tri(C₁-C₄-alkyl)silyl,
R¹⁵ represents a substituent at nitrogen:
C₁-C₆-alkyl, C₃-C₆-alkenyl, C₃-C₆-alkynyl, C₁-C₆-haloalkyl, C₃-C₆-haloalkenyl, C₃-C₆-haloalkynyl, C₃-C₆-cycloalkyl, C₃-C₆-halo-cycloalkyl, C₄-C₁₀-alkylcycloalkyl, C₄-C₁₀-cycloalkylalkyl, phenyl,
R¹_{g} represents a benzo-fused unsubstituted or substituted 5- or 6-membered heteroaryl, where the substituents independently of one another are selected from R¹⁶ and R¹⁷ and R¹⁶ represents a substituent at carbon:
halogen, cyano, hydroxyl, SH, amino, nitro, NR²⁵R²⁶, C₁-C₆-alkyl, C₂-C₆-alkenyl, C₂-C₆-alkynyl, C₁-C₆-haloalkyl, C₂-C₆-haloalkenyl, C₂-C₆-haloalkynyl, C₃-C₆-cycloalkyl, C₃-C₆-halocycloalkyl, C₄-C₁₀-alkylcycloalkyl, C₄-C₁₀-cycloalkylalkyl, C₆-C₁₄-cycloalkylcycloalkyl, C₅-C₁₀-alkylcycloalkylalkyl, C₂-C₄-alkoxyalkyl, C₂-C₄-alkylcarbonyl, C₂-C₆-alkoxycarbonyl, C₂-C₆-alkylaminocarbonyl, C₃-C₈-dialkylaminocarbonyl, C₁-C₄-hydroxyalkyl, C₁-C₄-alkoxy, C₁-C₄-haloalkoxy, C₂-C₆-alkylcarbonyloxy, C₂-C₆-alkylcarbonylthio, C₁-C₄-alkylthio, C₁-C₄-haloalkylthio, C₁-C₄-alkylsulphinyl, C₁-C₄-haloalkylsulphinyl, C₁-C₄-alkylsulphonyl, C₁-C₄-haloalkylsulphonyl, tri(C₁-C₄-alkyl)silyl or phenyl,
R¹⁷ represents a substituent at nitrogen:
C₁-C₆-alkyl, C₃-C₆-alkenyl, C₃-C₆-alkynyl, C₁-C₆-haloalkyl, C₃-C₆-haloalkenyl, C₃-C₆-haloalkynyl, C₃-C₆-cycloalkyl, C₃-C₆-halocycloalkyl, C₄-C₁₀-alkylcycloalkyl, C₄-C₁₀-cycloalkylalkyl or phenyl,
R¹ₕ represents unsubstituted or substituted C₅-C₁₅-heterocyclyl, where possible substituents independently of one another are selected from R¹⁸ and R¹⁹ and
R¹⁸ represents a substituent at carbon:
halogen, cyano, hydroxyl, SH, amino, nitro, NR²⁵R²⁶, C₁-C₆-alkyl, C₂-C₆-alkenyl, C₂-C₆-alkynyl, C₁-C₆-haloalkyl, C₂-C₆-haloalkenyl, C₂-C₆-haloalkynyl, C₃-C₆-cycloalkyl, C₃-C₆-halocycloalkyl, C₄-C₁₀-alkylcycloalkyl, C₄-C₁₀-cycloalkylalkyl, C₆-C₁₄-cycloalkylcycloalkyl, C₅-C₁₀-alkylcycloalkylalkyl, C₂-C₄-alkoxyalkyl, C₂-C₄-alkylcarbonyl, C₂-C₆-alkoxycarbonyl, C₂-C₆-alkylaminocarbonyl, C₃-C₈-dialkylaminocarbonyl, C₁-C₄-hydroxyalkyl, C₁-C₄-alkoxy, C₁-C₄-haloalkoxy, C₂-C₆-alkylcarbonyloxy, C₂-C₆-alkylcarbonylthio, C₁-C₄-alkylthio, C₁-C₄-haloalkylthio, C₁-C₄-alkylsulphinyl, C₁-C₄-haloalkylsulphinyl, C₁-C₄-alkylsulphonyl, C₁-C₄-haloalkylsulphonyl, tri(C₁-C₄-alkyl)silyl or phenyl,
R¹⁹ represents a substituent at nitrogen:
C₁-C₆-alkyl, C₃-C₆-alkenyl, C₃-C₆-alkynyl, C₁-C₆-haloalkyl, C₃-C₆-haloalkenyl, C₃-C₆-haloalkynyl, C₃-C₆-CyCloalkyl, C₃-C₆-halo-cycloalkyl, C₄-C₁₀-alkylcycloalkyl, C₄-C₁₀-cycloalkylalkyl or phenyl,
L² represents a direct bond or -O-,
L³ represents a direct bond,
Q' represents phenyl which may contain up to two substituents, where the substituents independently of one another are selected from R²² and R²² represents:
fluorine, chlorine, bromine, iodine, cyano, hydroxyl, SH, nitro, C₁-C₆-alkyl, C₁-C₆-haloalkyl, C₃-C₆-cycloalkyl, C₁-C₄-alkoxy, C₁-C₄-haloalkoxy, C₁-C₄-alkylthio or C₁-C₄-haloalkylthio,
Q" represents furan-2-yl, furan-3-yl, thiophen-2-yl, thiophen-3-yl, isoxazol-3-yl, isoxazol-4-yl, isoxazol-5-yl, pyrrol-1-yl, pyrrol-2-yl, pyrrol-3-yl, oxazol-2-yl, oxazol-4-yl, oxazol-5-yl, thiazol-2-yl, thiazol-4-yl, thiazol-5-yl, isothiazol-3-yl, isothiazol-4-yl, isothiazol-5-yl, pyrazol-1-yl, pyrazol-3-yl, pyrazol-4-yl, imidazol-1-yl, imidazol-2-yl, imidazol-4-yl, 1,2,4-oxadiazol-3-yl, 1,2,4-oxadiazol-5-yl, 1,3,4-oxadiazol-2-yl, tetrazol-5-yl, 1,2,4-thiadiazol-3-yl, 1,2,4-thiadiazol-5-yl, 1,3,4-thiadiazol-2-yl, 1,2,3-triazol-1-yl, 1,2,3-triazol-2-yl, 1.2.3-triazol-4-yl, 1,2,4-triazol-1-yl, 1.2.4-triazol-3-yl, 1,2,4-triazol-4-yl, tetrazol-5-yl, pyridin-2-yl, pyridin-3-yl, pyridin-4-yl, pyridazin-3-yl, pyridazin-4-yl, pyrimidin-2-yl, pyrimidin-4-yl, pyrimidin-5-yl, pyrazin-2-yl, 1,3,5-triazin-2-yl or 1,2,4-triazin-3-yl which may contain up to two substituents, where the substituents independently of one another are selected from R²³ and R²⁴ and
R²³ represents a substituent at carbon:
fluorine, chlorine, bromine, iodine, cyano, hydroxyl, SH, nitro, C₁-C₆-alkyl, C₁-C₆-haloalkyl, C₃-C₆-cycloalkyl, C₁-C₄-alkoxy, C₁-C₄-haloalkoxy, C₁-C₄-alkylthio or C₁-C₄-haloalkylthio,
R²⁴ represents a substituent at nitrogen:
C₁-C₆-alkyl, C₃-C₆-alkenyl, C₃-C₆-alkynyl, C₃-C₆-cycloalkyl or phenyl,
R⁵ represents hydrogen or methyl, ethyl, propyl, isopropyl, butyl, isobutyl or *tert*-butyl,
R⁷ represents hydrogen,
R⁸ independently of one another represent hydrogen, methyl or ethyl,
R²⁵ and R²⁶ independently of one another represent hydrogen, methyl, ethyl, propyl, isopropyl, butyl, isobutyl or *tert*-butyl,
and also agrochemically active salts thereof.

3. Compounds of the formula **(I)** according to one or more of Claims 1 and 2,
in which the symbols have the following meanings:
A represents phenyl which may contain up to two substituents, where the substituents independently of one another are selected from R² and R² represents:
fluorine, bromine, iodine, chlorine, cyano, nitro, methyl, ethyl, propyl, 1-methylethyl, 1,1-dimethylethyl, chlorofluoromethyl, dichloromethyl, dichlorofluoromethyl, difluoromethyl, trichloromethyl, trifluoromethyl, cyclopropyl, ethoxy, 1-methylethoxy, propoxy, methoxy, trifluoromethoxy, difluoromethoxy, 1-methylethylthio, methylthio, ethylthio, propylthio, difluoromethylthio or trifluoromethylthio,
A' represents a heteroaromatic radical selected from the group below: furan-2-yl, furan-3-yl, thiophen-2-yl, thiophen-3-yl, isoxazol-3-yl, isoxazol-4-yl, isoxazol-5-yl, pyrrol-1-yl, pyrrol-2-yl, pyrrol-3-yl, oxazol-2-yl, oxazol-4-yl, oxazol-5-yl, thiazol-2-yl, thiazol-4-yl, thiazol-5-yl, isothiazol-3-yl, isothiazol-4-yl, isothiazol-5-yl, pyrazol-1-yl, pyrazol-3-yl, pyrazol-4-yl, imidazol-1-yl, imidazol-2-yl, imidazol-4-yl, 1,2,3-triazol-1-yl, 1,2,4-triazol-1-yl, pyridin-2-yl, pyridin-3-yl, pyridin-4-yl, pyridazin-3-yl, pyridazin-4-yl, pyrimidin-2-yl, pyrimidin-4-yl or pyrimidin-5-yl which may contain up to two substituents, where the substituents independently of one another are selected from R³ and R⁴ and
R³ represents a substituent at carbon:
fluorine, chlorine, bromine, iodine, cyano, nitro, methyl, ethyl, propyl, 1-methylethyl, 1,1-dimethylethyl, chlorofluoromethyl, dichloromethyl, dichlorofluoromethyl, difluoromethyl, trichloromethyl, trifluoromethyl, cyclopropyl, ethoxy, 1-methylethoxy, propoxy, methoxy, trifluoromethoxy, difluoromethoxy, 1-methylethylthio, methylthio, ethylthio, propylthio, difluoromethylthio, trifluoromethylthio or phenyl,
R⁴ represents a substituent at nitrogen:
methyl, ethyl, propyl, 1-methylethyl, 2,2-trifluoroethyl, 2,2-difluoroethyl, 2,2-dichloro-2-fluoroethyl, 2-chloro-2-difluoroethyl or 2-chloro-2-fluoroethyl,
G represents G¹,
T represents *-C(=O) CH₂-#, *-C (=O) CH₂C (R⁸) 2-#, *-C(=O)CH=CH-#, *-C=CC(=O)-#, *-CH=CHC(=O)-#, *-CH₂CH₂C(=O)-#, *-C(=S)CH₂-#, *-C(=S)CH₂C(R⁸)₂-#, *-CH₂CH₂C (=S)-#, *-C (=NR⁹) CH₂-#, *-C (=NR⁹) CH=CH-#, *-CH=CHC(-NOH)-# or *-CH=CHC (N-O-C₁-C₄-alkyl) CH₂-#,
L¹ represents CH₂,
X represents CH,
Y represents oxygen,
R¹ₐ represents 1,1-dimethylethyl, 3,3-dimethylbutyl, 2,2-dimethylpropyl, 1,2,2-trimethylpropyl, pentyl, 1-ethylpropyl, butyl, 2-methylpropyl, 1-methylethyl, ethyl, propyl, 4-methylpentyl or hexyl,
R¹_{b}, represents cyclopentyl, cyclohexyl or cycloheptyl, each of which may contain up to two substituents, where the substituents independently of one another are selected from R¹⁰ and R¹⁰ represents:
cyano, chlorine, fluorine, bromine, iodine, methyl, ethyl, propyl, 1-methylethyl, 1,1-dimethylethyl, ethenyl, phenyl, methoxy, ethoxy, propyloxy, trifluoromethoxy, ethynyl, 2-propynyloxy, methylthio, ethylthio or trifluoromethylthio,
R¹_{c}, represents cyclopentenyl, cyclohexenyl or cycloheptenyl, each of which may contain up to two substituents, where the substituents independently of one another are selected from R¹¹ and R¹¹ represents:
methyl, ethyl, methoxy, ethoxy, trifluoromethoxy, ethynyl, 2-propynyloxy, methylthio, ethylthio or trifluoromethylthio,
R¹_{d} furthermore represents phenyl which may contain up to three substituents, where the substituents independently of one another are selected from -L²Q' or R¹² and R¹² represents:
fluorine, chlorine, bromine, iodine, cyano, nitro, methyl, ethyl, propyl, 1-methylethyl, butyl, 1,1-dimethylethyl, 1,2-dimethylethyl, ethenyl, ethynyl, trifluoromethyl, difluoromethyl, trichloromethyl, dichloromethyl, cyclopropyl, methoxy, ethoxy, propoxy, 1-methylethoxy, 1,1-dimethylethoxy, methylcarbonyl, ethylcarbonyl, trifluoromethylcarbonyl, methoxycarbonyl, ethoxycarbonyl, propoxycarbonyl, 1-methylethoxycarbonyl, 1,1-dimethylethoxycarbonyl, 2-propenyloxy, 2-propynyloxy, methylthio, ethylthio, methylsulphinyl or methylsulphonyl,
R¹ₑ represents naphthalen-1-yl, naphthalen-2-yl, 1,2,3,4-tetrahydronaphthalen-1-yl, 1,2,3,4-tetrahydronaphthalen-2-yl, 5,6,7,8-tetrahydronaphthalen-1-yl, 5,6,7,8-tetrahydronaphthalen-2-yl, decalin-1-yl, decalin-2-yl, 1H-inden-1-yl, 1H-inden-2-yl, 1H-inden-3-yl, 1H-inden-4-yl, 1H-inden-5-yl, 1H-inden-6-yl, 1H-inden-7-yl, indan-1-yl, indan-2-yl, indan-3-yl, indan-4-yl or indan-5-yl which may contain up to three substituents, where the substituents independently of one another are selected from R¹³ and R¹³ represents:
methyl, methoxy, cyano, fluorine, chlorine, bromine, iodine,
R¹_{f} represents furan-2-yl, furan-3-yl, thiophen-2-yl, thiophen-3-yl, isoxazol-3-yl, isoxazol-4-yl, isoxazol-5-yl, pyrrol-1-yl, pyrrol-2-yl, pyrrol-3-yl, oxazol-2-yl, oxazol-4-yl, oxazol-5-yl, thiazol-2-yl, thiazol-4-yl, thiazol-5-yl, isothiazol-3-yl, isothiazol-4-yl, isothiazol-5-yl, pyrazol-1-yl, pyrazol-3-yl, pyrazol-4-yl, imidazol-1-yl, imidazol-2-yl, imidazol-4-yl, 1,2,4-oxadiazol-3-yl, 1,2,4-oxadiazol-5-yl, 1,3,4-oxadiazol-2-yl, 1,2,4-thiadiazol-3-yl, 1,2,4-thiadiazol-5-yl, 1,3,4-thiadiazol-2-yl, 1,2,3-triazol-1-yl, 1,2,3-triazol-2-yl, 1,2,3-triazol-4-yl, 1,2,4-triazol-1-yl, 1,2,4-triazol-3-yl, 1,2,4-triazol-4-yl, pyridin-2-yl, pyridin-3-yl, pyridin-4-yl, pyridazin-3-yl, pyridazin-4-yl, pyrimidin-2-yl, pyrimidin-4-yl, pyrimidin-5-yl or pyrazin-2-yl, which may each contain up to two substituents, where the substituents independently of one another are selected from R¹⁴ and R¹⁵ and
R¹⁴ represents a substituent at carbon:
chlorine, fluorine, bromine, iodine, cyano, nitro, methyl, ethyl, propyl, 1-methylethyl, butyl, 1,1-dimethylethyl, ethenyl, ethynyl, trifluoromethyl, difluoromethyl, cyclopropyl, cyclopentyl, cyclohexyl, methylcarbonyl, ethylcarbonyl, methoxycarbonyl, ethoxycarbonyl, methoxy, ethoxy, propoxy, 1-methylethoxy, 2-propynyloxy, trifluoromethoxy, methylcarbonyloxy, methylcarbonylthio, methylthio, ethylthio, trifluoromethylthio, methylsulphinyl, ethylsulphinyl, trifluoromethylsulphinyl, methylsulphonyl, ethylsulphonyl or trifluoromethylsulphonyl,
R¹⁵ represents a substituent at nitrogen :
methyl, ethyl, propyl, cyclopropyl, cyclohexyl, phenyl or 2-propynyl,
R¹_{g} represents indol-1-yl, indol-2-yl, indol-3-yl, indol-4-yl, indol-5-yl, indol-6-yl, indol-7-yl, benzimidazol-1-yl, benzimidazol-2-yl, benzimidazol-4-yl, benzimidazol-5-yl, indazol-1-yl, indazol-3-yl, indazol-4-yl, indazol-5-yl, indazol-6-yl, indazol-7-yl, indazol-2-yl, 1-benzofuran-2-yl, 1-benzofuran-3-yl, 1-benzofuran-4-yl, 1-benzofuran-5-yl, 1-benzofuran-6-yl, 1-benzofuran-7-yl, 1-benzothiophen-2-yl, 1-benzothiophen-3-yl, 1-benzothiophen-4-yl, 1-benzothiophen-5-yl, 1-benzothiophen-6-yl, 1-benzothiophen-7-yl, 1,3-benzothiazol-2-yl, 1,3-benzothiazol-4-yl, 1,3-benzothiazol-5-yl, 1,3-benzothiazol-6-yl, 1,3-benzothiazol-7-yl, 1,3-benzoxazol-2-yl, 1,3-benzoxazol-4-yl, 1,3-benzoxazol-5-yl, 1,3-benzoxazol-6-yl, 1,3-benzoxazol-7-yl, quinolin-2-yl, quinolin-3-yl, quinolin-4-yl, quinolin-5-yl, quinolin-6-yl, quinolin-7-yl, quinolin-8-yl, isoquinolin-1-yl, isoquinolin-3-yl, isoquinolin-4-yl, isoquinolin-5-yl, isoquinolin-6-yl, isoquinolin-7-yl or isoquinolin-8-yl, each of which may contain up to two substituents, where the substituents independently of one another are selected from R¹⁶ and R¹⁷ and
R¹⁶ represents a substituent at carbon:
fluorine, chlorine, bromine, iodine, methyl, methoxy, 2-propynyloxy, 2-propenyloxy,
R¹⁷ represents a substituent at nitrogen:
methyl, ethyl, propyl, cyclopropyl, cyclohexyl, phenyl or 2-propynyl,
L² represents a direct bond,
Q represents phenyl,
R⁵ represents hydrogen,
R⁷ represents hydrogen,
R⁸ represents hydrogen, methyl,
and also agrochemically active salts thereof.

4. Method for controlling phytopathogenic harmful fungi, **characterized in that** compounds of the formula **(I)** according to one or more of Claims 1 to 3 are applied to the phytopathogenic harmful fungi and/or their habitat.

5. Composition for controlling phytopathogenic harmful fungi, **characterized in that** it comprises at least one compound of the formula **(I)** according to one or more of Claims 1 to 3, in addition to extenders and/or surfactants.

6. Use of ketoheteroarylpiperidine and -piperazine derivatives of the formula **(I)** according to one or more of Claims 1 to 3 for controlling phytopathogenic harmful fungi.

7. Process for preparing compositions for controlling phytopathogenic harmful fungi, **characterized in that** ketoheteroarylpiperidine and -piperazine derivatives of the formula **(I)** according to one or more of Claims 1 to 3 are mixed with extenders and/or surfactants.

8. Use of compounds of the formula **(I)** according to Claims 1 to 3 for treating seed.

9. Use of compounds of the formula **(I)** according to Claims 1 to 3 for treating transgenic plants.

10. Use of compounds of the formula **(I)** according to Claims 1 to 3 for treating transgenic seed.

## Revendications

1. Composés de formule (I) 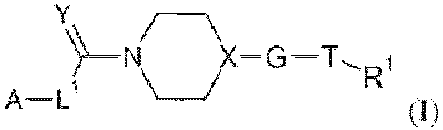 dans laquelle les symboles ont les significations suivantes :
A représente le groupe phényle, qui peut contenir jusqu'à trois substituants, les substituants étant choisis indépendamment les uns des autres parmi R²,
ou
A' représente un groupe hétéroaryle à 5 ou 6 chaînons, éventuellement benzo-condensé, substitué ou non substitué, les substituants sur l'atome de carbone étant choisis indépendamment les uns des autres parmi R³ et les substituants sur l'atome d'azote étant choisis indépendamment les uns des autres parmi R⁴,
L¹ représente NR⁵ ou C(R⁶)₂,
X représente CH ou un atome d'azote,
Y représente un atome de soufre ou d'oxygène,
G représente 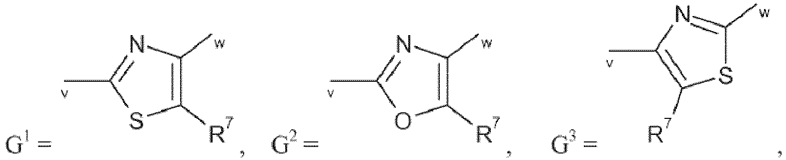 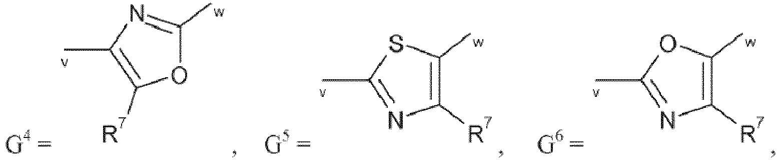 où la liaison identifiée par "v" est directement liée à X, et la liaison identifiée par "w" est directement liée à T,
T représente *-C(=O)CH₂-#, *-C(=O) CH₂C(R⁸)₂-#, *-C(=O)CH=CH-#, *-C(=O)C=C-#, *-C(=O) CH₂C(=O)-#, *-C=CC (=O) -#, *-CH=CHC (=O) -#, *-CH₂CH₂C(=O)-#, *-C(=S) CH₂-#, *-C(=S) CH₂C(R⁸)₂-#, *-C(=S)CH=CH-#, *-C(=S) C=C-#, *-C≡CC (=S) -#, *-CH=CHC (=S ) -#, *-CH₂CH₂C (=S)-#, *-C (=NR⁹) CH₂-#, *-C (=NR⁹)CH₂C (R⁸)₂-# ou *-CH₂CH₂C (=NR⁹)-#, *-C(=NR⁹) CH=CH-#, *-CH=CHC(=NR⁹)-#, *-CH=CHC(NOH)-#, *-CH=CHC(NO-(alkyle en C₁-C₄))CH₂-#,
où la liaison identifiée par * est directement liée à G, et la liaison identifiée par # est directement liée à R¹,
R¹ est choisi dans le groupe contenant R¹ₐ, R¹_{b}, R¹_{c}, R¹_{d}, R¹e, R¹_{f}, R¹g, ou R¹ₕ, où
R¹ₐ représente un groupe alkyle en C₁-C₆, halogènalkyle en C₁-C₆, alcényle en C₂-C₆, alcynyle en C₂-C₆, alcoxyalkyle en C₂-C₈, ou cycloalcoxyalkyle en C₅-C₉, ou
R¹_{b}, représente un groupe cycloalkyle en C₃-C₁₀, non substitué ou substitué, les substituants étant choisis indépendamment les uns des autres parmi -Q ou R¹⁰, ou
R¹_{c} représente un groupe cycloalcényle en C₅-C₁₀ non substitué ou substitué, les substituants étant choisis indépendamment les uns des autres parmi R¹¹, ou
R¹_{d} représente un groupe phényle non substitué ou substitué, les substituants étant choisis indépendamment les uns des autres parmi -L²Q ou R¹²,
R¹_{c} représente un groupe naphtalèn-1-yle, naphtalèn-2-yle, 1,2,3,4-tétrahydronaphtalèn-1-yle, 1,2,3,4-tétrahydronaphtalèn-2-yle, 5,6,7,8-tétrahydronaphtalèn-1-yle, 5,6,7,8-tétrahydronaphtalèn-2-yle, décalin-1-yle, décalin-2-yle, 1H-indèn-1-yle, 1H-indèn-2-yle, 1H-indèn-3-yle, 1H-indèn-4-yle, 1H-indèn-5-yl, 1H-indèn-6-yle, 1H-indèn-7-yle, indan-1-yle, indan-2-yle, indan-3-yle, indan-4-yle ou indan-5-yle, substitué ou non substitué,
les substituants étant choisis indépendamment les uns des autres parmi R¹³, ou
R¹_{f} représente un radical hétéroaryle à 5 ou 6 chaînons non substitué ou substitué, les substituants sur l'atome de carbone étant choisis indépendamment les uns des autres parmi -L²Q ou R¹⁴, et les substituants sur l'atome d'azote étant choisis indépendamment les uns des autres parmi -L³Q ou R¹⁵, ou
R¹_{g} représente un groupe hétéroaryle à 5 ou 6 chaînons benzo-condensé, non substitué ou substitué, les substituants sur l'atome de carbone étant choisis indépendamment les uns des autres parmi R¹⁶, et les substituants sur l'atome d'azote étant choisis indépendamment les uns des autres parmi R¹⁷, ou
R¹ₕ représente un groupe hétérocyclyle en C₅-C₁₅, non substitué ou substitué, les substituants sur l'atome de carbone étant choisis indépendamment les uns des autres parmi R¹⁸, et les substituants sur l'atome d'azote étant choisis indépendamment les uns des autres parmi R¹⁹,
L² représente une liaison directe, -O-, -C(R²⁰)₂- ou -NR²¹-,
L³ représente une liaison directe ou -C(R²⁰)₂-,
Q est choisi dans le groupe contenant Q', Q" ou Q"', où
Q' représente un groupe phényle, qui peut contenir jusqu'à deux substituants, les substituants étant choisis indépendamment les uns des autres parmi R²², ou
Q" représente un radical hétéroaryle à 5 ou 6 chaînons, qui peut contenir jusqu'à deux substituants, les substituants sur l'atome de carbone étant choisis indépendamment les uns des autres parmi R²³, et les substituants sur l'atome d'azote étant choisis indépendamment les uns des autres parmi R²⁴,
Q"' représente un groupe cycloalkyle en C₃-C₁₀, saturé ou partiellement insaturé,
R², R¹² et R²² représentent chacun indépendamment des autres un groupe halogéno, cyano, hydroxy, SH, amino, nitro, phényle, C(=O)H, C(=O)OH, CONR²⁵R²⁶, NR²⁵R²⁶, alkyle en C₁-C₆, alcényle en C₂-C₆, alcynyle en C₂-C₆, halogènalkyle en C₁-C₆, halogènalcényle en C₂-C₆, halogènalcynyle en C₂-C₆, cycloalkyle en C₃-C₈, halogénocycloalkyle en C₃-C₈, alkylcycloalkyle en C₄-C₁₀, cycloalkylalkyle en C₄-C₁₀, cycloalkylcycloalkyle en C₆-C₁₄, halogénocycloalkylalkyle en C₄-C₁₀, alkylcycloalkylalkyle en C₅-C₁₀, cycloalcényle en C₃-C₈, halogénocycloalcényle en C₃-C₈, alcoxyalkyle en C₂-C₆, cycloalcoxyalkyle en C₄-C₁₀, alcoxyalcoxyalkyle en C₃-C₈, alkylthioalkyle en C₂-C₆, alkylsulfinylalkyle en C₂-C₆, alkylsulfonylalkyle en C₂-C₆, alkylaminoalkyle en C₂-C₆, dialkylaminoalkyle en C₃-C₈, halogènalkylaminoalkyle en C₂-C₆, cycloalkylaminoalkyle en C₄-C₁₀, alkylcarbonyle en C₂-C₆, halogènalkylcarbonyle en C₂-C₆, cycloalkylcarbonyle en C₄-C₈, alcoxycarbonyle en C₂-C₆, cycloalcoxycarbonyle en C₄-C₈, cycloalkylalcoxycarbonyle en C₅-C₁₀, alkylaminocarbonyle en C₂-C₆, dialkylaminocarbonyle en C₃-C₈, cycloalkylaminocarbonyle en C₄-C₈, halogènalcoxyalkyle en C₂-C₆, hydroxyalkyle en C₁-C₆, alcoxy en C₁-C₆, halogènalcoxy en C₁-C₆, cycloalcoxy en C₃-C₈, halogénocycloalcoxy en C₃-C₈, cycloalkylalcoxy en C₄-C₁₀, alcényloxy en C₃-C₆, halogènalcényloxy en C₃-C₆, alcynyloxy en C₃-C₆, halogènalcynyloxy en C₃-C₆, alcoxyalcoxy en C₂-C₆, alkylcarbonyloxy en C₂-C₆, halogènalkylcarbonyloxy en C₂-C₆, cycloalkylcarbonyloxy en C₄-C₈, alkylcarbonylalcoxy en C₃-C₆, alkylthio en C₁-C₆, halogènalkylthio en C₁-C₆, cycloalkylthio en C₃-C₆, alkylsulfinyle en C₁-C₆, halogènalkylsulfinyle en C₁-C₆, alkylsulfonyle en C₁-C₆, halogènalkylsulfonyle en C₁-C₆, cycloalkylsulfonyle en C₃-C₈, tri (alkyle en C₁-C₄) silyle, alkylsulfonylamino en C₁-C₆, ou halogènalkylsulfonylamino en C₁-C₆,
R³, R¹⁴, R¹⁶, R¹⁸ et R²³ représentent chacun indépendamment des autres un groupe halogéno, cyano, hydroxy, SH, amino, nitro, NR²⁵R²⁶, alkyle en C₁-C₆, alcényle en C₂-C₆, alcynyle en C₂-C₆, halogènalkyle en C₁-C₆, halogènalcényle en C₂-C₆, halogènalcynyle en C₂-C₆, cycloalkyle en C₃-C₆, halogénocycloalkyle en C₃-C₆, alkylcycloalkyle en C₄-C₁₀, cycloalkylalkyle en C₄-C₁₀, cycloalkylcycloalkyle en C₆-C₁₄, alkylcycloalkylalkyle en C₅-C₁₀, alcoxyalkyle en C₂-C₄, alkylcarbonyle en C₂-C₄, alcoxycarbonyle en C₂-C₆, alkylaminocarbonyle en C₂-C₆, dialkylaminocarbonyle en C₃-C₈, hydroxyalkyle en C₁-C₄, alcoxy en C₁-C₄, alcényloxy en C₃-C₆, alcynyloxy en C₃-C₆, halogènalcoxy en C₁-C₄, alkylcarbonyloxy en C₂-C₆, alkylcarbonylthio en C₂-C₆, alkylthio en C₁-C₄, halogènalkylthio en C₁-C₄, alkylsulfinyle en C₁-C₄, halogènalkylsulfinyle en C₁-C₄, alkylsulfonyle en C₁-C₄, halogènalkylsulfonyle en C₁-C₄, tri (alkyle en C₁-C₄) silyle ou phényle,
R⁴, R¹⁵, R¹⁷, R¹⁸ et R²⁴ représentent chacun indépendamment des autres un groupe alkyle en C₁-C₆, alcényle en C₂-C₆, alcynyle en C₂-C₆, halogènalkyle en C₁-C₆, halogènalcényle en C₃-C₆, halogènalcynyle en C₃-C₆, cycloalkyle en C₃-C₆, halogénocycloalkyle en C₃-C₆, alkylcycloalkyle en C₄-C₁₀, cycloalkylalkyle en C₄-C₁₀, phényle, benzyle, alkylsulfonyle en C₁-C₄, C(=O)H, alkylcarbonyloxy en C₂-C₄, alcoxycarbony en C₂-C₅, ou alkylcarbonyle en C₂-C₅,
R⁵ est un atome d'hydrogène ou un groupe alkyle en C₁-C₄,
R⁶ représente indépendamment les uns des autres un atome d'hydrogène, un groupe alkyle en C₁-C₄, halogènalkyle en C₁-C₄, cyclopropyle, halogéno,
ou les deux radicaux R⁶ forment ensemble avec l'atome d'azote auquel ils sont liés un noyau cyclopropyle,
R⁷ représente un atome d'hydrogène, un groupe alkyle en C₁-C₃, halogènalkyle en C₁-C₃, alcoxy en C₁-C₄, alcoxycarbonyle en C₂-C₅ ou halogéno,
R⁸ représente indépendamment l'un de l'autre un atome d'hydrogène ou un groupe alkyle en C₁-C₄,
R⁹ représente OH, un groupe nitro, cyano, alkyle en C₁-C₄, alkylamino en C₁-C₄, dialkylamino en C₂-C₄, alcoxy en C₁-C₄, halogènalcoxy en C₁-C₄ ou cyclopropyloxy.
R¹⁰ et R¹¹ représentent chacun indépendamment de l'autre un groupe cyano, halogéno, alkyle en C₁-C₆, halogènalkyle en C₁-C₆, cycloalkyle en C₃-C₆, alcényle en C₂-C₆, halogènalcényle en C₂-C₆, alcynyle en C₂-C₆, halogènalcynyle en C₂-C₆, tri (alkyle en C₁-C₄) silyle, phényle, hydroxy, oxo, alcoxy en C₁-C₆, halogènalcoxy en C₁-C_{6,} alcényloxy en C₃-C₆, alcynyloxy en C₃-C₆, alkylthio en C₁-C₆ ou halogènalkylthio en C₁-C₆,
R¹³ représente indépendamment l'un de l'autre un groupe cyano, nitro, halogéno, alkyle en C₁-C₆, halogènalkyle en C₁-C₄, cycloalkyle en C₃-C₆, alcényle en C₂-C₆, halogènalcényle en C₂-C₆, alcynyle en C₂-C₆, halogènalcynyle en C₂-C₆, tri (alkyle en C₁-C₄) silyle, benzyle, phényle, hydroxy, SH, alcoxy en C₁-C₆, halogènalcoxy en C₁-C₆, alcényloxy en C₃-C₆, alcynyloxy en C₃-C₆, alkylthio en C₁-C₆ ou halogènalkylthio en C₁-C₆,
R²⁰ représente chacun indépendamment de l'autre un atome d'hydrogène, un groupe halogéno, alkyle en C₁-C₄ ou halogènalkyle en C₁-C₄,
R²¹ représente un groupe alkyle en C₁-C₆, halogènalkyle en C₁-C₆, cycloalkyle en C₃-C₈, alkylcarbonyle en C₂-C₆, halogènalkylcarbonyle en C₂-C₆, alcoxycarbonyle en C₂-C₆ ou halogènalcoxycarbonyle en C₂-C₆,
R²⁵ et R²⁶ représentent chacun indépendamment de l'autre un atome d'hydrogène, un groupe alkyle en C₁-C₄, alcényle en C₂-C₆, alcynyle en C₂-C₆, halogènalkyle en C₁-C₃, cycloalkyle en C₃-C₆, benzyle ou phényle, ainsi que les sels agrochimiquement actifs de ceux-ci.

2. Composés de formule (I) selon la revendication 1, dans laquelle les symboles ont les significations suivantes :
A représente un groupe phényle qui peut contenir jusqu'à deux substituants, les substituants étant choisis indépendamment l'un de l'autre dans la liste R², et R² représente :
un groupe halogéno, cyano, hydroxy, amino, nitro, alkyle en C₁-C₆, alcényle en C₂-C₆, alcynyle en C₂-C₆, cycloalkyle en C₃-C₈, halogènalkyle en C₁-C₆, halogènalcényle en C₂-C₆, halogènalcynyle en C₂-C₆, halogénocycloalkyle en C₃-C₆, alcoxy en C₁-C₄, halogènalcoxy en C₁-C₄, alkylthio en C₁-C₄, alkylsulfonyle en C₁-C₄, halogènalkylthio en C₁-C₄, halogènalkylsulfonyle en C₁-C₄, alcoxyalkyle en C₂-C₄, alkylcarbonyle en C₂-C₅, alcoxycarbonyle en C₂-C₆, alkylcarbonyloxy en C₂-C₆, ou C(=O)H,
A' représente un radical hétéroaromatique choisi dans le groupe suivant : furann-2-yle, furann-3-yle, thiophèn-2-yle, thiophèn-3-yle, isoxazol-3-yle, isoxazol-4-yle, isoxazol-5-yle, pyrrol-1-yle, pyrrol-2-yle, pyrrol-3-yle, oxazol-2-yle, oxazol-4-yle, oxazol-5-yle, thiazol-2-yle, thiazol-4-yle, thiazol-5-yle, isothiazol-3-yle, isothiazol-4-yle, isothiazol-5-yle, pyrazol-1-yle, pyrazol-3-yle, pyrazol-4-yle, imidazol-1-yle, imidazol-2-yle, imidazol-4-yle, 1,2,3-triazol-1-yle, 1,2,4-triazol-1-yle, pyridin-2-yle, pyridin-3-yle, pyridin-4-yle, pyridazin-3-yle, pyridazin-4-yle, pyrazin-2-yle, pyrazin-3-yle, pyrimidin-2-yle, pyrimidin-4-yle ou pyrimidin-5-yle, qui peut contenir jusqu'à deux substituants, les substituants étant choisis indépendamment l'un de l'autre parmi R³ et R⁴, et
R³ représente un substituant sur l'atome de carbone : un groupe halogéno, cyano, hydroxy, amino, nitro, alkyle en C₁-C₆, alcényle en C₂-C_{6,} alcynyle en C₂-C_{6,} cycloalkyle en C₃-C₈-, halogènalkyle en C₁-C₆, halogènalcényle en C₂-C₆, halogènalcynyle en C₂-C₆, halogénocycloalkyle en C₃-C₆, alcoxy en C₁-C₄, halogènalcoxy en C₁-C₄, alkylthio en C₁-C₄, alkylsulfonyle en C₁-C₄, halogènalkylthio en C₁-C₄, halogènalkylsulfonyle en C₁-C₄, alcoxyalkyle en C₂-C₄, alcoxycarbonyle en C₂-C₆, alkylcarbonyloxy en C₂-C₆, C(=O)H ou phényle,
R⁴ représente un substituant sur l'atome d'azote :
un groupe alkyle en C₁-C₆, halogènalkyle en C₁-C₄, alcényle en C₃-C₆, halogènalcényle en C₃-C₆, alcynyle en C₃-C₆ ou halogènalcynyle en C₃-C₆,
G représente G¹ ou G²,
T représente *-C(=O)CH₂-#, *-C(=O)CH₂C(R⁸)₂-#, *-C(=O)CH=CH-#, *-C(=O)C≡C-#, *-C≡CC(=O)-#, *-CH=CHC(=O)-#, *-CH₂CH₂C(=O)-#, *-C(=S)CH₂-#, *-C(=S)CH₂C(R⁸)₂-#, *-CH₂CH₂C(=S)-#, *-C(=NR⁹)CH₂-#, *-C(=NR⁹)CH=CH-#, *-CH=CHC(=NR⁹)-#, ^{*}CH=CHC(NOH)-#, ou *-CH=CHC(NO-(alkyle en C₁-C₄))CH₂-#,
L¹ représente CH₂ ou NR⁵,
X représente CH,
Y représente un atome d'oxygène,
R¹ₐ représente un groupe alkyle en C₁-C₆, halogènalkyle en C₁-C₆, alcényle en C₂-C₆ ou alcynyle en C₂-C₆, alcoxyalkyle en C₂-C₆,
R¹_{b} représente un groupe cycloalkyle en C₃-C₁₀ non substitué ou substitué, les substituants étant choisis indépendamment les uns des autres parmi -Q ou R¹⁰, et R¹⁰ représente :
un groupe cyano, halogéno, alkyle en C₁-C₆, halogènalkyle en C₁-C₆, cycloalkyle en C₃-C₆, alcényle en C₂-C₆, halogènalcényle en C₂-C₆, alcynyle en C₂-C₆, halogènalcynyle en C₂-C₆, tri (alkyle en C₁-C₄)silyle, hydroxy, oxo, alcoxy en C₁-C₆, halogènalcoxy en C₁-C₆, alcényloxy en C₃-C₆, alcynyloxy en C₃-C₆, alkylthio en C₁-C₆, halogènalkylthio en C₁-C₆,
R¹_{c} représente un groupe cycloalcényle en C₅-C₁₀ non substitué ou substitué, les substituants étant choisis indépendamment les uns des autres parmi R¹¹, et R¹¹ représente :
un groupe cyano, chloro, fluoro, alkyle en C₁-C₆, halogènalkyle en C₁-C₆, cycloalkyle en C₃-C₆, alcényle en C₂-C₆, halogènalcényle en C₂-C₆, alcynyle en C₂-C₆, halogènalcynyle en C₂-C₆, phényle, oxo, alcoxy en C₁-C_{6,} halogènalcoxy en C₁-C₆, alcényloxy en C₃-C₆, alcynyloxy en C₃-C₆, alkylthio en C₁-C₆ ou halogènalkylthio en Ci-Cr,
R¹_{d} représente un groupe phényle non substitué ou substitué, les substituants étant choisis indépendamment les uns des autres parmi -L²Q ou R¹², et R¹² représente :
un groupe halogéno, cyano, hydroxy, SH, amino, nitro, C(=O)H, C(=O)OH, CONR²⁵R²⁶, NR²⁵R²⁶, alkyle en C₁-C₆, alcényle en C₂-C₆, alcynyle en C₂-C₆, halogènalkyle en C₁-C₆, halogènalcényle en C₂-C₆, halogènalcynyle en C₂-C₆, cycloalkyle en C₃-C₈, halogénocycloalkyle en C₃-C₈, alkylcycloalkyle en C₄-C₁₀, cycloalkylalkyle en C₄-C₁₀, cycloalkylcycloalkyle en C₆-C₁₄, halogénocycloalkylalkyle en C₄-C₁₀, alkylcycloalkylalkyle en C₅-C₁₀, cycloalcényle en C₃-C₈, halogénocycloalcényle en C₃-C₈, alcoxyalkyle en C₂-C₆, cycloalcoxyalkyle en C₄-C₁₀, alcoxyalcoxyalkyle en C₃-C₈, alkylthioalkyle en C₂-C₆, alkylsulfinylalkyle en C₂-C₆, alkylsulfonylalkyle en C₂-C₆, alkylaminoalkyle en C₂-C₆, dialkylaminoalkyle en C₃-C₈, halogènalkylaminoalkyle en C₂-C₆, cycloalkylaminoalkyle en C₄-C₁₀, alkylcarbonyle en C₂-C₆, halogènalkylcarbonyle en C₂-C₆, cycloalkylcarbonyle en C₄-C₈, alcoxycarbonyle en C₂-C₆, cycloalcoxycarbonyle en C₄-C₈, cycloalkylacoxycarbonyle en C₅-C₁₀, alkylaminocarbonyle en C₂-C₆, dialkylaminocarbonyle en C₃-C₈, cycloalkylaminocarbonyle en C₄-C₈, halogènalcoxyalkyle en C₂-C₆, hydroxyalkyle en C₁-C₆, alcoxy en C₁-C₆, halogènalcoxy en C₁-C₆, cycloalcoxy en C₃-C₈, halogénocycloalcoxy en C₃-C₈, cycloalkylalcoxy en C₄-C₁₀, alcényloxy en C₃-C₆, halogènalcényloxy en C₃-C₆, alcynyloxy en C₃-C₆, halogènalcynyloxy en C₃-C₆, alcoxyalcoxy en C₂-C₆, alkylcarbonyloxy en C₂-C₆, halogènalkylcarbonyloxy en C₂-C₆, cycloalkylcarbonyloxy en C₄-C₈, alkylcarbonylalcoxy en C₃-C₆, alkylthio en C₁-C₆, halogènalkylthio en C₁-C_{6,} cycloalkylthio en C₃-C₆, alkylsulfinyle en C₁-C₆, halogènalkylsulfinyle en C₁-C₆, alkylsulfonyle en C₁-C₆, halogènalkylsulfonyle en C₁-C₆, cycloalkylsulfonyle en C₃-C₈, tri (alkyle en C₁-C₄) silyle, alkylsulfonylamino en C₁-C₆, halogènalkylsulfonylamino en C₁-C₆,
R¹ₑ représente un groupe naphtalèn-1-yle, naphtalèn-2-yle, 1,2,3,4-tétrahydronaphtalèn-1-yle, 1,2,3,4-tétrahydronaphtalèn-2-yle, 5,6,7,8-tétrahydronaphtalèn-1-yle, 5,6,7,8-tétrahydronaphtalèn-2-yle, décalin-1-yle, décalin-2-yle, 1H-indèn-1-yle, 1H-indèn-2-yle, 1H-indèn-3-yle, 1H-indèn-4-yle, 1H-indèn-5-yle, 1H-indèn-6-yle, 1H-indèn-7-yle, indan-1-yle, indan-2-yle, indan-3-yle, indan-4-yle ou indan-5-yle, non substitué ou substitué, les substituants étant choisis indépendamment les uns des autres parmi R¹³, et R¹³ représente :
un groupe cyano, halogéno, alkyle en C₁-C₆, halogènalkyle en C₁-C₄, alcényle en C₂-C₆, alcynyle en C₂-C₆, benzyle, phényle, alcoxy en C₁-C₆, halogènalcoxy en C₁-C₆, alcényloxy en C₃-C₆, alcynyloxy en C₃-C₆ ou alkylthio en C₁-C₆,
R¹_{f} représente un radical hétéroaryle à 5 ou 6 chaînons, non substitué ou substitué, les substituants étant choisis indépendamment les uns des autres parmi -L²Q ou R¹⁴ et -L³Q ou R¹⁵, et R¹⁴ représente un substituant sur l'atome de carbone :
un groupe halogéno, cyano, hydroxy, SH, amino, nitro, NR²⁵R²⁶, alkyle en C₁-C₆, alcényle en C₂-C₆, alcynyle en C₂-C₆, halogènalkyle en C₁-C₆, halogènalcényle en C₂-C₆, halogènalcynyle en C₂-C₆, cycloalkyle en C₃-C₆, halogénocycloalkyle en C₃-C₆, alkylcycloalkyle en C₄-C₁₀, cycloalkylalkyle en C₄-C₁₀, cycloalkylcycloalkyle en C₆-C₁₄, alkylcycloalkylalkyle en C₅-C₁₀, alcoxyalkyle en C₂-C₄, alcényloxy en C₃-C₆, alcynyloxy en C₃-C₆, alkylcarbonyle en C₂-C₄, alcoxycarbonyle en C₂-C₆, alkylaminocarbonyle en C₂-C₆, dialkylaminocarbonyle en C₃-C₈, hydroxyalkyle en C₁-C₄, alcoxy en C₁-C₄, halogènalcoxy en C₁-C₄, alkylcarbonyloxy en C₂-C₆, alkylcarbonylthio en C₂-C₆, alkylthio en C₁-C₄, halogènalkylthio en C₁-C₄, alkylsulfinyle en C₁-C₄, halogènalkylsulfinyle en C₁-C₄, alkylsulfonyle en C₁-C₄, halogènalkylsulfonyle en C₁-C₄, tri (alkyle en C₁-C₄)silyle,
R¹⁵ représente un substituant sur l'atome d'azote :
un groupe alkyle en C₁-C₆, alcényle en C₃-C₆, alcynyle en C₃-C₆, halogènalkyle en C₁-C₆, halogènalcényle en C₃-C₆, halogènalcynyle en C₃-C₆, cycloalkyle en C₃-C₆, halogénocycloalkyle en C₃-C₆, alkylcycloalkyle en C₄-C₁₀, cycloalkylalkyle en C₄-C₁₀, phényle,
R¹_{g} représente un groupe hétéroaryle à 5 ou 6 chaînons, benzo-condensé, non substitué ou substitué, les substituants étant choisis indépendamment les uns des autres parmi R¹⁶ et R¹⁷, et R¹⁶ représente un substituant sur l'atome de carbone :
un groupe halogéno, cyano, hydroxy, SH, amino, nitro, NR²⁵R²⁶, alkyle en C₁-C₆, alcényle en C₂-C₆, alcynyle en C₂-C₆, halogènalkyle en C₁-C₆, halogènalcényle en C₂-C₆, halogènalcynyle en C₂-C₆, cycloalkyle en C₃-C₆, halogénocycloalkyle en C₃-C₆, alkylcycloalkyle en C₄-C₁₀, cycloalkylalkyle en C₄-C₁₀, cycloalkylcycloalkyle en C₆-C₁₄, alkylcycloalkylalkyle en C₅-C₁₀, alcoxyalkyle en C₂-C₄, alkylcarbonyle en C₂-C₄, alcoxycarbonyle en C₂-C₆, alkylaminocarbonyle en C₂-C₆, dialkylaminocarbonyle en C₃-C₈, hydroxyalkyle en C₁-C₄, alcoxy en C₁-C₄, halogènalcoxy en C₁-C₄, alkylcarbonyloxy en C₂-C₆, alkylcarbonylthio en C₂-C₆, alkylthio en C₁-C₄, halogènalkylthio en C₁-C₄, alkylsulfinyle en C₁-C₄, halogènalkylsulfinyle en C₁-C₄, alkylsulfonyle en C₁-C₄, halogènalkylsulfonyle en C₁-C₄, tri(alkyle en C₁-C₄) silyle ou phényle,
R¹⁷ représente un substituant sur l'atome d'azote :
un groupe alkyle en C₁-C₆, alcényle en C₃-C₆, alcynyle en C₃-C₆, halogènalkyle en C₁-C₆, halogènalcényle en C₃-C₆, halogènalcynyle en C₃-C₆, cycloalkyle en C₃-C₆, halogénocycloalkyle en C₃-C₆, alkylcycloalkyle en C₄-C₁₀, cycloalkylalkyle en C₄-C₁₀ ou phényle,
R¹ₕ représente un groupe hétérocyclyle en C₅-C₁₅ non substitué ou substitué, les substituants possibles étant choisis indépendamment les uns des autres parmi R¹⁸ et R¹⁹, et
R¹⁸ représente un substituant sur l'atome de carbone :
un groupe halogéno, cyano, hydroxy, SH, amino, nitro, NR²⁵R²⁶, alkyle en C₁-C₆,alcényle en C₂-C₆, alcynyle en C₂-C₆, halogènalkyle en C₁-C₆, halogènalcényle en C₂-C₆, halogènalcynyle en C₂-C₆, cycloalkyle en C₃-C₆, halogénocycloalkyle en C₃-C₆, alkylcycloalkyle en C₄-C₁₀, cycloalkylalkyle en C₄-C₁₀, cycloalkylcyloalkyle en C₆-C₁₄, alkylcycloalkylalkyle en C₅-C₁₀, alcoxyalkyle en C₂-C₄, alkylcarbonyle en C₂-C₄, alcoxycarbonyle en C₂-C₆, alkylaminocarbonyle en C₂-C₆, dialkylaminocarbonyle en C₃-C₈, hydroxyalkyle en C₁-C₄, alcoxy en C₁-C₄, halogènalcoxy en C₁-C₄, alkylcarbonyloxy en C₂-C₆, alkylcarbonylthio en C₂-C₆, alkylthio en C₁-C₄, halogènalkylthio en C₁-C₄, alkylsulfinyle en C₁-C₄, halogènalkylsulfinyle en C₁-C₄, alkylsulfonyle en C₁-C₄, halogènalkylsulfonyle en C₁-C₄, tri(alkyle en C₁-C₄) silyle ou phényle,
R¹⁹ représente un substituant sur l'atome d'azote :
un groupe alkyle en C₁-C₆, alcényle en C₃-C₆, alcynyle en C₃-C₆, halogènalkyle en C₁-C₆, halogènalcényle en C₃-C₆, halogènalcynyle en C₃-C₆, cycloalkyle en C₃-C₆, halogénocycloalkyle en C₃-C₆, alkylcycloalkyle en C₄-C₁₀, cycloalkylalkyle en C₄-C₁₀ ou phényle,
L² représente une liaison directe ou -O-,
L³ représente une liaison directe,
Q' représente un groupe phényle qui peut contenir jusqu'à deux substituants, les substituants étant choisis indépendamment l'un de l'autre parmi R²², et R²² représente :
un groupe fluoro, chloro, bromo, iodo, cyano, hydroxy, SH, nitro, alkyle en C₁-C₆, halogènalkyle en C₁-C₆, cycloalkyle en C₃-C₆, alcoxy en C₁-C₄, halogènalcoxy en C₁-C₄, alkylthio en C₁-C₄ ou halogènalkylthio en C₁-C₄,
Q" représente un groupe furann-2-yle, furann-3-yle, thiophèn-2-yle, thiophèn-3-yle, isoxazol-3-yle, isoxazol-4-yle, isoxazol-5-yle, pyrrol-1-yle, pyrrol-2-yle, pyrrol-3-yle, oxazol-2-yle, oxazol-4-yle, oxazol-5-yle, thiazol-2-yle, thiazol-4-yle, thiazol-5-yle, isothiazol-3-yle, isothiazol-4-yle, isothiazol-5-yle, pyrazol-1-yle, pyrazol-3-yle, pyrazol-4-yle, imidazol-1-yle, imidazol-2-yle, imidazol-4-yle, 1,2,4-oxadiazol-3-yle, 1,2,4-oxadiazol-5-yle, 1,3,4-oxadiazol-2-yle, tétrazol-5-yle, 1,2,4-thiadiazol-3-yle, 1,2,4-thiadiazol-5-yle, 1,3,4-thiadiazol-2-yle, 1,2,3-triazol-1-yle, 1,2,3-triazol-2-yle, 1,2,3-triazol-4-yle, 1,2,4-triazol-1-yle, 1,2,4-triazol-3-yle, 1,2,4-triazol-4-yle, tétrazol-5-yle, pyridin-2-yle, pyridin-3-yle, pyridin-4-yle, pyridazin-3-yle, pyridazin-4-yle, pyrimidin-2-yle, pyrimidin-4-yle, pyrimidin-5-yle, pyrazin-2-yle, 1,3,5-triazin-2-yle ou 1,2,4-triazin-3-yle, dont chacun peut contenir jusqu'à deux substituants, les substituants étant choisis indépendamment l'un de l'autre parmi R²³ et R²⁴, et
R²³ représente un substituant sur l'atome de carbone :
un groupe fluoro, chloro, bromo, iodo, cyano, hydroxy, SH, nitro, alkyle en C₁-C₆, halogènalkyle en C₁-C₆, cycloalkyle en C₃-C₆, alcoxy en C1-C₄, halogènalcoxy en C₁-C₄, alkylthio en C₁-C₄ ou halogènalkylthio en C₁-C₄,
R²⁴ représente un substituant sur l'atome d'azote :
un groupe alkyle en C₁-C₆, alcényle en C₃-C₆, alcynyle en C₃-C₆, cycloalkyle en C₃-C₆ ou phényle,
R⁵ représente un atome d'hydrogène ou le groupe méthyle, éthyle, propyle, isopropyle, butyle, isobutyle ou tert-butyle,
R⁷ représente un atome d'hydrogène,
R⁸ représente, indépendamment l'un de l'autre, un atome d'hydrogène, le groupe méthyle ou éthyle,
R²⁵ et R²⁶ représentent chacun indépendamment de l'autre un atome d'hydrogène, les groupes méthyle, éthyle, propyle, isopropyle, butyle, isobutyle et tert-butyle, ainsi que les sels agrochimiquement actifs de ceux-ci.

3. Composés de formule (I) selon l'une ou plusieurs des revendications 1 à 2, dans lesquels les symboles ont les significations suivantes :
A représente un groupe phényle qui peut contenir jusqu'à deux substituants, les substituants étant choisis indépendamment l'un de l'autre parmi R², et R² représente :
le groupe fluoro, bromo, iodo, chloro, cyano, nitro, méthyle, éthyle, propyle, 1-méthyléthyle, 1,1-diméthyléthyle, chlorofluorométhyle, dichlorométhyle, dichlorofluorométhyle, difluorométhyle, trichlorométhyle, trifluorométhyle, cyclopropyle, éthoxy, 1-méthyléthoxy, propoxy, méthoxy, trifluorométhoxy, difluorométhoxy, 1-méthyléthylthio, méthylthio, éthylthio, propylthio, difluorométhylthio ou trifluorométhylthio,
A' représente un radical hétéroaromatique choisi dans le groupe suivant : les groupes furann-2-yle, furann-3-yle, thiophèn-2-yle, thiophèn-3-yle, isoxazol-3-yle, isoxazol-4-yle, isoxazol-5-yle, pyrrol-1-yle, pyrrol-2-yle, pyrrol-3-yle, oxazol-2-yle, oxazol-4-yle, oxazol-5-yle, thiazol-2-yle, thiazol-4-yle, thiazol-5-yle, isothiazol-3-yle, isothiazol-4-yle, isothiazol-5-yle, pyrazol-1-yle, pyrazol-3-yle, pyrazol-4-yle, imidazol-1-yle, imidazol-2-yle, imidazol-4-yle, 1,2,3-triazol-1-yle, 1,2,4-triazol-1-yle, pyridin-2-yle, pyridin-3-yle, pyridin-4-yle, pyridazin-3-yle, pyridazin-4-yle, pyrimidin-2-yle, pyrimidin-4-yle ou pyrimidin-5-yle, qui peut contenir jusqu'à deux substituants, les substituants étant choisis indépendamment l'un de l'autre parmi R³ et R⁴, et
R³ représente un substituant sur l'atome de carbone :
le groupe fluoro, chloro, bromo, iodo, cyano, nitro, méthyle, éthyle, propyle, 1-méthyléthyle, 1,1-diméthyléthyle, chlorofluorométhyle, dichlorométhyle, dichlorofluorométhyle, difluorométhyle, trichlorométhyle, trifluorométhyle, cyclopropyle, éthoxy, 1-méthyléthoxy, propoxy, méthoxy, trifluorométhoxy, difluorométhoxy, 1-méthyléthylthio, méthylthio, éthylthio, propylthio, difluorométhylthio, trifluorométhylthio ou phényle,
R⁴ représente un substituant sur l'atome d'azote:
le groupe méthyle, éthyle, propyle, 1-méthyléthyle, 2,2-trifluoroéthyle, 2,2-difluoroéthyle, 2,2-dichloro-2-fluoroéthyle, 2-chloro-2-difluoroéthyle ou 2-chloro-2-fluoroéthyle,
G représente G¹,
T représente *-C(=O)CH₂-#, *-C(=O) CH₂C(R⁸)₂-#, *-C(=O)CH=CH-#, *-C=CC(=O)-#, *-CH=CHC(=O)-#, *-CH₂CH₂C(=O)-#, *-C(=S)CH₂-#, *-C(=S)CH₂C(R⁸)₂-#, *-CH₂CH₂C(=S)-#, *-C(=NR⁹)CH₂-#, *-C(=NR⁹)CH=CH-#, *-CH=CHC(-NOH)-# ou *-CH=CHC (N-O-(alkyle en C₁-C₄))CH₂-#,
L¹ représente CH₂,
X représente CH,
Y représente un atome d'oxygène,
R¹ₐ représente le groupe 1,1-diméthyléthyle, 3,3-diméthylbutyle, 2,2-diméthylpropyle, 1,2,2-triméthylpropyle, pentyle, 1-éthylpropyle, butyle, 2-méthylpropyle, 1-méthyléthyle, éthyle, propyle, 4-méthylpentyle ou hexyle,
R¹_{b} représente un groupe cyclopentyle, cyclohexyle ou cycloheptyle, dont chacun peut contenir jusqu'à deux substituants, les substituants étant choisis indépendamment l'un de l'autre parmi R¹⁰, et R¹⁰ représente :
le groupe cyano, chloro, fluoro, bromo, iodo, méthyle, éthyle, propyle, 1-méthyléthyle, 1,1-diméthyléthyle, éthényle, phényle, méthoxy, éthoxy, propyloxy, trifluorométhoxy, éthynyle, 2-propynyloxy, méthylthio, éthylthio ou trifluorométhylthio,
R¹_{c} représente un groupe cyclopentényle, cyclohexényle ou cyloheptényle, dont chacun peut contenir jusqu'à deux substituants, les substituants étant choisis indépendamment l'un de l'autre parmi R¹¹, et R¹¹ représente :
le groupe méthyle, éthyle, méthoxy, éthoxy, trifluorométhoxy, éthynyle, 2-propynyloxy, méthylthio, éthylthio ou trifluorométhylthio,
R¹_{d} représente par ailleurs un groupe phényle qui peut contenir jusqu'à trois substituants, les substituants étant choisis indépendamment les uns des autres parmi -L²Q' ou R¹², et R¹² représente :
le groupe fluoro, chloro, bromo, iodo, cyano, nitro, méthyle, éthyle, propyle, 1-méthyléthyle, butyle, 1,1-diméthyléthyle, 1,2-diméthyléthyle, éthényle, éthynyle, trifluorométhyle, difluorométhyle, trichlorométhyle, dichlorométhyle, cyclopropyle, méthoxy, éthoxy, propoxy, 1-méthyléthoxyle, 1,1-diméthyléthoxyle, méthylcarbonyle, éthylcarbonyle, trifluorométhylcarbonyle, méthoxycarbonyle, éthoxycarbonyle, propoxycarbonyle, 1-méthyléthoxycarbonyle, 1,1-diméthyléthoxycarbonyle, 2-propényloxy, 2-propynyloxy, méthylthio, éthylthio, méthylsulfinyle ou méthylsulfonyle,
R¹ₑ représente un groupe naphtalèn-1-yle, naphtalèn-2-yle, 1,2,3,4-tétrahydronaphtalèn-1-yle, 1,2,3,4-tétrahydronaphtalèn-2-yle, 5,6,7,8-tétrahydronaphtalèn-1-yle, 5,6,7,8-tétrahydronaphtalèn-2-yle, décalin-1-yle, décalin-2-yle, 1H-indèn-1-yle 1H-indèn-2-yle, 1H-indèn-3-yle, 1H-indèn-4-yle, 1H-indèn-5-yle, 1H-indèn-6-yle, 1H-indèn-7-yle, indan-1-yle, indan-2-yle, indan-3-yle, indan-4-yle ou indan-5-yle, qui peut contenir jusqu'à trois substituants, les substituants étant choisis indépendamment les uns des autres parmi R¹³, et R¹³ représente
le groupe méthyle, méthoxy, cyano, fluoro, chloro, bromo, iodo,
R¹_{f} représente un groupe furann-2-yle, furann-3-yle, thiophèn-2-yle, thiophèn-3-yle, isoxazol-3-yle, isoxazol-4-yle, isoxazol-5-yle, pyrrol-1-yle, pyrrol-2-yle, pyrrol-3-yle, oxazol-2-yle, oxazol-4-yle, oxazol-5-yle, thiazol-2-yle, thiazol-4-yle, thiazol-5-yle, isothiazol-3-yle, isothiazol-4-yle, isothiazol-5-yle, pyrazol-1-yle, pyrazol-3-yle, pyrazol-4-yle, imidazol-1-yle, imidazol-2-yle, imidazol-4-yle, 1,2,4-oxadiazol-3-yle, 1,2,4-oxadiazol-5-yle, 1,3,4-oxadiazol-2-yle, 1,2,4-thiadiazol-3-yle, 1,2,4-thiadiazol-5-yle, 1,3,4-thiadiazol-2-yle, 1,2,3-triazol-1-yle, 1,2,3-triazol-2-yle, 1,2,3-triazol-4-yle, 1,2,4-triazol-1-yle, 1,2,4-triazol-3-yle, 1,2,4-triazol-4-yle, pyridin-2-yle, pyridin-3-yle, pyridin-4-yle, pyridazin-3-yle, pyridazin-4-yle, pyrimidin-2-yle, pyrimidin-4-yle, pyrimidin-5-yle ou pyrazin-2-yle, dont chacun peut contenir jusqu'à deux substituants, les substituants étant choisis indépendamment l'un de l'autre parmi R¹⁴ et R¹⁵, et
R¹⁴ représente un substituant sur l'atome de carbone :
le groupe chloro, fluoro, bromo, iodo, cyano, nitro, méthyle, éthyle, propyle, 1-méthyléthyle, butyle, 1,1-diméthyléthyle, éthényle, éthynyle, trifluorométhyle, difluorométhyle, cyclopropyle, cyclopentyle, cyclohexyle, méthylcarbonyle, éthylcarbonyle, méthoxycarbonyle, éthoxycarbonyle, méthoxy, éthoxy, propoxy, 1-méthyléthoxy, 2-propynyoxy, trifluorométhoxy, méthylcarbonyloxy, méthylcarbonylthio, méthylthio, éthylthio, trifluorométhylthio, méthylsulfinyle, éthylsulfinyle, trifluorométhylsulfinyle, méthylsulfonyle, éthylsulfonyle ou trifluorométhylsulfonyle,
R¹⁵ représente un substituant sur l'atome d'azote :
le groupe méthyle, éthyle, propyle, cyclopropyle, cyclohexyle, phényle ou 2-propynyle,
R¹_{g} représente un groupe indol-1-yle, indol-2-yle, indol-3-yle, indol-4-yle, indol-5-yle, indol-6-yle, indol-7-yle, benzimidazol-1-yle, benzimidazol-2-yle, benzimidazol-4-yle, benzimidazol-5-yle, indazol-1-yle, indazol-3-yle, indazol-4-yle, indazol-5-yle, indazol-6-yle, indazol-7-yle, indazol-2-yle, 1-benzofurann-2-yle, 1-benzofurann-3-yle, 1-benzofurann-4-yle, 1-benzofurann-5-yle, 1-benzofurann-6-yle, 1-benzofurann-7-yle, 1-benzothiophèn-2-yle, 1-benzothiophèn-3-yle, 1-benzothiophèn-4-yle, 1-benzothiophèn-5-yle, 1-benzothiophèn-6-yle, 1-benzothiophèn-7-yle, 1,3-benzothiazol-2-yle, 1,3-benzothiazol-4-yle, 1,3-benzothiazol-5-yle, 1,3-benzothiazol-6-yle, 1,3-benzothiazol-7-yle, 1,3-benzoxazol-2-yle, 1,3-benzoxazol-4-yle, 1,3-benzoxazol-5-yle, 1,3-benzoxazol-6-yle, 1,3-benzoxazole-7-yle, quinoléin-2-yle, quinoléin-3-yle, quinoléin-4-yle, quinoléin-5-yle, quinoléin-6-yle, quinoléin-7-yle, quinoléin-8-yle, isoquinoléin-1-yle, isoquinoléin-3-yle, isoquinoléin-4-yle, isoquinoléin-5-yle, isoquinoléin-6-yle, isoquinoléin-7-yle ou isoquinoléin-8-yle, dont chacun peut contenir jusqu'à deux substituants, les substituants étant choisis indépendamment l'un de l'autre parmi R¹⁶ et R¹⁷, et
R¹⁶ représente un substituant sur l'atome de carbone :
le groupe fluoro, chloro, bromo, iodo, méthyle, méthoxy, 2-propynyloxy, 2-propényloxy,
R¹⁷ représente un substituant sur l'atome d'azote :
le groupe méthyle, éthyle, propyle, cyclopropyle, cyclohexyle, phényle ou 2-propynyle,
L² représente une liaison directe,
Q' représente le groupe phényle,
R⁵ représente un atome d'hydrogène;
R⁷ représente un atome d'hydrogène,
R⁸ représente un atome d'hydrogène, le groupe méthyle,
ainsi que les sels agrochimiquement actifs de ceux-ci.

4. Procédé pour lutter contre les champignons nuisibles phytopathogènes, **caractérisé en ce qu'**on épand sur les champignons nuisibles phytopathogènes et/ou leurs biotopes des composés de formule (I) selon l'une ou plusieurs des revendication 1 à 3.

5. Produit pour lutter contre les champignons nuisibles phytopathogènes, **caractérisé en ce qu'**il contient au moins un composé de formule (I) selon l'une ou plusieurs des revendications 1 à 3, en plus de diluants et/ou de substances tensioactives.

6. Utilisation de dérivés de cétohétéroaryl-pipéridine et -pipérazine de formule (I) selon l'une ou plusieurs des revendications 1 à 3 pour lutter contre les champignons nuisibles phytopathogènes.

7. Procédé de fabrication de produits pour lutter contre les champignons nuisibles phytopathogènes, **caractérisé en ce qu'**on mélange à des diluants et/ou des substances tensioactives des dérivés de cétohétéroaryl-pipéridine et -pipérazine de formule (I) selon l'une ou plusieurs des revendications 1 à 3.

8. Utilisation de composés de formule (I) selon les revendications 1 à 3 pour traiter des semences.

9. Utilisation de composés de formule (I) selon les revendications 1 à 3 pour le traitement de plantes transgéniques.

10. Utilisation de composés de formule (I) selon les revendications 1 à 3 pour le traitement de semences transgéniques.
